# EUROPEAN PATENT APPLICATION

(11) **EP 4 818 159 A1**
(43) Date of publication of application: **07.10.2026**
(21) Application number: 24912982.6
(22) Date of filing: 26.12.2024
(51) Int. Cl.: C07D 215/26, A61K 31/4365

(54) **NITROGEN-CONTAINING CONDENSED RING COMPOUND**

(30) Priority: 29.12.2023 JP 2023223739
(71) Applicant: Kissei Pharmaceutical Co., Ltd., Matsumoto-Shi, Nagano 399-8710 (JP)
(72) Inventor: KIYOSAWA, Seina, Azumino-shi, Nagano 399-8304 (JP); MUTAI, Yousuke, Azumino-shi, Nagano 399-8304 (JP); ISHIKAWA, Takehiro, Azumino-shi, Nagano 399-8304 (JP); TANADA, Fumiya, Azumino-shi, Nagano 399-8304 (JP); MATSUMOTO, Kyohei, Azumino-shi, Nagano 399-8304 (JP); KONDO, Atsushi, Azumino-shi, Nagano 399-8304 (JP); OKI, Tomoya, Azumino-shi, Nagano 399-8304 (JP); SHIOGAI, Akihiro, Azumino-shi, Nagano 399-8304 (JP)
(74) Representative: Isarpatent
(86) International application number: PCT/JP2024/046101
(87) International publication number: WO 2025/143096

(57) **Abstract**

An object of the present invention is to provide a novel compound which has thyroid-stimulating hormone receptor antagonist activity and is useful for treating thyroid-related diseases. The present invention relates to a nitrogen-containing condensed ring compound represented by the following Formula (A-I) or a pharmacologically acceptable salt thereof. The compound of the present invention or a pharmacologically acceptable salt thereof has antagonist activity against a thyroid-stimulating hormone receptor and is useful as a therapeutic agent for thyroid-related diseases (for example, hyperthyroidism, Graves' disease, thyroid eye disease and thyroid cancer).

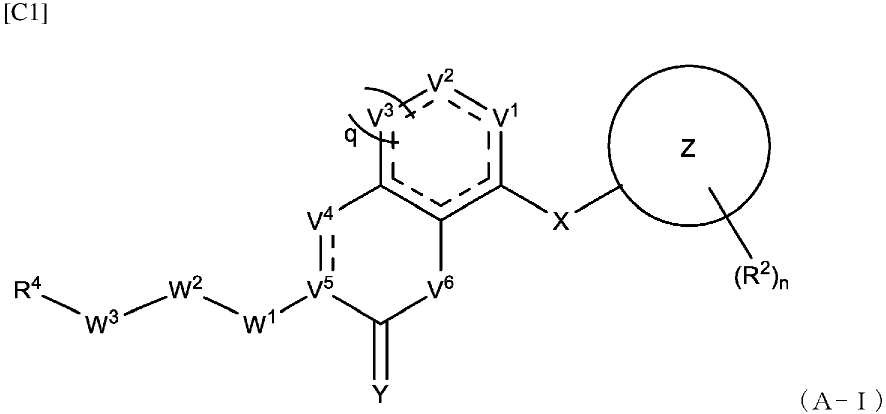

## Description

### [Technical Field]

The present invention relates to a nitrogen-containing condensed ring compound useful as a pharmaceutical. More specifically, the present invention relates to a nitrogen-containing condensed ring compound or a pharmacologically acceptable salt thereof which has antagonist activity against thyroid-stimulating hormone receptor (TSHR) and is useful as a therapeutic agent for thyroid-related diseases.

### [Background Art]

The thyroid hormones triiodothyronine (T3) and thyroxine (T4) play important roles in development, growth, and metabolism, and their synthesis and secretion are precisely regulated by the thyroid-stimulating hormone (TSH) secreted by the pituitary gland.

In hyperthyroidism, these thyroid hormones are excessively secreted for some reason, and the hormone action thereof causes various undesirable effects on the mind and body, such as goiter, tachycardia, high blood pressure, fatigue, weight loss, palpitation, sleep disorders, and menstrual irregularities.

There are various causes of hyperthyroidism, and among these, Graves' disease (Basedow's disease) is the most common. In Graves' disease, due to the autoimmune mechanism, one's own thyroid gland is recognized as a foreign body, and autoantibodies against TSHR present on thyroid follicular cells, called TSHR antibodies (TRAb), are produced. It is thought that this TRAb acts as an agonist on TSHR, and when TSHR is excessively stimulated, more of thyroid hormones than necessary is secreted, and hyperthyroidism develops.

In addition, as a disease associated with Graves' disease, thyroid eye disease is also known. Thyroid eye disease is an autoimmune inflammatory disease that exhibits various ocular symptoms, and is thought to be mainly caused by the agonist action of TRAb on TSHR in the orbital tissue. It often develops at roughly the same time as hyperthyroidism, but may not be associated with thyroid dysfunction.

Currently, antithyroid drugs such as thiamazole and propylthiouracil, which inhibit biosynthesis of thyroid hormones, are used for drug therapy for Graves' disease, but they have problems of a low remission rate, a long treatment period until remission is achieved, and a high frequency of side effects. Therefore, there is currently a need for a therapeutic agent for Graves' disease with a new mechanism of action.

Blocking TSHR or inhibiting signal transduction induced through TSHR inhibits thyroid hormone production and secretion and thyroid cell proliferation. Therefore, a TSHR antagonist is thought to have a therapeutic effect for Graves' disease and thyroid eye disease caused by the agonist action of TRAb on TSHR (PTL 1 and 2). NCGC00242364 is known as a TSHR antagonist, and it is found that it has a blood T4 concentration lowering effect in mice in which a TSH-releasing hormone (TRH) has been administered and mice in which a thyroid stimulating antibody M22 has been administered (NPL 1).

Compounds having TSHR antagonist activity are described in PTL 1 to 3, and NPL 1.

However, the nitrogen-containing condensed ring compound of the present invention is not mentioned in PTL 1 to 3 and NPL 1.

### [Citation List]

### [Patent Literature]

[PTL 1] U.S. Patent Application Publication No. 2011/0172267
[PTL 2] U.S. Patent Application Publication No. 2012/0315217
[PTL 3] U.S. Patent Application Publication No. 2019/0134024

### [Non Patent Literature]

[NPL 1] Susanne Neumann et al., "Endocrinology" 2014, Vol. 155, No. 1, pp. 310-314

### [Summary of Invention]

### [Technical Problem]

An object of the present invention is to provide a novel compound which has TSHR antagonist activity and is useful for treating thyroid-related diseases.

### [Solution to Problem]

The present invention relates to a compound represented by the following Formula (A-I) or a pharmacologically acceptable salt thereof.

Specifically, the present invention relates to the following [A-1] to [A-6], [1] to [9] and the like.

### [A-1]

A compound represented by Formula (A-I): 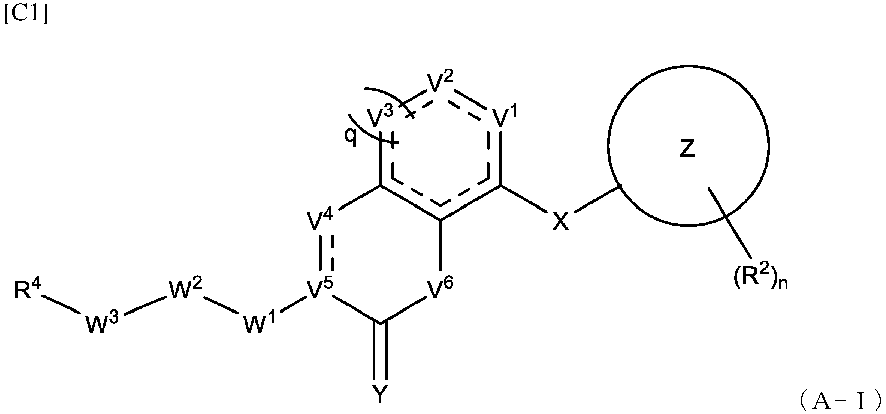 [wherein,
the ring Z is C₆₋₁₀ aryl, 5- or 6-membered heteroaryl, C₃₋₈ cycloalkyl, or 3- to 8-membered heterocycloalkyl;
V¹ is CR^{V1}R^{V1'}, CR^{V1}, NR^{V1}, N, O or S;
V² is CR^{V2}R^{V2'}, CR^{V2}, NR^{V2}, N, O or S;
V³ is CR^{V3}R^{V3'}, CR^{V3}, NR^{V3}, N, O, or S;
V⁴ is a single bond, CR^{T1}R^{T2}, CR^{T1}, O, NR^{T3}, or N;
V⁵ is CR³, C, or N;
V⁶ is NR¹;
X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0}, R^{X0'}, R^{X1}, and R^{X2} are each independently a hydrogen atom, a halogen atom, hydroxy, C₁₋₆ alkyl, or halo C₁₋₆ alkyl;
R^{X3} is a hydrogen atom, C₁₋₆ alkyl, or halo C₁₋₆ alkyl;
R^{X0} may, together with R^{X0'}, form a 3- to 8-membered saturated carbocyclic ring which is unsubstituted or substituted with 1 to 6 groups selected from Substituent Group B;
R^{X1} may, together with R^{X2}, form a 3- to 8-membered saturated carbocyclic ring which is unsubstituted or substituted with 1 to 6 groups selected from Substituent Group B;
R^{X1} may, together with R^{X3}, form a 3- to 8-membered saturated heterocyclic ring which is unsubstituted or substituted with 1 to 6 groups selected from Substituent Group B;
R¹ is a hydrogen atom, C₁₋₆ alkyl, or halo C₁₋₆ alkyl;
R¹ may, together with R^{X0}, R^{X1}, or R^{X3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
when there are two or more groups selected from Substituent Group B, these groups may be the same as or different from each other;
R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
R^{X0} may, together with R², form a 5- to 8-membered ring which is unsubstituted or substituted with 1 to 6 groups selected from Substituent Group B;
R^{X1} may, together with R², form a 5- to 8-membered ring which is unsubstituted or substituted with 1 to 6 groups selected from Substituent Group B;
R^{X3} may, together with R², form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
q is 0 or 1;
R^{V1}, R^{V1'}, R^{V2}, R^{V2'}, R^{V3}, and R^{V3'} are each independently a hydrogen atom, a halogen atom, hydroxy, cyano, C₁₋₆ alkyl, C₁₋₆ alkoxy, halo C₁₋₆ alkyl, hydroxy C₁₋₆ alkyl, C₁₋₆ alkoxy C₁₋₆ alkyl, C₆₋₁₀ aryl C₁₋₆ alkyl, halo C₁₋₆ alkoxy, C₆₋₁₀ aryl C₁₋₆ alkoxy, NR^{V4}COR^{V5}, NR^{V4}COOR^{V6}, or NR^{V4}CONR^{V7}R^{V8};
R^{V4} is a hydrogen atom or C₁₋₆ alkyl;
R^{V5}, R^{V6}, R^{V7}, and R^{V8} are each independently a hydrogen atom or a group selected from the group consisting of the following (i) to (ix):
   (i) C₁₋₆ alkyl which is unsubstituted or substituted with 1 to 6 groups selected from Substituent Group A,
   (ii) C₃₋₈ cycloalkyl which is unsubstituted or substituted with 1 to 6 groups selected from Substituent Group A,
   (iii) C₃₋₈ cycloalkyl C₁₋₆ alkyl which is unsubstituted or substituted with 1 to 6 groups selected from Substituent Group A,
   (iv) C₆₋₁₀ aryl which is unsubstituted or substituted with 1 to 6 groups selected from Substituent Group A,
   (v) C₆₋₁₀ aryl C₁₋₆ alkyl which is unsubstituted or substituted with 1 to 6 groups selected from Substituent Group A,
   (vi) 3- to 8-membered heterocycloalkyl which is unsubstituted or substituted with 1 to 6 groups selected from Substituent Group A,
   (vii) 3- to 8-membered heterocycloalkyl C₁₋₆ alkyl which is unsubstituted or substituted with 1 to 6 groups selected from Substituent Group A,
   (viii) 5- to 10-membered heteroaryl which is unsubstituted or substituted with 1 to 6 groups selected from Substituent Group A, and
   (ix) 5- to 10-membered heteroaryl C₁₋₆ alkyl which is unsubstituted or substituted with 1 to 6 groups selected from Substituent Group A;
Substituent Group A is a group consisting of a halogen atom, cyano, hydroxy, N(C₁₋₆ alkyl)₂, C₁₋₆ alkyl, halo C₁₋₆ alkyl, C₁₋₆ alkoxy, halo C₁₋₆ alkoxy, C₆₋₁₀ aryl C₁₋₆ alkoxy, and C₃₋₈ cycloalkyl;
when there are two or more groups selected from Substituent Group A, these groups may be the same as or different from each other;
R^{T1} and R^{T2} are each independently a hydrogen atom, a halogen atom, hydroxy, C₁₋₆ alkyl, or halo C₁₋₆ alkyl;
R^{T3} is a hydrogen atom, C₁₋₆ alkyl, or halo C₁₋₆ alkyl;
R³ is a hydrogen atom, a halogen atom, or C₁₋₆ alkyl;
R⁴ is C₁₋₆ alkyl, halo C₁₋₆ alkyl, C₁₋₆ alkoxy C₁₋₆ alkyl, OR⁵, NR⁵R^{5'}, C₃₋₈ cycloalkyl, 3- to 8-membered heterocycloalkyl which is unsubstituted or substituted with 1 to 6 groups selected from Substituent Group C, C₆₋₁₀ aryl or 5- or 6-membered heteroaryl which is unsubstituted or substituted with 1 to 4 groups selected from Substituent Group C;
Substituent Group C is a group consisting of a halogen atom, cyano, hydroxy, C₁₋₆ alkyl, halo C₁₋₆ alkyl, hydroxy C₁₋₆ alkyl, C₁₋₆ alkoxy, halo C₁₋₆ alkoxy, oxo, CONH₂, CONH-(C₁₋₆ alkyl), and CONH-(C₃₋₈ cycloalkyl);
when there are two or more groups selected from Substituent Group C, these groups may be the same as or different from each other;
R⁵ and R^{5'} are each independently a hydrogen atom, C₁₋₆ alkyl, halo C₁₋₆ alkyl, or C₃₋₈ cycloalkyl;
R⁵ may, together with R^{5'}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
R³ may, together with R^{T1}, R^{T2}, or R^{T3}, form a 3- to 10-membered ring which is unsubstituted or substituted with 1 to 6 groups selected from Substituent Group B;
R³ may, together with R⁵, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
R⁴ may, together with R^{T1}, R^{T2}, or R^{T3}, form a 3- to 10-membered ring which is unsubstituted or substituted with 1 to 6 groups selected from Substituent Group B;
R⁵ may, together with R^{T1}, R^{T2}, or R^{T3}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
R^{V7} may, together with R^{V8}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
W¹ is CR^{W1}R^{W2}, O, NR^{W3}, or a single bond;
W² is CR^{W1}R^{W2}, O, NR^{W3}, or a single bond;
R^{W1} and R^{W2} are each independently a hydrogen atom, a halogen atom, C₁₋₆ alkyl, or halo C₁₋₆ alkyl;
R^{W3} is a hydrogen atom, C₁₋₆ alkyl, or halo C₁₋₆ alkyl;
W³ is CO, CNR^{W4}, SO, SO₂, or a single bond;
R^{W4} is a hydrogen atom, C₁₋₆ alkyl, cyano, hydroxy, C₁₋₆ alkoxy, CO-(C₁₋₆ alkyl), or SO₂-(C₁₋₆ alkyl);
n is an integer of 0 to 3;
when n is 2 or 3, these R²s may be the same as or different from each other;
R⁴ and R⁵ may, together with R^{W1} or R^{W3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
Y is O, S, or NR^{Y};
R^{Y} is a hydrogen atom or C₁₋₆ alkyl;
R^{Y} may, together with R¹, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B; 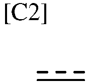
indicates a single bond or a double bond]
or a pharmacologically acceptable salt thereof;
(provided that, in Formula (A-I), when W¹ is a single bond; W² is CH₂ or NH; W³ is CO; and R⁴ is C₁₋₆ alkyl, halo C₁₋₆ alkyl, NR⁵R^{5'}, or C₃₋₈ cycloalkyl, any of the following (a) to (f) is satisfied;
   (a) R^{T1} and R^{T2} are not both hydrogen atoms
   (b) X is not O, and when X is CHR^{X0}, R^{X0} is neither a hydrogen atom nor C₁₋₆ alkyl
   (c) R¹ is halo C₁₋₆ alkyl
   (d) V² is CR^{V2}, and R^{V2} is halo C₁₋₆ alkoxy, NR^{V4}COR^{V5}, NR^{V4}COOR^{V6}, or NR^{V4}CONR^{V7}R^{V8}
   (e) Y is not O
   (f) q is 0).

### [A-2-1]

The compound according to the above [A-1],
wherein the compound is represented by Formula (A-II): 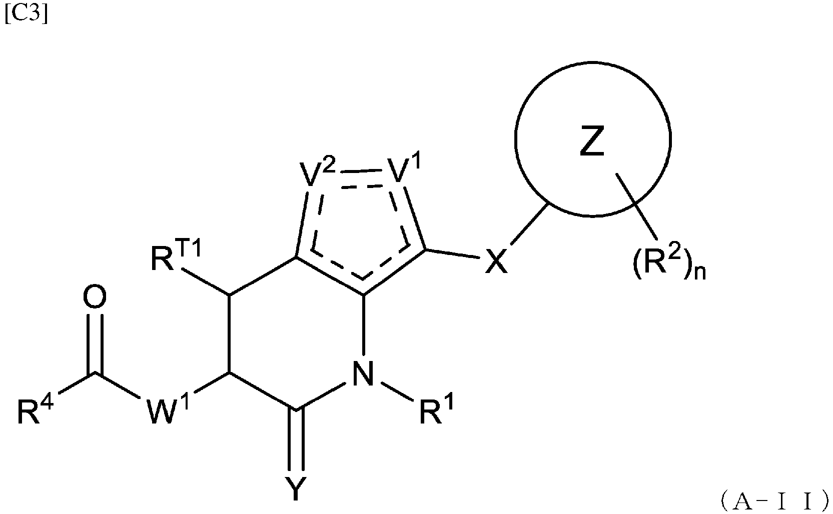 [wherein,
the ring Z is C₆₋₁₀ aryl, 5- or 6-membered heteroaryl, C₃₋₈ cycloalkyl, or 3- to 8-membered heterocycloalkyl;
V¹ is CR^{V1}R^{V1'}, CR^{V1}, NR^{V1}, N, O or S;
V² is CR^{V2}R^{V2'}, CR^{V2}, NR^{V2}, N, O or S;
R^{V1}, R^{V1'}, R^{V2}, and R^{V2'} are each independently a hydrogen atom, a halogen atom, hydroxy, cyano, C₁₋₆ alkyl, C₁₋₆ alkoxy, halo C₁₋₆ alkyl, hydroxy C₁₋₆ alkyl, C₁₋₆ alkoxy C₁₋₆ alkyl, halo C₁₋₆ alkoxy, NR^{V4}COR^{V5}, NR^{V4}COOR^{V6}, or NR^{V4}CONR^{V7}R^{V8};
R^{V4} is a hydrogen atom or C₁₋₆ alkyl;
R^{V5}, R^{V6}, R^{V7}, and R^{V8} are each independently a hydrogen atom or a group selected from the following groups (i) to (v):
   (i) C₁₋₆ alkyl which is unsubstituted or substituted with 1 to 6 groups selected from Substituent Group A,
   (ii) C₃₋₈ cycloalkyl which is unsubstituted or substituted with 1 to 6 groups selected from Substituent Group A,
   (iii) C₃₋₈ cycloalkyl C₁₋₆ alkyl which is unsubstituted or substituted with 1 to 6 groups selected from Substituent Group A,
   (iv) C₆₋₁₀ aryl which is unsubstituted or substituted with 1 to 6 groups selected from Substituent Group A, and
   (v) C₆₋₁₀ aryl C₁₋₆ alkyl which is unsubstituted or substituted with 1 to 6 groups selected from Substituent Group A;
Substituent Group A is a group consisting of a halogen atom, C₁₋₆ alkyl, halo C₁₋₆ alkyl, C₁₋₆ alkoxy, halo C₁₋₆ alkoxy, and C₃₋₈ cycloalkyl;
when there are two or more groups selected from Substituent Group A, these groups may be the same as or different from each other;
X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0}, R^{X0'}, R^{X1}, and R^{X2} are each independently a hydrogen atom, a halogen atom, hydroxy, C₁₋₆ alkyl, or halo C₁₋₆ alkyl;
R^{X3} is a hydrogen atom, C₁₋₆ alkyl, or halo C₁₋₆ alkyl;
R^{X0} may, together with R^{X0'}, form a 3- to 8-membered saturated carbocyclic ring which is unsubstituted or substituted with 1 to 6 groups selected from Substituent Group B;
R^{X1} may, together with R^{X2}, form a 3- to 8-membered saturated carbocyclic ring which is unsubstituted or substituted with 1 to 6 groups selected from Substituent Group B;
R^{X1} may, together with R^{X3}, form a 3- to 8-membered saturated heterocyclic ring which is unsubstituted or substituted with 1 to 6 groups selected from Substituent Group B;
R¹ is a hydrogen atom, C₁₋₆ alkyl, or halo C₁₋₆ alkyl;
R¹ may, together with R^{X0}, R^{X1}, or R^{X3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
when there are two or more groups selected from Substituent Group B, these groups may be the same as or different from each other;
R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
R^{T1} is a hydrogen atom, a halogen atom, hydroxy, C₁₋₆ alkyl, or halo C₁₋₆ alkyl;
R⁴ is C₁₋₆ alkyl, halo C₁₋₆ alkyl, C₁₋₆ alkoxy C₁₋₆ alkyl, OR⁵, NR⁵R^{5'}, C₃₋₈ cycloalkyl, or 3- to 8-membered heterocycloalkyl;
R⁵ and R^{5'} are each independently a hydrogen atom, C₁₋₆ alkyl, halo C₁₋₆ alkyl, or C₃₋₈ cycloalkyl;
R⁵ may, together with R^{5'}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
W¹ is CR^{W1}R^{W2}, O, NR^{W3}, or a single bond;
R^{W1} and R^{W2} are each independently a hydrogen atom, a halogen atom, C₁₋₆ alkyl, or halo C₁₋₆ alkyl;
R^{W3} is a hydrogen atom, C₁₋₆ alkyl, or halo C₁₋₆ alkyl;
n is an integer of 0 to 3;
when n is 2 or 3, these R²s may be the same as or different from each other; and
Y is O or S;

   [C4] - - -
indicates a single bond or a double bond]
or a pharmacologically acceptable salt thereof.

### [A-2-2]

The compound according to the above [A-1] or [A-2-1],
wherein R^{T1} is a hydrogen atom,
or a pharmacologically acceptable salt thereof.

### [A-2-3]

The compound according to any one of the above [A-1] to [A-2-2],
wherein R¹ is a hydrogen atom or C₁₋₆ alkyl,
or a pharmacologically acceptable salt thereof.

### [A-2-4]

The compound according to any one of the above [A-1] to [A-2-3],
wherein the ring Z is C₆₋₁₀ aryl or 5- or 6-membered heteroaryl,
or a pharmacologically acceptable salt thereof.

### [A-2-5]

The compound according to any one of the above [A-1] to [A-2-4],
wherein X is CR^{X0}R^{X0'}, O, CO, or -(CR^{X1}R^{X2})₂-; and
R^{X0} and R^{X0'} are each independently a hydrogen atom, C₁₋₆ alkyl, or halo C₁₋₆ alkyl, or a pharmacologically acceptable salt thereof.

### [A-2-6]

The compound according to any one of the above [A-1] to [A-2-5],
wherein R^{V1}, R^{V1'}, R^{V2}, and R^{V2'} are each independently a hydrogen atom, a halogen atom, cyano, C₁₋₆ alkyl, C₁₋₆ alkoxy, halo C₁₋₆ alkyl, hydroxy C₁₋₆ alkyl, or C₁₋₆ alkoxy C₁₋₆ alkyl,
or a pharmacologically acceptable salt thereof.

### [A-2-7]

The compound according to any one of the above [A-1] to [A-2-6],
wherein Y is O,
or a pharmacologically acceptable salt thereof.

### [A-2-8]

The compound according to any one of the above [A-1] to [A-2-7],
wherein R⁴ is NR⁵R^{5'}; and
R⁵ and R^{5'} have the same meanings as in the above [A-1],
or a pharmacologically acceptable salt thereof.

### [A-2-9]

The compound according to any one of the above [A-1] to [A-2-8],
wherein W¹ is NR^{W3}; and
R^{W3} has the same meaning as in the above [A-1],
or a pharmacologically acceptable salt thereof.

### [A-2-10]

The compound according to any one of the above [A-1] to [A-2-9],
wherein R⁵ and R^{5'} are a hydrogen atom,
or a pharmacologically acceptable salt thereof.

### [A-2-11]

The compound according to the above [A-2-1],
wherein the ring Z is C₆₋₁₀ aryl;
V¹ is CR^{V1}, N, or S;
V² is CR^{V2} or S;
R^{V1} and R^{V2} are each independently a hydrogen atom, a halogen atom, cyano, or hydroxy C₁₋₆ alkyl;
X is CHR^{X0} or CO;
R^{X0} is a hydrogen atom or C₁₋₆ alkyl;
R¹ is C₁₋₆ alkyl;
R² is a halogen atom;
R^{T1} is a hydrogen atom;
R⁴ is NH₂;
W¹ is NH;
n is 2; and
Y is O,
or a pharmacologically acceptable salt thereof.

### [A-2-12]

The compound according to any one of the above [A-1] to [A-2-11],
wherein the compound is selected from the group consisting of the following compounds: 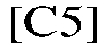 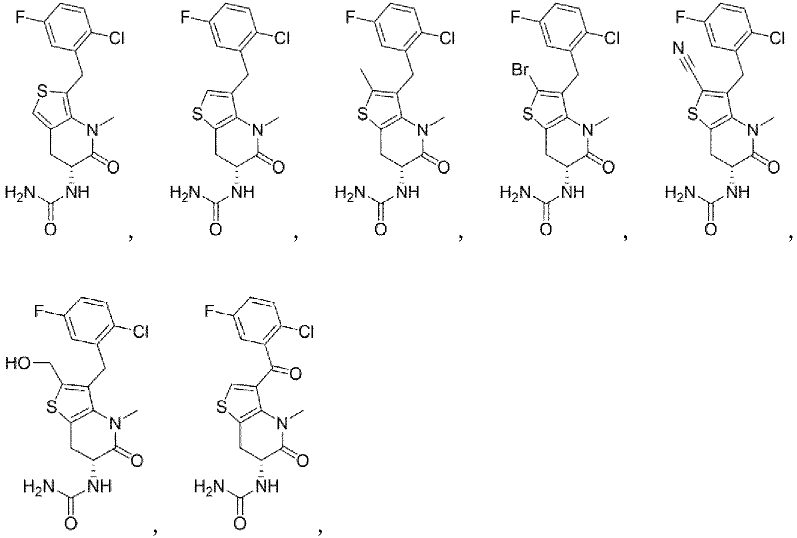 and 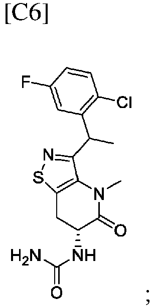 or a pharmacologically acceptable salt thereof.

### [A-3-1]

The compound according to the above [A-1],
wherein the compound is represented by Formula (A-III): 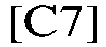 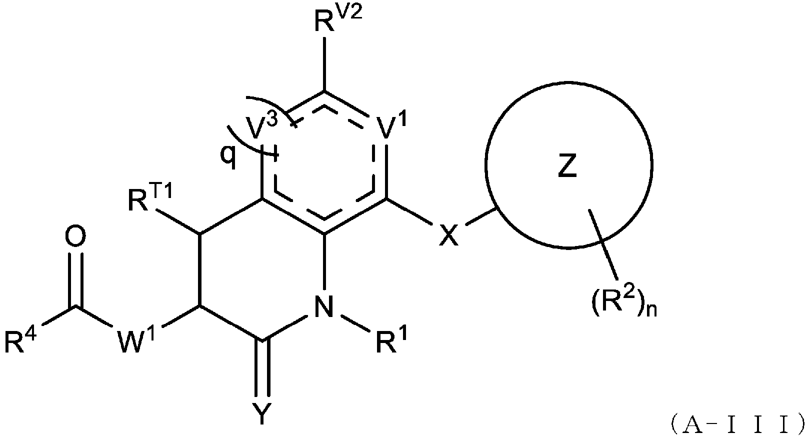 [wherein,
the ring Z is C₆₋₁₀ aryl, 5- or 6-membered heteroaryl, C₃₋₈ cycloalkyl, or 3- to 8-membered heterocycloalkyl;
V¹ is CR^{V1}R^{V1'}, CR^{V1}, NR^{V1}, N, O or S;
V³ is CR^{V3}R^{V3'}, CR^{V3}, NR^{V3}, N, O, or S;
R^{V1}, R^{V1'}, R^{V3}, and R^{V3'} are each independently a hydrogen atom, a halogen atom, hydroxy, cyano, C₁₋₆ alkyl, C₁₋₆ alkoxy, halo C₁₋₆ alkyl, hydroxy C₁₋₆ alkyl, C₁₋₆ alkoxy C₁₋₆ alkyl, halo C₁₋₆ alkoxy, NR^{V4}COR^{V5}, NR^{V4}COOR^{V6}, or NR^{V4}CONR^{V7}R^{V8};
R^{V2} is halo C₁₋₆ alkoxy, NR^{V4}COR^{V5}, NR^{V4}COOR^{V6}, or NR^{V4}CONR^{V7}R^{V8};
R^{V4} is a hydrogen atom or C₁₋₆ alkyl;
R^{V5}, R^{V6}, R^{V7}, and R^{V8} are each independently a hydrogen atom or a group selected from the group consisting of the following (i) to (ix):
   (i) C₁₋₆ alkyl which is unsubstituted or substituted with 1 to 6 groups selected from Substituent Group A,
   (ii) C₃₋₈ cycloalkyl which is unsubstituted or substituted with 1 to 6 groups selected from Substituent Group A,
   (iii) C₃₋₈ cycloalkyl C₁₋₆ alkyl which is unsubstituted or substituted with 1 to 6 groups selected from Substituent Group A,
   (iv) C₆₋₁₀ aryl which is unsubstituted or substituted with 1 to 6 groups selected from Substituent Group A,
   (v) C₆₋₁₀ aryl C₁₋₆ alkyl which is unsubstituted or substituted with 1 to 6 groups selected from Substituent Group A,
   (vi) 3- to 8-membered heterocycloalkyl which is unsubstituted or substituted with 1 to 6 groups selected from Substituent Group A,
   (vii) 3- to 8-membered heterocycloalkyl C₁₋₆ alkyl which is unsubstituted or substituted with 1 to 6 groups selected from Substituent Group A,
   (viii) 5- to 10-membered heteroaryl which is unsubstituted or substituted with 1 to 6 groups selected from Substituent Group A, and
   (ix) 5- to 10-membered heteroaryl C₁₋₆ alkyl which is unsubstituted or substituted with 1 to 6 groups selected from Substituent Group A;
Substituent Group A is a group consisting of a halogen atom, cyano, hydroxy, N(C₁₋₆ alkyl)₂, C₁₋₆ alkyl, halo C₁₋₆ alkyl, C₁₋₆ alkoxy, halo C₁₋₆ alkoxy, C₆₋₁₀ aryl C₁₋₆ alkoxy, and C₃₋₈ cycloalkyl;
when there are two or more groups selected from Substituent Group A, these groups may be the same as or different from each other;
X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0}, R^{X0'}, R^{X1}, and R^{X2} are each independently a hydrogen atom, a halogen atom, hydroxy, C₁₋₆ alkyl, or halo C₁₋₆ alkyl;
R^{X3} is a hydrogen atom, C₁₋₆ alkyl, or halo C₁₋₆ alkyl;
R^{X0} may, together with R^{X0'}, form a 3- to 8-membered saturated carbocyclic ring which is unsubstituted or substituted with 1 to 6 groups selected from Substituent Group B;
R^{X1} may, together with R^{X2}, form a 3- to 8-membered saturated carbocyclic ring which is unsubstituted or substituted with 1 to 6 groups selected from Substituent Group B;
R^{X1} may, together with R^{X3}, form a 3- to 8-membered saturated heterocyclic ring which is unsubstituted or substituted with 1 to 6 groups selected from Substituent Group B;
R¹ is a hydrogen atom, C₁₋₆ alkyl, or halo C₁₋₆ alkyl;
R¹ may, together with R^{X0}, R^{X1}, or R^{X3}, form a 5- to 8-membered ring which is unsubstituted or substituted with 1 to 6 groups selected from Substituent Group B;
Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
when there are two or more groups selected from Substituent Group B, these groups may be the same as or different from each other;
R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
R^{T1} is a hydrogen atom, a halogen atom, hydroxy, C₁₋₆ alkyl, or halo C₁₋₆ alkyl;
R⁴ is C₁₋₆ alkyl, halo C₁₋₆ alkyl, C₁₋₆ alkoxy C₁₋₆ alkyl, OR⁵, NR⁵R^{5'}, C₃₋₈ cycloalkyl, or 3- to 8-membered heterocycloalkyl;
R⁵ and R^{5'} are each independently a hydrogen atom, C₁₋₆ alkyl, halo C₁₋₆ alkyl, or C₃₋₈ cycloalkyl;
R⁵ may, together with R^{5'}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
W¹ is CR^{W1}R^{W2}, O, NR^{W3}, or a single bond;
R^{W1} and R^{W2} are each independently a hydrogen atom, a halogen atom, C₁₋₆ alkyl, or halo C₁₋₆ alkyl;
R^{W3} is a hydrogen atom, C₁₋₆ alkyl, or halo C₁₋₆ alkyl;
n is an integer of 0 to 3;
when n is 2 or 3, these R²s may be the same as or different from each other;
q is 0 or 1; and
Y is O or S;

   [C8] - - -
indicates a single bond or a double bond],
or a pharmacologically acceptable salt thereof.

### [A-3-2]

The compound according to the above [A-1] or [A-3-1],
wherein R^{T1} is a hydrogen atom,
or a pharmacologically acceptable salt thereof.

### [A-3-3]

The compound according to the above [A-1], the above [A-3-1], or the above [A-3-2],
wherein R¹ is a hydrogen atom or C₁₋₆ alkyl,
or a pharmacologically acceptable salt thereof.

### [A-3-4]

The compound according to the above [A-1] or any one of the above [A-3-1] to [A-3-3],
wherein the ring Z is C₆₋₁₀ aryl or 5- or 6-membered heteroaryl,
or a pharmacologically acceptable salt thereof.

### [A-3-5]

The compound according to the above [A-1] or any one of the above [A-3-1] to [A-3-4],
wherein X is CR^{X0}R^{X0'}, O, CO, or -(CR^{X1}R^{X2})₂-,
or a pharmacologically acceptable salt thereof.

### [A-3-6]

The compound according to the above [A-1] or any one of the above [A-3-1] to [A-3-5],
wherein R^{V1}, R^{V1'}, R^{V3}, and R^{V3'} are each independently a hydrogen atom, a halogen atom, C₁₋₆ alkyl, halo C₁₋₆ alkyl, or hydroxy C₁₋₆ alkyl,
or a pharmacologically acceptable salt thereof.

### [A-3-7]

The compound according to the above [A-1] or any one of the above [A-3-1] to [A-3-6],
wherein Y is O,
or a pharmacologically acceptable salt thereof.

### [A-3-8]

The compound according to the above [A-1] or any one of the above [A-3-1] to [A-3-7],
wherein R⁴ is NR⁵R^{5'}; and
R⁵ and R^{5'} have the same meanings as in the above [A-1],
or a pharmacologically acceptable salt thereof.

### [A-3-9]

The compound according to the above [A-1] or any one of the above [A-3-1] to [A-3-8],
wherein q is 1,
or a pharmacologically acceptable salt thereof.

### [A-3-10]

The compound according to the above [A-1] or any one of the above [A-3-1] to [A-3-9],
wherein W¹ is CH₂, NH, or a single bond,
or a pharmacologically acceptable salt thereof.

### [A-3-11]

The compound according to the above [A-1] or any one of the above [A-3-1] to [A-3-10],
wherein R⁵ and R^{5'} are a hydrogen atom,
or a pharmacologically acceptable salt thereof.

### [A-3-12]

The compound according to the above [A-3-1],
wherein the ring Z is C₆₋₁₀ aryl or 5- or 6-membered heteroaryl;
V¹ is CH;
V³ is CH;
R^{V2} is NHCOOR^{V6};
R^{V6} is a group selected from the following groups (i) to (iii):
   (i) C₁₋₆ alkyl which is unsubstituted or substituted with 1 to 6 groups selected from Substituent Group A,
   (ii) C₃₋₈ cycloalkyl which is unsubstituted or substituted with 1 to 6 groups selected from Substituent Group A, and
   (iii) C₃₋₈ cycloalkyl C₁₋₆ alkyl which is unsubstituted or substituted with 1 to 6 groups selected from Substituent Group A;
Substituent Group A is a group consisting of a halogen atom and C₁₋₆ alkyl;
when there are two or more groups selected from Substituent Group A, these groups may be the same as or different from each other;
X is O;
R¹ is C₁₋₆ alkyl;
R² is cyano or halo C₁₋₆ alkyl;
R^{T1} is a hydrogen atom;
R⁴ is NH₂;
W¹ is CH₂, NH or a single bond;
n is 1;
q is 1; and
Y is O,
or a pharmacologically acceptable salt thereof.

### [A-4]

A pharmaceutical composition comprising the compound or a pharmacologically acceptable salt thereof according to any one of the above [A-1] to [A-3-12] and a pharmaceutical additive.

### [A-5]

The pharmaceutical composition according to the above [A-4], which is a pharmaceutical composition for treating thyroid-related diseases.

### [A-6]

The pharmaceutical composition according to the above [A-5], wherein the thyroid-related diseases are hyperthyroidism, Graves' disease or thyroid eye disease.

As one embodiment, the present invention relates to a method of treating thyroid-related diseases, including administering a required amount of the pharmaceutical composition according to the above [A-4] to a patient.

As one embodiment, the present invention relates to a use of the compound or a pharmacologically acceptable salt thereof according to any one of the above [A-1] to [A-3-12], for manufacturing a pharmaceutical composition for treating thyroid-related diseases.

[1] A compound represented by Formula (I): 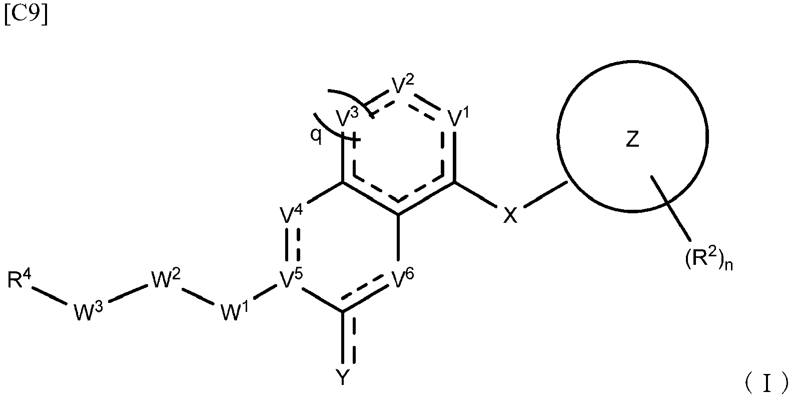 [wherein,
   the ring Z is C₆₋₁₀ aryl, 5- or 6-membered heteroaryl, C₃₋₈ cycloalkyl, or 3- to 8-membered heterocycloalkyl;
   V¹ is CR^{V1}R^{V1'}, CR^{V1}, NR^{V1}, N, O or S;
   V² is CR^{V2}R^{V2'}, CR^{V2}, NR^{V2}, N, O or S;
   V³ is CR^{V3}R^{V3'}, CR^{V3}, NR^{V3}, N, O, or S;
   V⁴ is a single bond, CR^{T1}R^{T2}, CR^{T1}, O, NR^{T3}, or N;
   V⁵ is CR³, C, or N;
   V⁶ is CR¹, CR¹R^{1'}, N or NR¹;
   X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
   R^{X0}, R^{X0'}, R^{X1}, and R^{X2} are each independently a hydrogen atom, a halogen atom, hydroxy, C₁₋₆ alkyl, or halo C₁₋₆ alkyl;
   R^{X3} is a hydrogen atom, C₁₋₆ alkyl, or halo C₁₋₆ alkyl;
   R^{X0} may, together with R^{X0'}, form a 3- to 8-membered saturated carbocyclic ring which is unsubstituted or substituted with 1 to 6 groups selected from Substituent Group B;
   R^{X1} may, together with R^{X2}, form a 3- to 8-membered saturated carbocyclic ring which is unsubstituted or substituted with 1 to 6 groups selected from Substituent Group B;
   R^{X1} may, together with R^{X3}, form a 3- to 8-membered saturated heterocyclic ring which is unsubstituted or substituted with 1 to 6 groups selected from Substituent Group B;
   R¹ and R^{1'} are each independently a hydrogen atom, C₁₋₆ alkyl, or halo C₁₋₆ alkyl;
   R^{X0} may, together with R¹, form a 5- to 8-membered ring which is unsubstituted or substituted with 1 to 6 groups selected from Substituent Group B;
   R^{X1} may, together with R¹, form a 5- to 8-membered ring which is unsubstituted or substituted with 1 to 6 groups selected from Substituent Group B;
   R^{X3} may, together with R¹, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
   Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
   when there are two or more groups selected from Substituent Group B, these groups may be the same as or different from each other;
   R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
   R^{X0} may, together with R², form a 5- to 8-membered ring which is unsubstituted or substituted with 1 to 6 groups selected from Substituent Group B;
   R^{X1} may, together with R², form a 5- to 8-membered ring which is unsubstituted or substituted with 1 to 6 groups selected from Substituent Group B;
   R^{X3} may, together with R², form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
   q is 0 or 1;
   R^{V1}, R^{V1'}, R^{V2}, R^{V2'}, R^{V3}, and R^{V3'} are each independently a hydrogen atom, a halogen atom, hydroxy, C₁₋₆ alkyl, C₁₋₆ alkoxy, halo C₁₋₆ alkyl, hydroxy C₁₋₆ alkyl, C₆₋₁₀ aryl C₁₋₆ alkyl, halo C₁₋₆ alkoxy, C₆₋₁₀ aryl C₁₋₆ alkoxy, NR^{V4}COR^{V5}, NR^{V4}COOR^{V6}, or NR^{V4}CONR^{V7}R^{V8};
   R^{V4} is a hydrogen atom or C₁₋₆ alkyl;
   R^{V5}, R^{V6}, R^{V7}, and R^{V8} are each independently a hydrogen atom or a group selected from the group consisting of the following (i) to (ix):
      (i) C₁₋₆ alkyl which is unsubstituted or substituted with 1 to 6 groups selected from Substituent Group A,
      (ii) C₃₋₈ cycloalkyl which is unsubstituted or substituted with 1 to 6 groups selected from Substituent Group A,
      (iii) C₃₋₈ cycloalkyl C₁₋₆ alkyl which is unsubstituted or substituted with 1 to 6 groups selected from Substituent Group A,
      (iv) C₆₋₁₀ aryl which is unsubstituted or substituted with 1 to 6 groups selected from Substituent Group A,
      (v) C₆₋₁₀ aryl C₁₋₆ alkyl which is unsubstituted or substituted with 1 to 6 groups selected from Substituent Group A,
      (vi) 3- to 8-membered heterocycloalkyl which is unsubstituted or substituted with 1 to 6 groups selected from Substituent Group A,
      (vii) 3- to 8-membered heterocycloalkyl C₁₋₆ alkyl which is unsubstituted or substituted with 1 to 6 groups selected from Substituent Group A,
      (viii) 5- to 10-membered heteroaryl which is unsubstituted or substituted with 1 to 5 groups selected from Substituent Group A, and
      (ix) 5- to 10-membered heteroaryl C₁₋₆ alkyl which is unsubstituted or substituted with 1 to 6 groups selected from Substituent Group A;
   Substituent Group A is a group consisting of a halogen atom, cyano, hydroxy, N(C₁₋₆ alkyl)₂, C₁₋₆ alkyl, halo C₁₋₆ alkyl, C₁₋₆ alkoxy, halo C₁₋₆ alkoxy, C₆₋₁₀ aryl C₁₋₆ alkoxy, and C₃₋₈ cycloalkyl;
   when there are two or more groups selected from Substituent Group A, these groups may be the same as or different from each other;
   R^{T1} and R^{T2} are each independently a hydrogen atom, a halogen atom, hydroxy, C₁₋₆ alkyl, or halo C₁₋₆ alkyl;
   R^{T3} is a hydrogen atom, C₁₋₆ alkyl, or halo C₁₋₆ alkyl;
   R³ is a hydrogen atom, a halogen atom, or C₁₋₆ alkyl;
   R⁴ is C₁₋₆ alkyl, halo C₁₋₆ alkyl, OR⁵, NR⁵R^{5'}, C₃₋₈ cycloalkyl, 3- to 8-membered heterocycloalkyl which is unsubstituted or substituted with 1 to 6 groups selected from Substituent Group C, C₆₋₁₀ aryl or 5- or 6-membered heteroaryl which is unsubstituted or substituted with 1 to 4 groups selected from Substituent Group C;
   Substituent Group C is a group consisting of a halogen atom, cyano, hydroxy, C₁₋₆ alkyl, halo C₁₋₆ alkyl, hydroxy C₁₋₆ alkyl, C₁₋₆ alkoxy, halo C₁₋₆ alkoxy, oxo, CONH₂, CONH-(C₁₋₆ alkyl), or CONH-(C₃₋₈ cycloalkyl);
   when there are two or more groups selected from Substituent Group C, these groups may be the same as or different from each other;
   R⁵ and R⁵' are each independently a hydrogen atom, C₁₋₆ alkyl, halo C₁₋₆ alkyl, or C₃₋₈ cycloalkyl;
   R³ may, together with R⁴, R^{T1}, R^{T2}, or R^{T3}, form a 3- to 10-membered ring which is unsubstituted or substituted with 1 to 6 groups selected from Substituent Group B;
   R³ may, together with R⁵, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
   R⁴ may, together with R^{T1}, R^{T2}, or R^{T3}, form a 3- to 10-membered ring which is unsubstituted or substituted with 1 to 6 groups selected from Substituent Group B;
   R⁵ may, together with R^{T1}, R^{T2}, or R^{T3}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
   R^{V7} may, together with R^{V8}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
   W¹ is CR^{W1}R^{W2}, O, NR^{W3}, or a single bond;
   W² is CR^{W1}R^{W2}, O, NR^{W3}, or a single bond;
   R^{W1} and R^{W2} are each independently a hydrogen atom, a halogen atom, C₁₋₆ alkyl, or halo C₁₋₆ alkyl;
   R^{W3} is a hydrogen atom, C₁₋₆ alkyl, or halo C₁₋₆ alkyl;
   W³ is CO, CNR^{W4}, SO, SO₂, or a single bond;
   R^{W4} is a hydrogen atom, C₁₋₆ alkyl, cyano, hydroxy, C₁₋₆ alkoxy, CO-(C₁₋₆ alkyl), or SO₂-(C₁₋₆ alkyl);
   n is an integer of 0 to 3;
   when n is 2 or 3, these R²s may be the same as or different from each other;
   R⁴ and R⁵ may, together with R^{W1} or R^{W3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
   Y is O, OR^{Y}, S, SR^{Y}, NR^{Y}, or NR^{Y}R^{Y'};
   R^{Y} and R^{Y'} are a hydrogen atom or C₁₋₆ alkyl;
   R^{Y} may, together with R¹, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;

      [C10] - - -
   indicates a single bond or a double bond]
   or a pharmacologically acceptable salt thereof;
   (provided that, in Formula (I), when W¹ is a single bond; W² is CH₂ or NH; W³ is CO; and R⁴ is C₁₋₆ alkyl, halo C₁₋₆ alkyl, NR⁵R⁵', or C₃₋₈ cycloalkyl, any of the following (a) to (f) is satisfied;
      (a) R^{T1} and R^{T2} are not both hydrogen atoms
      (b) X is not O, and when X is CHR^{X0}, R^{X0} is neither a hydrogen atom nor C₁₋₆ alkyl
      (c) when V⁶ is NR¹, R¹ is halo C₁₋₆ alkyl
      (d) V² is CR^{V2}, and R^{V2} is halo C₁₋₆ alkoxy, NR^{V4}COR^{V5}, NR^{V4}COOR^{V6}, or NR^{V4}CONR^{V7}R^{V8}
      (e) Y is not O
      (f) q is 0).

### [2-A1]

The compound according to the above [1],
wherein the compound is represented by Formula (I-A1): 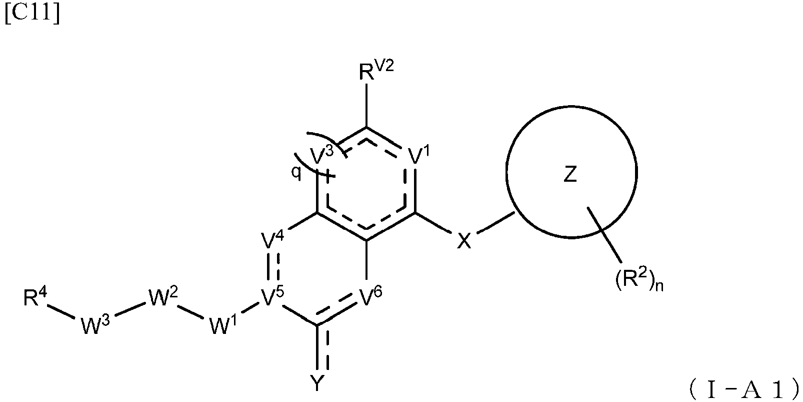 [wherein,
the ring Z, X, V¹, V³, V⁴, V⁵, V⁶, W¹, W², W³, R², R⁴, n, Y, and q have the same meanings as in the above [1]; and
R^{V2} is halo C₁₋₆ alkoxy, NR^{V4}COR^{V5}, NR^{V4}COOR^{V6}, or NR^{V4}CONR^{V7}R^{V8};

   [C12] - - -
indicates a single bond or a double bond;
R^{V4}, R^{V5}, R^{V6}, R^{V7} and R^{V8} have the same meanings as in the above [1]]
or a pharmacologically acceptable salt thereof.

### [2-A2]

The compound according to the above [1] or [2-A1],
wherein the compound is represented by Formula (I-A2): 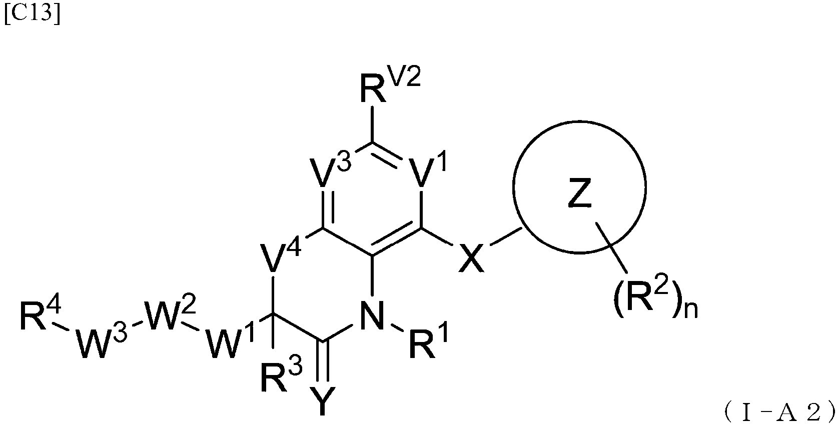 [wherein,
the ring Z, X, W¹, W², W³, R¹, R², R³, R⁴, and n have the same meanings as in the above [1];
R^{V2} has the same meaning as in the above [2-A1];
V¹ is CR^{V1} or N;
V³ is CR^{V3} or N;
V⁴ is a single bond, CR^{T1}R^{T2}, O, or NR^{T3};
Y is O, S, or NR^{Y};
R^{V1}, R^{V3}, R^{T1}, R^{T2}, R^{T3}, and R^{Y} have the same meanings as in the above [1]]
or a pharmacologically acceptable salt thereof.

### [2-A3]

The compound according to any one of the above [1] to [2-A2],
wherein the compound is represented by Formula (I-A3): 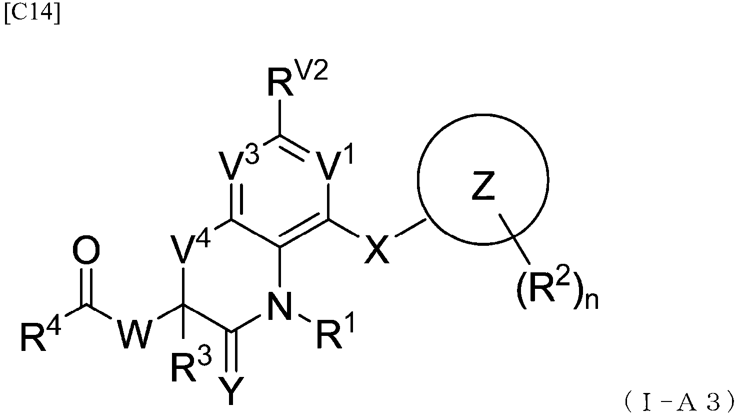 [wherein,
the ring Z, R¹, R², R³, R⁴, and n have the same meanings as in the above [1];
X is CR^{X0}R^{X0'}, O, NR^{X3}, CO, or -C(=CH₂)-;
Y is O or S;
R^{V2} has the same meaning as in the above [2-A1];
V¹, V³, and V⁴ have the same meanings as in the above [2-A2];
W is a single bond, CH₂, or NH; and
R^{X0}, R^{X0'}, and R^{X3} have the same meanings as in the above [1]]
or a pharmacologically acceptable salt thereof.

### [2-A4]

The compound according to any one of the above [1] to [2-A3],
wherein R^{V2} is NR^{V4}COOR^{V6}, or a pharmacologically acceptable salt thereof.

### [2-A5]

The compound according to any one of the above [1] to [2-A4],
wherein R^{V6} is a group selected from the following groups (i) to (iii):
   (i) C₁₋₆ alkyl which is unsubstituted or substituted with 1 to 6 groups selected from Substituent Group A,
   (ii) C₃₋₈ cycloalkyl which is unsubstituted or substituted with 1 to 6 groups selected from Substituent Group A,
   (iii) C₃₋₈ cycloalkyl C₁₋₆ alkyl which is unsubstituted or substituted with 1 to 6 groups selected from Substituent Group A; and
when there are two or more groups selected from Substituent Group A, these groups may be the same as or different from each other,
or a pharmacologically acceptable salt thereof.

### [2-A6]

The compound or a pharmacologically acceptable salt thereof according to any one of the above [1] to [2-A5],
wherein Substituent Group A is a group consisting of a halogen atom, C₁₋₆ alkyl, halo C₁₋₆ alkyl, and C₃₋₈ cycloalkyl.

### [2-B1]

The compound or a pharmacologically acceptable salt thereof according to the above [1],
wherein the compound is represented by Formula (I-B1): 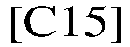 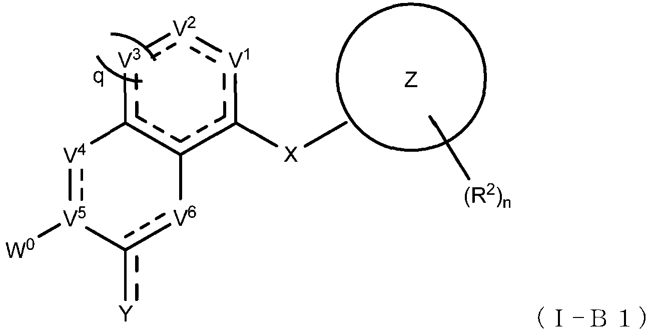 [wherein,
the ring Z, X, V¹, V², V³, V⁴, V⁵, V⁶, R², n, Y, and q have the same meanings as in the above [1]; and
W⁰ is a group represented by the following Formula (w-1): 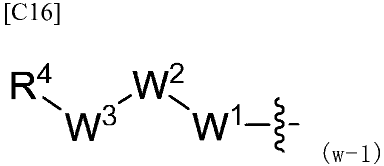 (wherein,
W¹, W², W³, and R⁴ have the same meanings as in the above [1];
provided that, when W¹ is a single bond, any of the following (g) to (i) is satisfied;
(g) when W² is CR^{W1}R^{W2}, R^{W1} and R^{W2} are not both hydrogen atoms, and when W² is NR^{W3}, R^{W3} is not a hydrogen atom
(h) R⁴ is not C₁₋₆ alkyl, halo C₁₋₆ alkyl, NR⁵R⁵', or C₃₋₈ cycloalkyl
(i) W³ is not CO
when W² is a single bond, any of the following (j) to (l) is satisfied;
(j) when W¹ is CR^{W1}R^{W2}, R^{W1} and R^{W2} are not both hydrogen atoms, and when W¹ is NR^{W3}, R^{W3} is not a hydrogen atom
(k) R⁴ is not C₁₋₆ alkyl, halo C₁₋₆ alkyl, NR⁵R^{5'}, or C₃₋₈ cycloalkyl
(l) W³ is not CO);

   [C17] - - -
indicates a single bond or a double bond; and
R^{W1}, R^{W2}, R^{W3}, R⁵, and R^{5'} have the same meanings as in the above [1]]
or a pharmacologically acceptable salt thereof.

### [2-B2]

The compound according to the above [1] or [2-B1],
wherein the compound is represented by Formula (I-B2): 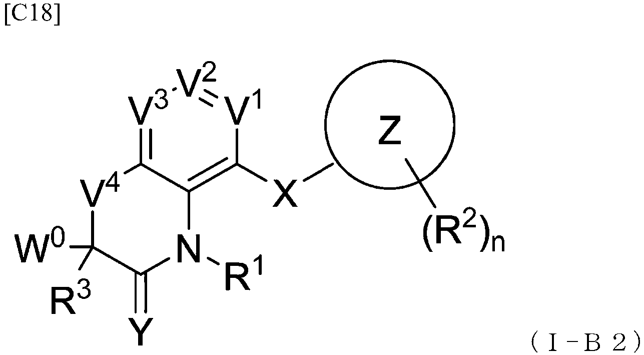 [wherein,
the ring Z, X, R¹, R², R³, and n have the same meanings as in the above [1];
W⁰ has the same meaning as in the above [2-B1];
V¹ is CR^{V1} or N;
V² is CR^{V2} or N;
V³ is CR^{V3} or N;
V⁴ is a single bond, CR^{T1}R^{T2}, O, or NR^{T3};
Y is O, S, or NR^{Y};
R^{V1}, R^{V2}, R^{V3}, R^{T1}, R^{T2}, R^{T3} and R^{Y} have the same meanings as in the above [1]] or a pharmacologically acceptable salt thereof.

### [2-B3]

The compound according to the above [1], [2-B1], or the above [2-B2],
wherein the compound is represented by Formula (I-B3): 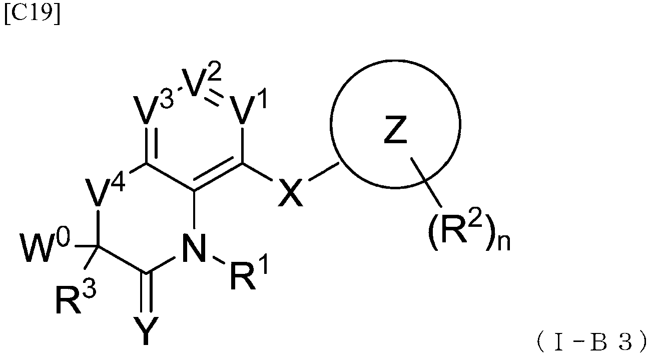 [wherein,
the ring Z, R¹, R², R³, and n have the same meanings as in the above [1];
W⁰ has the same meaning as in the above [2-B1];
V¹, V², V³, and V⁴ have the same meanings as in the above [2-B2];
X is CR^{X0}R^{X0'}, O, NR^{X3}, CO, or -C(=CH₂)-;
Y is O or S; and
R^{X0}, R^{X0'}, and R^{X3} have the same meanings as in the above [1]]
or a pharmacologically acceptable salt thereof.

### [2-B4]

The compound according to any one of the above [2-B1] to [2-B3],
wherein W⁰ is the following group: 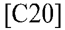 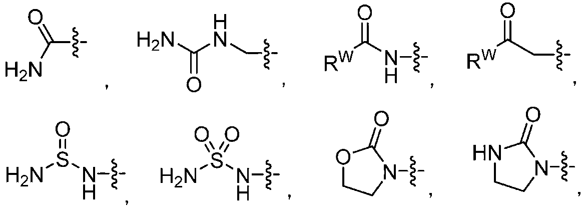 or 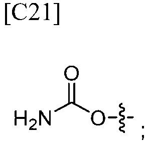 wherein, R^{W} is C₁₋₆ alkoxy, 3- to 8-membered heterocycloalkyl or C₆₋₁₀ aryl, or a pharmacologically acceptable salt thereof.

### [2-C1]

The compound according to the above [1],
wherein the compound is represented by Formula (I-C1): 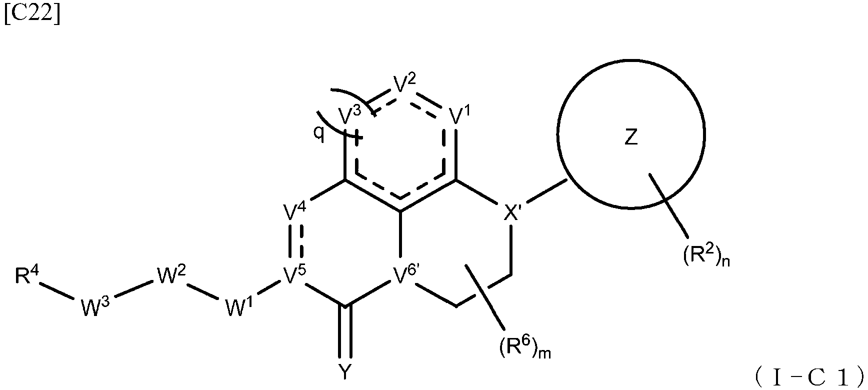 [wherein,
the ring Z, V¹, V², V³, V⁴, V⁵, W¹, W², W³, R², R⁴, n, and q have the same meanings as in the above [1];
X' is CR^{X0} or N;
V^{6'} is CR¹ or N;
Y is O or S;
R⁶ is a halogen atom, hydroxy, C₁₋₆ alkyl, halo C₁₋₆ alkyl or oxo; and
m is an integer of 0 to 4;

   [C23] - - -
indicates a single bond or a double bond; and
R^{X0} and R¹ have the same meanings as in the above [1]]
or a pharmacologically acceptable salt thereof.

### [2-C2]

The compound according to the above [1] or [2-C1],
wherein the compound is represented by Formula (I-C2): 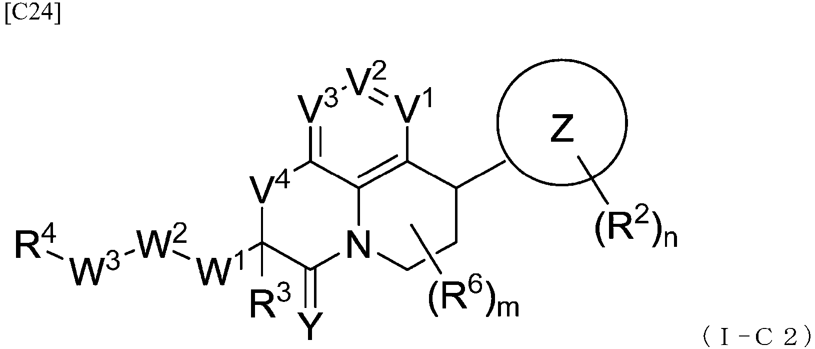 [wherein,
the ring Z, W¹, W², W³, R², R³, R⁴ and n have the same meanings as in the above [1];
V¹ is CR^{V1} or N;
V² is CR^{V2} or N;
V³ is CR^{V3} or N;
V⁴ is a single bond, CR^{T1}R^{T2}, O, or NR^{T3};
R^{V1}, R^{V2}, R^{V3}, R^{T1}, R^{T2}, and R^{T3} have the same meanings as in the above [1]; and
Y, R⁶ and m have the same meanings as in the above [2-C1]]
or a pharmacologically acceptable salt thereof.

### [2-C3]

The compound according to the above [1], [2-C1], or the above [2-C2],
wherein the compound is represented by Formula (I-C3): 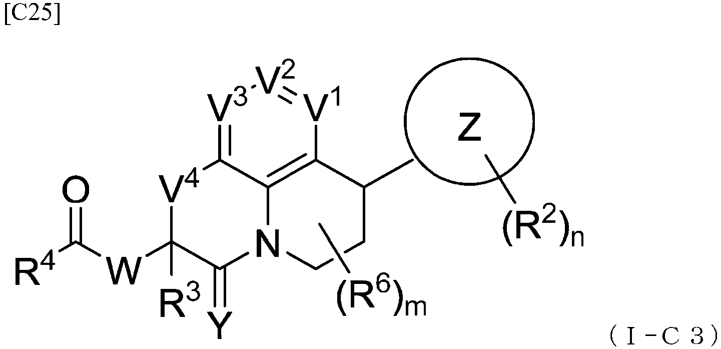 [wherein,
the ring Z, R², R³, R⁴, and n have the same meanings as in the above [1];
Y, R⁶, and m have the same meanings as in the above [2-C1];
V¹, V², V³, and V⁴ have the same meanings as in the above [2-C2]; and
W is CH₂ or NH]
or a pharmacologically acceptable salt thereof.

### [2-C4]

The compound according to the above [1] or any one of the above [2-C1] to [2-C3],
wherein R⁶ is C₁₋₆ alkyl or oxo,
or a pharmacologically acceptable salt thereof.

### [2-D1]

The compound according to the above [1],
wherein the compound is represented by Formula (I-D1): 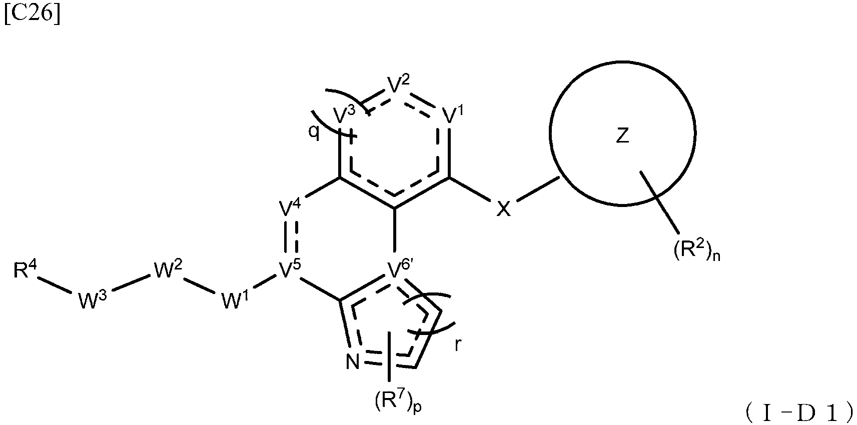 [wherein,
the ring Z, X, V¹, V², V³, V⁴, V⁵, W¹, W², W³, R², R⁴, n and q have the same meanings as in the above [1];
V^{6'} is C, CR¹ or N;
R⁷ is a halogen atom, C₁₋₆ alkyl, or halo C₁₋₆ alkyl;
r is an integer of 1 to 4;
p is an integer of 0 to 3;

   [C27] - - -
indicates a single bond or a double bond;
R¹ has the same meaning as in the above [1]]
or a pharmacologically acceptable salt thereof.

### [2-D2]

The compound according to the above [1] or [2-D1],
wherein the compound is represented by Formula (I-D2): 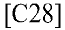 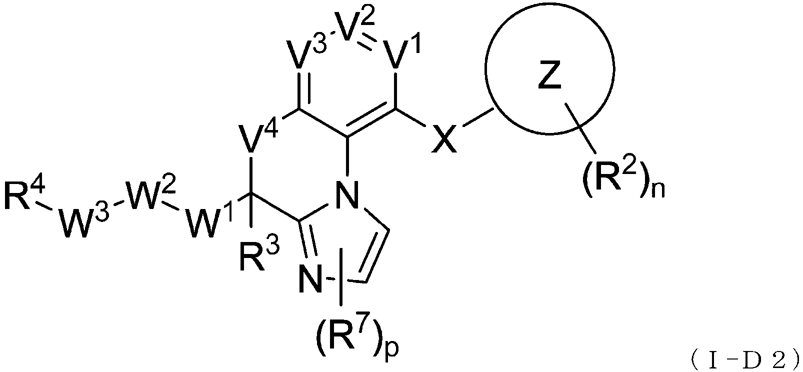 [wherein,
the ring Z, X, W¹, W², W³, R², R³, R⁴, and n have the same meanings as in the above [1];
R⁷ and p have the same meanings as in the above [2-D1];
V¹ is CR^{V1} or N;
V² is CR^{V2} or N;
V³ is CR^{V3} or N;
V⁴ is a single bond, CR^{T1}R^{T2}, O, or NR^{T3};
R^{V1}, R^{V2}, R^{V3}, R^{T1}, R^{T2} and R^{T3} have the same meanings as in the above [1]]
or a pharmacologically acceptable salt thereof.

### [2-D3]

The compound according to the above [1], [2-D1] or [2-D2],
wherein the compound is represented by Formula (I-D3): 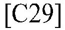 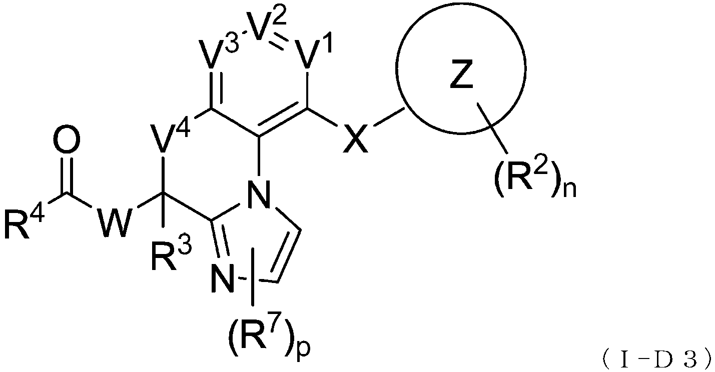 [wherein,
the ring Z, R², R³, R⁴ and n have the same meanings as in the above [1];
R⁷ and p have the same meanings as in the above [2-D1];
V¹, V², V³ and V⁴ have the same meanings as in the above [2-D2];
X is CR^{X0}R^{X0'}, O, NR^{X3}, CO, or -C(=CH₂)-;
W is CH₂ or NH;
R^{X0}, R^{X0'} and R^{X3} have the same meanings as in the above [1]]
or a pharmacologically acceptable salt thereof.

### [2-D4]

The compound according to the above [1] or any one of the above [2-D1] to [2-D3],
wherein R⁷ is C₁₋₆ alkyl,
or a pharmacologically acceptable salt thereof.

### [2-E1]

The compound according to any one of the above [1] to [2-D4],
wherein, when V⁴ is CR^{T1}R^{T2}, R^{T1} and R^{T2} are not both hydrogen atoms,
or a pharmacologically acceptable salt thereof.

### [2-E2]

The compound according to any one of the above [1] to [2-E1],
wherein V⁴ is CR^{T1}R^{T2},
or a pharmacologically acceptable salt thereof.

### [2-E3]

The compound according to any one of the above [1] to [2-E2],
wherein R^{T1} and R^{T2} are each independently a hydrogen atom or hydroxy,
or a pharmacologically acceptable salt thereof.

### [2-F1]

The compound according to any one of the above [1] to [2-E3],
wherein X is CR^{X0}R^{X0'}, NR^{X3}, CO, -C(=CH₂)-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0} and R^{X0'} are each independently a hydrogen atom, a halogen atom, hydroxy, C₁₋₆ alkyl, or halo C₁₋₆ alkyl;
provided that, when X is CHR^{X0}, R^{X0} is neither a hydrogen atom nor C₁₋₆ alkyl,
or a pharmacologically acceptable salt thereof.

### [2-F2]

The compound according to any one of the above [1] to [2-F1],
wherein X is CR^{X0}R^{X0'}, CO, -C(=CH₂)-, or OCR^{X1}R^{X2},
or a pharmacologically acceptable salt thereof.

### [2-F3]

The compound according to any one of the above [1] to [2-F2],
wherein R^{X0} and R^{X0'} are each independently a hydrogen atom, a halogen atom, hydroxy, C₁₋₆ alkyl, or halo C₁₋₆ alkyl,
or a pharmacologically acceptable salt thereof.

### [2-F4]

The compound according to any one of the above [1] to [2-F3],
wherein R^{X1} and R^{X2} are each independently a hydrogen atom, hydroxy, or C₁₋₆ alkyl, or a pharmacologically acceptable salt thereof.

### [2-G]

The compound according to any one of the above [1] to [2-F4],
wherein, when V⁶ is NR¹, R¹ is halo C₁₋₆ alkyl,
or a pharmacologically acceptable salt thereof.

### [2-H1]

The compound according to any one of the above [1] to [2-G],
wherein Y is S or NR^{Y};
R^{Y} is a hydrogen atom or C₁₋₆ alkyl;
R^{Y} may, together with R¹, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
when there are two or more groups selected from Substituent Group B, these groups may be the same as or different from each other,
or a pharmacologically acceptable salt thereof.

### [2-H2]

The compound according to any one of the above [1] to [2-H1],
wherein Y is S,
or a pharmacologically acceptable salt thereof.

[3]
The compound according to any one of the above [1] to [2-H2],
wherein the ring Z is C₆₋₁₀ aryl or 5- or 6-membered heteroaryl,
or a pharmacologically acceptable salt thereof.

[4]
The compound according to any one of the above [1] to [3],
wherein R³ is a hydrogen atom,
or a pharmacologically acceptable salt thereof.

[5]
The compound according to any one of the above [1] to [4],
wherein R² is a halogen atom, cyano, or halo C₁₋₆ alkyl,
or a pharmacologically acceptable salt thereof.

### [6-A1]

The compound according to any one of the above [1] to [5],
wherein R^{V1}, R^{V1'}, R^{V2}, R^{V2'}, R^{V3} and R^{V3'} are each independently a hydrogen atom, a halogen atom, hydroxy, C₁₋₆ alkyl, C₁₋₆ alkoxy, halo C₁₋₆ alkyl, hydroxy C₁₋₆ alkyl, C₆₋₁₀ aryl C₁₋₆ alkyl, or C₆₋₁₀ aryl C₁₋₆ alkoxy,
or a pharmacologically acceptable salt thereof.

### [6-A2]

The compound according to any one of the above [1] to [6-A1],
wherein V¹ is CR^{V1};
V² is CH;
V³ is CH; and
R^{V1} is a hydrogen atom, a halogen atom, C₁₋₆ alkyl, halo C₁₋₆ alkyl, or hydroxy C₁₋₆ alkyl, or a pharmacologically acceptable salt thereof.

### [6-A3]

The compound according to any one of the above [1] to [6-A2],
wherein, when V⁶ is NR¹, R^{V1} is a hydrogen atom or C₁₋₆ alkyl, or a pharmacologically acceptable salt thereof.

### [6-B1]

The compound according to any one of the above [1] to [6-A3],
wherein W¹ is a single bond;
W² is CH₂, NH or a single bond; and
W³ is CO,
or a pharmacologically acceptable salt thereof.

### [6-B2]

The compound according to any one of the above [1] to [6-B1],
wherein R⁴ is C₁₋₆ alkyl, halo C₁₋₆ alkyl, -NR⁵R^{5'}, or C₃₋₈ cycloalkyl, or a pharmacologically acceptable salt thereof.

### [6-B3]

The compound according to any one of the above [1] to [6-B2],
wherein R⁴ is -NH₂,
or a pharmacologically acceptable salt thereof.

### [6-B4]

The compound according to any one of the above [1] to [6-B3],
wherein W² is NH,
or a pharmacologically acceptable salt thereof.

### [6-C]

The compound according to any one of the above [1] to [6-B4],
wherein V⁴ is CH₂,
or a pharmacologically acceptable salt thereof.

### [6-D1]

The compound according to any one of the above [1] to [6-C],
wherein X is O or CHR^{X0},
or a pharmacologically acceptable salt thereof.

### [6-D2]

The compound according to any one of the above [1] to [6-D1],
wherein X is O,
or a pharmacologically acceptable salt thereof.

### [6-E]

The compound according to any one of the above [1] to [6-D2],
wherein R¹ is C₁₋₆ alkyl,
or a pharmacologically acceptable salt thereof.

### [6-F]

The compound according to any one of the above [1] to [6-E],
wherein Y is O,
or a pharmacologically acceptable salt thereof.

A pharmaceutical composition comprising the compound or a pharmacologically acceptable salt thereof according to any one of the above [1] to [6-F] and a pharmaceutical additive.

[8] The pharmaceutical composition according to the above [7], which is a pharmaceutical composition for treating thyroid-related diseases.

[9] The pharmaceutical composition according to the above [8], wherein the thyroid-related diseases are hyperthyroidism, Graves' disease or thyroid eye disease.

As one embodiment, the present invention relates to a method of treating thyroid-related diseases, including administering a required amount of the pharmaceutical composition according to the above [7] to a patient.

As one embodiment, the present invention relates to a use of the compound or a pharmacologically acceptable salt thereof according to any one of the above [1] to [6-F], for manufacturing a pharmaceutical composition for treating thyroid-related diseases.

### [Advantageous Effects of Invention]

The compound of the present invention has excellent TSHR antagonist activity. Therefore, the compound of the present invention or a pharmacologically acceptable salt thereof is useful as a therapeutic agent for thyroid-related diseases.

### [Description of Embodiments]

Hereinafter, embodiments of the present invention will be described in more detail.

In the present invention, unless otherwise specified, respective terms have the following meanings.

"Halogen atom" refers to a fluorine atom, a chlorine atom, a bromine atom, or an iodine atom.
"C₁₋₆ alkyl" refers to a linear or branched alkyl group having 1 to 6 carbon atoms. Examples thereof include methyl, ethyl, propyl, isopropyl, butyl, isobutyl, sec-butyl, and tert-butyl.
"C₁₋₆ alkylene" refers to a linear or branched alkylene group having 1 to 6 carbon atoms. Examples thereof include methylene, ethylene, and 1-propylene.
"C₂₋₆ alkenyl" refers to an alkenyl group having 2 to 6 carbon atoms and having at least one double bond. Examples thereof include ethenyl, 1-propenyl, and 2-propenyl.
"C₂₋₆ alkynyl" refers to an alkynyl group having 2 to 6 carbon atoms and having at least one triple bond. Examples thereof include ethynyl, 1-propynyl, 2-propynyl, and 1-butynyl.
"C₁₋₆ alkoxy" refers to a linear or branched alkoxy group having 1 to 6 carbon atoms. Examples thereof include methoxy, ethoxy, propoxy, and isopropoxy.
"Hydroxy C₁₋₆ alkyl" refers to C₁₋₆ alkyl substituted with one or two hydroxy groups. Examples thereof include hydroxymethyl, 2-hydroxyethyl, 3-hydroxypropyl, and 2-hydroxypropan-2-yl.
"Halo C₁₋₆ alkyl" refers to C₁₋₆ alkyl substituted with 1 to 5 identical or different halogen atoms. Examples thereof include monofluoromethyl, 2-fluoroethyl, difluoromethyl, trifluoromethyl, 1,1-difluoroethyl, 2,2,2-trifluoroethyl, 3,3,3-trifluoropropyl, 4,4,4-trifluorobutyl, and pentafluoroethyl.
"Halo C₁₋₆ alkoxy" refers to C₁₋₆ alkoxy substituted with 1 to 5 identical or different halogen atoms. Examples thereof include monofluoromethoxy, difluoromethoxy, trifluoromethoxy, 1,1,2,2-tetrafluoroethoxy, and pentafluoroethoxy.
"C₁₋₆ alkoxy C₁₋₆ alkyl" refers to C₁₋₆ alkyl substituted with one C₁₋₆ alkoxy. Examples thereof include methoxymethyl, methoxyethyl, ethoxymethyl, and ethoxyethyl.
"Carboxy C₁₋₆ alkyl" refers to C₁₋₆ alkyl substituted with one carboxy. Examples thereof include carboxymethyl and carboxyethyl.
"Halohydroxy C₁₋₆ alkyl" refers to C₁₋₆ alkyl substituted with 1 to 6 identical or different halogen atoms and one or two hydroxy groups. Examples thereof include 1,1,1,3,3,3-hexafluoro-2-hydroxypropan-2-yl.
"C₆₋₁₀ aryl" refers to a monocyclic or polycyclic aromatic hydrocarbon group having 6 to 10 carbon atoms, and a part of the ring may be partially saturated. Examples thereof include phenyl, naphthyl, and indanyl.
"5- or 6-membered heteroaryl" refers to a 5- or 6-membered aromatic heterocyclic group including 1 to 4 heteroatoms selected from among an oxygen atom, a nitrogen atom and a sulfur atom in the ring. Examples thereof include pyridyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, and 1,2,4-oxadiazolyl.
"5- to 10-membered heteroaryl" refers to a 5- to 10-membered aromatic heterocyclic group including 1 to 4 heteroatoms selected from among an oxygen atom, a nitrogen atom and a sulfur atom in the ring, and a part of the ring may be partially saturated. Examples thereof include pyridyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, 1,2,4-oxadiazolyl, 2,3-dihydrobenzofuranyl, 1,2,3,4-tetrahydroquinolyl, and 1,2,3,4-tetrahydroisoquinolyl.
"C₃₋₈ cycloalkyl" refers to a 3- to 8-membered saturated hydrocarbon group, and includes those having a partially crosslinked structure. Examples thereof include cyclopropyl, cyclobutyl, cyclopentyl, cyclohexyl, bicyclo[1.1.1]pentyl, and bicyclo[2.2.2]octyl.
"3- to 8-membered heterocycloalkyl" refers to a 3- to 8-membered cycloalkyl group in which carbon atoms in the ring are substituted with one or two heteroatoms selected from among an oxygen atom, a nitrogen atom and a sulfur atom, and includes those having a partially crosslinked structure. Examples thereof include aziridinyl, azetidinyl, pyrrolidinyl, piperidinyl, piperazinyl, morpholinyl, thiomorpholinyl, azepanyl, azabicyclo[2.2.2]octyl, 2-oxa-5-azabicyclo[2.2.1]heptanyl, 6-oxa-3-azabicyclo[2.2.1]heptanyl, 8-oxa-3-azabicyclo[3.2.1]octanyl, 3-oxa-8-azabicyclo[3.2.1]octanyl, 2-azaspiro[3.3]heptanyl, 2-oxa-6-azaspiro[3.3]heptanyl, pyrrolidonyl, piperidonyl, oxiranyl, oxetanyl, tetrahydrofuranyl, tetrahydropyranyl, and octahydrocyclopenta[c]pyrrolyl.
"3- to 10-membered saturated carbocyclic ring" refers to a cyclic saturated hydrocarbon that is monocyclic or polycyclic and has 3 to 10 carbon atoms.
"3- to 8-membered saturated carbocyclic ring" refers to a cyclic saturated hydrocarbon that is monocyclic or polycyclic and has 3 to 8 carbon atoms.
"5- to 8-membered saturated carbocyclic ring" refers to a cyclic saturated hydrocarbon that is monocyclic or polycyclic and has 5 to 8 carbon atoms.
"3- to 10-membered unsaturated carbocyclic ring" refers to a cyclic hydrocarbon that is monocyclic or polycyclic, is completely unsaturated or partially unsaturated, and has 3 to 10 carbon atoms.
"5- to 8-membered unsaturated carbocyclic ring" refers to a cyclic hydrocarbon that is monocyclic or polycyclic ring, is completely unsaturated or partially unsaturated, and has 5 to 8 carbon atoms.
"3- to 10-membered saturated heterocyclic ring" refers to a 3- to 10-membered saturated heterocyclic ring that contains 1 to 5 heteroatoms selected from among a nitrogen atom, an oxygen atom and a sulfur atom in the ring, and is monocyclic or polycyclic.
"3- to 8-membered saturated heterocyclic ring" refers to a 3- to 8-membered saturated heterocyclic ring that contains 1 to 3 heteroatoms selected from among a nitrogen atom, an oxygen atom and a sulfur atom in the ring and is monocyclic or polycyclic.
"5- to 8-membered saturated heterocyclic ring" refers to a 5- to 8-membered saturated heterocyclic ring that contains 1 to 3 heteroatoms selected from among a nitrogen atom, an oxygen atom and a sulfur atom in the ring and is monocyclic or polycyclic.
"3- to 10-membered unsaturated heterocyclic ring" refers to a 3- to 10-membered heterocyclic ring that contains 1 to 5 heteroatoms selected from among a nitrogen atom, an oxygen atom and a sulfur atom in the ring, is monocyclic or polycyclic ring, and is completely unsaturated or partially unsaturated.
"5- to 8-membered unsaturated heterocyclic ring" refers to a 5- to 8-membered heterocyclic ring that contains 1 to 3 heteroatoms selected from among a nitrogen atom, an oxygen atom and a sulfur atom in the ring, is monocyclic or polycyclic, and is completely unsaturated or partially unsaturated.
"3- to 10-membered ring" refers to a 3- to 10-membered saturated carbocyclic ring, a 3- to 10-membered saturated heterocyclic ring, a 3- to 10-membered unsaturated carbocyclic ring, or a 3- to 10-membered unsaturated heterocyclic ring.
"5- to 8-membered ring" refers to a 5- to 8-membered saturated carbocyclic ring, a 5-to 8-membered saturated heterocyclic ring, a 5- to 8-membered unsaturated carbocyclic ring, or a 5- to 8-membered unsaturated heterocyclic ring.
"C₆₋₁₀ aryloxy" refers to a group represented by (C₆₋₁₀ aryl)-O-. Examples thereof include phenoxy.
"C₆₋₁₀ aryl C₁₋₆ alkoxy" refers to C₁₋₆ alkoxy substituted with one C₆₋₁₀ aryl. Examples thereof include benzyloxy.
"C₆₋₁₀ aryl C₁₋₆ alkyl" refers to C₁₋₆ alkyl substituted with one C₆₋₁₀ aryl. Examples thereof include benzyl.
"C₆₋₁₀ aryl C₂₋₆ alkynyl" refers to C₂₋₆ alkynyl substituted with one C₆₋₁₀ aryl. Examples thereof include phenylethynyl.
"5- or 6-membered heteroaryl C₁₋₆ alkyl" refers to C₁₋₆ alkyl substituted with one 5- or 6-membered heteroaryl.
"5- or 6-membered heteroaryl C₂₋₆ alkynyl" refers to C₂₋₆ alkynyl substituted with one 5- or 6-membered heteroaryl. Examples thereof include pyridin-4-ylethynyl and pyridin-3-ylethynyl.
"5- to 10-membered heteroaryl C₁₋₆ alkyl" refers to C₁₋₆ alkyl substituted with one 5-to 10-membered heteroaryl.
"C₃₋₈ cycloalkyl C₁₋₆ alkyl" refers to C₁₋₆ alkyl substituted with one C₃₋₈ cycloalkyl. Examples thereof include cyclopropylethyl.
"C₃₋₈ cycloalkyl C₂₋₆ alkynyl" refers to C₂₋₆ alkynyl substituted with one C₃₋₈ cycloalkyl. Examples thereof include cyclopropylethynyl.
"3- to 8-membered heterocycloalkyl C₁₋₆ alkyl" refers to C₁₋₆ alkyl substituted with one 3- to 8-membered heterocycloalkyl. Examples thereof include morpholinoethyl.

The following abbreviations in the text, drawings and tables have the following meanings.
BF₃-Et₂O: boron trifluoride-diethyl ether complex
BH₃-THF: borane-tetrahydrofuran complex
Boc: tert-butoxycarbonyl
Boc₂O: di-tert-butyl dicarbonate
Cbz: benzyloxycarbonyl
CDI: carbonyldiimidazole
DBU: diazabicycloundecene
DCE: 1,2-dichloroethane
DCM: dichloromethane
DIBAL-H: diisobutylaluminum hydride
DIPEA: N,N-diisopropylethylamine
DMAP: 4-dimethylaminopyridine
DMF: N,N-dimethylformamide
DMP: Dess-Martin periodinane
DMT-MM: 4-(4,6-dimethoxy-1,3,5-triazin-2-yl)-4-methylmorpholinium chloride
DPPA: diphenylphosphoryl azide
Et₂O: diethyl ether
HATU: O-(7-azabenzotriazol-1-yl)-N,N,N',N'-tetramethyluronium hexafluorophosphate
LDA: lithium diisopropylamide
MeCN: acetonitrile
MTBE: methyl tert-butyl ether
NBS: N-bromosuccinimide
NCS: N-chlorosuccinimide
NMP: N-methylpyrrolidone
TBAF: tetrabutylammonium fluoride
TBS: tert-butyldimethylsilyl
TBSCl: tert-butyldimethylsilyl chloride
TEA: triethylamine
TFA: trifluoroacetic acid
THF: tetrahydrofuran
TMS: trimethylsilyl
TMSCl: trimethylsilyl chloride
T3P: propylphosphonic anhydride (cyclic trimer)
Xantphos: 4,5-bis(diphenylphosphino)-9,9-dimethylxanthene
Xphos: 2-dicyclohexylphosphino-2',4',6'-triisopropylbiphenyl
10% Pd/C: 10% palladium on carbon (wetted with about 55% water)
Lawesson's reagent: 2,4-bis(4-methoxyphenyl)-1,3-dithia-2,4-diphosphetane 2,4-disulfide
APS: aminopropylated silica gel
Method A: column chromatography in which a silica gel column is connected to the bottom of an aminopropylated silica gel column
Method B: column chromatography in which a dihydroxypropylated silica gel column is connected to the bottom of a silica gel column
ODS: octadecylsilylated silica gel
PLC: preparative thin-layer chromatography
Ref. No.: Reference Example Number
Str.: structural formula
Ex. No.: Example Number
Phys. data: physical property value
IC₅₀: 50% inhibitory concentration
¹H-NMR: proton nuclear magnetic resonance spectrum
DMSO: dimethyl sulfoxide
DMSO-d6: dimethyl sulfoxide-d6
CDCl₃: chloroform-d1
MS: mass spectrometry
(The measured values in the table were measured by an electrospray ionization method or a multi-ionization method including an electrospray ionization method and an atmospheric pressure chemical ionization method.)
cAMP: adenosine 3',5'-cyclic monophosphate
CHO: Chinese hamster ovary
FBS: fetal bovine serum
HEPES: 2-(4-(2-hydroxyethyl)-1-piperazinyl)ethanesulfonic acid
IBMX: 3-isobutyl-1-methylxanthine

In the compounds represented by Formulae (A-I) to (AIII) or Formulae (I) to (I-D3), when there are one or more asymmetric carbon atoms, the present invention also includes a compound in which respective asymmetric carbon atoms are in the R configuration, a compound in which respective asymmetric carbon atoms are in the S configuration, and any combination of these compounds. In addition, the scope of the present invention also includes racemic compounds, racemic mixtures, single enantiomers and diastereomeric mixtures thereof.

In the compounds represented by Formulae (A-I) to (AIII) or Formulae (I) to (I-D3), when there are cis-trans isomers, the present invention includes all of these cis-trans isomers.

In the compounds represented by Formulae (A-I) to (AIII) or Formulae (I) to (I-D3), when there are tautomers, the present invention includes all of these tautomers.

In the present invention, the determination of stereochemistry can be performed by a method known in the art.

As necessary, the compounds represented by Formulae (A-I) to (AIII) or Formulae (I) to (I-D3) can be converted into a pharmacologically acceptable salt thereof according to a conventional method. Examples of such salts include acid addition salts and salts with bases.

Examples of acid addition salts include acid addition salts with mineral acids such as hydrochloric acid, hydrobromic acid, hydroiodic acid, sulfuric acid, nitric acid, and phosphoric acid, and acid addition salts with organic acids such as formic acid, acetic acid, trifluoroacetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, benzoic acid, glutamic acid, and aspartic acid.

Examples of salts with bases include salts with inorganic bases such as lithium salts, sodium salts, potassium salts, calcium salts, and magnesium salts, and salts with organic bases such as N-methyl-D-glucamine, N,N'-dibenzylethylenediamine, TEA, piperidine, morpholine, pyrrolidine, arginine, lysine, and choline.

Unless otherwise specified, for example, suffixes "hydrochloride," or "HCl" to chemical names or structural formulas for salts are not stoichiometric descriptions, but simply refer to salt forms.

When the compounds represented by Formulae (A-I) to (AIII) or Formulae (I) to (I-D3) or pharmacologically acceptable salts thereof are present as, for example, a crystal, the present invention also includes any crystal form. For example, pharmacologically acceptable salts also include solvates with pharmaceutically acceptable solvents such as water or ethanol, and cocrystals with appropriate cocrystal formers (Coformer).

In the compounds represented by Formulae (A-I) to (AIII) or Formulae (I) to (I-D3), some atoms may be substituted with corresponding isotopes. The present invention also includes compounds substituted with these isotopes. Examples of isotopes include isotopes of a hydrogen atom, a carbon atom, a chlorine atom, a fluorine atom, an iodine atom, a nitrogen atom, an oxygen atom, and a sulfur atom represented by ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, and ³⁵S. As one embodiment, compounds in which some hydrogen atoms of compounds represented by Formulae (A-I) to (AIII) or Formulae (I) to (I-D3) are substituted with ²H (D: deuterium atom) may be exemplified.

In the compounds represented by Formulae (A-I) to (AIII) or Formulae (I) to (I-D3), compounds in which some atoms are substituted with isotopes can be manufactured by the same method as a manufacturing method to be described below using commercially available building blocks into which isotopes are introduced. In addition, methods described in the literature (for example, refer to Journal of Synthetic Organic Synthesis, Vol. 65, No. 12, pp. 1179-1190, 2007 and refer to RADIOISOTOPES, Vol. 56, No. 11, pp. 741-750, 2007) can be used for manufacturing.

The compounds represented by Formulae (A-I) to (AIII) or Formulae (I) to (I-D3) of the present invention can be manufactured, for example, by methods shown in Schemes 1 to 30 or methods similar thereto, or by methods described in the literature or methods similar thereto. In the Schemes, the compounds represented by Formulae (A-I) to (AIII) or Formulae (I) to (I-D3) correspond to compounds represented by Formulae (I-1) to (I-25).

The compounds represented by Formulae (A-I) to (AIII) or Formulae (I) to (I-D3) of the present invention can be manufactured by the following methods, but the following manufacturing methods are simply general manufacturing methods, and are not intended to limit the manufacturing methods.

In the reaction of each step, when raw materials and reagents are commercially available, commercially available products can be used.

In the reaction of each step, the reaction time varies depending on the raw materials used, solvent, reaction temperature and the like, but is generally 30 minutes to 3 days unless otherwise specified.

In the reaction of each step, the reaction temperature varies depending on the raw materials used, solvent and the like, but is generally -78°C to a reflux temperature unless otherwise specified.

In the reaction of each step, the pressure varies depending on the raw materials used, solvent, reaction temperature and the like, but is generally 1 to 20 atm unless otherwise specified.

In the reaction of each step, a microwave reaction device such as an Initiator (commercially available from Biotage) can be used. When the reaction is performed using a microwave reaction device, the reaction can be performed under varying conditions depending on the raw materials used, solvent, and model, pressure range: 1 to 30 bar, power range: 1 to 400 W, reaction temperature: room temperature to 300°C, and reaction time: 1 minute to 1 day.

Unless otherwise specified, the reaction in each step is performed without a solvent or using an appropriate solvent. Examples of appropriate solvents include solvents inert to the reaction. Specific examples of solvents used include solvents described in reference examples and examples corresponding to the steps and the following solvents. Two or more of the following solvents may be used in combination at an appropriate ratio. Alcohols: methanol, ethanol, tert-butyl alcohol, 2-propanol, etc.;
ethers: diethyl ether, THF, 1,2-dimethoxyethane, 1,4-dioxane, cyclopentyl methyl ether, MTBE, etc.;
aromatic hydrocarbons: benzene, chlorobenzene, 1,2-dichlorobenzene, toluene, xylene, etc.; saturated hydrocarbons: cyclohexane, n-hexane, n-pentane, etc.;
amides: DMF, N,N-dimethylacetamide, NMP, etc.;
halogenated hydrocarbons : DCM, DCE, carbon tetrachloride, etc.;
nitriles: MeCN, etc.;
sulfoxides: DMSO, etc.;
aromatic organic bases: pyridine, etc.;
acid anhydrides: acetic anhydride, etc.;
organic acids: formic acid, acetic acid, TFA, methanesulfonic acid, etc.;
esters: ethyl acetate, methyl acetate, isopropyl acetate, etc.;
ketones: acetone, methyl ethyl ketone, etc.; and
water.

In the reaction of each step, when a base is used, the reaction is performed using a base suitable for the reaction. Specific examples of bases used include bases described in reference examples and examples corresponding to the steps and the following bases. inorganic bases: sodium hydroxide, lithium hydroxide, potassium hydroxide, etc.; basic salts: sodium carbonate, sodium bicarbonate, potassium carbonate, cesium carbonate, etc.; organic bases: TEA, DIPEA, diethylamine, pyridine, DMAP, 2,6-lutidine, DBU, imidazole, piperidine, etc.;
metal alkoxides: sodium ethoxide, sodium methoxide, potassium tert-butoxide, etc.;
alkali metal hydrides: sodium hydride, etc.;
metal amides: sodium amide, LDA, lithium hexamethyldisilazide, sodium
hexamethyldisilazide, potassium hexamethyldisilazide, etc.;
organomagnesiums: isopropylmagnesium chloride, etc.;
organolithiums: methyllithium, n-butyllithium, sec-butyllithium, tert-butyllithium, etc.

In the reaction of each step, when an acid is used, the reaction is performed using an acid suitable for the reaction. Specific examples of acids used include acids described in reference examples and examples corresponding to the steps and the following acids. inorganic acids: hydrochloric acid, sulfuric acid, nitric acid, hydrobromic acid, phosphoric acid, etc.;
organic acids: acetic acid, TFA, citric acid, methanesulfonic acid, p-toluenesulfonic acid, 10-camphorsulfonic acid, trifluoromethanesulfonic acid, etc.;
Lewis acids: BF₃-Et₂O, zinc iodide, aluminum chloride, zinc chloride, titanium(IV) chloride, bismuth(III) trifluoromethanesulfonate, etc.

In the reaction of each step, when a condensing agent is used, the reaction is performed using a condensing agent suitable for the reaction. Specific examples of condensing agents used include condensing agents described in reference examples and examples corresponding to the steps and the following condensing agents.
carbodiimides: 1-ethyl-3-(3-dimethylaminopropyl)carbodiimide hydrochloride, N,N'-dicyclohexylcarbodiimide, etc.;
imidazoles: CDI, etc.;
uronium salts, phosphonium salts: HATU,
1H-benzotriazol-1-yloxytris(dimethylamino)phosphonium hexafluorophosphate, etc.; triazines: DMT-MM, etc.;
others: T3P, etc.

In the reaction of each step, when a reducing agent is used, the reaction is performed using a reducing agent suitable for the reaction. Specific examples of reducing agents used include reducing agents described in reference examples and examples corresponding to the steps and the following reducing agents.
metal hydrides: lithium aluminum hydride, lithium borohydride, sodium borohydride, sodium triacetoxyborohydride, sodium cyanoborohydride, DIBAL-H, etc.;
boranes: BH₃-THF, picoline-borane complex, decaborane, etc.

In the reaction of each step, when an oxidizing agent is used, the reaction is performed using an oxidizing agent suitable for the reaction. Specific examples of oxidizing agents used include oxidizing agents described in reference examples and examples corresponding to the steps and the following oxidizing agents.
peracids: m-chloroperbenzoic acid, hydrogen peroxide, tert-butyl hydroperoxide, etc.;
chlorates: sodium hypochlorite, sodium chlorite, etc.;
iodates: sodium periodate, etc.;
hypervalent iodine reagents: iodobenzenediacetate, DMP, etc.;
chromium-containing reagents: pyridinium dichromate, Jones reagent, etc.;
manganese-containing reagents: manganese dioxide, potassium permanganate, etc.

In the reaction of each step, when a carbonyl group introduction reagent is used, the reaction is performed using a carbonyl group introduction reagent suitable for the reaction. Specific examples of carbonyl group introduction reagents used include carbonyl group introduction reagents described in reference examples and examples corresponding to the steps and the following carbonyl group introduction reagents.
phosgenes: phosgene, diphosgene, triphosgene, etc.;
chloroformate esters: 4-nitrophenyl chloroformate, etc.;
imidazoles: CDI, etc.

In each step, when a protecting group is required according to the type of functional groups, introduction and removal operations can be performed in an appropriate combination according to a conventional method. Regarding the type of protecting groups, protection, and deprotection, for example, the methods described in "Greene's Protective Groups in Organic Synthesis," fifth edition, Wiley-Interscience, 2014 edited by Peter G. M. Wuts may be referred to.

In each step, when hydrolysis is performed, the reaction can be performed in the presence of an acid or a base. Examples of acids and bases used include those exemplified above.

In each step, when a catalytic reduction is performed, the reaction can be performed in the presence of hydrogen and a catalyst. Examples of catalysts used include palladium on carbon powder, a Perlman catalyst, platinum carbon powder, Raney nickel, and (1,5-cyclooctadiene) (pyridine) (tricyclohexylphosphine)iridium(I) hexafluorophosphate. Here, as necessary, an acid may be used in the reaction.

In each step, when reduction is performed, the reaction can be performed in the presence of a reducing agent. Examples of reducing agents used include those exemplified above.

In each step, when a metal reduction is performed, the reaction can be performed in the presence of a metal or the like. Examples of metals used or the like include iron powder, zinc powder, tin chloride, and titanium trichloride. Here, as necessary, an acid may be used in the reaction.

In each step, when oxidation is performed, the reaction can be performed in the presence of an oxidizing agent. Examples of oxidizing agents used include those exemplified above. When a nitroxy radical oxidation catalyst is used, the reaction can be performed in the presence of a re-oxidizing agent. Examples of nitroxy radical oxidation catalysts used include 2,2,6,6-tetramethylpiperidine 1-oxyl and 2-azaadamantane-N-oxyl. Examples of re-oxidizing agents used include sodium hypochlorite and iodobenzenediacetate. Here, as necessary, the reaction may be performed by adding an additive such as tetrabutylammonium bisulfate or potassium bromide.

In each step, when amidation is performed, the reaction can be performed using a condensing agent in the presence or absence of a base. Examples of condensing agents and bases used include those exemplified above. Here, when a carbodiimide is used as the condensing agent, the reaction may be performed by adding additives such as 1-hydroxybenzotriazole and DMAP as necessary. In addition, the reaction can be performed using an acyl halide or an acid anhydride in the presence or absence of a base.

In each step, when an aromatic nucleophilic substitution reaction is performed, the reaction can be performed in the presence of a base. Examples of bases include those exemplified above.

In each step, when a Negishi coupling reaction is performed, the reaction can be performed in the presence of an organozinc compound, a palladium catalyst and a ligand. Examples of palladium catalysts used include palladium(II) acetate and tris(dibenzylideneacetone)palladium(0). Examples of ligands include Xphos, 2-dicyclohexylphosphino-2',6'-dimethoxybiphenyl, and tris(2-methylphenyl)phosphine.

In each step, when a Suzuki-Miyaura cross-coupling reaction is performed, the reaction can be performed in the presence of a palladium catalyst and a base. Examples of palladium catalysts used include bis(di-tert-butyl(4-dimethylaminophenyl)phosphine)dichloropalladium(II), bis(triphenylphosphine)palladium(II) chloride, and tetrakis(triphenylphosphine)palladium(0), (1,1'-bis(diphenylphosphino)ferrocene)palladium(II) dichloride dichloromethane adducts. Examples of bases used include those exemplified above.

In each step, when a Mizoroki-Heck cross-coupling reaction is performed, the reaction can be performed in the presence of a palladium catalyst, a ligand and a base. Examples of palladium catalysts used include palladium(II) acetate. Examples of ligands used include tris(2-methylphenyl)phosphine. Examples of bases used include those exemplified above.

In each step, when a Buchwald-Hartwig cross coupling reaction is performed, the reaction can be performed in the presence of a palladium catalyst, a ligand and a base. Examples of palladium catalysts used include tris(dibenzylideneacetone)dipalladium(0). Examples of ligands used include Xantphos. Examples of bases used include those exemplified above.

In each step, when bromination is performed, the reaction can be performed in the presence of a bromoating reagent. Examples of bromoating reagents used include NBS.

In each step, when chlorination is performed, the reaction can be performed in the presence of a chlorinating agent. Examples of chlorinating agents used include NCS.

In each step, when a Wittig reaction is performed, the reaction can be performed in the presence of a base. Examples of bases used include those exemplified above.

In each step, when a Horner-Wadsworth-Emmons reaction is performed, the reaction can be performed in the presence of a base. Examples of bases used include those exemplified above.

In each step, when Brown hydroboration is performed, the reaction can be performed in the presence of a boronating agent. Examples of boronating agents used include BH₃-THF.

In each step, when a Curtius rearrangement reaction is performed, the reaction can be performed in the presence of an azide source. Examples of azide sources used include sodium azide and DPPA.

In each step, when carbamate formation or urea formation is performed, the reaction can be performed using a carbonyl group introduction reagent in the presence or absence of a base. Examples of carbonyl group introduction reagents and bases used include those exemplified above.

In each step, when asymmetric alkylation is performed, the reaction can be performed in the presence of a base and an optically active phase-transfer catalyst. Examples of bases used include those exemplified above. Examples of optically active phase-transfer catalysts used include
(S)-4,4-dibutyl-2,6-bis(3,4,5-trifluorophenyl)-4,5-dihydro-3H-dinaphtho[2,1-c:1',2'-e]azepini um bromide,
(11bS,11'bS)-2,6-bis(3,4,5-trifluorophenyl)-3,3',5,5'-tetrahydro-4,4'-spirobi[dinaphtho[2,1-c :1',2'-e]azepinium]-4-ium bromide,
6,10-dibenzyl-N,N'-dimethyl-N,N,N',N'-tetrakis(4-methylbenzyl)-1,4-dioxaspiro[4.5]decane -(2S,3S)diylbis(methylammonium) bis(tetrafluoroborate) salt, N-benzylcinchoninium chloride, N-benzylcinchoninium bromide, N-anthranylcinchoninium chloride, and N-anthranylcinchoninium bromide.

In each step, when oxidative cleavage is performed, the reaction can be performed in the presence of an oxidative cleavage agent. Examples of oxidative cleavage agents used include a combination of osmium(VIII) tetroxide and sodium periodate.

The compound represented by Formula (I-1) can be manufactured, for example, according to the method described in Scheme 1. 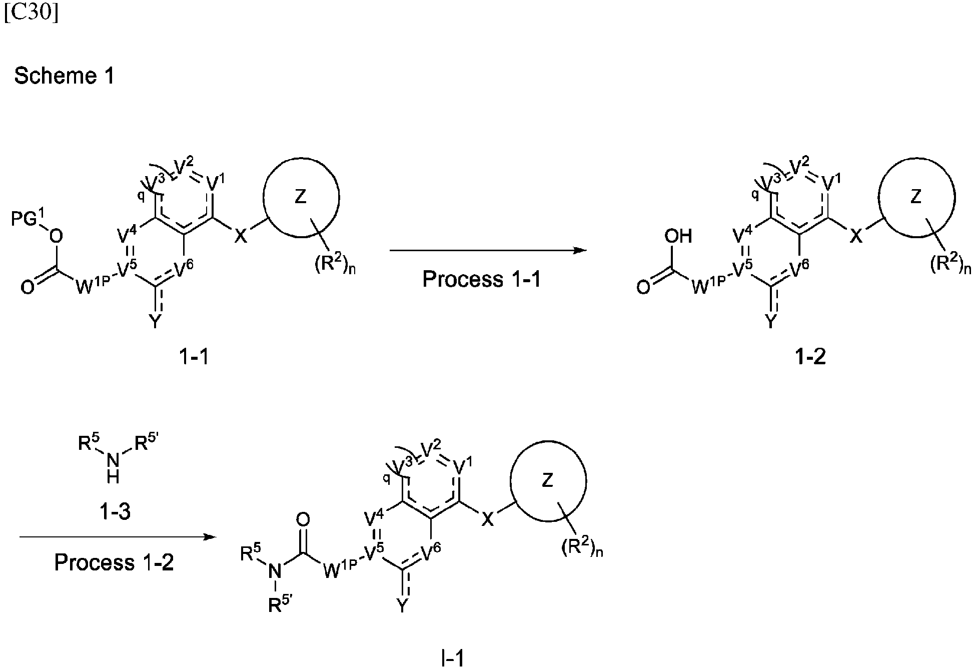 The symbols in the formulae have the same meanings as above. PG¹ is a protecting group, and W^{1P} is CR^{W1}R^{W2} or a single bond.

### Process 1-1

Compound (1-2) can also be manufactured by removing the protecting group of Compound (1-1).

### Process 1-2

Compound (I-1) can also be manufactured by amidation of Compound (1-2) and Compound (1-3).

The compound represented by Formula (I-2) can be manufactured, for example, according to the method described in Scheme 2. 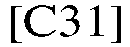 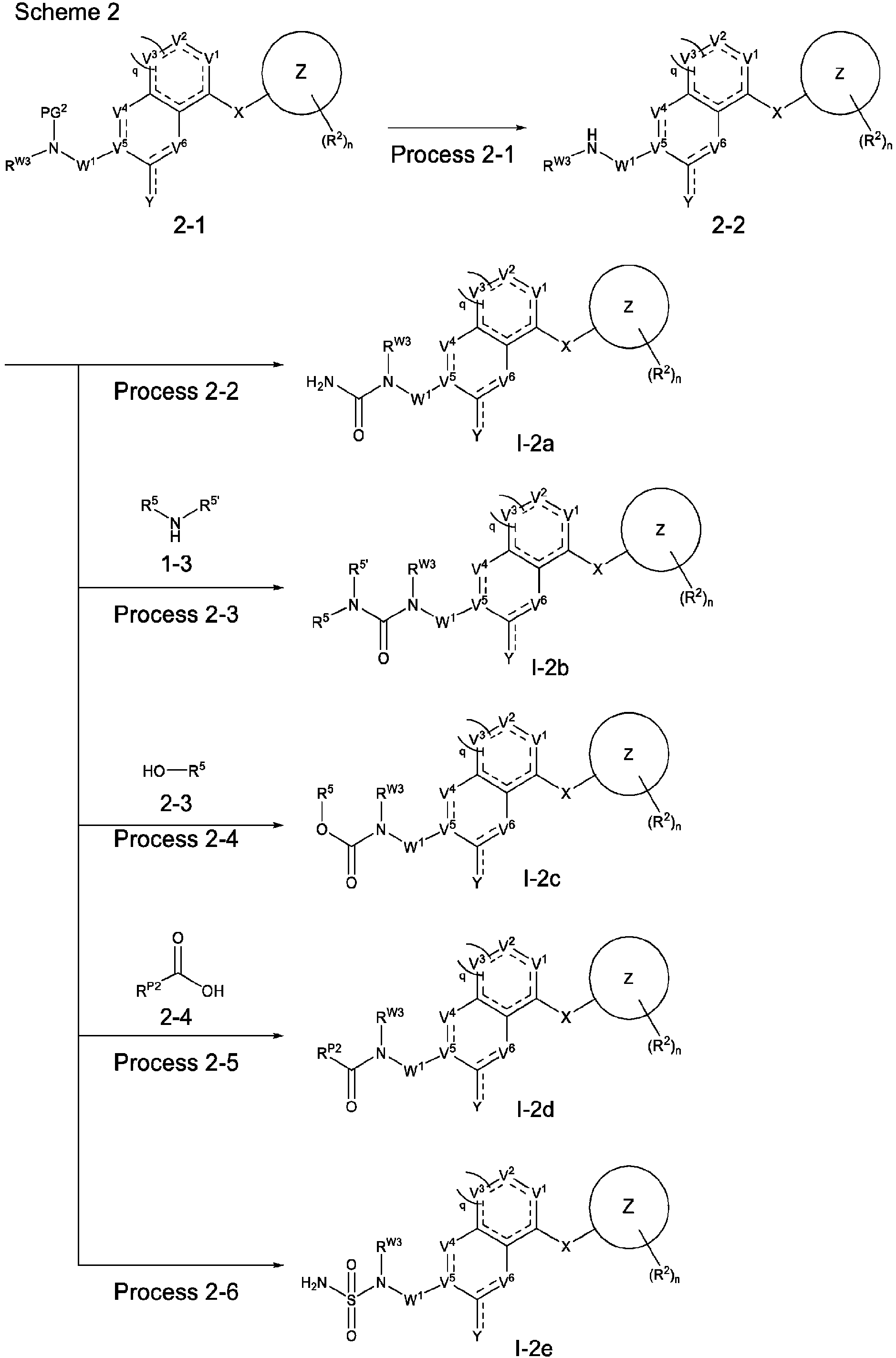 The symbols in the formulae have the same meanings as above. PG² is a protecting group, R^{P2} is C₁₋₆ alkyl, halo C₁₋₆ alkyl, C₁₋₆ alkoxy C₁₋₆ alkyl, C₃₋₈ cycloalkyl, 3- to 8-membered heterocycloalkyl which is unsubstituted or substituted with 1 to 6 groups selected from Substituent Group C, C₆₋₁₀ aryl or 5- or 6-membered heteroaryl which is unsubstituted or substituted with 1 to 4 groups selected from Substituent Group C.

### Process 2-1

Compound (2-2) can also be manufactured by removing the protecting group of Compound (2-1).

### Process 2-2

Compound (I-2a) can also be manufactured by reacting Compound (2-2) with trimethylsilyl isocyanate. As another method, Compound (I-2a) can also be manufactured by reacting Compound (2-2) with potassium cyanate or sodium cyanate in the presence of an acid.

### Process 2-3

Compound (I-2b) can also be manufactured by urea formation of Compound (2-2) and Compound (1-3).

### Process 2-4

Compound (I-2c) can also be manufactured by carbamate formation of Compound (2-2) and Compound (2-3).

### Process 2-5

Compound (I-2d) can also be manufactured by amidation of Compound (2-2) and Compound (2-4).

### Process 2-6

Compound (I-2e) can also be manufactured by reacting Compound (2-2) with tert-butyl N-(chlorosulfonyl)carbamate, followed by a reaction with an acid.

Compound represented by Formula (1-1) can be manufactured, for example, according to the method described in Scheme 3.

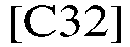

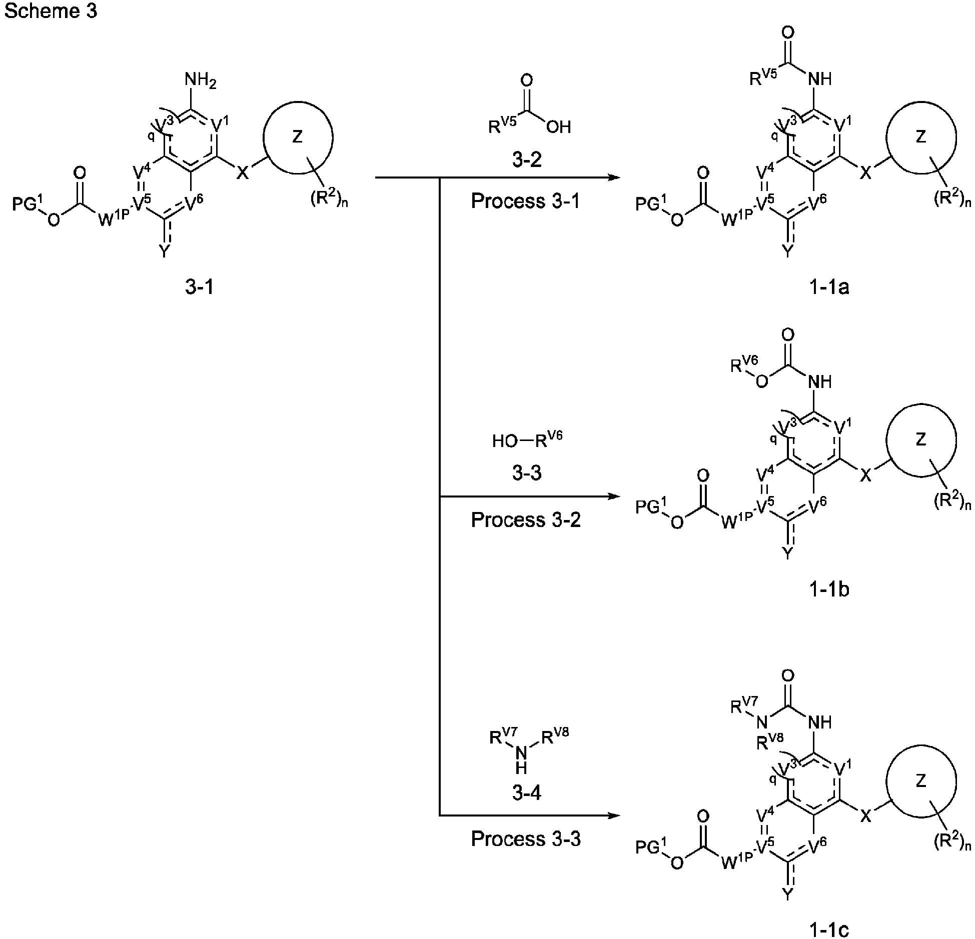

The symbols in the formulae have the same meanings as above.

### Process 3-1

Compound (1-1a) can also be manufactured by amidation of Compound (3-1) and Compound (3-2).

### Process 3-2

Compound (1-1b) can also be manufactured by carbamate formation of Compound (3-1) and Compound (3-3).

### Process 3-3

Compound (1-1c) can also be manufactured by urea formation of Compound (3-1) and Compound (3-4).

The compound represented by Formula (2-1) can be manufactured, for example, according to the method described in Scheme 4.

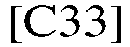

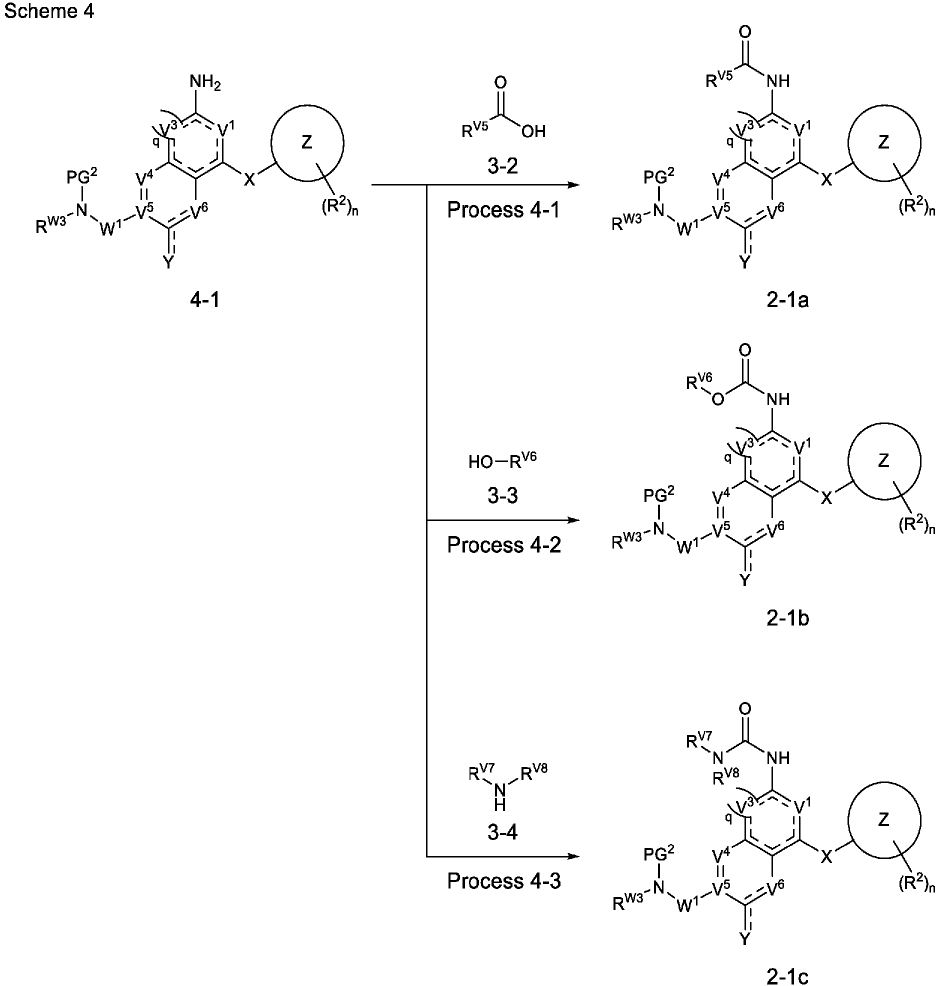

The symbols in the formulae have the same meanings as above.

### Process 4-1

Compound (2-1a) can also be manufactured by amidation of Compound (4-1) and Compound (3-2).

### Process 4-2

Compound (2-1b) can also be manufactured by carbamate formation of Compound (4-1) and Compound (3-3).

### Process 4-3

Compound (2-1c) can also be manufactured by urea formation of Compound (4-1) and Compound (3-4).

The compound represented by Formula (3-1) can be manufactured, for example, according to the method described in Scheme 5. 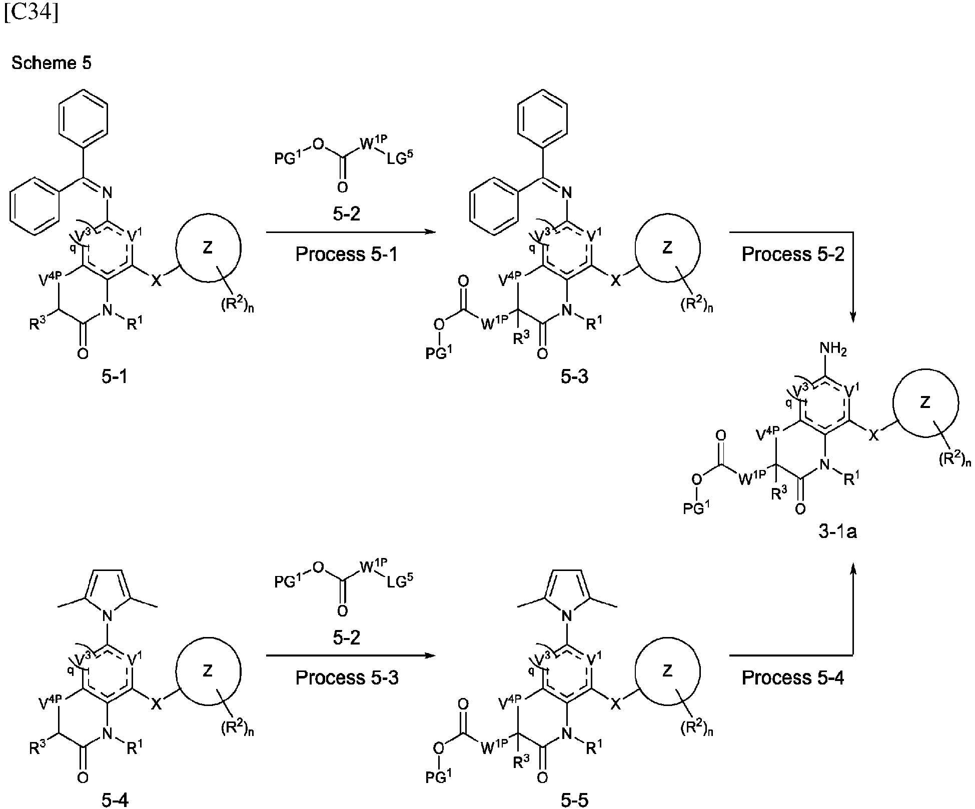 The symbols in the formulae have the same meanings as above. LG⁵ is a chlorine atom, a bromine atom, an iodine atom, OCO-(C₁₋₆ alkyl), or a methanesulfonyloxy group, and V^{4P} is a single bond, CR^{T1}R^{T2}, O, or NR^{T3}.

### Process 5-1

Compound (5-3) can also be manufactured by reacting Compound (5-1) with Compound (5-2) in the presence of a base.

### Process 5-2

Compound (3-1a) can also be manufactured by reacting Compound (5-3) with an acid and a hydroxylamine.

### Process 5-3

Compound (5-5) can also be manufactured by reacting Compound (5-4) with Compound (5-2) in the presence of a base.

### Process 5-4

Compound (3-1a) can also be manufactured by reacting Compound (5-5) with an acid and a hydroxylamine.

The compound represented by Formula (4-1) can be manufactured, for example, according to the method described in Scheme 6.

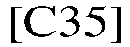

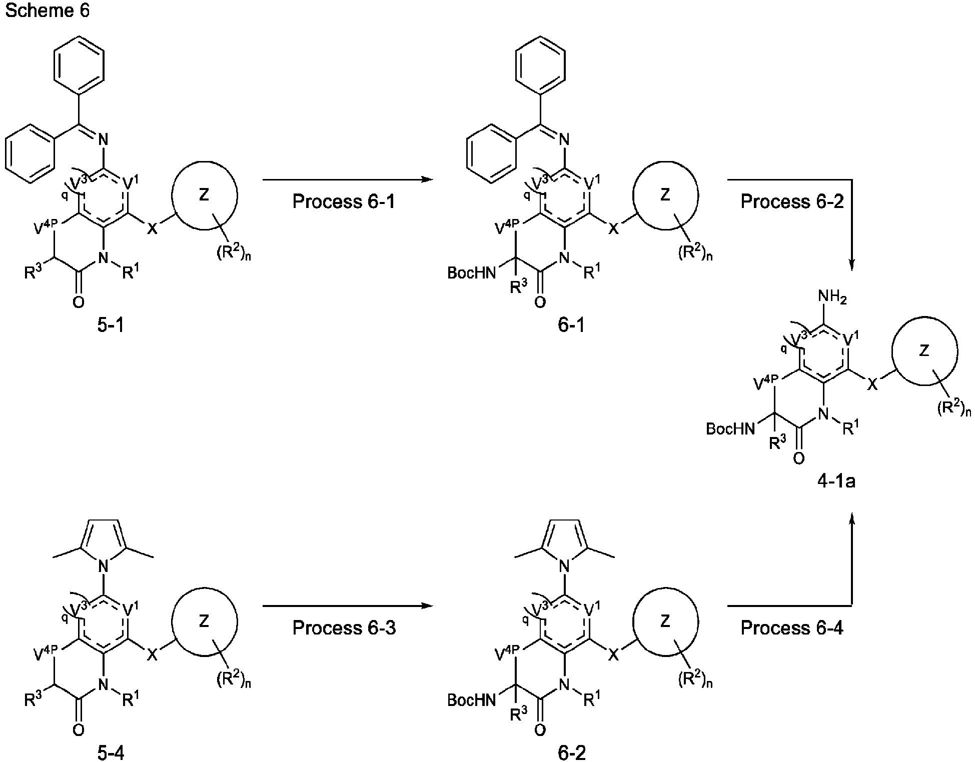

The symbols in the formulae have the same meanings as above.

### Process 6-1

Compound (6-1) can also be manufactured by reacting Compound (5-1) with DPPA and Boc₂O in the presence of a base.

### Process 6-2

Compound (4-1a) can also be manufactured by reacting Compound (6-1) with an acid and a hydroxylamine, followed by a reaction with Boc₂O.

### Process 6-3

Compound (6-2) can also be manufactured by reacting Compound (5-4) with DPPA and Boc₂O in the presence of a base.

### Process 6-4

Compound (4-1a) can also be manufactured by reacting Compound (6-2) with an acid and a hydroxylamine, followed by a reaction with Boc₂O.

The compounds represented by Formula (1-1), Formula (2-1), and Formula (2-2) can be manufactured, for example, according to the method described in Scheme 7. 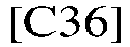 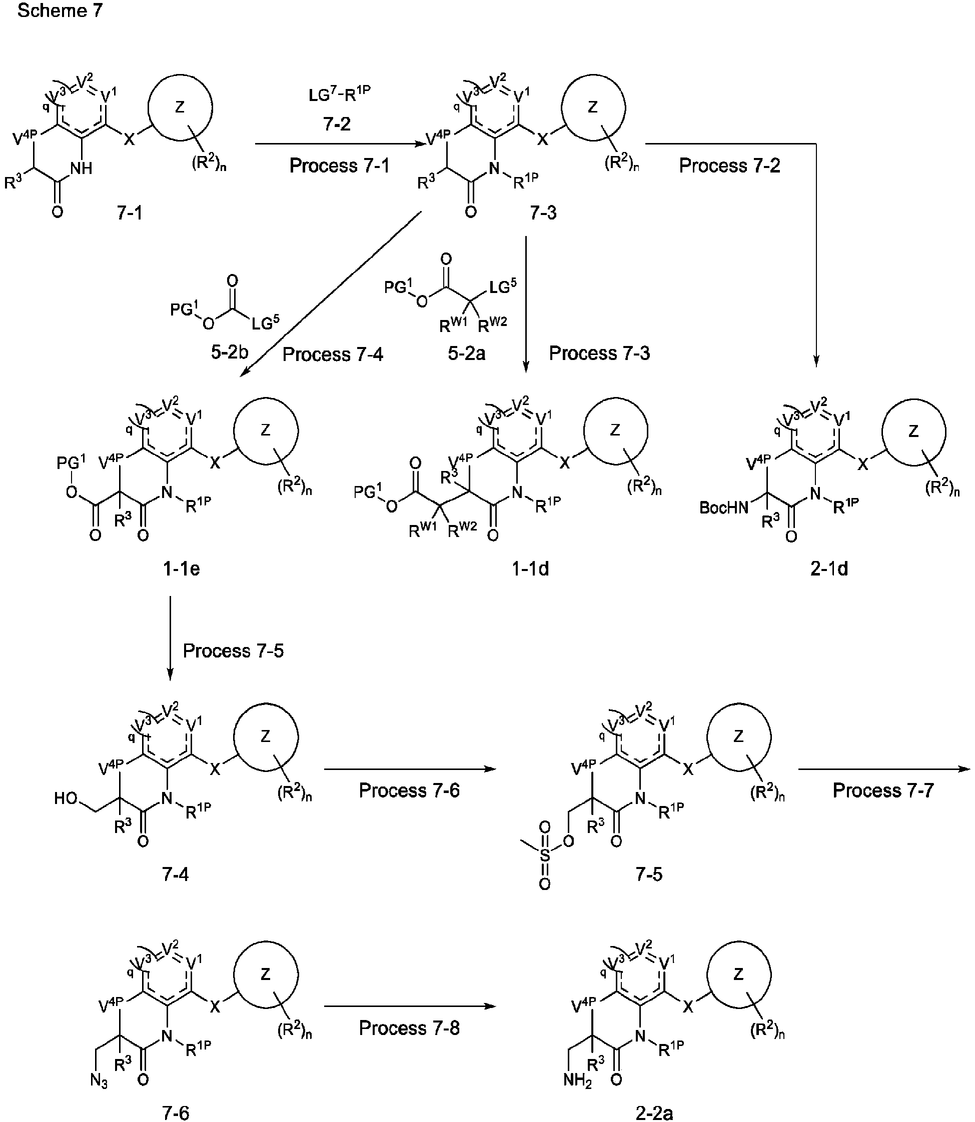 The symbols in the formulae have the same meanings as above. LG⁷ is a chlorine atom, a bromine atom, an iodine atom, or a methanesulfonyloxy group, and R^{1P} is C₁₋₆ alkyl or halo C₁₋₆ alkyl.

### Process 7-1

Compound (7-3) can also be manufactured by reacting Compound (7-1) with Compound (7-2) in the presence of a base.

### Process 7-2

Compound (2-1d) can also be manufactured by reacting Compound (7-3) with DPPA and Boc₂O in the presence of a base.

### Process 7-3

Compound (1-1d) can also be manufactured by reacting Compound (7-3) with Compound (5-2a) in the presence of a base.

### Process 7-4

Compound (1-1e) can also be manufactured by reacting Compound (7-3) with Compound (5-2b) in the presence of a base.

### Process 7-5

Compound (7-4) can also be manufactured by reduction of Compound (1-1e).

### Process 7-6

Compound (7-5) can also be manufactured by reacting Compound (7-4) with methanesulfonyl chloride.

### Process 7-7

Compound (7-6) can also be manufactured by reacting Compound (7-5) with sodium azide.

### Process 7-8

Compound (2-2a) can also be manufactured by catalytic reduction of Compound (7-6).

The compound represented by Formula (5-1) can be manufactured, for example, according to the method described in Scheme 8.

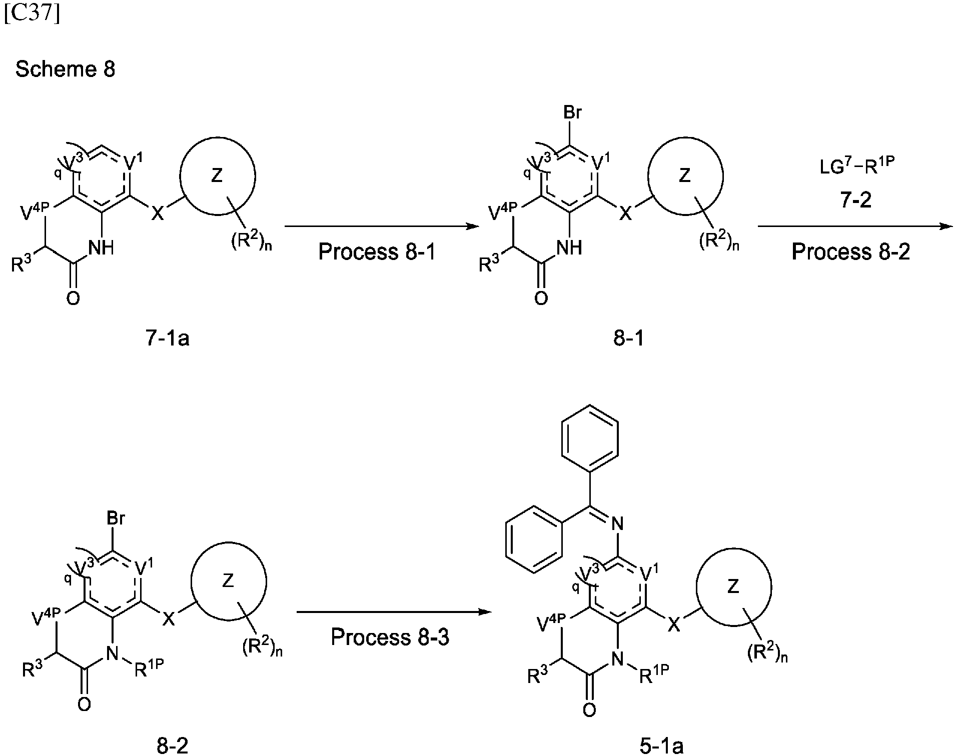

The symbols in the formulae have the same meanings as above.

### Process 8-1

Compound (8-1) can also be manufactured by bromination of Compound (7-1a).

### Process 8-2

Compound (8-2) can also be manufactured by reacting Compound (8-1) with Compound (7-2) in the presence of a base.

### Process 8-3

Compound (5-1a) can also be manufactured by a Buchwald-Hartwig cross coupling reaction of Compound (8-2) and benzophenone imine.

The compound represented by Formula (7-1) can be manufactured, for example, according to the method described in Scheme 9. 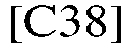 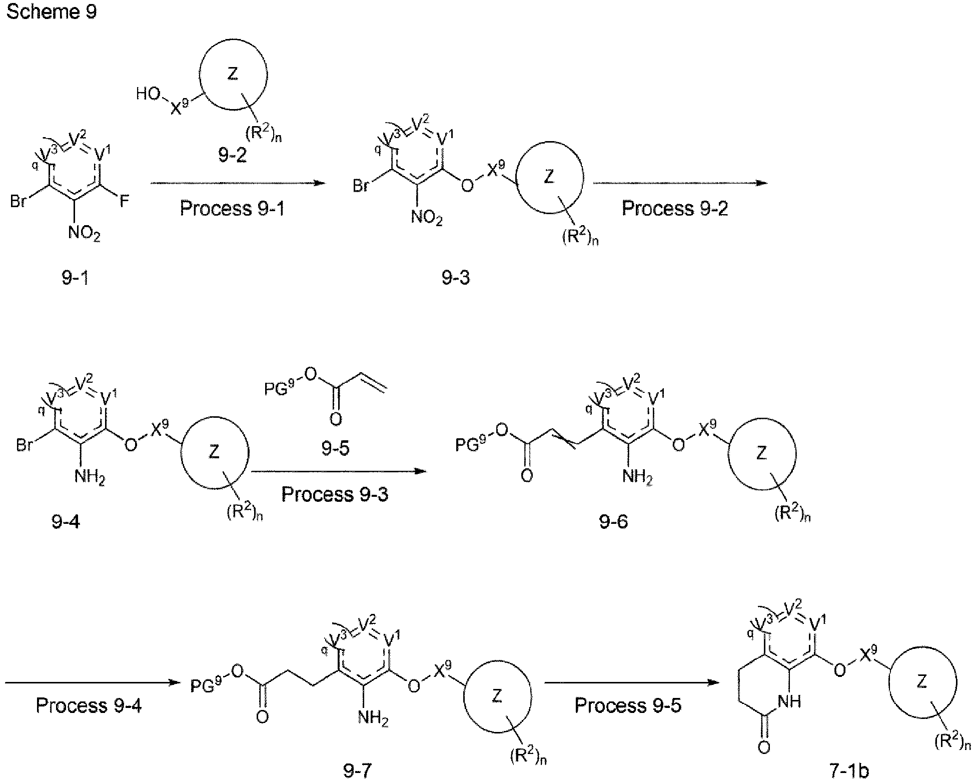 The symbols in the formulae have the same meanings as above. X⁹ is a single bond or C₁₋₆ alkylene, and PG⁹ is a protecting group.

### Process 9-1

Compound (9-3) can also be manufactured by an aromatic nucleophilic substitution reaction of Compound (9-1) and Compound (9-2).

### Process 9-2

Compound (9-4) can also be manufactured by metal reduction of Compound (9-3).

### Process 9-3

Compound (9-6) can also be manufactured by a Mizoroki-Heck cross-coupling reaction of Compound (9-4) and Compound (9-5).

### Process 9-4

Compound (9-7) can also be manufactured by catalytic reduction of Compound (9-6).

### Process 9-5

Compound (7-1b) can also be manufactured by reacting Compound (9-7) with a base.

The compound represented by Formula (5-4) can be manufactured, for example, according to the method described in Scheme 10. 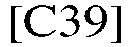 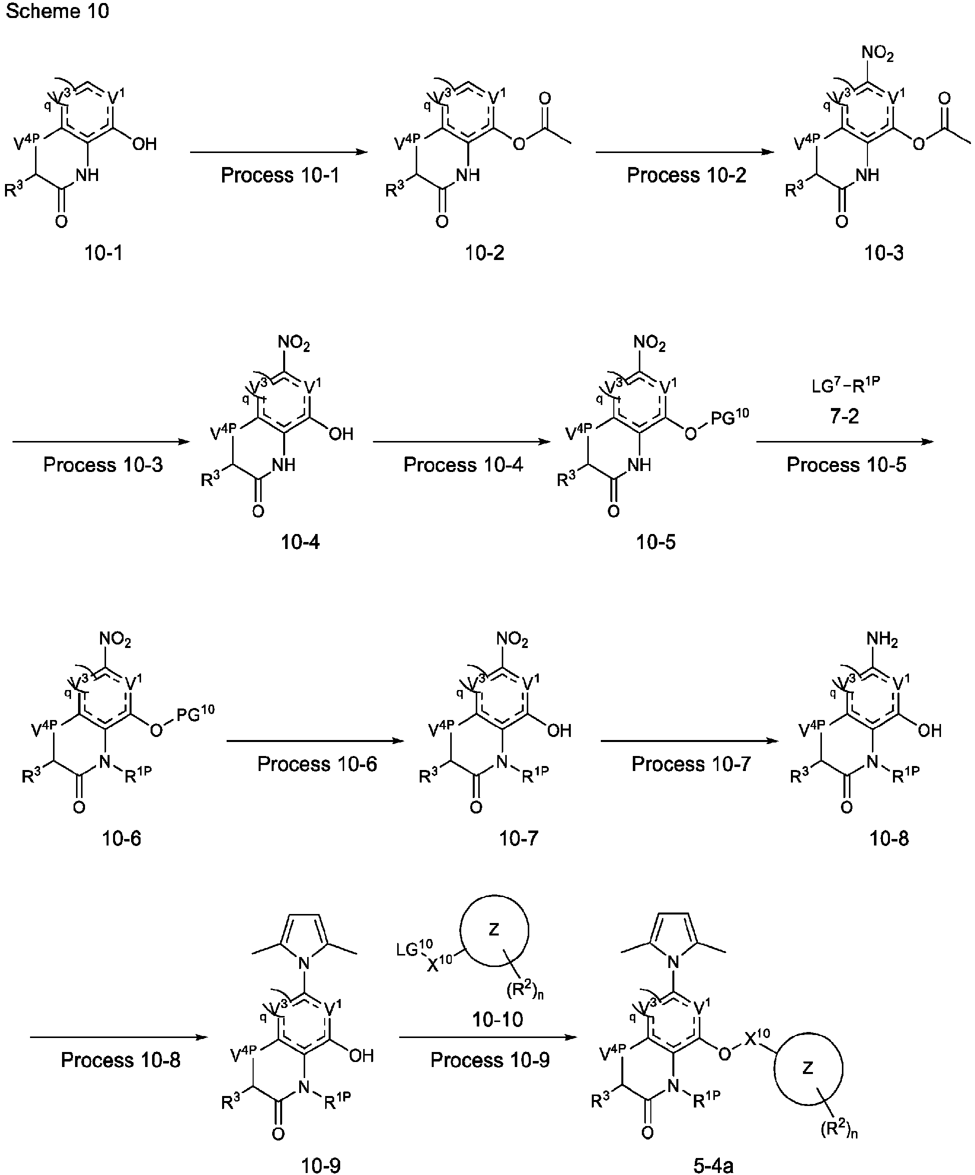 The symbols in the formulae have the same meanings as above. PG¹⁰ is a protecting group, LG¹⁰ is a fluorine atom, a chlorine atom, a bromine atom, an iodine atom, or a methanesulfonyloxy group, and X¹⁰ is a single bond or C₁₋₆ alkylene.

### Process 10-1

Compound (10-2) can also be manufactured by reacting Compound (10-1) with acetic anhydride in the presence of an acid.

### Process 10-2

Compound (10-3) can also be manufactured by reacting Compound (10-2) with concentrated nitric acid in the presence of an acid.

### Process 10-3

Compound (10-4) can also be manufactured by hydrolysis of Compound (10-3).

### Process 10-4

Compound (10-5) can also be manufactured by protecting the hydroxyl group of Compound (10-4).

### Process 10-5

Compound (10-6) can also be manufactured by reacting Compound (10-5) with Compound (7-2) in the presence of a base.

### Process 10-6

Compound (10-7) can also be manufactured by removing the protecting group of Compound (10-6).

### Process 10-7

Compound (10-8) can also be manufactured by reduction of Compound (10-7).

### Process 10-8

Compound (10-9) can also be manufactured by reacting Compound (10-8) with an acetonylacetone in the presence of an acid.

### Process 10-9

Compound (5-4a) can also be manufactured by reacting Compound (10-9) with Compound (10-10) in the presence of a base.

The compounds represented by Formula (2-1) and Formula (2-2) can be manufactured, for example, according to the method described in Scheme 11. 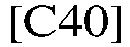 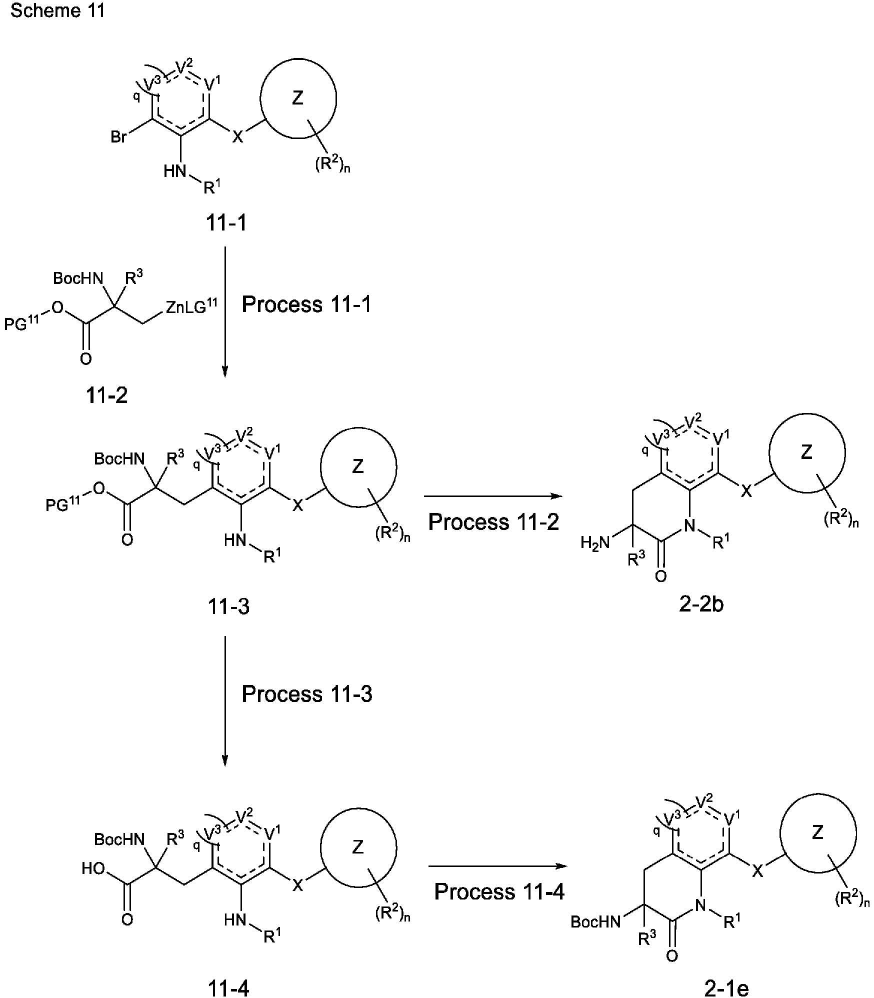 The symbols in the formulae have the same meanings as above. PG¹¹ is a protecting group, and LG¹¹ is a bromine atom or an iodine atom.

### Process 11-1

Compound (11-3) can also be manufactured by a Negishi coupling reaction of Compound (11-1) and Compound (11-2).

### Process 11-2

Compound (2-2b) can also be manufactured by reacting Compound (11-3) with an acid.

### Process 11-3

Compound (11-4) can also be manufactured by hydrolysis of Compound (11-3).

### Process 11-4

Compound (2-1e) can also be manufactured by amidation of Compound (11-4).

The compound represented by Formula (11-1) can be manufactured, for example, according to the method described in Scheme 12. 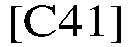 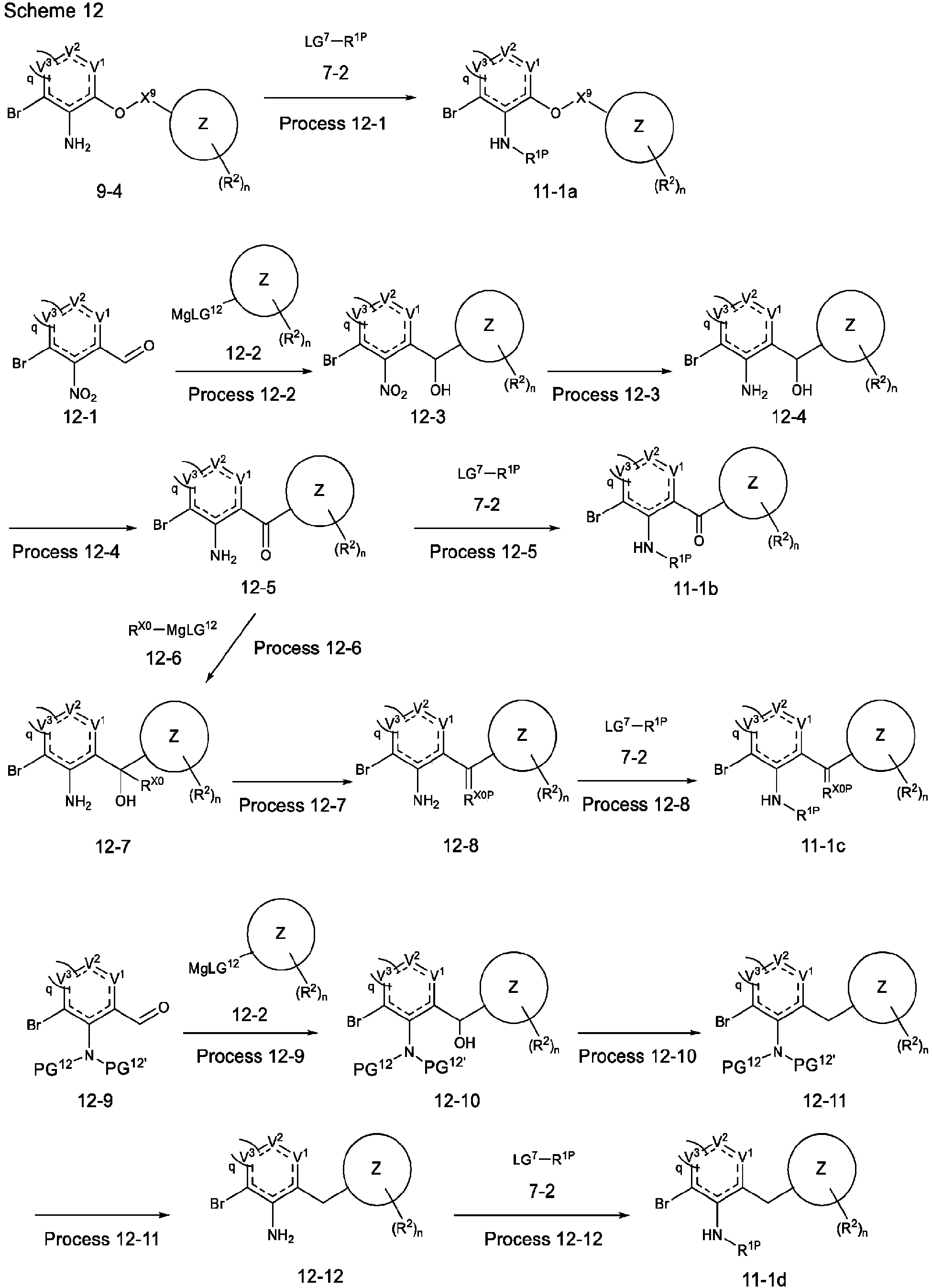 The symbols in the formulae have the same meanings as above. LG¹² is a chlorine atom, a bromine atom, or an iodine atom, R^{X0P} is C₂₋₆ alkenyl, and PG¹² and PG^{12'} are each independently a protecting group.

### Process 12-1

Compound (11-1a) can also be manufactured by reacting Compound (9-4) with Compound (7-2) in the presence of a base.

### Process 12-2

Compound (12-3) can also be manufactured by reacting Compound (12-1) with Compound (12-2).

### Process 12-3

Compound (12-4) can also be manufactured by metal reduction of Compound (12-3).

### Process 12-4

Compound (12-5) can also be manufactured by oxidation of Compound (12-4).

### Process 12-5

Compound (11-1b) can also be manufactured by reacting Compound (12-5) with Compound (7-2) in the presence of a base.

### Process 12-6

Compound (12-7) can also be manufactured by reacting Compound (12-5) with Compound (12-6).

### Process 12-7

Compound (12-8) can also be manufactured by reacting Compound (12-7) with triethylsilane in the presence of an acid.

### Process 12-8

Compound (11-1c) can also be manufactured by reacting Compound (12-8) with Compound (7-2) in the presence of a base.

### Process 12-9

Compound (12-10) can also be manufactured by reacting Compound (12-9) with Compound (12-2).

### Process 12-10

Compound (12-11) can also be manufactured by reacting Compound (12-10) with triethylsilane in the presence of an acid.

### Process 12-11

Compound (12-12) can also be manufactured by removing the protecting group of Compound (12-11).

### Process 12-12

Compound (11-1d) can also be manufactured by reacting Compound (12-12) with Compound (7-2) in the presence of a base.

The compound represented by Formula (7-3) can be manufactured, for example, according to the method described in Scheme 13.

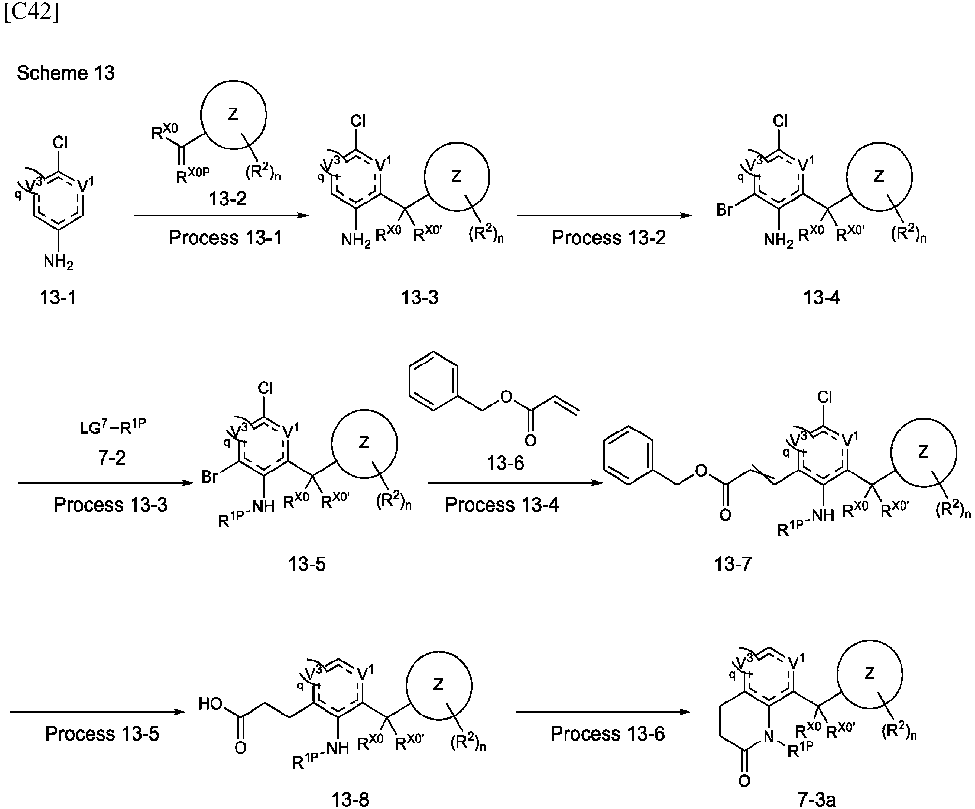

The symbols in the formulae have the same meanings as above.

### Process 13-1

Compound (13-3) can also be manufactured by reacting Compound (13-1) with Compound (13-2) in the presence of an acid.

### Process 13-2

Compound (13-4) can also be manufactured by bromination of Compound (13-3).

### Process 13-3

Compound (13-5) can also be manufactured by reacting Compound (13-4) with Compound (7-2) in the presence of a base.

### Process 13-4

Compound (13-7) can also be manufactured by a Mizoroki-Heck cross-coupling reaction of Compound (13-5) and Compound (13-6).

### Process 13-5

Compound (13-8) can also be manufactured by catalytic reduction of Compound (13-7).

### Process 13-6

Compound (7-3a) can also be manufactured by amidation of Compound (13-8).

The compound represented by Formula (I-14) can be manufactured, for example, according to the method described in Scheme 14. 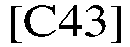 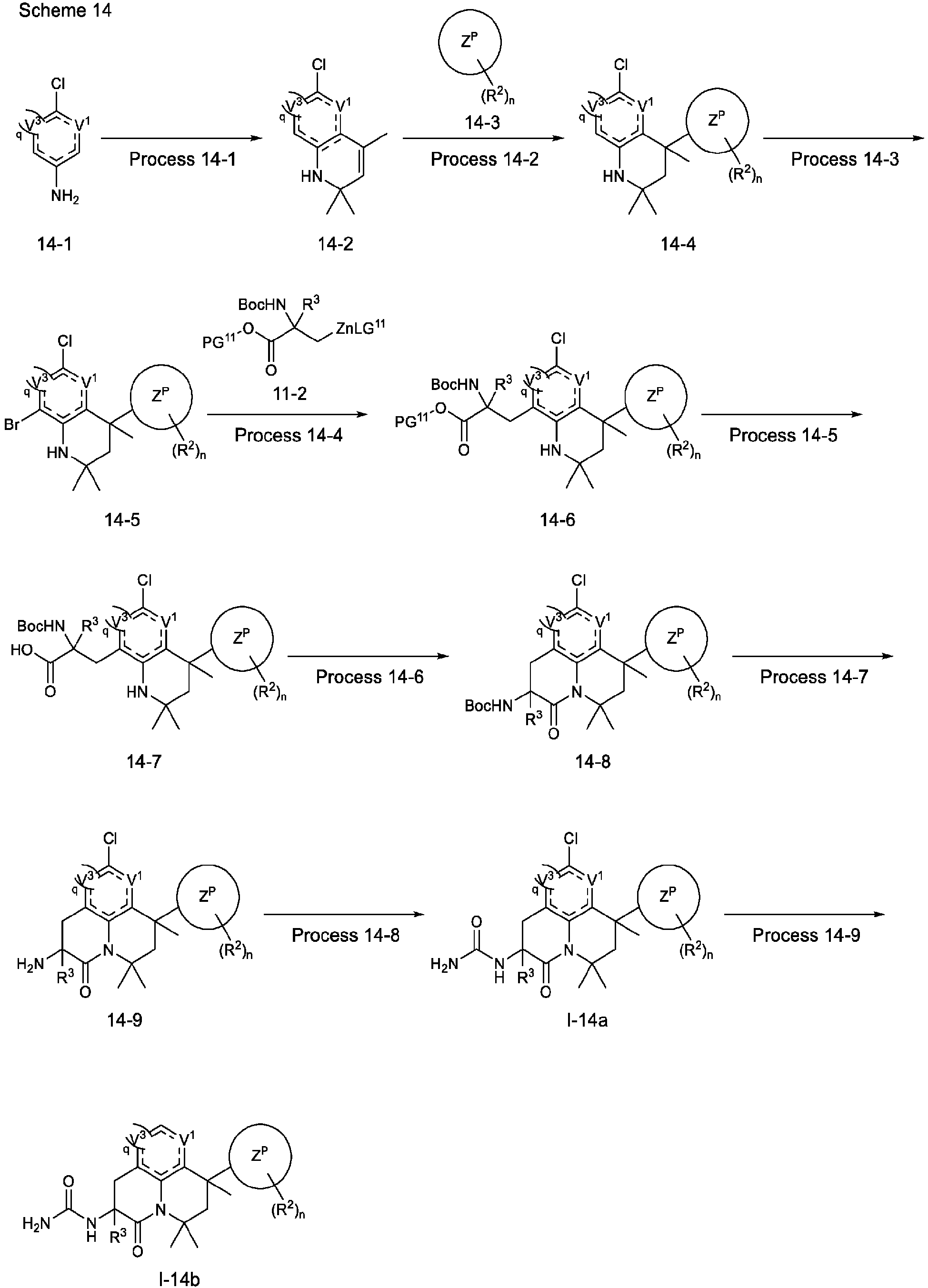 The symbols in the formulae have the same meanings as above. Z^{P} is C₆₋₁₀ aryl or 5- or 6-membered heteroaryl.

### Process 14-1

Compound (14-2) can also be manufactured by reacting Compound (14-1) with acetone in the presence of an acid.

### Process 14-2

Compound (14-4) can also be manufactured by reacting Compound (14-2) with Compound (14-3) in the presence of an acid.

### Process 14-3

Compound (14-5) can also be manufactured by bromination of Compound (14-4).

### Process 14-4

Compound (14-6) can also be manufactured by a Negishi coupling reaction of Compound (14-5) and Compound (11-2).

### Process 14-5

Compound (14-7) can also be manufactured by hydrolysis of Compound (14-6).

### Process 14-6

Compound (14-8) can also be manufactured by amidation of Compound (14-7).

### Process 14-7

Compound (14-9) can also be manufactured by removing the Boc group of Compound (14-8).

### Process 14-8

Compound (I-14a) can also be manufactured by reacting Compound (14-9) with trimethylsilyl isocyanate. As another method, Compound (I-14a) can also be manufactured by reacting Compound (14-9) with potassium cyanate or sodium cyanate in the presence of an acid.

### Process 14-9

Compound (I-14b) can also be manufactured by catalytic reduction of Compound (I-14a).

The compound represented by Formula (I-15) can be manufactured, for example, according to the method described in Scheme 15.

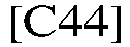

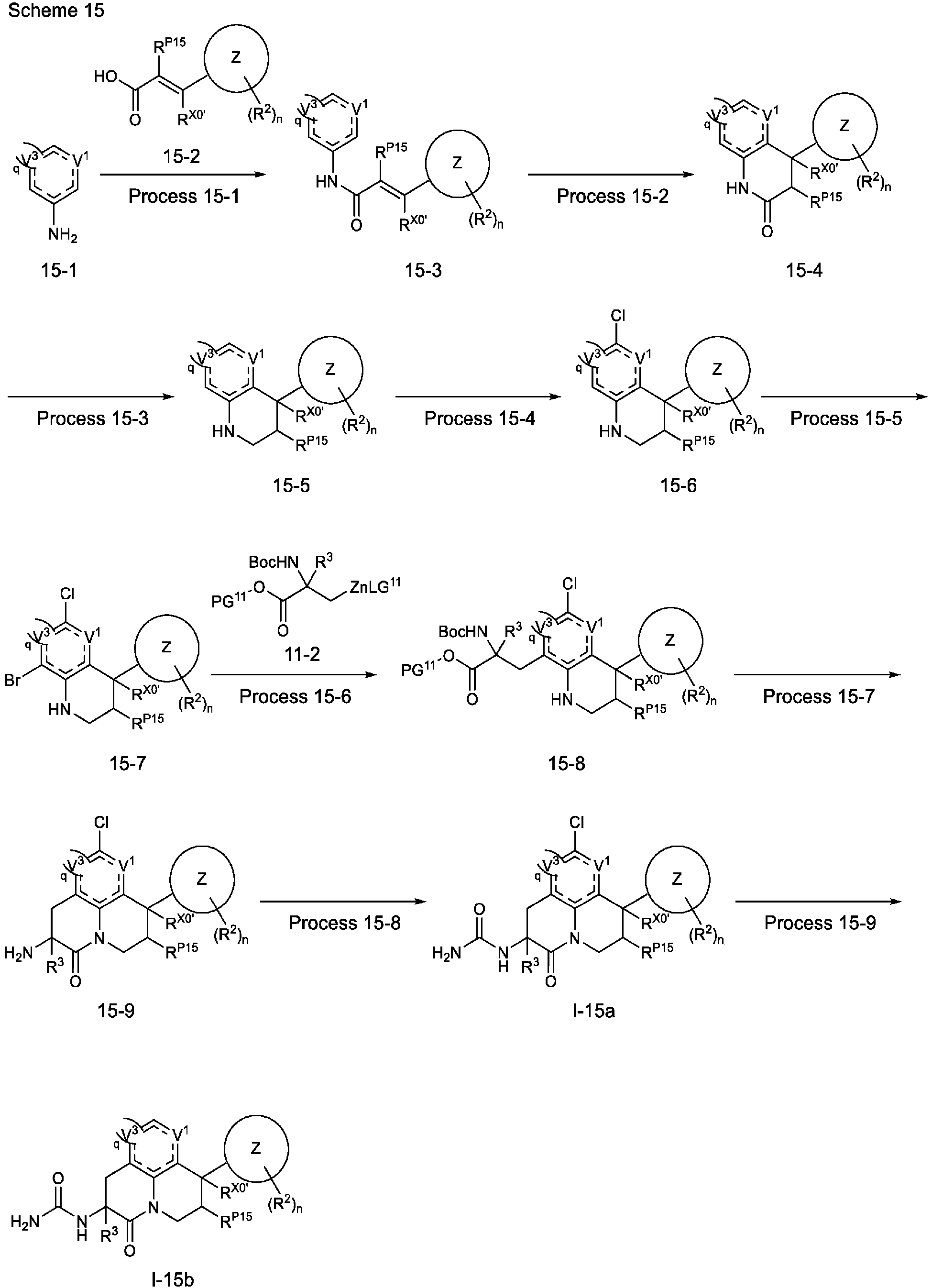

The symbols in the formulae have the same meanings as above. R^{P15} is a hydrogen atom, a halogen atom, hydroxy, C₁₋₆ alkyl, halo C₁₋₆ alkyl, C₁₋₆ alkoxy or halo C₁₋₆ alkoxy.

### Process 15-1

Compound (15-3) can also be manufactured by amidation of Compound (15-1) and Compound (15-2).

### Process 15-2

Compound (15-4) can also be manufactured by reacting Compound (15-3) with an acid.

### Process 15-3

Compound (15-5) can also be manufactured by reduction of Compound (15-4).

### Process 15-4

Compound (15-6) can also be manufactured by chlorination of Compound (15-5).

### Process 15-5

Compound (15-7) can also be manufactured by bromination of Compound (15-6).

### Process 15-6

Compound (15-8) can also be manufactured by a Negishi coupling reaction of Compound (15-7) and Compound (11-2).

### Process 15-7

Compound (15-9) can also be manufactured by reacting Compound (15-8) with an acid.

### Process 15-8

Compound (I-15a) can also be manufactured by reacting Compound (15-9) with trimethylsilyl isocyanate. As another method, Compound (I-15a) can also be manufactured by reacting Compound (15-9) with potassium cyanate or sodium cyanate in the presence of an acid.

### Process 15-9

Compound (I-15b) can also be manufactured by catalytic reduction of Compound (I-15a).

The compound represented by Formula (I-16) can be manufactured, for example, according to the method described in Scheme 16. 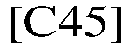 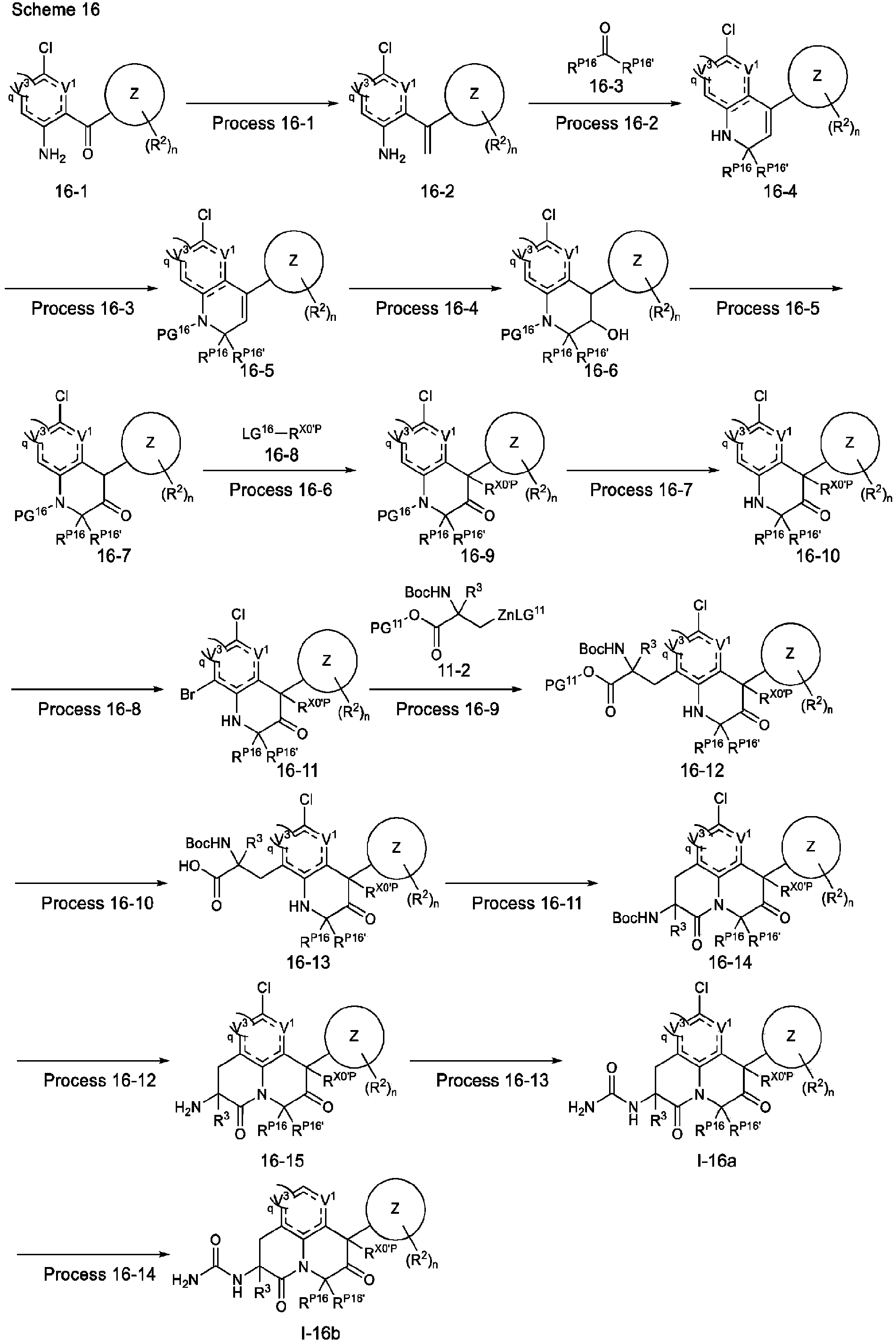 The symbols in the formulae have the same meanings as above. R^{P16}, R^{P16'}, and R^{X0'P} are each independently C₁₋₆ alkyl or halo C₁₋₆ alkyl, LG¹⁶ is a chlorine atom, a bromine atom, an iodine atom, or a methanesulfonyloxy group, and PG¹⁶ is a protecting group.

### Process 16-1

Compound (16-2) can also be manufactured by a Wittig reaction of Compound (16-1) and methyltriphenylphosphonium bromide.

### Process 16-2

Compound (16-4) can also be manufactured by reacting Compound (16-2) with Compound (16-3) in the presence of an acid.

### Process 16-3

Compound (16-5) can also be manufactured by protecting the amino group of Compound (16-4).

### Process 16-4

Compound (16-6) can also be manufactured by a Brown hydroboration reaction of Compound (16-5), followed by a reaction with a sodium hydroxide aqueous solution and hydrogen peroxide.

### Process 16-5

Compound (16-7) can also be manufactured by oxidation of Compound (16-6).

### Process 16-6

Compound (16-9) can also be manufactured by reacting Compound (16-7) with Compound (16-8) in the presence of a base.

### Process 16-7

Compound (16-10) can also be manufactured by removing the protecting group of Compound (16-9).

### Process 16-8

Compound (16-11) can also be manufactured by bromination of Compound (16-10).

### Process 16-9

Compound (16-12) can also be manufactured by a Negishi coupling reaction of Compound (16-11) and Compound (11-2).

### Process 16-10

Compound (16-13) can also be manufactured by hydrolysis of Compound (16-12).

### Process 16-11

Compound (16-14) can also be manufactured by amidation of Compound (16-13).

### Process 16-12

Compound (16-15) can also be manufactured by removing the protecting group of Compound (16-14).

### Process 16-13

Compound (I-16a) can also be manufactured by reacting Compound (16-15) with trimethylsilyl isocyanate. As another method, Compound (I-16a) can also be manufactured by reacting Compound (16-15) with potassium cyanate or sodium cyanate in the presence of an acid.

### Process 16-14

Compound (I-16b) can also be manufactured by catalytic reduction of Compound (I-16a).

The compound represented by Formula (2-1) can be manufactured, for example, according to the method described in Scheme 17. 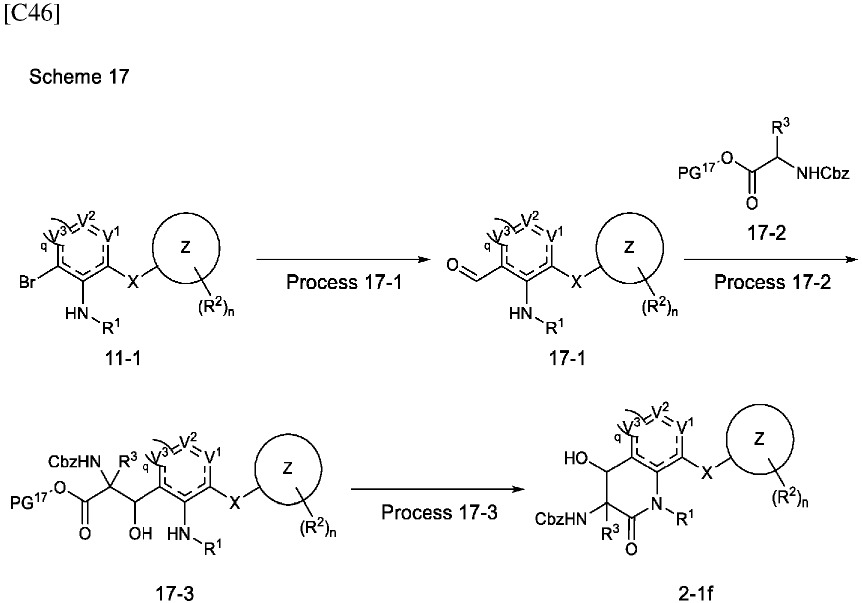 The symbols in the formulae have the same meanings as above. PG¹⁷ is a protecting group.

### Process 17-1

Compound (17-1) can also be manufactured by reacting Compound (11-1) with DMF in the presence of a base.

### Process 17-2

Compound (17-3) can also be manufactured by reacting Compound (17-1) with Compound (17-2) in the presence of a base.

### Process 17-3

Compound (2-1f) can also be manufactured by reacting Compound (17-3) with an acid.

The compound represented by Formula (I-18) can be manufactured, for example, according to the method described in Scheme 18. 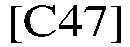 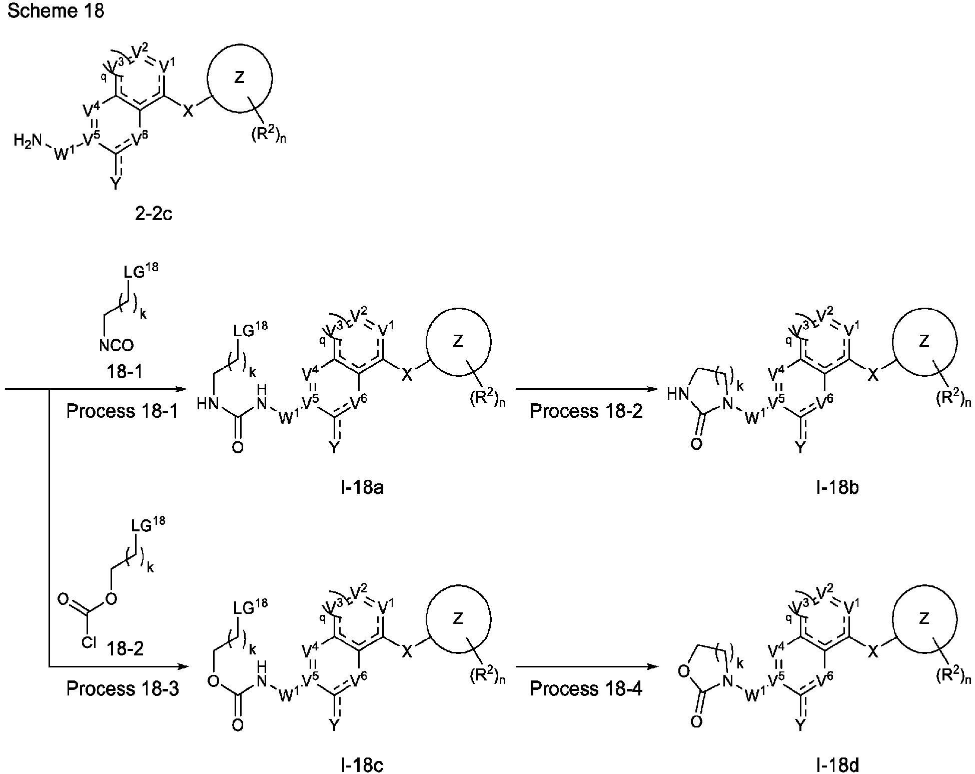 The symbols in the formulae have the same meanings as above. LG¹⁸ is a chlorine atom, a bromine atom, an iodine atom, or a methanesulfonyloxy group, and k is an integer of 1 to 4.

### Process 18-1

Compound (I-18a) can also be manufactured by reacting Compound (2-2c) with Compound (18-1).

### Process 18-2

Compound (I-18b) can also be manufactured by reacting Compound (I-18a) with a base.

### Process 18-3

Compound (I-18c) can also be manufactured by reacting Compound (2-2c) with Compound (18-2).

### Process 18-4

Compound (I-18d) can also be manufactured by reacting Compound (I-18c) with a base.

The compound represented by Formula (I-19) can be manufactured, for example, according to the method described in Scheme 19. 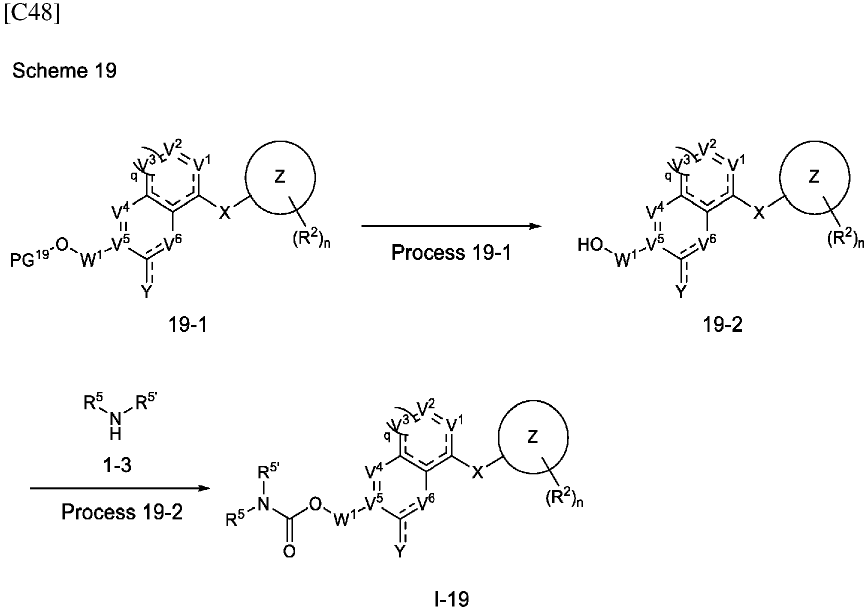 The symbols in the formulae have the same meanings as above. PG¹⁹ is a protecting group.

### Process 19-1

Compound (19-2) can also be manufactured by removing the protecting group of Compound (19-1).

### Process 19-2

Compound (I-19) can also be manufactured by carbamate formation of Compound (19-2) and Compound (1-3).

The compound represented by Formula (19-1) can be manufactured, for example, according to the method described in Scheme 20. 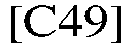 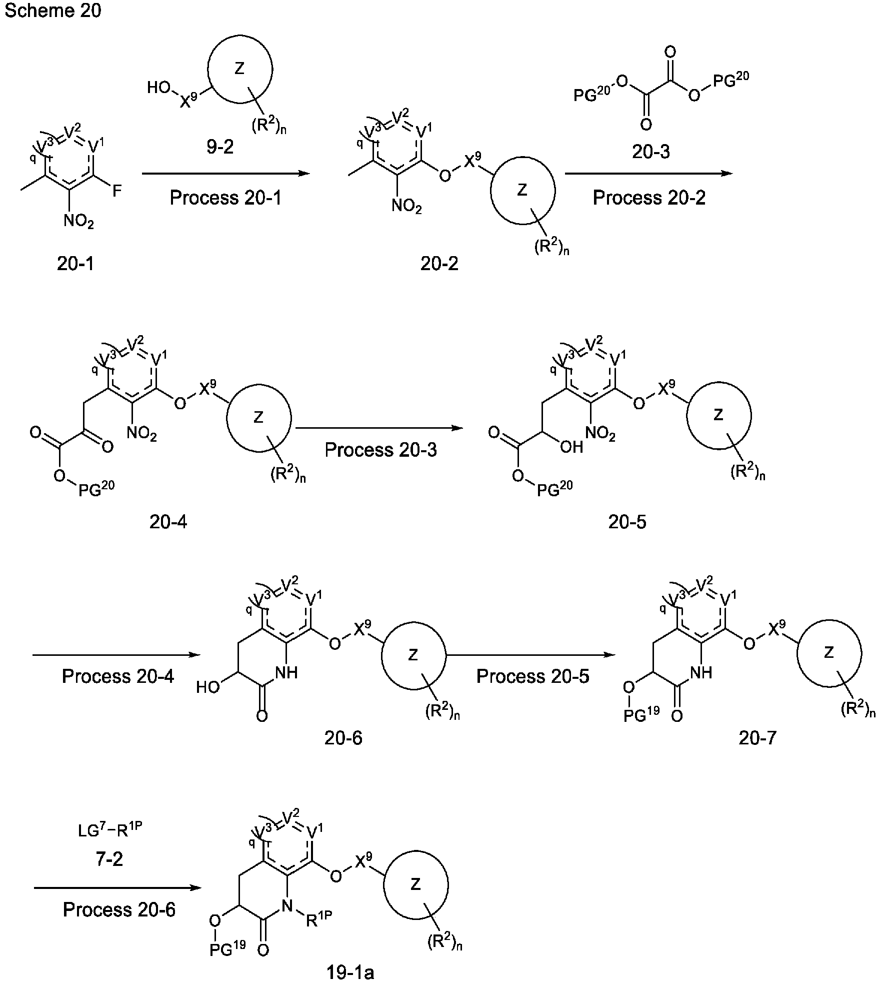 The symbols in the formulae have the same meanings as above. PG²⁰ is a protecting group.

### Process 20-1

Compound (20-2) can also be manufactured by reacting Compound (20-1) with Compound (9-2) in the presence of a base.

### Process 20-2

Compound (20-4) can also be manufactured by reacting Compound (20-2) with Compound (20-3) in the presence of a base.

### Process 20-3

Compound (20-5) can also be manufactured by reduction of Compound (20-4).

### Process 20-4

Compound (20-6) can also be manufactured by metal reduction of Compound (20-5).

### Process 20-5

Compound (20-7) can also be manufactured by protecting the hydroxyl group of Compound (20-6).

### Process 20-6

Compound (19-1a) can also be manufactured by reacting Compound (20-7) with Compound (7-2) in the presence of a base.

The compound represented by Formula (2-2) can be manufactured, for example, according to the method described in Scheme 21.

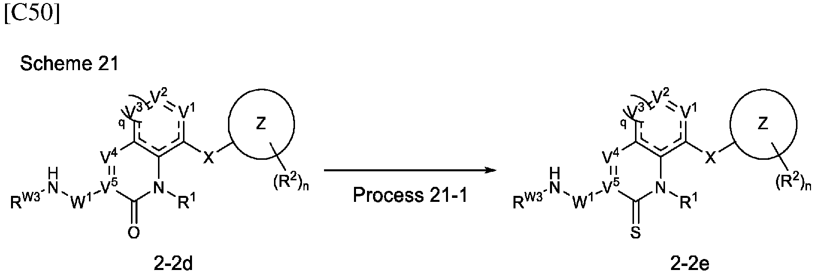

The symbols in the formulae have the same meanings as above.

### Process 21-1

Compound (2-2e) can also be manufactured by reacting Compound (2-2d) with a Lawesson's reagent.

The compound represented by Formula (2-1) can be manufactured, for example, according to the method described in Scheme 22.

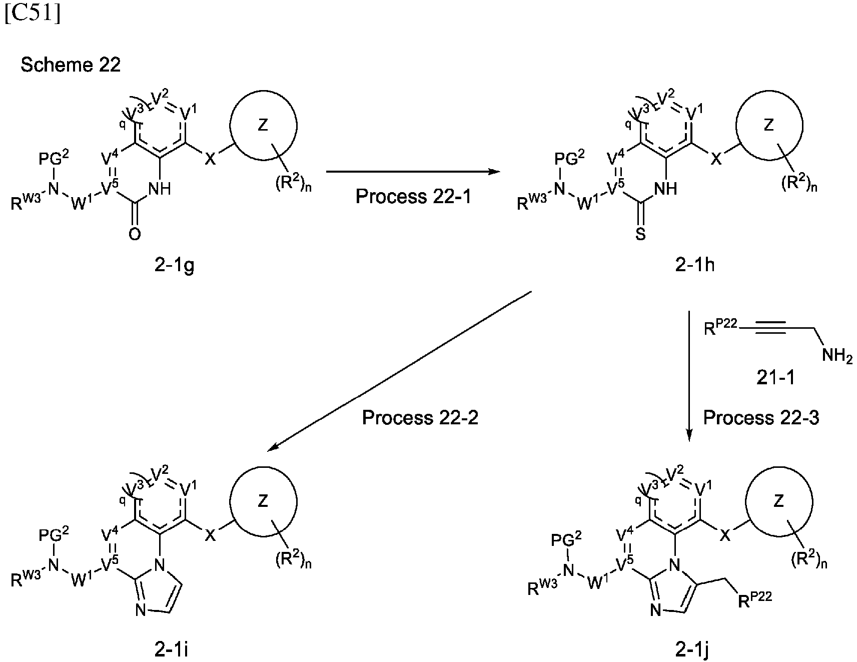

The symbols in the formulae have the same meanings as above. R^{P22} is a hydrogen atom or C₁₋₅ alkyl.

### Process 22-1

Compound (2-1h) can also be manufactured by reacting Compound (2-1g) with a Lawesson's reagent.

### Process 22-2

Compound (2-1i) can also be manufactured by reacting Compound (2-1h) with aminoacetaldehyde diethyl acetal, followed by a reaction with an acid.

### Process 22-3

Compound (2-1j) can also be manufactured by reacting Compound (2-1h) with Compound (22-1).

The compound represented by Formula (I-23) can be manufactured, for example, according to the method described in Scheme 23.

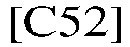

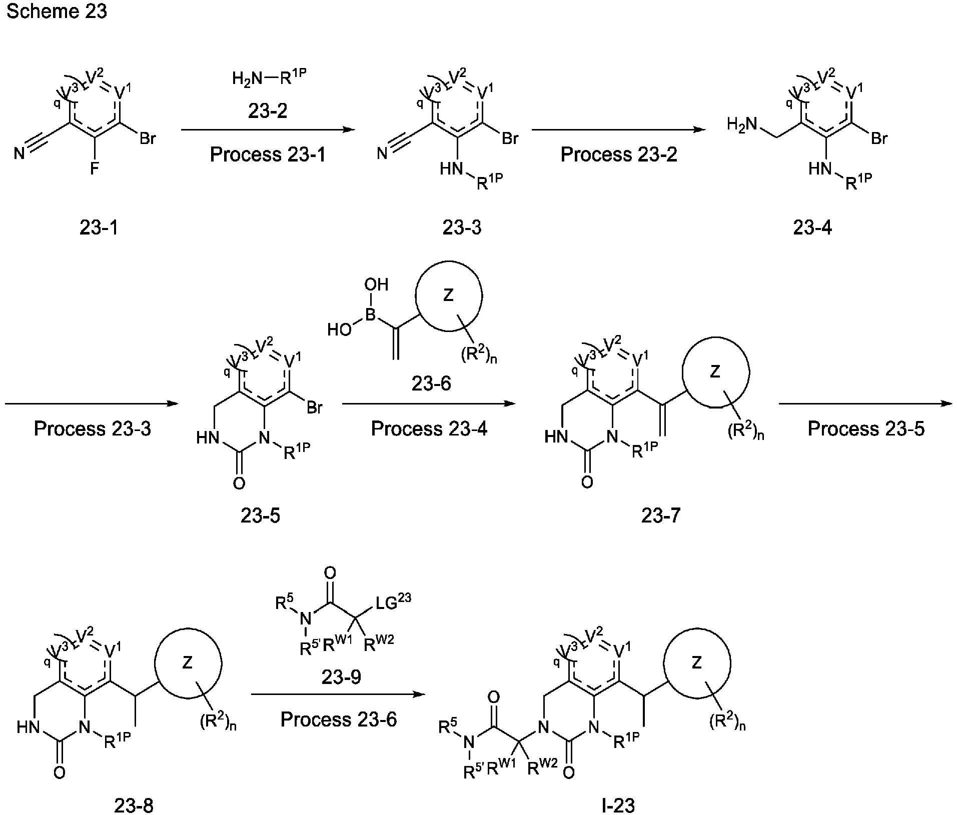

The symbols in the formulae have the same meanings as above. LG²³ is a chlorine atom, a bromine atom, an iodine atom, or a methanesulfonyloxy group.

### Process 23-1

Compound (23-3) can also be manufactured by an aromatic nucleophilic substitution reaction of Compound (23-1) and Compound (23-2).

### Process 23-2

Compound (23-4) can also be manufactured by reduction of Compound (23-3).

### Process 23-3

Compound (23-5) can also be manufactured by urea formation of Compound (23-4).

### Process 23-4

Compound (23-7) can also be manufactured by a Suzuki-Miyaura cross-coupling reaction of Compound (23-5) and Compound (23-6).

### Process 23-5

Compound (23-8) can also be manufactured by catalytic reduction of Compound (23-7).

### Process 23-6

Compound (I-23) can also be manufactured by reacting Compound (23-8) with Compound (23-9).

The compound represented by Formula (2-1) can be manufactured, for example, according to the method described in Scheme 24. 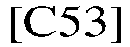 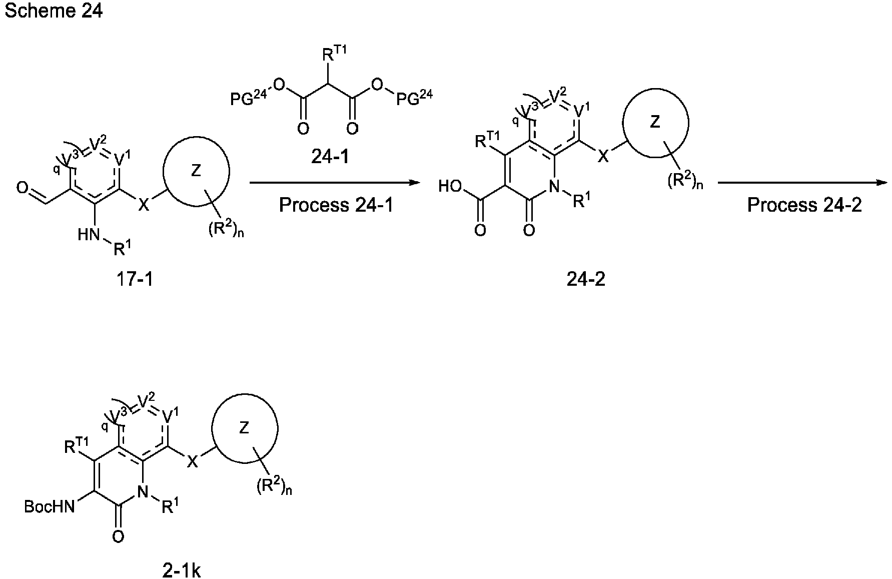 The symbols in the formulae have the same meanings as above. PG²⁴ is a protecting group.

### Process 24-1

Compound (24-2) can also be manufactured by reacting Compound (17-1) with Compound (24-1) in the presence of a base.

### Process 24-2

Compound (2-1k) can also be manufactured by a Curtius rearrangement reaction of Compound (24-2).

The compound represented by Formula (I-25) can be manufactured, for example, according to the method described in Scheme 25. 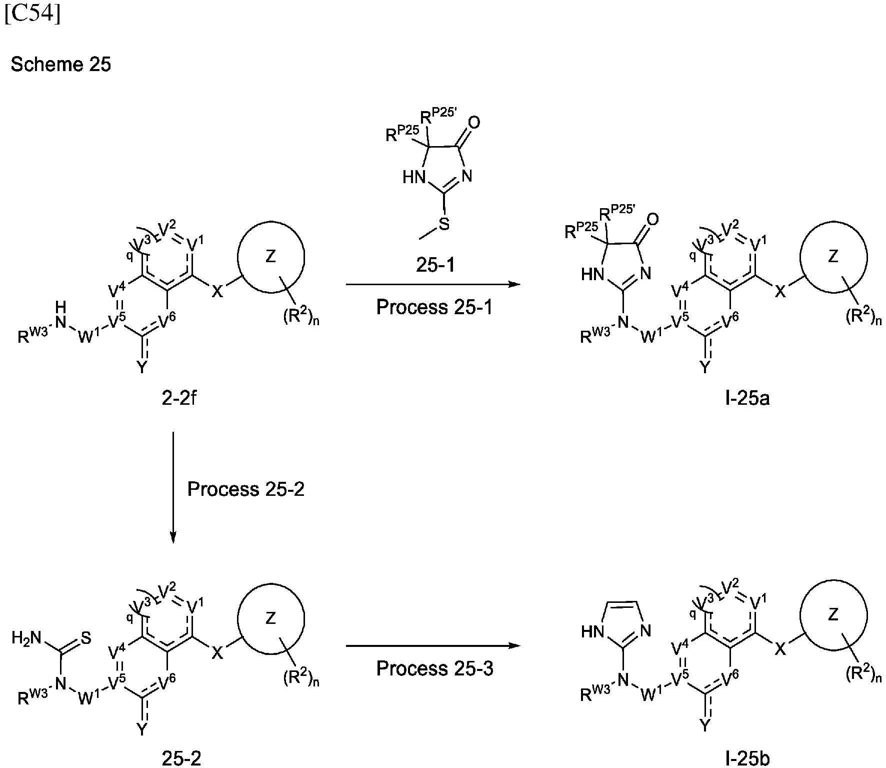 The symbols in the formulae have the same meanings as above. R²⁵ and R^{25'} are each independently a halogen atom, cyano, hydroxy, C₁₋₆ alkyl, halo C₁₋₆ alkyl, hydroxy C₁₋₆ alkyl, C₁₋₆ alkoxy, halo C₁₋₆ alkoxy, oxo, CONH₂, CONH-(C₁₋₆ alkyl), or CONH-(C₃₋₈ cycloalkyl).

### Process 25-1

Compound (I-25a) can also be manufactured by reacting Compound (2-2f) with Compound (25-1).

### Process 25-2

Compound (25-2) can also be manufactured by reacting Compound (2-2f) with benzoyl isothiocyanate, followed by a reaction with a base.

### Process 25-3

Compound (I-25b) can also be manufactured by reacting Compound (25-2) with methyl iodide and subsequently with 2,2-diethoxyethane-1-amine, followed by a reaction with an acid.

The compound represented by Formula (2-1) can be manufactured, for example, according to the method described in Scheme 26. 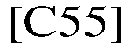 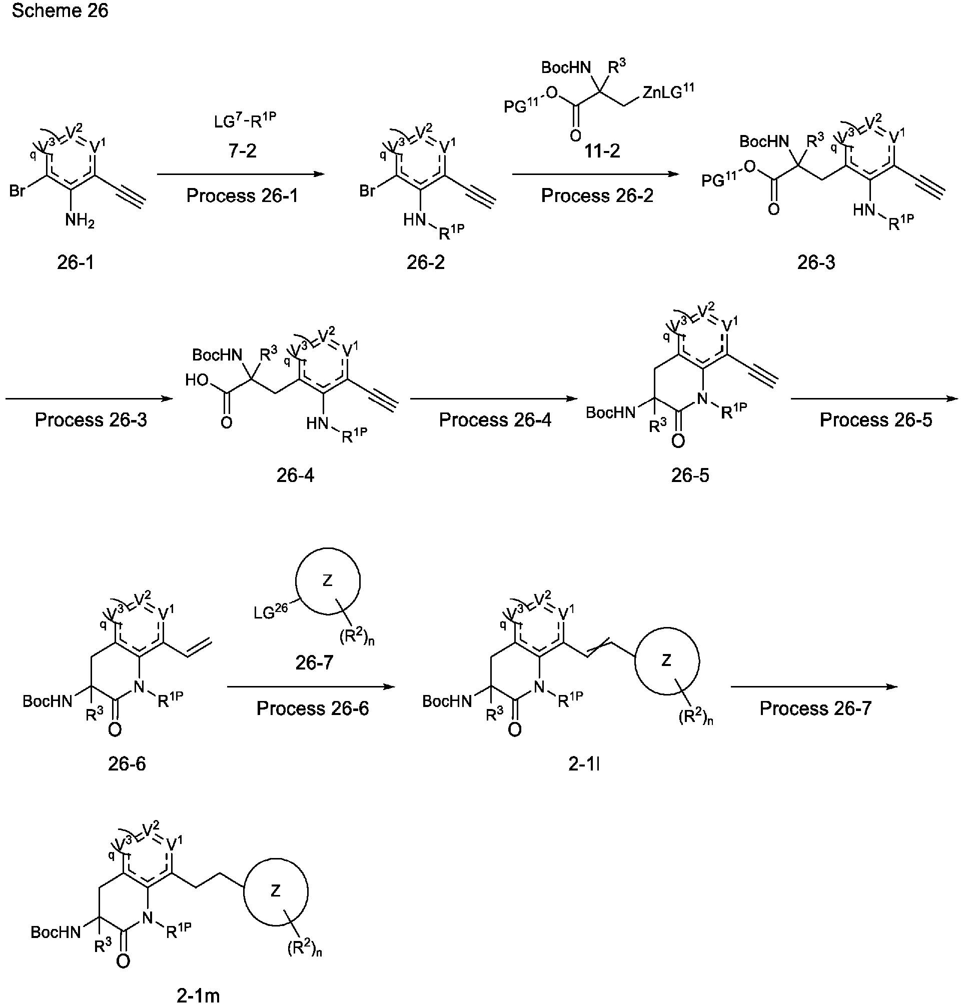 The symbols in the formulae have the same meanings as above. LG²⁶ is a chlorine atom, a bromine atom, or an iodine atom.

### Process 26-1

Compound (26-2) can also be manufactured by reacting Compound (26-1) with Compound (7-2) in the presence of a base.

### Process 26-2

Compound (26-3) can also be manufactured by a Negishi coupling reaction of Compound (26-2) and Compound (11-2).

### Process 26-3

Compound (26-4) can also be manufactured by hydrolysis of Compound (26-3).

### Process 26-4

Compound (26-5) can also be manufactured by amidation of Compound (26-4).

### Process 26-5

Compound (26-6) can also be manufactured by catalytic reduction of Compound (26-5).

### Process 26-6

Compound (2-1l) can also be manufactured by a Mizoroki-Heck cross-coupling reaction of Compound (26-6) and Compound (26-7).

### Process 26-7

Compound (2-1m) can also be manufactured by catalytic reduction of Compound (2-1l).

The compounds represented by Formula (2-2) and Formula (11-3) can be manufactured, for example, according to the method described in Scheme 27. 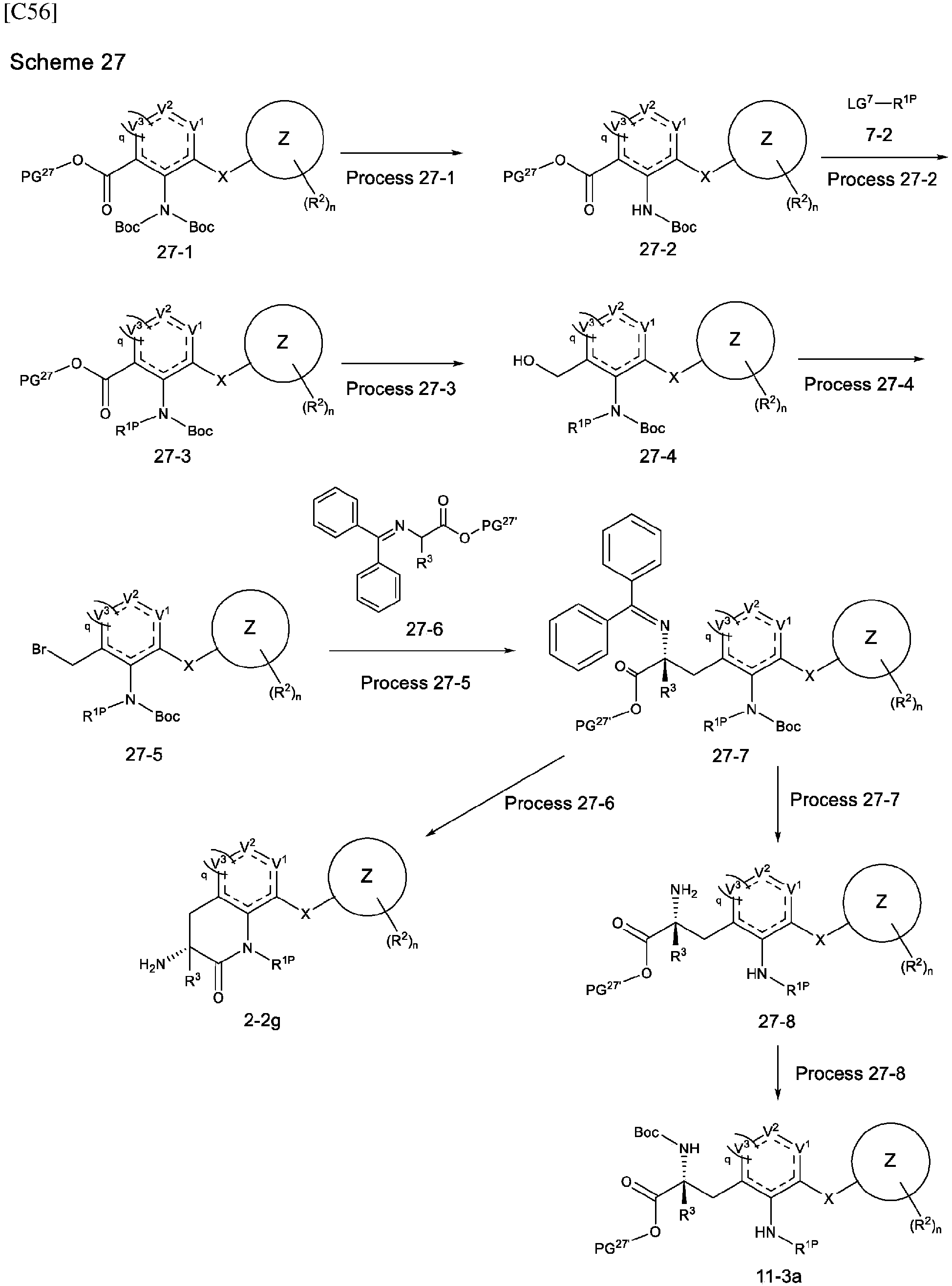 The symbols in the formulae have the same meanings as above. PG²⁷ and PG^{27'} are each independently a protecting group.

### Process 27-1

Compound (27-2) can also be manufactured by removing one Boc group of Compound (27-1).

### Process 27-2

Compound (27-3) can also be manufactured by reacting Compound (27-2) with Compound (7-2) in the presence of a base.

### Process 27-3

Compound (27-4) can also be manufactured by reduction of Compound (27-3).

### Process 27-4

Compound (27-5) can also be manufactured by reacting Compound (27-4) with methanesulfonyl chloride, followed by a reaction with lithium bromide.

### Process 27-5

Compound (27-7) can also be manufactured by asymmetric alkylation of Compound (27-5) and Compound (27-6).

### Process 27-6

Compound (2-2g) can also be manufactured by reacting Compound (27-7) with an acid.

### Process 27-7

Compound (27-8) can also be manufactured by reacting Compound (27-7) with an acid.

### Process 27-8

Compound (11-3a) can also be manufactured by converting the amino group of Compound (27-8) into a -NHBoc group.

The compound represented by Formula (27-1) can be manufactured, for example, according to the method described in Scheme 28. 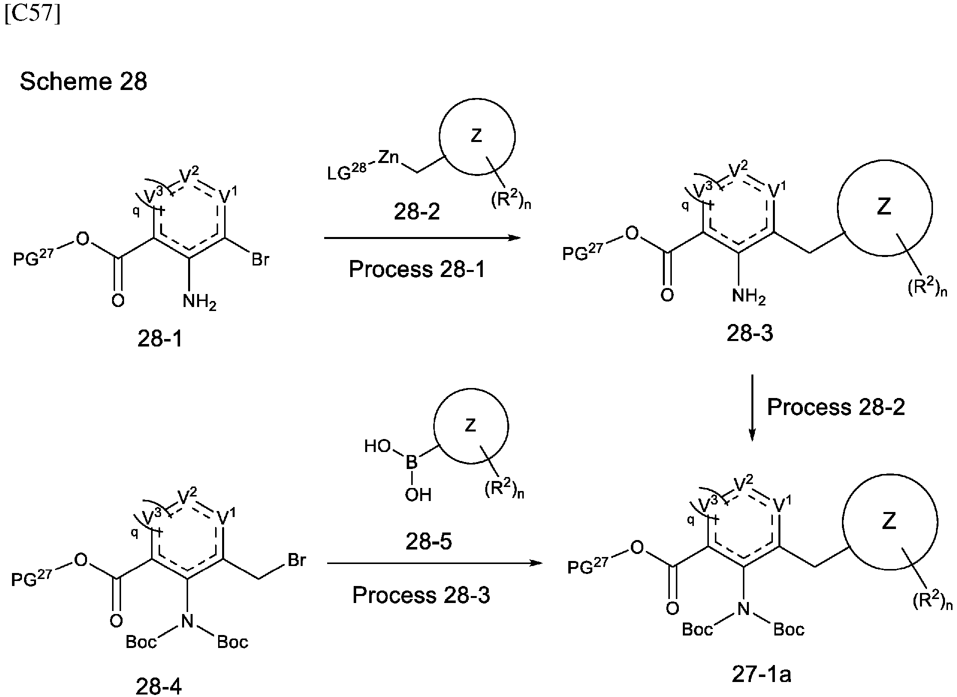 The symbols in the formulae have the same meanings as above. LG²⁸ is a chlorine atom, a bromine atom, or an iodine atom.

### Process 28-1

Compound (28-3) can also be manufactured by a Negishi coupling reaction of Compound (28-1) and Compound (28-2).

### Process 28-2

Compound (27-1a) can also be manufactured by converting the amino group of Compound (28-3) into a -N(boc)₂ group.

### Process 28-3

Compound (27-1a) can also be manufactured by a Suzuki-Miyaura cross-coupling reaction of Compound (28-4) and Compound (28-5).

The compound represented by Formula (2-1) can be manufactured, for example, according to the method described in Scheme 29.

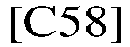

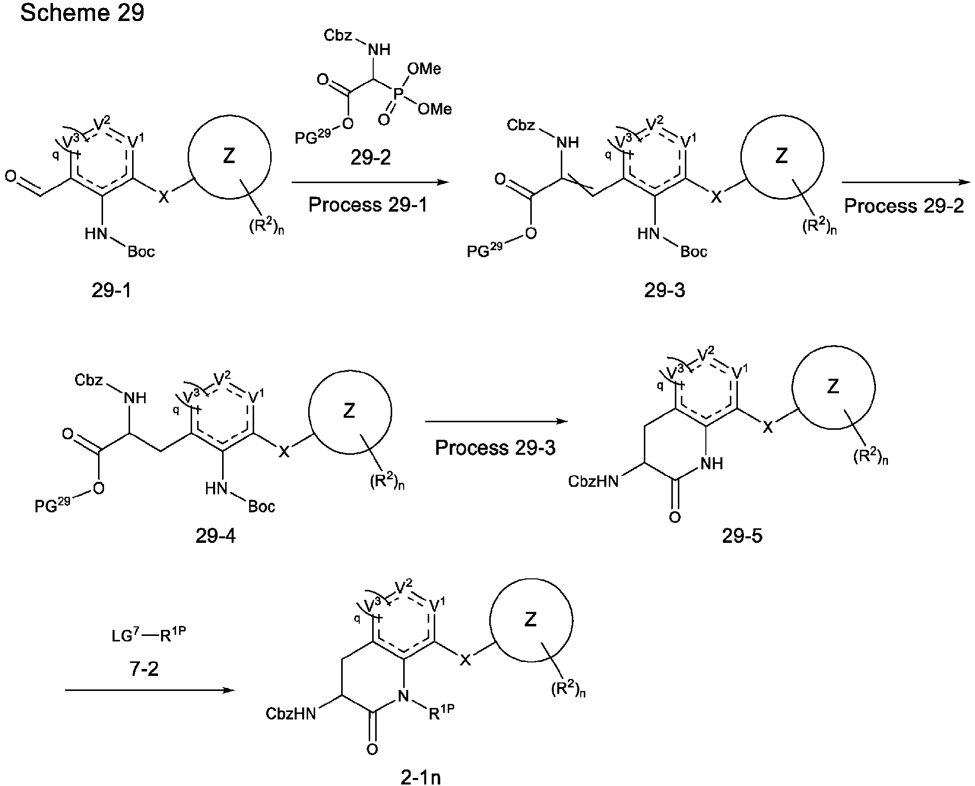

The symbols in the formulae have the same meanings as above. PG²⁹ is a protecting group.

### Process 29-1

Compound (29-3) can also be manufactured by a Horner-Wadsworth-Emmons reaction of Compound (29-1) and Compound (29-2).

### Process 29-2

Compound (29-4) can also be manufactured by catalytic reduction of Compound (29-3).

### Process 29-3

Compound (29-5) can also be manufactured by reacting Compound (29-4) with an acid.

### Process 29-4

Compound (2-1n) can also be manufactured by reacting Compound (29-5) with Compound (7-2) in the presence of a base.

The compound represented by Formula (29-1) can be manufactured, for example, according to the method described in Scheme 30. 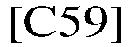 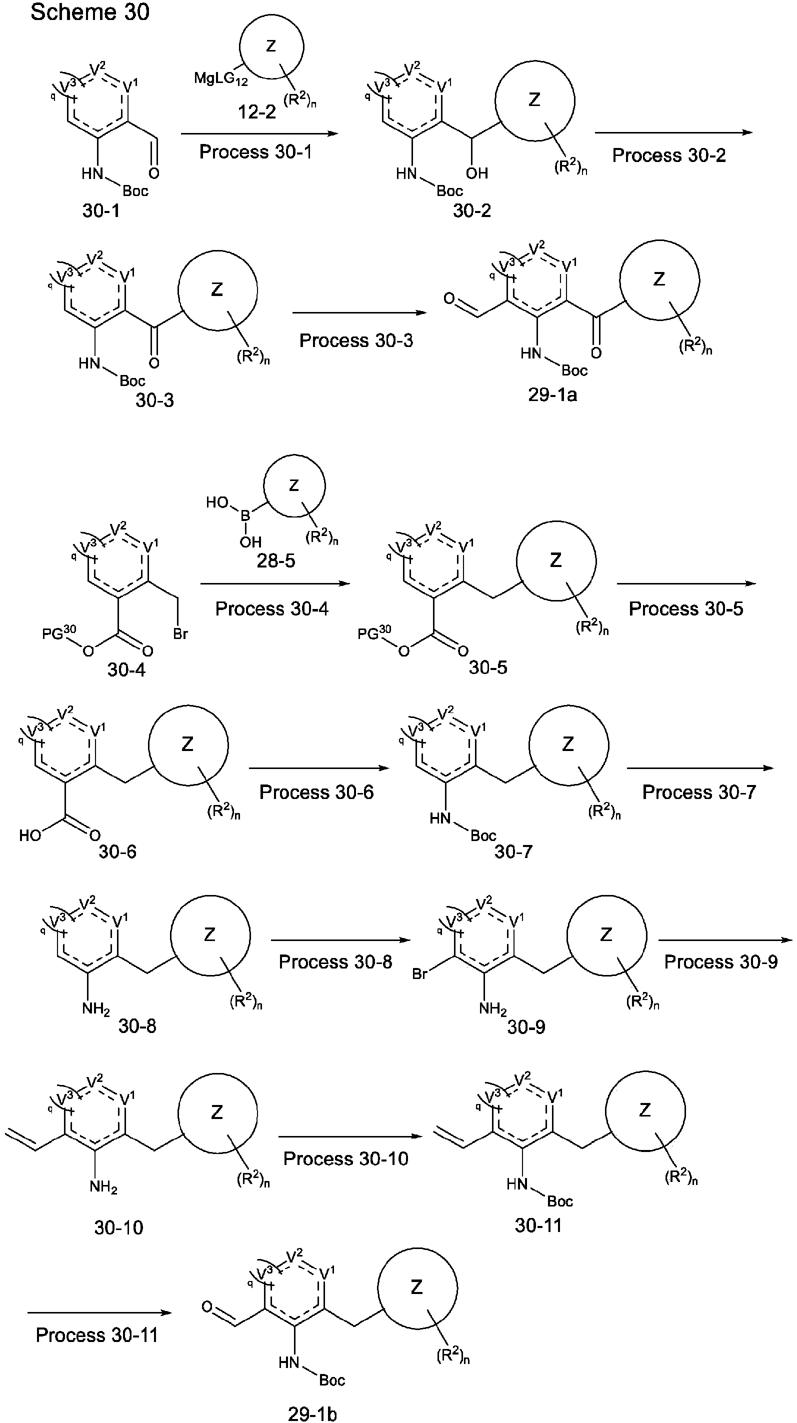 The symbols in the formulae have the same meanings as above. PG³⁰ is a protecting group.

### Process 30-1

Compound (30-2) can also be manufactured by reacting Compound (30-1) with Compound (12-2).

### Process 30-2

Compound (30-3) can also be manufactured by oxidation of Compound (30-2).

### Process 30-3

Compound (29-1a) can also be manufactured by reacting Compound (30-3) with DMF in the presence of a base.

### Process 30-4

Compound (30-5) can also be manufactured by a Suzuki-Miyaura cross-coupling reaction of Compound (30-4) and Compound (28-5).

### Process 30-5

Compound (30-6) can also be manufactured by hydrolysis of Compound (30-5).

### Process 30-6

Compound (30-7) can also be manufactured by a Curtius rearrangement reaction of Compound (30-6).

### Process 30-7

Compound (30-8) can also be manufactured by removing the Boc group of Compound (30-7).

### Process 30-8

Compound (30-9) can also be manufactured by bromination of Compound (30-8).

### Process 30-9

Compound (30-10) can also be manufactured by a Suzuki-Miyaura cross-coupling reaction of Compound (30-9) and potassium vinyltrifluoroborate.

### Process 30-10

Compound (30-11) can also be manufactured by converting the amino group of Compound (30-10) into a -NHBoc group.

### Process 30-11

Compound (29-1b) can also be manufactured by oxidative cleavage of Compound (30-11).

The compounds represented by Formulae (A-I) to (AIII) or Formulae (I) to (I-D3) and their intermediates during manufacturing can be isolated and purified as necessary by isolation and purification methods well known to those skilled in the art such as solvent extraction, crystallization, recrystallization, chromatography, and preparative high - performance liquid chromatography.

The compound of the present invention can be used as a therapeutic agent for thyroid-related diseases because it has excellent TSHR antagonist activity. In the present invention, the thyroid-related diseases include, for example, hyperthyroidism, Graves' disease, thyroid eye disease and thyroid cancer. Preferably, the compound of the present invention can be used as a therapeutic agent for hyperthyroidism, Graves' disease, or thyroid eye disease (refer to Endocrinology, 2014, 155 (1), pp. 310-314). More preferably, the compound of the present invention can be used as a therapeutic agent for hyperthyroidism or Graves' disease.

In addition, as one embodiment, hyperthyroidism includes, for example, hyperthyroidism caused by any of Graves' disease, thyroiditis, Plummer's disease, toxic multinodular goiter, TSH-producing pituitary adenoma, hyperemesis gravidarum, ovarian goiter, gestational trophoblastic tumor, and germ cell tumor. Preferably, the compound of the present invention can be used as a therapeutic agent for hyperthyroidism caused by Graves' disease.

In addition, as one embodiment, the thyroid-related diseases are diseases and symptoms associated with abnormalities in thyroid hormone levels. The diseases and symptoms associated with abnormalities in thyroid hormone levels include, for example, diseases and symptoms caused by TRAb.

In the present invention, "treatment" includes the meaning of "prevention." For example, the treatment of hyperthyroidism, Graves' disease or thyroid eye disease includes the meaning of "prevention of relapse/recurrence" and "maintenance of remission." In addition, as one embodiment, the compound of the present invention can be used to prevent the development of thyroid eye disease in patients with Graves' disease.

In the present invention, "antagonist" refers to a drug that inhibits or blocks the function of a target protein, regardless of its binding site. For example, "antagonist" includes the meaning of "allosteric antagonist" and "negative allosteric modulator (NAM)."

The therapeutic effect of the compound of the present invention on thyroid-related diseases can be confirmed according to methods well known in the related art. Examples of methods for confirming the effect of hyperthyroidism or Graves' disease in model animals include methods described in Endocrinology 2007, 148(5), pp. 2335-2344 or similar methods.

The pharmaceutical composition of the present invention may be used in various dosage forms depending on the usage. Examples of such dosage forms include powdered drugs, granules, fine granules, dry syrups, tablets, capsules, injections, liquid drugs, ointments, suppositories, patches, eye drops, and enema agents.

The pharmaceutical composition of the present invention includes a compound represented by Formulae (A-I) to (AIII) or Formulae (I) to (I-D3) or a pharmacologically acceptable salt thereof as an active ingredient.

The pharmaceutical composition of the present invention is prepared using a compound represented by Formulae (A-I) to (AIII) or Formulae (I) to (I-D3) or a pharmacologically acceptable salt thereof, and at least one pharmaceutical additive. These pharmaceutical compositions can also be prepared by being appropriately mixed, diluted or dissolved with appropriate pharmaceutical additives such as an excipient, a disintegrant, a binder, a lubricant, a diluent, a buffer, an isotonizing agent, a preservative, a wetting agent, an emulsifying agent, a dispersing agent, a stabilizer, and a solubilizing agent according to a pharmaceutically known method depending on a dosage form.

When the pharmaceutical composition of the present invention is used for treatment, the dosage of the compound represented by Formulae (A-I) to (AIII) or Formulae (I) to (I-D3) or a pharmacologically acceptable salt thereof is appropriately determined depending on the age, sex, weight, degree of disease and treatment of patients and the like. The daily dosage may be administered separately once, twice, three times or four times a day.

In the case of oral administration, the dosage for adults can be set, for example, in a range of 0.1 to 5,000 mg/day. As one embodiment, in the case of oral administration, the dosage can be set in a range of 1 to 1,500 mg/day, and preferably in a range of 1 to 500 mg/day.

In the case of parenteral administration, the dosage for adults can be set to, for example, 0.01 to 5,000 mg/day. As one embodiment, in the case of parenteral administration, the dosage can be set in a range of 0.1 to 1,500 mg/day, and preferably in a range of 0.1 to 500 mg/day.

As one embodiment, the pharmaceutical composition of the present invention can also be used in combination with drugs other than TSHR antagonist. Examples of other drugs that can be used in combination in the treatment of thyroid-related diseases include antithyroid drugs (for example, thiamazole, propylthiouracil, etc.), inorganic iodine, lithium carbonate, and thyroid hormone formulations.

When the compound represented by Formulae (A-I) to (AIII) or Formulae (I) to (I-D3) or a pharmacologically acceptable salt thereof is used in combination with other drugs, a formulation containing these active ingredients together or a formulation in which each of these active ingredients is separately formulated can be administered. When the components are formulated separately, the formulations can be administered separately or simultaneously. In addition, the dosage of the compound represented by Formulae (A-I) to (AIII) or Formulae (I) to (I-D3) or a pharmacologically acceptable salt thereof may be appropriately reduced depending on the dosage of other drugs used in combination.

The compounds represented by Formulae (A-I) to (AIII) or Formulae (I) to (I-D3) that are appropriately converted into prodrugs may be used. For example, the prodrugs of the compounds represented by Formulae (A-I) to (AIII) or Formulae (I) to (I-D3) can also be manufactured by introducing a prodrug-constituting group using a corresponding prodrug formation reagent such as a halide and performing purification. Examples of prodrug-constituting groups include groups described in "Development of Medicine" (Hirokawa Shoten, 1990), Vol. 7, pp. 163-198.

### [Examples]

Hereinafter, the present invention will be described in more detail with reference to reference examples, examples and test examples, but the present invention is not limited to the contents thereof.

The compounds described in the following examples, except for commercially available reagents, were named using ChemDraw Professional (PerkinElmer), MarvinSketch (ChemAxon) and the like. The stereo configuration of the chiral center marked with "*" in a compound name means that it is a relative configuration.

### Reference Example 1

### 1-bromo-2-nitro-3-phenoxybenzene

Potassium carbonate (10.1 g) was added to a mixture of 1-bromo-3-fluoro-2-nitrobenzene (4.01 g), phenol (1.89 g) and DMF (40 mL) at room temperature, and the mixture was stirred at 80°C for 6 hours. The reaction mixture was cooled to room temperature, water was then added, and the mixture was extracted twice with ethyl acetate. The combined extract was washed with water and then dried over anhydrous sodium sulfate, and concentrated under reduced pressure. The residue was purified by APS column chromatography (eluent: n-hexane/ethyl acetate=80/20 to 50/50) to give the title compound (5.58 g).

### Reference Example 2

### 2-bromo-6-phenoxyaniline

Iron powder (3.05 g) was added to a mixture of Reference Example 1 (5.58 g), acetic acid (43 mL) and ethanol (43 mL) at room temperature, and the mixture was stirred at 91°C for 11 hours. The reaction mixture was cooled to room temperature and then filtered through Celite. The filtrate was concentrated under reduced pressure. The residue was purified by silica gel column chromatography (eluent: n-hexane/ethyl acetate=100/0 to 94/6) to give the title compound (4.68 g).

### Reference Example 3

### ethyl (E)-3-(2-amino-3-phenoxyphenyl)acrylate

A mixture of Reference Example 2 (1.11 g), ethyl acrylate (1.25 g), palladium(II) acetate (0.093 g), tris(2-methylphenyl)phosphine (0.255 g), TEA (2.11 g) and MeCN (21 mL) was stirred at 130°C for 1 hour under microwave irradiation. The reaction mixture was poured into water, and the mixture was extracted with DCM. The extract was dried over anhydrous sodium sulfate and concentrated under reduced pressure. The residue was purified by silica gel column chromatography (eluent: n-hexane/ethyl acetate=100/0 to 86/14) to give the title compound (1.08 g).

¹H-NMR (CDCl₃)δ ppm:1.35 (3H, t, J=7.2 Hz), 4.21 (2H, br), 4.28 (2H, q, J=7.2 Hz), 6.40 (1H, d, J=16.0 Hz), 6.66-6.76 (1H, m), 6.86 (1H, dd, J=1.3, 7.8 Hz), 6.96-7.02 (2H, m), 7.06-7.13 (1H, m), 7.21 (1H, dd, J=1.3, 8.0 Hz), 7.29-7.37 (2H, m), 7.85 (1H, d, J=16.0 Hz)

### Reference Example 4

### 8-phenoxy-3,4-dihydroquinolin-2(1H)-one

Under an argon atmosphere, 10% Pd/C (0.199 g) was added to a mixture of Reference Example 3 (1.07 g) and methanol (15 mL) at room temperature, and the mixture was stirred at the same temperature for 7.5 hours under a hydrogen atmosphere. The reaction mixture was filtered through Celite, and the filtrate was concentrated under reduced pressure.

A mixture of the residue and ethanol (15 mL) was stirred at 83°C for 15 hours. Lithium hydroxide monohydrate (0.159 g) was added to the reaction mixture, and the mixture was stirred at 83°C for 40 minutes. The reaction mixture was cooled to room temperature and then concentrated under reduced pressure. Ethyl acetate and water were added to the residue, and the aqueous layer was separated. 2 mol/L hydrochloric acid was added to the aqueous layer, and the mixture was extracted with ethyl acetate. The combined organic layer was dried over anhydrous sodium sulfate and concentrated under reduced pressure. The residue was purified by silica gel column chromatography (eluent: n-hexane/ethyl acetate=90/10 to 55/45) to give the title compound (0.803 g).

### Reference Example 5

### 6-bromo-1-methyl-8-phenoxy-3,4-dihydroquinolin-2(1H)-one

NBS (0.096 g) was added to a mixture of Reference Example 4 (0.108 g) and DMF (3.0 mL) at 50°C, and the mixture was stirred at the same temperature for 1 hour. After the reaction mixture was cooled to room temperature, a saturated sodium sulfite aqueous solution was added, and the mixture was extracted with ethyl acetate. The extract was washed with water, then dried over anhydrous magnesium sulfate and concentrated under reduced pressure.

Methyl iodide (0.128 g) and sodium hydride (about 60%) (0.023 g) were added to a mixture of the residue and DMF (3.0 mL) at room temperature, and the mixture was stirred at the same temperature for 30 minutes. Ice was added to the reaction mixture, and the mixture was stirred at room temperature for 10 minutes. The mixture was extracted with ethyl acetate. The extract was washed with water, then dried over anhydrous magnesium sulfate and concentrated under reduced pressure. The residue was purified by silica gel column chromatography (eluent: n-hexane/ethyl acetate=74/26 to 45/55) to give the title compound (0.147 g).

¹H-NMR (CDCl₃)δ ppm:2.55-2.67 (2H, m), 2.82-2.93 (2H, m), 3.36 (3H, s), 6.85-7.04 (3H, m), 7.05-7.19 (2H, m), 7.28-7.41 (2H, m)

### Reference Example 6

### 6-((diphenylmethylene)amino)-1-methyl-8-phenoxy-3,4-dihydroquinolin-2(1H)-one

Under an argon atmosphere, a mixture of Reference Example 5 (0.104 g), benzophenone imine (0.085 g), tris(dibenzylideneacetone)dipalladium(0) (0.029 g), Xantphos (0.036 g), sodium tert-butoxide (0.060 g) and toluene (3.0 mL) was stirred at 90°C for 1 hour. The reaction mixture was cooled to room temperature, water was then added, and the mixture was extracted with ethyl acetate. The extract was dried over anhydrous magnesium sulfate and concentrated under reduced pressure. The residue was purified by silica gel column chromatography (eluent: n-hexane/ethyl acetate=65/35 to 26/74) to give the title compound (0.122 g).

### Reference Example 7

### tert-butyl 6-((diphenylmethylene)amino)-1-methyl-2-oxo-8-phenoxy-1,2,3,4-tetrahydroquinoline-3-car boxylate

LDA (1.09 mol/L in THF/n-hexane) (0.171 mL) was added to a mixture of Reference Example 6 (0.054 g) and THF (1.0 mL) at -78°C, and the mixture was stirred at the same temperature for 30 minutes. A mixture of Boc₂O (0.054 g) and THF (0.3 mL) was added to the reaction mixture at -78°C, and the mixture was stirred at the same temperature for 15 minutes and under ice cooling for 30 minutes. A saturated ammonium chloride aqueous solution was added to the reaction mixture, and the mixture was extracted with ethyl acetate. The extract was concentrated under reduced pressure. The residue was purified by silica gel column chromatography (eluent: n-hexane/ethyl acetate=80/20 to 51/49) to give the title compound (0.039 g).

### Reference Example 8

### 6-amino-tert-butyl 1-methyl-2-oxo-8-phenoxy-1,2,3,4-tetrahydroquinoline-3-carboxylate

Hydroxylammonium chloride (0.015 g) and sodium acetate (0.015 g) were added to a mixture of Reference Example 7 (0.039 g) and methanol (1.0 mL) at room temperature, and the mixture was stirred at the same temperature for 30 minutes. Water was added to the reaction mixture, and the mixture was extracted with ethyl acetate. The extract was concentrated under reduced pressure. The residue was purified by silica gel column chromatography (eluent: n-hexane/ethyl acetate=70/30 to 0/100) to give the title compound (0.027 g).

### Reference Example 9

### tert-butyl 2-(6-((diphenylmethylene)amino)-1-methyl-2-oxo-8-phenoxy-1,2,3,4-tetrahydroquinoline-3-yl)acetate

Reference Example 9 was synthesized in the same method as in Reference Example 7 except that tert-butyl bromoacetate was used in place of Boc₂O.

### Reference Example 10

### tert-butyl 2-(6-amino-1-methyl-2-oxo-8-phenoxy-1,2,3,4-tetrahydroquinoline-3-yl)acetate

Reference Example 10 was synthesized in the same method as in Reference Example 8 except that Reference Example 9 was used in place of Reference Example 7.

### Reference Example 11

### 6-nitro-2-oxo-1,2,3,4-tetrahydroquinoline-8-yl acetate

A mixture of 8-hydroxy-3,4-dihydroquinoline-2(1H)-one (2.98 g), acetic anhydride (15 mL) and concentrated sulfuric acid (0.090 g) was stirred at 80°C for 2 hours. Acetic acid (15 mL) and concentrated nitric acid (1.5 mL) were added to the reaction mixture under ice cooling, and the mixture was stirred under ice cooling for 10 minutes and at room temperature for 1 hour. Water was added to the reaction mixture under ice cooling, and the mixture was stirred for 10 minutes under ice cooling. Insoluble substances were collected by filtration, and the obtained solid was washed with water and then dried under reduced pressure to give the title compound (4.57 g).

### Reference Example 12

### 8-hydroxy-6-nitro-3,4-dihydroquinolin-2(1H)-one

Potassium carbonate (3.03 g) was added to a mixture of Reference Example 11 (4.57 g), methanol (48 mL) and water (9.0 mL) at room temperature, and the mixture was stirred at the same temperature for 30 minutes. The reaction mixture was concentrated under reduced pressure. Water, 1 mol/L hydrochloric acid (36.5 mL) and methanol (3.0 mL) were added to the residue, and the mixture was stirred at room temperature for 30 minutes. Insoluble substances were collected by filtration, and the obtained solid was washed with water and then dried under reduced pressure to give the title compound (3.40 g).

### Reference Example 13

### 8-((tert-Butyldimethylsilyl)oxy)-6-nitro-3,4-dihydroquinolin-2(1H)-one

Imidazole (1.31 g) and TBSCl (1.74 g) were added to a mixture of Reference Example 12 (2.0 g) and DMF (20 mL) under ice cooling, and the mixture was stirred at room temperature for 2 hours. TBSCl (0.724 g) was added to the reaction mixture at room temperature, and the mixture was stirred at the same temperature for 1 hour and at 40°C for 1 hour. After the reaction mixture was cooled to room temperature, a saturated ammonium chloride aqueous solution and water were added, and the mixture was stirred at room temperature for 10 minutes. Insoluble substances were collected by filtration, and the obtained solid was washed with water and n-hexane and then dried under reduced pressure to give the title compound (2.10 g).

### Reference Example 14

### 8-hydroxy-1-methyl-6-nitro-3,4-dihydroquinolin-2(1H)-one

Sodium hydride (about 60%) (0.714 g) was added to a mixture of Reference Example 13 (2.88 g), methyl iodide (1.90 g) and DMF (30 mL) under ice cooling, and the mixture was stirred at room temperature for 1 hour. A saturated ammonium chloride aqueous solution and water were added to the reaction mixture under ice cooling, and the mixture was extracted with ethyl acetate. The extract was washed with water and a saturated saline solution, then dried over anhydrous sodium sulfate, and concentrated under reduced pressure. The residue was suspended in n-hexane/ethyl acetate (3/1), and insoluble substances were collected by filtration. The obtained solid was dried under reduced pressure to give the title compound (1.28 g).

### Reference Example 15

### 6-amino-8-hydroxy-1-methyl-3,4-dihydroquinolin-2(1H)-one

Nickel bromide(II) (0.126 g) and sodium borohydride (0.654 g) were added to a mixture of Reference Example 14 (1.28 g), THF (25 mL) and methanol (25 mL) under ice cooling, and the mixture was stirred under ice cooling for 1 hour. A saturated ammonium chloride aqueous solution, water and ethyl acetate were added to the reaction mixture, and the mixture was concentrated under reduced pressure. The residue was purified by silica gel column chromatography (eluent: n-hexane/ethyl acetate/methanol=48.5/48.5/3 to 0/97/3) to give the title compound (0.950 g).

### Reference Example 16

### 6-(2,5-dimethyl-1H-pyrrol-1-yl)-8-hydroxy-1-methyl-3,4-dihydroquinolin-2(1H)-one

Using a device including a Dean-Stark tube, a mixture of Reference Example 15 (0.950 g), acetonylacetone (0.733 g), p-toluenesulfonic acid monohydrate (0.470 g) and toluene (50 mL) was refluxed for 2 hours. The reaction mixture was concentrated under reduced pressure. The residue was purified by silica gel column chromatography (eluent: n-hexane/ethyl acetate=70/30 to 0/100) to give the title compound (0.610 g).

### Reference Example 17

### 2-((6-(2,5-dimethyl-1H-pyrrol-1-yl)-1-methyl-2-oxo-1,2,3,4-tetrahydroquinoline-8-yl)oxy)be nzonitrile

Potassium carbonate (0.460 g) was added to a mixture of Reference Example 16 (0.300 g), 2-fluorobenzonitrile (0.202 g) and NMP (3.0 mL) at room temperature, and the mixture was stirred at 100°C for 10 hours. After the reaction mixture was cooled to room temperature, water and ethyl acetate were added, and the mixture was extracted with n-hexane/ethyl acetate (1/3). The extract was washed with water and a saturated saline solution, then dried over anhydrous sodium sulfate, and concentrated under reduced pressure. The residue was purified by Method A (eluent: n-hexane/ethyl acetate=80/20 to 30/70) to give the title compound (0.300 g).

### Reference Example 18

### tert-butyl 2-(8-(2-cyanophenoxy)-6-(2,5-dimethyl-1H-pyrrol-1-yl)-1-methyl-2-oxo-1,2,3,4-tetrahydroq uinoline-3-yl)acetate

Reference Example 18 was synthesized in the same method as in Reference Example 7 except that Reference Example 17 was used in place of Reference Example 6 and tert-butyl bromoacetate was used in place of Boc₂O.

### Reference Example 19

### ethyl 2-(6-amino-8-(2-cyanophenoxy)-1-methyl-2-oxo-1,2,3,4-tetrahydroquinoline-3-yl)acetate

A mixture of Reference Example 18 (0.140 g), concentrated hydrochloric acid (0.481 mL) and ethanol (3.0 mL) was refluxed for 1 hour. Hydroxylamine hydrochloride (0.200 g) was added to the reaction mixture, and the mixture was refluxed for 1 hour. A 2 mol/L sodium hydroxide aqueous solution (4.5 mL) was added to the reaction mixture under ice cooling, and the mixture was extracted with ethyl acetate. The extract was washed with a saturated saline solution, then dried over anhydrous sodium sulfate, and concentrated under reduced pressure. The residue was purified by APS column chromatography (eluent: n-hexane/ethyl acetate=80/20 to 20/80) to give the title compound (0.060 g).

### Reference Example 20

### tert-butyl (8-(2-cyanophenoxy)-6-(2,5-dimethyl-1H-pyrrol-1-yl)-1-methyl-2-oxo-1,2,3,4-tetrahydroqui noline-3-yl)carbamate

LDA (1.08 mol/L in THF/n-hexane) (1.56 mL) was added to a mixture of Reference Example 17 (0.250 g) and THF (2.0 mL) at -78°C, and the mixture was stirred at the same temperature for 1 hour. A mixture of DPPA (0.463 g) and THF (1.0 mL) was added to the reaction mixture at -78°C, and the mixture was stirred at the same temperature for 30 minutes. A mixture of Boc₂O (0.294 g) and THF (1.0 mL) was added to the reaction mixture at -78°C, and the mixture was stirred at the same temperature for 30 minutes and under ice cooling for 1 hour. Water was added to the reaction mixture, and the mixture was stirred at 50°C for 1 hour. After the reaction mixture was cooled to room temperature, a saturated ammonium chloride aqueous solution was added, and the mixture was extracted with ethyl acetate. The extract was washed with a saturated saline solution, then dried over anhydrous sodium sulfate, and concentrated under reduced pressure. The residue was purified by Method A (eluent: n-hexane/ethyl acetate=90/10 to 50/50) to give the title compound (0.130 g).

### Reference Example 21

### tert-butyl (6-amino-8-(2-cyanophenoxy)-1-methyl-2-oxo-1,2,3,4-tetrahydroquinoline-3-yl)carbamate

A mixture of Reference Example 20 (0.120 g), hydroxylamine hydrochloride (0.171 g), ethanol (1.0 mL) and water (0.5 mL) was stirred at 100°C for 4 hours. Hydroxylamine hydrochloride (0.514 g) and water (0.5 mL) were added to the reaction mixture, and the mixture was stirred at 100°C for 8 hours. A 2 mol/L sodium hydroxide aqueous solution (5.0 mL) was added to the reaction mixture under ice cooling, and the mixture was extracted with ethyl acetate. The extract was washed with a saturated saline solution, then dried over anhydrous sodium sulfate, and concentrated under reduced pressure.

Boc₂O (0.081 g) was added to a mixture of the residue and THF (2.0 mL) at room temperature, and the mixture was stirred at the same temperature for 30 minutes. Methanol was added to the reaction mixture, and the mixture was concentrated under reduced pressure. The residue was purified by APS column chromatography (eluent: n-hexane/ethyl acetate=80/20 to 20/80) to give the title compound (0.070 g).

### Reference Example 22

### 6-(2,5-dimethyl-1H-pyrrol-1-yl)-1-methyl-8-((6-(trifluoromethyl)pyridin-2-yl)oxy)-3,4-dihyd roquinolin-2(1H)-one

Reference Example 22 was synthesized in the same method as in Reference Example 17 except that 2-chloro-6-(trifluoromethyl)pyridine was used in place of 2-fluorobenzonitrile.

### Reference Example 23

### tert-butyl (6-(2,5-dimethyl-1H-pyrrol-1-yl)-1-methyl-2-oxo-8-((6-(trifluoromethyl)pyridin-2-yl)oxy)-1, 2,3,4-tetrahydroquinoline-3-yl)carbamate

Reference Example 23 was synthesized in the same method as in Reference Example 20 except that Reference Example 22 was used in place of Reference Example 17.

### Reference Example 24

### tert-butyl (6-amino-1-methyl-2-oxo-8-((6-(trifluoromethyl)pyridin-2-yl)oxy)-1,2,3,4-tetrahydroquinolin e-3-yl)carbamate

Reference Example 24 was synthesized in the same method as in Reference Example 21 except that Reference Example 23 was used in place of Reference Example 20.

### Reference Example 25

### 1-methyl-8-phenoxy-3,4-dihydroquinolin-2(1H)-one

Sodium hydride (about 60%) (0.037 g) was added to a mixture of Reference Example 4 (0.170 g), methyl iodide (0.202 g) and THF (2.0 mL) under ice cooling, and the mixture was stirred at room temperature for 1 hour. Ice water was added to the reaction mixture, and the mixture was extracted with ethyl acetate. The extract was concentrated under reduced pressure. The residue was purified by silica gel column chromatography (eluent: n-hexane/ethyl acetate=74/26 to 57/43) to give the title compound (0.178 g).

### Reference Example 26

### tert-butyl 1-methyl-2-oxo-8-phenoxy-1,2,3,4-tetrahydroquinoline-3-carboxylate

Reference Example 26 was synthesized in the same method as in Reference Example 7 except that Reference Example 25 was used in place of Reference Example 6.

### Reference Example 27

### 1-methyl-2-oxo-8-phenoxy-1,2,3,4-tetrahydroquinoline-3-carboxylic acid

A mixture of Reference Example 26 (0.237 g) and hydrogen chloride (4 mol/L in 1,4-dioxane) (2.0 mL) was stirred at room temperature for 30 minutes and at 50°C for 2 hours. The reaction mixture was cooled to room temperature and then concentrated under reduced pressure to give the title compound (0.216 g).

### Reference Example 28

### 3-(hydroxymethyl)-1-methyl-8-phenoxy-3,4-dihydroquinolin-2(1H)-one

Isobutyl chloroformate (0.025 g) was added to a mixture of Reference Example 27 (0.050 g), N-methylmorpholine (0.020 g) and THF (1.0 mL) under ice cooling, and the mixture was stirred at room temperature for 30 minutes. The reaction mixture was filtered, sodium borohydride (0.013 g) was added to the filtrate under ice cooling, and the mixture was stirred at room temperature for 1 hour. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate=100/0 to 0/100) to give the title compound (0.025 g).

### Reference Example 29

### methyl (1-methyl-2-oxo-8-phenoxy-1,2,3,4-tetrahydroquinoline-3-yl)methanesulfonate

Methanesulfonyl chloride (0.012 g) was added to a mixture of Reference Example 28 (0.025 g), TEA (0.012 g) and THF (1.0 mL) under ice cooling, and the mixture was stirred under ice cooling for 30 minutes. A saturated ammonium chloride aqueous solution was added to the reaction mixture, and the mixture was extracted with MTBE. The extract was washed with water, then dried over anhydrous magnesium sulfate and concentrated under reduced pressure. The residue was purified by silica gel column chromatography (eluent: n-hexane/ethyl acetate=90/10 to 0/100) to give the title compound (0.008 g).

### Reference Example 30

### 3-(aminomethyl)-1-methyl-8-phenoxy-3,4-dihydroquinolin-2(1H)-one

A mixture of Reference Example 29 (0.008 g), sodium azide (0.007 g) and DMF (1.0 mL) was stirred at 50°C for 3 hours. The reaction mixture was cooled to room temperature, water was then added, and the mixture was extracted with MTBE. The extract was washed with water, then dried over anhydrous magnesium sulfate, and concentrated under reduced pressure to give 3-(azidomethyl)-1-methyl-8-phenoxy-3,4-dihydroquinolin-2(1H)-one (0.008 g).

A mixture of the obtained Compound (0.006 g), 10% Pd/C (0.010 g) and methanol (1.0 mL) was stirred under a hydrogen atmosphere at room temperature for 1 hour. The reaction mixture was filtered through Celite, and the filtrate was concentrated under reduced pressure to give the title compound (0.006 g).

### Reference Example 31

### (2-amino-3-bromophenyl) (2-chlorophenyl)methanol

Isopropyl magnesium chloride (2 mol/L in THF) (1.43 mL) was added to a mixture of 1-chloro-2-iodobenzene (0.680 g) and THF (3.0 mL) at -40°C, and the mixture was stirred under ice cooling for 30 minutes. A mixture of 3-bromo-2-nitrobenzaldehyde (0.328 g) and THF (3.0 mL) was added to the reaction mixture at -40°C, and the mixture was stirred at the same temperature for 10 minutes and under ice cooling for 1 hour. A saturated ammonium chloride aqueous solution and water were added to the reaction mixture, and the mixture was extracted with ethyl acetate. The extract was washed with a saturated saline solution, then dried over anhydrous magnesium sulfate, and concentrated under reduced pressure.

Iron powder (0.399 g) and ammonium chloride (1.91 g) were added to a mixture of the residue, ethanol (3.0 mL) and water (1.0 mL), and the mixture was stirred at 80°C for 1.5 hours. The reaction mixture was cooled to room temperature, and then filtered through Celite, and the organic layer of the filtrate was separated. The organic layer was washed with water and a saturated saline solution, then dried over anhydrous magnesium sulfate and concentrated under reduced pressure. The residue was purified by silica gel column chromatography (eluent: n-hexane/ethyl acetate=100/0 to 65/35) to give the title compound (0.375 g).

### Reference Example 32

### (2-amino-3-bromophenyl) (2-chlorophenyl)methanone

Manganese(IV) dioxide (0.278 g) was added to a mixture of Reference Example 31 (0.200 g) and DCM (5.0 mL) at room temperature, and the mixture was stirred at 50°C for 2 hours and at room temperature for 10 hours. The reaction mixture was filtered through Celite, and the filtrate was concentrated under reduced pressure. The residue was purified by silica gel column chromatography (eluent: n-hexane/ethyl acetate=100/0 to 75/25) to give the title compound (0.171 g).

### Reference Example 33

### (3-bromo-2-(methylamino)phenyl) (2-chlorophenyl)methanone

Pyridine (0.057 g) and trifluoroacetic anhydride (0.150 g) were added to a mixture of Reference Example 32 (0.171 g) and ethyl acetate (2.0 mL) under ice cooling, and the mixture was stirred at room temperature for 30 minutes. Pyridine (0.057 g) and trifluoroacetic anhydride (0.150 g) were added to the reaction mixture, and the mixture was stirred at room temperature for 30 minutes. Pyridine (0.057 g) and trifluoroacetic anhydride (0.150 g) were added to the reaction mixture, and the mixture was stirred at room temperature for 30 minutes. Water was added to the reaction mixture, and the mixture was extracted with ethyl acetate. The extract was washed with a saturated saline solution, then dried over anhydrous magnesium sulfate, and concentrated under reduced pressure.

Methyl iodide (0.117 g) and potassium carbonate (0.152 g) were added to a mixture of the residue and DMF (1.5 mL) at room temperature, and the mixture was stirred at the same temperature for 1 hour. Water was added to the reaction mixture, and the mixture was extracted with ethyl acetate. The extract was washed with a saturated saline solution, then dried over anhydrous magnesium sulfate, and concentrated under reduced pressure.

A 5 mol/L sodium hydroxide aqueous solution (0.220 mL) was added to a mixture of the residue, methanol (1.5 mL) and THF (1.5 mL) at room temperature, and the mixture was stirred at the same temperature for 1 hour. Water was added to the reaction mixture, and the mixture was extracted with ethyl acetate. The extract was washed with a saturated saline solution, then dried over anhydrous magnesium sulfate, and concentrated under reduced pressure. The residue was purified by silica gel column chromatography (eluent: n-hexane/ethyl acetate=100/0 to 85/15) to give the title compound (0.148 g).

### Reference Example 34

### (R)-3-amino-8-(2-chlorobenzoyl)-1-methyl-3,4-dihydroquinolin-2(1H)-one

A mixture of methyl (S)-2-((tert-butoxycarbonyl)amino)-3-iodopropanoate (0.225 g) and THF (0.3 mL) was added to a mixture of zinc powder (0.066 g) and DMF (0.6 mL) under ice cooling, and the mixture was stirred at room temperature for 1 hour. A mixture of Reference Example 33 (0.148 g) and THF (0.6 mL), and palladium(II) acetate (0.005 g) and Xphos (0.022 g) were added to the reaction mixture, and the mixture was stirred under an argon atmosphere at 40°C for 2 hours and at room temperature overnight. A saturated ammonium chloride aqueous solution, water and n-hexane/ethyl acetate (1/1) were added to the reaction mixture, and the mixture was stirred at room temperature for 10 minutes. The mixture was filtered through Celite, and the organic layer of the filtrate was separated. The organic layer was washed with water and then concentrated under reduced pressure.

Methanesulfonic acid (0.131 g) was added to a mixture of the residue, ethanol (0.45 mL) and toluene (0.7 mL) at room temperature, and the mixture was stirred at 70°C for 3 hours. After the reaction mixture was cooled to room temperature, ethyl acetate and water were added, and the aqueous layer was separated. A saturated sodium bicarbonate aqueous solution was added to the aqueous layer, and the mixture was extracted with ethyl acetate. The extract was washed with a saturated saline solution, then dried over anhydrous magnesium sulfate, and concentrated under reduced pressure to give the title compound (0.050 g).

### Reference Example 35

### 1-(2-amino-3-bromophenyl)-1-(2-chlorophenyl)ethan-1-ol

Under an argon atmosphere, methylmagnesium bromide (3 mol/L in Et₂O) (0.521 mL) was added to a mixture of Reference Example 32 (0.231 g) and Et₂O (2.5 mL) under ice cooling, and the mixture was stirred under ice cooling for 15 minutes and at room temperature for 1 hour. A saturated ammonium chloride aqueous solution and water were added to the reaction mixture under ice cooling, and the mixture was extracted with DCM. The extract was concentrated under reduced pressure. The residue was purified by silica gel column chromatography (eluent: n-hexane/ethyl acetate=98/2 to 85/15) to give the title compound (0.247 g).

### Reference Example 36

### 2-bromo-6-(1-(2-chlorophenyl)vinyl)aniline

BF₃-Et₂O (0.127 g) and triethylsilane (0.174 g) were added to a mixture of Reference Example 35 (0.195 g) and DCE (3.0 mL) at room temperature, and the mixture was stirred at 70°C for 2 hours and at 50°C for 15 hours. A 5 mol/L sodium hydroxide aqueous solution (0.180 mL) was added to the reaction mixture under ice cooling, and the mixture was extracted with DCM. The extract was concentrated under reduced pressure. The residue was purified by silica gel column chromatography (eluent: n-hexane/ethyl acetate=98/2 to 85/15) to give the title compound (0.171 g).

### Reference Example 37

### 2-bromo-6-(1-(2-chlorophenyl)vinyl)-N-methylaniline

Methyllithium (1.04 mol/L in Et₂O) (0.585 mL) was added to a mixture of Reference Example 36 (0.171 g) and THF (1.7 mL) at -78°C, and the mixture was stirred at the same temperature for 30 minutes. Methyl iodide (0.094 g) was added to the reaction mixture at -78°C, and the mixture was stirred at the same temperature for 10 minutes and under ice cooling for 1.5 hours. A saturated ammonium chloride aqueous solution and water were added to the reaction mixture, and the mixture was extracted with DCM. The extract was concentrated under reduced pressure. The residue was purified by silica gel column chromatography (eluent: n-hexane/ethyl acetate=98/2 to 85/15) to give the title compound (0.168 g).

### Reference Example 38

### (R)-3-amino-8-(1-(2-chlorophenyl)vinyl)-1-methyl-3,4-dihydroquinolin-2(1H)-one

Under an argon atmosphere, a mixture of methyl (S)-2-((tert-butoxycarbonyl)amino)-3-iodopropanoate (0.257 g) and DMF (0.35 mL) was added to a mixture of zinc powder (0.075 g) and DMF (0.7 mL), and the mixture was stirred at room temperature for 1 hour. A mixture of Reference Example 37 (0.168 g) and DMF (0.7 mL), and palladium(II) acetate (0.006 g) and Xphos (0.025 g) were added to the reaction mixture at room temperature, and the mixture was stirred at 40°C for 2 hours and at room temperature for 15 hours. A saturated ammonium chloride aqueous solution, water and n-hexane/ethyl acetate (1/1) were added to the reaction mixture under ice cooling, and the mixture was stirred at room temperature for 10 minutes. The mixture was filtered through Celite, and the organic layer of the filtrate was separated. The organic layer was washed with water and a saturated saline solution, then dried over anhydrous magnesium sulfate and concentrated under reduced pressure.

Methanesulfonic acid (0.104 g) was added to a mixture of the residue, ethanol (0.6 mL) and toluene (0.8 mL) at room temperature, and the mixture was stirred at 80°C for 30 minutes. A 5 mol/L sodium hydroxide aqueous solution (0.217 mL) was added to the reaction mixture under ice cooling, and the mixture was extracted with DCM. The extract was concentrated under reduced pressure. The residue was purified by Method A (eluent: ethyl acetate/methanol=98/2 to 80/20) to give the title compound (0.024 g).

### Reference Example 39

### 4-chloro-2-(2-phenylpropan-2-yl)aniline

Trifluoromethanesulfonic acid (0.151 g) was added to a mixture of 4-chloroaniline (0.640 g) and prop-1-en-2-ylbenzene (0.593 g), and the mixture was stirred at 160°C for 8 hours. A saturated sodium bicarbonate aqueous solution was added to the reaction mixture under ice cooling, and the mixture was extracted with DCM. The extract was concentrated under reduced pressure. The residue was purified by silica gel column chromatography (eluent: n-hexane/ethyl acetate=98/2 to 80/20) to give the title compound (0.750 g).

### Reference Example 40

### 2-bromo-4-chloro-6-(2-phenylpropan-2-yl)aniline

NBS (0.652 g) was added to a mixture of Reference Example 39 (0.750 g) and DMF (7.0 mL) under ice cooling, and the mixture was stirred under ice cooling for 1 hour. A saturated sodium bicarbonate aqueous solution, a 1 mol/L sodium thiosulfate aqueous solution and n-hexane were added to the reaction mixture, and the mixture was extracted with ethyl acetate. The extract was washed with a saturated saline solution, then dried over anhydrous sodium sulfate, and concentrated under reduced pressure. The residue was purified by silica gel column chromatography (eluent: n-hexane/ethyl acetate=98/2 to 90/10) to give the title compound (0.640 g).

### Reference Example 41

### 2-bromo-4-chloro-N-methyl-6-(2-phenylpropan-2-yl)aniline

Reference Example 41 was synthesized in the same method as in Reference Example 37 except that Reference Example 40 was used in place of Reference Example 36.

### Reference Example 42

### benzyl (E)-3-(5-chloro-2-(methylamino)-3-(2-phenylpropan-2-yl)phenyl)acrylate

Palladium(II) acetate (0.023 g) was added to a mixture of Reference Example 41 (0.340 g), tris(2-methylphenyl)phosphine (0.061 g), TEA (0.406 g), benzyl acrylate (0.326 g) and MeCN (3.0 mL), and the mixture was stirred at 130°C for 1 hour under microwave irradiation. A saturated ammonium chloride aqueous solution was added to the reaction mixture, and the mixture was extracted with DCM. The extract was concentrated under reduced pressure. The residue was purified by Method A (eluent: n-hexane/ethyl acetate=100/0 to 90/10) to give the title compound (0.240 g).

### ¹H-NMR (CDCl₃)δ ppm:1.64 (6H, s), 2.19 (3H, d, J=5.6 Hz), 3.01 (1H, q, J=5.6 Hz), 5.21 (2H, s), 6.33 (1H, d, J=16.0 Hz), 7.17-7.51 (12H, m), 7.79 (1H, d, J=16.0 Hz)

### Reference Example 43

### 1-methyl-8-(2-phenylpropan-2-yl)-3,4-dihydroquinolin-2(1H)-one

10% Pd/C (0.100 g) was added to a mixture of Reference Example 42 (0.240 g), THF (3.0 mL) and methanol (5.0 mL), and the mixture was stirred under a hydrogen atmosphere at 50°C for 2 hours. The reaction mixture was cooled to room temperature and then filtered through Celite, and the filtrate was concentrated under reduced pressure.

DIPEA (0.222 g) was added to a mixture of the residue, HATU (0.326 g) and DMF (3.0 mL) at room temperature, and the mixture was stirred at the same temperature for 12 hours. A saturated ammonium chloride aqueous solution and n-hexane were added to the reaction mixture, and the mixture was extracted with ethyl acetate. The extract was washed with water and a saturated saline solution, then dried over anhydrous sodium sulfate, and concentrated under reduced pressure. The residue was purified by Method A (eluent: n-hexane/ethyl acetate=95/5 to 70/30) to give the title compound (0.110 g).

### Reference Example 44

### 6-chloro-2,2,4-trimethyl-1,2-dihydroquinoline

Bismuth(III) trifluoromethanesulfonate (1.20 g) was added to a mixture of 4-chloroaniline (2.00 g) and acetone (20 mL), and the mixture was refluxed overnight. 1 mol/L hydrochloric acid and ethyl acetate were added to the reaction mixture, and the mixture was filtered through Celite. A saturated saline solution was added to the filtrate, and the organic layer was separated. The organic layer was dried over anhydrous magnesium sulfate and concentrated under reduced pressure. The residue was purified by Method A (eluent: n-hexane/ethyl acetate=100/0 to 95/5) to give the title compound (1.02 g).

### Reference Example 45

### 6-chloro-2,2,4-trimethyl-4-phenyl-1,2,3,4-tetrahydroquinoline

Aluminum(III) chloride (1.96 g) was added to a mixture of Reference Example 44 (1.02 g) and benzene (15 mL) under ice cooling, and the mixture was stirred at room temperature for 1 hour and at 60°C overnight. A saturated ammonium chloride aqueous solution was added to the reaction mixture, and the mixture was extracted with ethyl acetate. The extract was washed with water and a saturated saline solution, then dried over anhydrous magnesium sulfate, and concentrated under reduced pressure. The residue was purified by Method A (eluent: n-hexane/ethyl acetate=100/0 to 90/10) to give the title compound (0.419 g).

### Reference Example 46

### 8-bromo-6-chloro-2,2,4-trimethyl-4-phenyl-1,2,3,4-tetrahydroquinoline

Reference Example 46 was synthesized in the same method as in Reference Example 40 except that Reference Example 45 was used in place of Reference Example 39.

### Reference Example 47

### methyl (2R)-2-amino-3-(6-chloro-2,2,4-trimethyl-4-phenyl-1,2,3,4-tetrahydroquinoline-8-yl)propano ate

Reference Example 47 was synthesized in the same method as in Reference Example 38 except that Reference Example 46 was used in place of Reference Example 37.

### Reference Example 48

### methyl (2R)-2-((tert-butoxycarbonyl)amino)-3-(6-chloro-2,2,4-trimethyl-4-phenyl-1,2,3,4-tetrahydro quinoline-8-yl)propanoate

Boc₂O (0.059 g) was added to a mixture of Reference Example 47 (0.070 g) and DCM (1.0 mL) at room temperature, and the mixture was stirred at the same temperature for 2 hours. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate=100/0 to 70/30) to give the title compound (0.122 g).

### Reference Example 49

### (2R)-2-((tert-butoxycarbonyl)amino)-3-(6-chloro-2,2,4-trimethyl-4-phenyl-1,2,3,4-tetrahydro quinoline-8-yl)propanoate

A 4 mol/L lithium hydroxide aqueous solution (0.125 mL) was added to a mixture of Reference Example 48 (0.114 g), methanol (0.3 mL) and THF (0.3 mL) at room temperature, and the mixture was stirred at the same temperature for 30 minutes. 2 mol/L hydrochloric acid (0.251 mL) was added to the reaction mixture, and the mixture was extracted with DCM. The extract was concentrated under reduced pressure. The residue was purified by silica gel column chromatography (eluent: n-hexane/ethyl acetate=70/30 to 0/100) to give the title compound (0.080 g).

### Reference Example 50

### (E)-N,3-diphenylbuta-2-enamide

A mixture of (E)-3-phenylbuta-2-enoic acid (1.15 g), aniline (0.759 g), HATU (3.24 g), DIPEA (0.916 g) and DCM (40 mL) was stirred at room temperature overnight. 1 mol/L hydrochloric acid was added to the reaction mixture, and the mixture was extracted with ethyl acetate. The extract was washed with a saturated sodium bicarbonate aqueous solution, water and a saturated saline solution, then dried over anhydrous magnesium sulfate, and concentrated under reduced pressure. The residue was purified by silica gel column chromatography (eluent: n-hexane/ethyl acetate=100/0 to 75/25) to give the title compound (1.42 g).

### Reference Example 51

### 4-methyl-4-phenyl-1,2,3,4-tetrahydroquinoline

A mixture of Reference Example 50 (1.42 g) and concentrated sulfuric acid (13.5 mL) was stirred at room temperature for 30 minutes. Ice and water were added to the reaction mixture, and the mixture was stirred for 30 minutes. Insoluble substances were collected by filtration, and the obtained solid was washed with water, and then dissolved in ethyl acetate. The mixture was washed with a saturated sodium bicarbonate aqueous solution, water and a saturated saline solution, then dried over anhydrous magnesium sulfate, and concentrated under reduced pressure to give 4-methyl-4-phenyl-3,4-dihydroquinoline-2(1H)-one (1.08 g).

Under an argon atmosphere, BH₃-THF (0.89 mol/L in THF) (4.67 mL) was added to a mixture of the obtained Compound (0.329 g) and THF (5.5 mL) at room temperature, and the mixture was stirred at the same temperature for 20 minutes and at 65°C for 3 hours. 2 mol/L hydrochloric acid (1.90 mL) was added to the reaction mixture under ice cooling, and the mixture was stirred at room temperature for 30 minutes. A 2 mol/L sodium hydroxide aqueous solution (1.90 mL) was added to the reaction mixture, and the mixture was extracted with ethyl acetate. The extract was washed with a saturated saline solution, then dried over anhydrous magnesium sulfate, and concentrated under reduced pressure. The residue was purified by APS column chromatography (eluent: n-hexane/ethyl acetate=100/0 to 90/10) to give the title compound (0.115 g).

### Reference Example 52

### 6-chloro-4-methyl-4-phenyl-1,2,3,4-tetrahydroquinoline

Reference Example 52 was synthesized in the same method as in Reference Example 40 except that Reference Example 51 was used in place of Reference Example 39, and NCS was used in place of NBS.

¹H-NMR (CDCl₃)δ ppm:1.72 (3H, s), 1.90-2.03 (1H, m), 2.08-2.23 (1H, m), 2.93-3.09 (1H, m), 3.15-3.31 (1H, m), 3.95 (1H, br), 6.46 (1H, d, J=8.4 Hz), 6.86-7.03 (2H, m), 7.08-7.22 (3H, m), 7.23-7.36 (2H, m)

### Reference Example 53

### 8-bromo-6-chloro-4-methyl-4-phenyl-1,2,3,4-tetrahydroquinoline

Reference Example 53 was synthesized in the same method as in Reference Example 40 except that Reference Example 52 was used in place of Reference Example 39.

### Reference Example 54

### (6R)-6-amino-9-chloro-1-methyl-1-phenyl-2,3,6,7-tetrahydro-1H,5H-pyrido[3,2,1-ij]quinolin -5-one

Reference Example 54 was synthesized in the same method as in Reference Example 38 except that Reference Example 53 was used in place of Reference Example 37.

### Reference Example 55

### 4-chloro-2-(1-phenylvinyl)aniline

Under an argon atmosphere, potassium hexamethyldisilazide (1 mol/L in THF) (16.5 mL) was added to a mixture of methyltriphenylphosphonium bromide (6.20 g) and THF (20 mL) at room temperature, and the mixture was stirred at the same temperature for 45 minutes. A mixture of (2-amino-5-chlorophenyl)(phenyl)methanone (2.01 g) and THF (6.9 mL) was added to the reaction mixture under ice cooling, and the mixture was stirred at room temperature for 45 minutes. A saturated ammonium chloride aqueous solution and water were added to the reaction mixture under ice cooling, and the mixture was extracted with ethyl acetate. The extract was washed with a saturated saline solution, then dried over anhydrous magnesium sulfate, and concentrated under reduced pressure. The residue was purified by silica gel column chromatography (eluent: n-hexane/ethyl acetate=90/10 to 70/30) to give the title compound (2.06 g).

### Reference Example 56

### 6-chloro-2,2-dimethyl-4-phenyl-1,2-dihydroquinoline

A mixture of Reference Example 55 (2.06 g), BF₃-Et₂O (0.122 g), acetone (6.3 mL) and toluene (18 mL) was stirred at 140°C for 10 hours under microwave irradiation. The reaction mixture was concentrated under reduced pressure. The residue was purified by silica gel column chromatography (eluent: n-hexane/ethyl acetate=100/0 to 90/10) to give the title compound (2.19 g).

### Reference Example 57

### tert-butyl 6-chloro-2,2-dimethyl-4-phenylquinoline-1(2H)-carboxylate

n-butyllithium (1.57 mol/L in n-hexane) (3.87 mL) was added to a mixture of Reference Example 56 (1.37 g) and THF (13 mL) at -78°C, and the mixture was stirred at the same temperature for 30 minutes. A mixture of Boc₂O (1.66 g) and THF (2.6 mL) was added to the reaction mixture at -78°C, and the mixture was stirred at room temperature for 1 hour. A saturated ammonium chloride aqueous solution and water were added to the reaction mixture, and the mixture was extracted with n-hexane. The extract was dried over anhydrous magnesium sulfate and concentrated under reduced pressure. The residue was purified by Method A (eluent: n-hexane/ethyl acetate=95/5) to give the title compound (1.65 g).

### Reference Example 58

### tert-butyl 6-chloro-3-hydroxy-2,2-dimethyl-4-phenyl-3,4-dihydroquinoline-1(2H)-carboxylate

Under an argon atmosphere, BH₃-THF (0.89 mol/L in THF) (15.0 mL) was added to a mixture of Reference Example 57 (1.65 g) and THF (23 mL) under ice cooling, and the mixture was stirred under ice cooling for 45 minutes at room temperature for 45 minutes and at 35°C for 5 hours. THF (7.7 mL), water (7.7 mL), a 5 mol/L sodium hydroxide aqueous solution (6.24 mL) and 30% hydrogen peroxide (1.37 mL) were added to the reaction mixture under ice cooling, and the mixture was stirred at room temperature for 16 hours. 1 mol/L hydrochloric acid was added to the reaction mixture, and the mixture was extracted with ethyl acetate. The extract was washed with a saturated sodium bicarbonate aqueous solution and a saturated saline solution, then dried over anhydrous magnesium sulfate, and concentrated under reduced pressure. The residue was purified by silica gel column chromatography (eluent: n-hexane/ethyl acetate=98/2 to 85/15) to give the title compound (0.856 g).

### Reference Example 59

### tert-butyl 6-chloro-2,2-dimethyl-3-oxo-4-phenyl-3,4-dihydroquinoline-1(2H)-carboxylate

Under an argon atmosphere, DMP (1.12 g) was added to a mixture of Reference Example 58 (0.856 g) and DCM (9.0 mL) under ice cooling, and the mixture was stirred under ice cooling for 40 minutes. A 1 mol/L sodium thiosulfate aqueous solution, a saturated sodium bicarbonate aqueous solution and water were added to the reaction mixture, and the mixture was extracted with DCM. The extract was concentrated under reduced pressure. The residue was purified by silica gel column chromatography (eluent: n-hexane/ethyl acetate=98/2 to 85/15) to give the title compound (0.581 g).

### Reference Example 60

### tert-butyl 6-chloro-2,2,4-trimethyl-3-oxo-4-phenyl-3,4-dihydroquinoline-1(2H)-carboxylate

Under an argon atmosphere, sodium hydride (about 60%) (0.090 g) was added to a mixture of Reference Example 59 (0.580 g), methyl iodide (1.07 g) and DMF (7.3 mL) under ice cooling, and the mixture was stirred under ice cooling for 30 minutes. A saturated ammonium chloride aqueous solution and water were added to the reaction mixture, and the mixture was extracted with n-hexane/ethyl acetate (1/1). The extract was washed with water and a saturated saline solution, then dried over anhydrous magnesium sulfate, and concentrated under reduced pressure. The residue was purified by silica gel column chromatography (eluent: n-hexane/ethyl acetate=100/0 to 90/10) to give the title compound (0.522 g).

### Reference Example 61

### 6-chloro-2,2,4-trimethyl-4-phenyl-1,4-dihydroquinolin-3(2H)-one

TFA (5.18 g) was added to a mixture of Reference Example 60 (0.521 g) and DCM (7.0 mL) under ice cooling, and the mixture was stirred at room temperature for 1.5 hours. A 2 mol/L sodium hydroxide aqueous solution (25 mL) was added to the reaction mixture under ice cooling, and the mixture was extracted with DCM. The extract was concentrated under reduced pressure. The residue was purified by silica gel column chromatography (eluent: n-hexane/ethyl acetate=98/2 to 90/10) to give the title compound (0.369 g).

### Reference Example 62

### 8-bromo-6-chloro-2,2,4-trimethyl-4-phenyl-1,4-dihydroquinolin-3(2H)-one

Reference Example 62 was synthesized in the same method as in Reference Example 40 except that Reference Example 61 was used in place of Reference Example 39.

### Reference Example 63

### methyl (2R)-2-((tert-butoxycarbonyl)amino)-3-(6-chloro-2,2,4-trimethyl-3-oxo-4-phenyl-1,2,3,4-tetr ahydroquinoline-8-yl)propanoate

Under an argon atmosphere, a mixture of methyl (S)-2-((tert-butoxycarbonyl)amino)-3-iodopropanoate (0.261 g) and DMF (0.35 mL) was added to a mixture of zinc powder (0.076 g) and DMF (0.7 mL), and the mixture was stirred at room temperature for 1 hour. A mixture of Reference Example 62 (0.200 g) and DMF (0.7 mL), and palladium(II) acetate (0.006 g) and Xphos (0.025 g) were added to the reaction mixture at room temperature, and the mixture was stirred at 40°C for 2 hours and then left at the same temperature for 13 hours. A saturated ammonium chloride aqueous solution, water and n-hexane/ethyl acetate (1/1) were added to the reaction mixture under ice cooling, and the mixture was stirred at room temperature for 10 minutes. The mixture was filtered through Celite, and the organic layer of the filtrate was separated. The organic layer was washed with water and a saturated saline solution, then dried over anhydrous magnesium sulfate and concentrated under reduced pressure. The residue was purified by silica gel column chromatography (eluent: n-hexane/ethyl acetate=98/2 to 70/30) to give the title compound (0.103 g).

### Reference Example 64

### (2R)-2-((tert-butoxycarbonyl)amino)-3-(6-chloro-2,2,4-trimethyl-3-oxo-4-phenyl-1,2,3,4-tetr ahydroquinoline-8-yl)propanoate

Reference Example 64 was synthesized in the same method as in Reference Example 49 except that Reference Example 63 was used in place of Reference Example 48.

### Reference Example 65

### 2-(benzyloxy)-6-bromoaniline

Potassium carbonate (0.628 g) was added to a mixture of 1-bromo-3-fluoro-2-nitrobenzene (0.500 g), benzyl alcohol (0.258 g) and DMF (3.0 mL) at room temperature, and the mixture was stirred at 110°C for 4 hours, at room temperature for 12 hours, and at 110°C for 10 hours. After the reaction mixture was cooled to room temperature, water and n-hexane were added, and the mixture was extracted with ethyl acetate. The extract was washed with water and a saturated saline solution, then dried over anhydrous sodium sulfate, and concentrated under reduced pressure.

A mixture of the residue, iron powder (0.635 g), ammonium chloride (3.04 g), water (2.0 mL) and ethanol (6.0 mL) was stirred at 80°C for 2 hours. After the reaction mixture was cooled to room temperature, water and ethyl acetate were added. The mixture was filtered through Celite, and the organic layer of the filtrate was separated. The organic layer was washed with a saturated saline solution, then dried over anhydrous sodium sulfate, and concentrated under reduced pressure. The residue was purified by silica gel column chromatography (eluent: n-hexane/ethyl acetate=98/2 to 80/20) to give the title compound (0.365 g).

### Reference Example 66

### 2-(benzyloxy)-6-bromo-N-methylaniline

Reference Example 66 was synthesized in the same method as in Reference Example 33 except that Reference Example 65 was used in place of Reference Example 32.

### Reference Example 67

### (R)-3-amino-8-(benzyloxy)-1-methyl-3,4-dihydroquinoline-2(1H)-one

Reference Example 67 was synthesized in the same method as in Reference Example 38 except that Reference Example 66 was used in place of Reference Example 37.

### Reference Example 68

### 6-bromo-2-(2-chloro-5-fluorophenoxy)-3-methylaniline

Potassium carbonate (0.360 g) was added to a mixture of 1-bromo-3-fluoro-4-methyl-2-nitrobenzene (0.305 g), 2-chloro-5-fluorophenol (0.200 g) and DMF (10 mL), and the mixture was stirred at 100°C for 2 hours. After the reaction mixture was cooled to room temperature, water, ethyl acetate and toluene were added, and the organic layer was separated. The organic layer was washed with water and then concentrated under reduced pressure. The residue was purified by silica gel column chromatography (eluent: n-hexane/ethyl acetate=100/0 to 55/45) to give 1-bromo-3-(2-chloro-5-fluorophenoxy)-4-methyl-2-nitrobenzene (0.439 g).

Ammonium chloride (1.74 g) and iron powder (0.362 g) were added to a mixture of the obtained Compound (0.439 g), ethanol (4.5 mL) and water (1.5 mL), and the mixture was stirred at 80°C for 1.5 hours. The reaction mixture was cooled to room temperature and then filtered through Celite. Water was added to the filtrate, and the mixture was extracted with ethyl acetate. The extract was concentrated under reduced pressure. The residue was purified by silica gel column chromatography (eluent: n-hexane/ethyl acetate=100/0 to 70/30) to give the title compound (0.355 g).

### Reference Example 69

### 6-bromo-2-(2-chloro-5-fluorophenoxy)-N,3-dimethylaniline

Methyllithium (1.04 mol/L in Et₂O) (1.3 mL) was added to a mixture of Reference Example 68 (0.355 g) and THF (3.0 mL) at -78°C, and the mixture was stirred at the same temperature for 1 hour. A mixture of methyl iodide (0.214 g) and THF (1.5 mL) was added to the reaction mixture at -78°C, and the mixture was stirred under ice cooling for 2 hours. A saturated ammonium chloride aqueous solution and water were added to the reaction mixture under water cooling, and the mixture was extracted with DCM. The extract was concentrated under reduced pressure. The residue was purified by silica gel column chromatography (eluent: n-hexane/ethyl acetate=100/0 to 90/10) to give the title compound (0.349 g).

### Reference Example 70

### (3R)-3-amino-8-(2-chloro-5-fluorophenoxy)-1,7-dimethyl-1,2,3,4-tetrahydroquinoline-2-one

Under an argon atmosphere, zinc powder (0.141 g) was added to a mixture of methyl (S)-2-((tert-butoxycarbonyl)amino)-3-iodopropanoate (0.393 g) and DMF (3.0 mL), and the mixture was stirred at room temperature for 1.5 hours. A mixture of Reference Example 69 (0.338 g) and DMF (1.0 mL), and palladium(II) acetate (0.012 g) and Xphos (0.047 g) were added to the reaction mixture, and the mixture was stirred at room temperature for 13 hours. A saturated ammonium chloride aqueous solution, water and ethyl acetate were added to the reaction mixture. The mixture was filtered through Celite, and the filtrate was extracted with ethyl acetate. The extract was washed with water and then concentrated under reduced pressure.

TFA (2.24 g) was added to a mixture of the residue and DCM (4.0 mL) under ice cooling, and the mixture was stirred at room temperature for 3 hours. A 5 mol/L sodium hydroxide aqueous solution (3.9 mL) was added to the reaction mixture under ice cooling, and the mixture was extracted with DCM. The extract was concentrated under reduced pressure. The residue was purified by APS column chromatography (eluent: n-hexane/ethyl acetate/methanol=50/50/0 to 0/100/0 to 0/80/20) to give the title compound (0.135 g).

### Reference Example 71

### 3-(2-chloro-5-fluorophenoxy)-4-methyl-2-(methylamino)benzaldehyde

Under an argon atmosphere, (1,1'-bis(diphenylphosphino)ferrocene)palladium(II) dichloride dichloromethane adduct (0.474 g) was added to a mixture of Reference Example 69 (2.00 g), sodium carbonate (0.923 g), triethylsilane (1.35 g) and NMP (10 mL), and the mixture was stirred under a carbon monoxide atmosphere at 100°C for 3 hours. After the reaction mixture was cooled to room temperature, water and n-hexane were added, and the mixture was extracted with ethyl acetate. The extract was washed with water and a saturated saline solution, then dried over anhydrous sodium sulfate, and concentrated under reduced pressure. The residue was purified by silica gel column chromatography (eluent: n-hexane/ethyl acetate=98/2 to 85/15) to give the title compound (1.40 g).

### Reference Example 72

### Ethyl (2R^{*},3S^{*})-2-(((benzyloxy)carbonyl)amino)-3-(3-(2-chloro-5-fluorophenoxy)-4-methyl-2-(met hylamino)phenyl)-3-hydroxypropanoate

LDA (1.07 mol/L in THF/n-hexane) (2.50 mL) was added to a mixture of ethyl((benzyloxy)carbonyl)glycine (0.363 g) and THF (3.0 mL) at -78°C, and the mixture was stirred at the same temperature for 1 hour. A mixture of Reference Example 71 (0.750 g) and THF (5.0 mL) was added to the reaction mixture at -78°C, and the mixture was stirred at the same temperature for 30 minutes and under ice cooling for 1 hour. A saturated ammonium chloride aqueous solution and water were added to the reaction mixture, and the mixture was extracted with DCM. The extract was concentrated under reduced pressure. The residue was purified by silica gel column chromatography (eluent: n-hexane/ethyl acetate=95/5 to 60/40) to give the title compound (0.090 g).

### Reference Example 73

### 2-bromo-6-(2-chloro-5-fluorophenoxy)aniline

Potassium carbonate (0.377 g) was added to a mixture of 1-bromo-3-fluoro-2-nitrobenzene (0.300 g), 2-chloro-5-fluorophenol (0.220 g) and DMF (10 mL), and the mixture was stirred at 100°C for 1 hour. The reaction mixture was cooled to room temperature, water was then added, and the mixture was extracted with Et₂O. The extract was washed with water, then dried over anhydrous magnesium sulfate and concentrated under reduced pressure.

Ammonium chloride (1.46 g) and iron powder (0.777 g) were added to a mixture of the residue, ethanol (9.0 mL) and water (3.0 mL), and the mixture was stirred at 90°C for 1 hour. The reaction mixture was cooled to room temperature and then filtered through Celite. The filtrate was extracted with ethyl acetate, and the extract was then concentrated under reduced pressure. The residue was purified by silica gel column chromatography (eluent: n-hexane/ethyl acetate=100/0 to 75/25) to give the title compound (0.417 g).

### Reference Example 74

### 2-bromo-6-(2-chloro-5-fluorophenoxy)-N-methylaniline

Methyllithium (1.06 mol/L in Et₂O) (1.37 mL) was added to a mixture of Reference Example 73(0.417 g) and THF (7.0 mL) at -78°C, and the mixture was stirred at the same temperature for 30 minutes. Methyl iodide (0.224 g) was added to the reaction mixture at -78°C, and the mixture was stirred at the same temperature for 10 minutes and under ice cooling for 30 minutes. A saturated ammonium chloride aqueous solution was added to the reaction mixture under ice cooling, and the mixture was extracted with ethyl acetate. The extract was washed with water and then concentrated under reduced pressure. The residue was purified by silica gel column chromatography (eluent: n-hexane/ethyl acetate=100/0 to 77/23) to give the title compound (0.436 g).

### Reference Example 75

### (3R)-3-amino-8-(2-chloro-5-fluorophenoxy)-1-methyl-1,2,3,4-tetrahydroquinoline-2-one

Under an argon atmosphere, zinc powder (0.215 g) was added to a mixture of methyl (S)-2-((tert-butoxycarbonyl)amino)-3-iodopropanoate (0.650 g) and DMF (7.0 mL), and the mixture was stirred at room temperature for 2 hours. A mixture of Reference Example 74 (0.435 g) and DMF (1.0 mL), and palladium(II) acetate (0.015 g) and Xphos (0.063 g) were added to the reaction mixture, and the mixture was stirred at room temperature for 16 hours. A saturated ammonium chloride aqueous solution was added to the reaction mixture, and the mixture was extracted with ethyl acetate. The extract was washed with water, then dried over anhydrous magnesium sulfate and concentrated under reduced pressure.

A mixture of the residue and hydrogen chloride (4 mol/L in 1,4-dioxane) (4.0 mL) was stirred at room temperature for 30 minutes. The reaction mixture was concentrated under reduced pressure. Ethyl acetate and a saturated sodium bicarbonate aqueous solution were added to the residue, and the organic layer was separated. The organic layer was washed with water and then concentrated under reduced pressure. The residue was purified by APS column chromatography (eluent: n-hexane/ethyl acetate/methanol=30/70/0 to 0/100/0 to 0/71/29) to give the title compound (0.198 g).

### Reference Example 76

### 1-(2-chloro-5-fluorophenoxy)-3-methyl-2-nitrobenzene

Potassium carbonate (0.976 g) was added to a mixture of 1-fluoro-3-methyl-2-nitrobenzene (0.730 g), 2-chloro-5-fluorophenol (0.724 g) and DMF (4.7 mL) at room temperature, and the mixture was stirred at 60°C for 1 hour and at 100°C for 68 hours. After the reaction mixture was cooled to room temperature, a saturated ammonium chloride aqueous solution was added, and the mixture was extracted with n-hexane/ethyl acetate (1/1). The extract was washed with water and a saturated saline solution, then dried over anhydrous magnesium sulfate, and concentrated under reduced pressure. The residue was purified by silica gel column chromatography (eluent: n-hexane/ethyl acetate=100/0 to 85/15) to give the title compound (1.26 g).

### Reference Example 77

### 8-(2-chloro-5-fluorophenoxy)-3-hydroxy-3,4-dihydroquinoline-2(1H)-one

Sodium hydride (about 60%) (0.891 g) was added to a mixture of Reference Example 76 (1.26 g), dimethyl oxalate (2.63 g) and DMF (13 mL) under ice cooling, and the mixture was stirred under ice cooling for 30 minutes and at room temperature for 20 hours. A saturated ammonium chloride aqueous solution was added to the reaction mixture under ice cooling, and the mixture was extracted with n-hexane/ethyl acetate (1/1). The extract was washed with water and a saturated saline solution, then dried over anhydrous magnesium sulfate, and concentrated under reduced pressure. The residue was suspended in n-hexane, and insoluble substances were collected by filtration. The obtained solid was washed with n-hexane and then dried under reduced pressure.

Sodium borohydride (0.054 g) was added to a mixture of the obtained compound and MeCN (5.3 mL) under ice cooling, and the mixture was stirred under ice cooling for 15 minutes. Acetic acid (16.3 mL) was added to the reaction mixture, and the temperature was raised to 50°C. Iron powder (0.594 g) was added to the reaction mixture, and the mixture was stirred at 50°C for 30 minutes, at 65°C for 45 minutes, and at 80°C for 11 hours. The reaction mixture was cooled to room temperature, and water was then added. Insoluble substances were collected by filtration, and the filtrate was extracted with ethyl acetate. The extract was washed with water, a saturated sodium bicarbonate aqueous solution and a saturated saline solution, then dried over anhydrous magnesium sulfate, and concentrated under reduced pressure. The residue was purified by silica gel column chromatography (eluent: n-hexane/ethyl acetate=85/15 to 20/80). The obtained product was suspended in n-hexane/DCM (4/1), and insoluble substances were collected by filtration. The obtained solid was washed with n-hexane and then dried under reduced pressure to give the title compound (0.528 g).

### Reference Example 78

### (3-((tert-butyldimethylsilyl)oxy)-8-(2-chloro-5-fluorophenoxy)-3,4-dihydroquinoline-2(1H)-one

TBSCl (0.220 g) was added to a mixture of Reference Example 77 (0.300 g), imidazole (0.100 g) and THF (3.0 mL) at room temperature, and the mixture was stirred at the same temperature for 20 minutes. DMF (1.5 mL) was added to the reaction mixture, and the mixture was stirred at room temperature for 2 hours, at 50°C for 3 hours, and at 65°C for 1 hour. The reaction mixture was cooled to room temperature, water was then added, and the mixture was extracted with n-hexane/ethyl acetate (1/1). The extract was washed with water and a saturated saline solution, then dried over anhydrous magnesium sulfate, and concentrated under reduced pressure. The residue was purified by silica gel column chromatography (eluent: n-hexane/ethyl acetate=100/0 to 60/40) to give the title compound (0.361 g).

### Reference Example 79

### 8-(2-chloro-5-fluorophenoxy)-3-hydroxy-1-methyl-3,4-dihydroquinoline-2(1H)-one

Methyl iodide (0.134 g) and potassium carbonate (0.177 g) were added to a mixture of Reference Example 78 (0.361 g) and DMF (1.8 mL), and the mixture was stirred at room temperature for 1 hour, at 60°C for 1 hour, and at 80°C for 2 hours. Methyl iodide (0.109 g) was added to the reaction mixture at room temperature, and the mixture was stirred at 80°C for 17 hours. The reaction mixture was cooled to room temperature, water was then added, and the mixture was extracted with n-hexane/ethyl acetate (1/1). The extract was washed with water and a saturated saline solution, then dried over anhydrous magnesium sulfate, and concentrated under reduced pressure.

TBAF (1 mol/L in THF) (1.03 mL) was added to a mixture of the residue and THF (1.8 mL) under ice cooling, and the mixture was stirred under ice cooling for 45 minutes. Water was added to the reaction mixture, and the mixture was extracted with DCM. The extract was concentrated under reduced pressure. The residue was purified by silica gel column chromatography (eluent: n-hexane/ethyl acetate=80/20 to 0/100) to give the title compound (0.230 g).

### Reference Example 80

### 8-bromo-1-methyl-3,4-dihydroquinazoline-2(1H)-one

A 40% methylamine aqueous solution (0.417 mL) was added to a mixture of 3-bromo-2-fluorobenzonitrile (0.500 g), potassium carbonate (0.691 g) and 1,4-dioxane (2.5 mL), and the mixture was stirred at 40°C for 14 hours. The reaction mixture was cooled to room temperature, water was then added, and the mixture was extracted with ethyl acetate. The extract was washed with a saturated saline solution, then dried over anhydrous magnesium sulfate, and concentrated under reduced pressure.

Under an argon atmosphere, BH₃-THF (1 mol/L in THF) (7.08 mL) was added to a mixture of the residue and THF (2.5 mL) under ice cooling, and the mixture was stirred at room temperature for 2 hours and at 40°C for 2 hours. Methanol (3.0 mL) and 6 mol/L hydrochloric acid (2.75 mL) were added to the reaction mixture under ice cooling, and the mixture was stirred at 60°C for 1 hour and at room temperature for 13 hours. A 2 mol/L sodium hydroxide aqueous solution (9.44 mL) was added to the reaction mixture under ice cooling, and the mixture was extracted twice with DCM. The combined extract was dried over anhydrous sodium sulfate and concentrated under reduced pressure.

CDI (0.402 g) was added to a mixture of the residue, pyridine (0.280 g) and DCE (15 mL), and the mixture was stirred at room temperature for 6 hours. 1 mol/L hydrochloric acid (11.8 mL) was added to the reaction mixture, and the mixture was extracted with DCM. The extract was washed with a saturated sodium bicarbonate aqueous solution and a saturated saline solution, then dried over anhydrous sodium sulfate, and concentrated under reduced pressure. MTBE was added to the residue, and the mixture was stirred at room temperature for 30 minutes. Insoluble substances were collected by filtration, and the obtained solid was washed with MTBE and then dried under reduced pressure to give the title compound (0.404 g).

### Reference Example 81

### 1-methyl-8-(1-phenylvinyl)-3,4-dihydroquinazoline-2(1H)-one

A mixture of Reference Example 80 (0.100 g), (1-phenylvinyl)boronic acid (0.092 g), bis(di-tert-butyl(4-dimethylaminophenyl)phosphine)dichloropalladium(II) (0.015 g), potassium carbonate (0.172 g), water (0.4 mL) and 1,4-dioxane (1.6 mL) was stirred at 120°C for 2 hours under microwave irradiation. Water was added to the reaction mixture, and the mixture was extracted with ethyl acetate. The extract was washed with a saturated saline solution, then dried over anhydrous magnesium sulfate, and concentrated under reduced pressure. The residue was purified by silica gel column chromatography (eluent: n-hexane/ethyl acetate=50/50 to 0/100) to give the title compound (0.096 g).

### Reference Example 82

### 1-methyl-8-(1-phenylethyl)-3,4-dihydroquinazoline-2(1H)-one

10% Pd/C (0.029 g) was added to a mixture of Reference Example 81 (0.095 g), THF (1.0 mL) and ethanol (1.0 mL) under ice cooling, and the mixture was stirred under a hydrogen atmosphere at room temperature for 3 hours. The reaction mixture was filtered through Celite, and the filtrate was concentrated under reduced pressure to give the title compound (0.096 g).

### Reference Example 83

### 8-(2-chloro-5-fluorophenoxy)-1,7-dimethyl-2-oxo-1,2-dihydroquinoline-3-carboxylic acid

Dimethyl malonate (0.621 g) and a 28% sodium methoxide methanol solution (1.21 g) were added to a mixture of Reference Example 71 (0.460 g) and methanol (5.0 mL), and the mixture was stirred at room temperature for 14 hours. Dimethyl malonate (0.621 g) and a 28% sodium methoxide methanol solution (1.81 g) were added to the reaction mixture, and the mixture was stirred at 50°C for 2 hours. Water was added to the reaction mixture, and the mixture was stirred at 50°C for 30 minutes. The mixture was extracted with ethyl acetate. The extract was washed with a saturated saline solution, then dried over anhydrous sodium sulfate, and concentrated under reduced pressure. The residue was suspended in MTBE, and insoluble substances were collected by filtration. The obtained solid was dried under reduced pressure to give the title compound (0.170 g).

### Reference Example 84

### 2-bromo-6-ethynylaniline

Under an argon atmosphere, dimethyl (1-diazo-2-oxopropyl)phosphonate (0.501 g) was added to a mixture of 3-bromo-2-nitrobenzaldehyde (0.500 g), potassium carbonate (0.601 g) and methanol (20 mL) at room temperature, and the mixture was stirred at the same temperature for 1 hour. The reaction mixture was concentrated under reduced pressure. Water was added to the residue, and the mixture was extracted with Et₂O. The extract was washed with a saturated sodium bicarbonate aqueous solution and a saturated saline solution, then dried over anhydrous sodium sulfate, and concentrated under reduced pressure.

Under an argon atmosphere, 4,4'-bipyridyl (0.003 g) was added to a mixture of the residue, tetrahydroxydiborone (0.584 g) and DMF (10 mL) at room temperature, and the mixture was stirred at the same temperature for 10 minutes. Water was added to the reaction mixture, and the mixture was extracted with n-hexane/ethyl acetate (1/1). The extract was dried over anhydrous sodium sulfate and concentrated under reduced pressure. The residue was purified by silica gel column chromatography (eluent: n-hexane/ethyl acetate=100/0 to 90/10) to give the title compound (0.271 g).

### Reference Example 85

### 2-bromo-6-ethynyl-N-methylaniline

Under an argon atmosphere, trifluoroacetic anhydride (0.377 g) was added to a mixture of Reference Example 84 (0.271 g) and ethyl acetate (5.0 mL) in an ice salt bath, and the mixture was stirred in the ice salt bath for 40 minutes. Methanol (0.056 mL) and water were added to the reaction mixture, and the mixture was extracted with ethyl acetate. The extract was dried over anhydrous sodium sulfate and concentrated under reduced pressure.

Under an argon atmosphere, potassium carbonate (0.382 g) was added to a mixture of the residue, methyl iodide (0.294 g) and DMF (4.0 mL) at room temperature, and the mixture was stirred at the same temperature for 1 hour. Water was added to the reaction mixture, and the mixture was extracted with n-hexane/ethyl acetate (1/1). The extract was dried over anhydrous sodium sulfate and concentrated under reduced pressure.

Under an argon atmosphere, a mixture of a 48% potassium hydroxide aqueous solution (0.155 g) and methanol (0.8 mL) was added to a mixture of the residue and THF (4.0 mL) at room temperature, and the mixture was stirred at the same temperature for 13 hours. Water was added to the reaction mixture, and the mixture was extracted with ethyl acetate. The extract was dried over anhydrous sodium sulfate and concentrated under reduced pressure. The residue was sequentially purified by silica gel column chromatography (eluent: n-hexane/ethyl acetate=100/0 to 90/10) and silica gel column chromatography (eluent: n-hexane/ethyl acetate=95/5) to give the title compound (0.178 g).

### Reference Example 86

### methyl (R)-2-((tert-butoxycarbonyl)amino)-3-(3-ethynyl-2-(methylamino)phenyl)propanoate

Under an argon atmosphere, TMSCl (0.018 g) was added to a mixture of zinc powder (0.122 g) and DMF (2.0 mL) at room temperature, and the mixture was stirred at the same temperature for 20 minutes. A mixture of methyl (S)-2-((tert-butoxycarbonyl)amino)-3-iodopropanoate (0.558 g) and THF (1.0 mL) was added to the reaction mixture under water cooling, and the mixture was stirred under water cooling for 30 minutes. To the reaction mixture, palladium(II) acetate (0.010 g) and Xphos (0.040 g) were added, a mixture of Reference Example 85 (0.178 g) and THF (2.0 mL) was then added under water cooling, and the mixture was stirred at 40°C for 2 hours. The reaction mixture was cooled to room temperature, water was then added, and the mixture was extracted with n-hexane/ethyl acetate (1/1). The extract was dried over anhydrous sodium sulfate and concentrated under reduced pressure. The residue was purified by silica gel column chromatography (eluent: n-hexane/ethyl acetate=100/0 to 60/40) to give the title compound (0.258 g).

### Reference Example 87

### tert-butyl (R)-(8-ethynyl-1-methyl-2-oxo-1,2,3,4-tetrahydroquinoline-3-yl)carbamate

Under an argon atmosphere, a 5 mol/L sodium hydroxide aqueous solution (0.466 mL) was added to a mixture of Reference Example 86 (0.258 g), methanol (1.0 mL) and THF (1.0 mL) at room temperature, and the mixture was stirred at the same temperature for 30 minutes. 2 mol/L hydrochloric acid (1.36 mL) was added to the reaction mixture, and the mixture was extracted with DCM. The extract was dried over anhydrous sodium sulfate and concentrated under reduced pressure.

Under an argon atmosphere, HATU (0.310 g) was added to a mixture of the residue, DIPEA (0.201 g) and MeCN (4.0 mL) at room temperature, and the mixture was stirred at the same temperature for 10 minutes. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate=100/0 to 50/50) to give the title compound (0.080 g).

### Reference Example 88

### tert-butyl (4-bromo-2-formylthiophen-3-yl)(tert-butoxycarbonyl)carbamate

Under an argon atmosphere, a mixture of methyl 3-amino-4-bromothiophene-2-carboxylate (0.502 g) and THF (2.0 mL) was added to a mixture of lithium aluminum hydride (0.323 g) and THF (8.0 mL) under ice cooling, and the mixture was stirred at room temperature for 1 hour. Sodium sulfate decahydrate (4.11 g) was added to the reaction mixture under ice cooling, and the mixture was stirred under ice cooling for 10 minutes and at room temperature for 30 minutes. The reaction mixture was filtered through Celite, and the filtrate was concentrated under reduced pressure.

Under an argon atmosphere, manganese(IV) dioxide (0.923 g) was added to a mixture of the residue and ethyl acetate (10 mL) at room temperature, and the mixture was stirred at the same temperature for 18 hours. The reaction mixture was filtered through Celite, and the filtrate was concentrated under reduced pressure.

Under an argon atmosphere, a mixture of Boc₂O (0.927 g) and THF (1.0 mL) was added to a mixture of the residue, TEA (0.430 g), DMAP (0.052 g) and THF (5.0 mL) at room temperature, and the mixture was stirred at the same temperature for 1 hour. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate=100/0 to 80/20) to give the title compound (0.265 g).

### Reference Example 89

### 4-bromo-2-(2-chloro-5-fluorobenzyl)thiophene-3-amine

Under an argon atmosphere, isopropyl magnesium chloride (2 mol/L in THF) (0.326 mL) was added to a mixture of 1-chloro-4-fluoro-2-iodobenzene (0.167 g) and THF (1.5 mL) at -78°C, and the mixture was stirred under ice cooling for 30 minutes. A mixture of Reference Example 88 (0.265 g) and THF (1.5 mL) was added to the reaction mixture at -78°C, and the mixture was stirred at the same temperature for 10 minutes, and under ice cooling for 1 hour. A saturated ammonium chloride aqueous solution was added to the reaction mixture, and the mixture was extracted with DCM. The extract was dried over anhydrous sodium sulfate and concentrated under reduced pressure.

Under an argon atmosphere, TFA (0.743 g) and triethylsilane (0.152 g) were added to a mixture of the residue and DCM (3.0 mL) at room temperature, and the mixture was stirred at the same temperature for 62 hours. A 5 mol/L sodium hydroxide aqueous solution was added to the reaction mixture under ice cooling, and the mixture was extracted with DCM. The extract was dried over anhydrous sodium sulfate and concentrated under reduced pressure. The residue was purified by silica gel column chromatography (eluent: n-hexane/ethyl acetate=100/0 to 80/20) to give the title compound (0.104 g).

### Reference Example 90

### 4-bromo-2-(2-chloro-5-fluorobenzyl)-N-methylthiophene-3-amine

Reference Example 90 was synthesized in the same method as in Reference Example 85 except that Reference Example 89 was used in place of Reference Example 84.

### Reference Example 91

### methyl (R)-2-((tert-butoxycarbonyl)amino)-3-(5-(2-chloro-5-fluorobenzyl)-4-(methylamino)thiophen -3-yl)propanoate

Reference Example 91 was synthesized in the same method as in Reference Example 86 except that Reference Example 90 was used in place of Reference Example 85.

### Reference Example 92

### methyl 3-amino-4-(2-chloro-5-fluorobenzyl)thiophene-2-carboxylate

Under an argon atmosphere, a mixture of 2-(bromomethyl)-1-chloro-4-fluorobenzene (0.243 g) and DMF (0.65 mL) was added to a mixture of zinc powder (0.104 g) and DMF (1.3 mL), and the mixture was stirred at room temperature for 1 hour. A mixture of methyl 3-amino-4-bromothiophene-2-carboxylate (0.171 g) and DMF (1.3 mL), and palladium(II) acetate (0.008 g) and Xphos (0.035 g) were added to the reaction mixture at room temperature, and the mixture was stirred at 40°C for 2 hours. After the reaction mixture was cooled to room temperature, a saturated ammonium chloride aqueous solution, water and n-hexane/ethyl acetate (1/1) were added, and the mixture was stirred at room temperature for 10 minutes. The mixture was filtered through Celite, and the organic layer of the filtrate was separated. The organic layer was washed with water and a saturated saline solution, then dried over anhydrous magnesium sulfate and concentrated under reduced pressure. The residue was purified by silica gel column chromatography (eluent: n-hexane/ethyl acetate=100/0 to 70/30) to give the title compound (0.176 g).

### Reference Example 93

### methyl 3-(bis(tert-butoxycarbonyl)amino)-4-(2-chloro-5-fluorobenzyl)thiophene-2-carboxylate

A mixture of Boc₂O (0.363 g) and THF (1.1 mL), and TEA (0.062 g) and DMAP (0.007 g) were added to a mixture of Reference Example 92 (0.166 g) and THF (1.1 mL) at room temperature, and the mixture was stirred at the same temperature for 45 minutes, at 50°C for 2 hours, and at room temperature for 60 hours. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate=100/0 to 80/20) to give the title compound (0.228 g).

### Reference Example 94

### methyl 3-((tert-butoxycarbonyl)(methyl)amino)-4-(2-chloro-5-fluorobenzyl)thiophene-2-carboxylate

TFA (0.078 g) was added to a mixture of Reference Example 93 (0.228 g) and DCM (1.5 mL), and the mixture was stirred at room temperature for 4 hours. A 5 mol/L sodium hydroxide aqueous solution (0.140 mL) was added to the reaction mixture under ice cooling, and the mixture was extracted with DCM. The extract was concentrated under reduced pressure.

Potassium carbonate (0.094 g) was added to a mixture of the residue, methyl iodide (0.097 g) and DMF (1.5 mL) at room temperature, and the mixture was stirred at 60°C for 17 hours. Methyl iodide (0.097 g) and potassium carbonate (0.094 g) were added to the reaction mixture at room temperature, and the mixture was stirred at 80°C for 2 hours. After the reaction mixture was cooled to room temperature, a saturated ammonium chloride aqueous solution and water were added, and the mixture was extracted with n-hexane/ethyl acetate (1/1). The extract was washed with water and a saturated saline solution, then dried over anhydrous magnesium sulfate, and concentrated under reduced pressure. The residue was purified by silica gel column chromatography (eluent: n-hexane/ethyl acetate=95/5 to 80/20) to give the title compound (0.172 g).

### Reference Example 95

### tert-butyl (4-(2-chloro-5-fluorobenzyl)-2-(hydroxymethyl)thiophen-3-yl)(methyl)carbamate

Under an argon atmosphere, DIBAL-H (1.03 mol/L in n-hexane) (1.41 mL) was added to a mixture of Reference Example 94 (0.200 g) and DCM (2.0 mL) at -78°C, and the mixture was stirred at the same temperature for 20 minutes. Methanol and a 30% Rochelle salt aqueous solution were added to the reaction mixture, and the mixture was stirred at room temperature for a while. The mixture was extracted with DCM, and the extract was concentrated under reduced pressure. The residue was purified by Method A (eluent: n-hexane/ethyl acetate=90/10 to 60/40) to give the title compound (0.169 g).

### Reference Example 96

### tert-butyl (R)-3-(3-((tert-butoxycarbonyl)(methyl)amino)-4-(2-chloro-5-fluorobenzyl)thiophen-2-yl)-2-((diphenylmethylene)amino)propanoate

Methanesulfonyl chloride (0.058 g) was added to a mixture of Reference Example 95 (0.163 g), TEA (0.068 g) and ethyl acetate (2.1 mL) under ice cooling, and the mixture was stirred under ice cooling for 30 minutes. The reaction mixture was filtered, and insoluble substances were washed with ethyl acetate (2.1 mL). Lithium bromide monohydrate (0.133 g) was added to the filtrate under ice cooling, and the mixture was stirred at room temperature for 45 minutes. A saturated ammonium chloride aqueous solution was added to the reaction mixture, and the mixture was extracted with ethyl acetate. The extract was washed with a saturated saline solution, then dried over anhydrous magnesium sulfate, and concentrated under reduced pressure.

A mixture of potassium hydroxide (0.773 g) and water (1.0 mL) was added to a mixture of the residue, tert-butyl 2-((diphenylmethylene)amino)acetate (0.172 g), (S)-4,4-dibutyl-2,6-bis(3,4,5-trifluorophenyl)-4,5-dihydro-3H-dinaphtho[2,1-c:1',2'-e]azepini um bromide (0.029 g) and toluene (2.0 mL) under ice cooling, and the mixture was stirred under ice cooling for 45 minutes. A saturated ammonium chloride aqueous solution was added to the reaction mixture, and the mixture was extracted with DCM. The extract was concentrated under reduced pressure. The residue was sequentially purified by silica gel column chromatography (eluent: n-hexane/ethyl acetate=100/0 to 80/20) and Method A (eluent: n-hexane/ethyl acetate=95/5 to 85/15) to give the title compound (0.249 g).

### Reference Example 97

### (R)-6-amino-3-(2-chloro-5-fluorobenzyl)-4-methyl-6,7-dihydrothieno[3,2-b]pyridine-5(4H)-o ne

Methanesulfonic acid (0.491 g) was added to a mixture of Reference Example 96 (0.169 g), ethanol (2.0 mL) and toluene (1.0 mL), and the mixture was stirred at 80°C for 4.5 hours. A 5 mol/L sodium hydroxide aqueous solution (1.02 mL) and water were added to the reaction mixture under ice cooling, and the mixture was extracted with DCM. The extract was concentrated under reduced pressure. The residue was purified by APS column chromatography (eluent: n-hexane/ethyl acetate/methanol=70/30/0 to 0/100/0 to 0/80/20) to give the title compound (0.055 g).

### Reference Example 98

### methyl 3-((tert-butoxycarbonyl)(methyl)amino)-4-(2-chloro-5-fluorobenzyl)-5-methylthiophene-2-ca rboxylate

LDA (1.07 mol/L in THF/n-hexane) (1.03 mL) was added to a mixture of Reference Example 94 (0.303 g) and THF (3.7 mL) at -78°C, and the mixture was stirred at the same temperature for 15 minutes. Methyl iodide (0.520 g) was added to the reaction mixture, and the mixture was stirred at -78°C for 1.5 hours. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate=100/0 to 80/20) to give the title compound (0.306 g).

### Reference Example 99

### tert-butyl (4-(2-chloro-5-fluorobenzyl)-2-(hydroxymethyl)-5-methylthiophen-3-yl)(methyl)carbamate

Reference Example 99 was synthesized in the same method as in Reference Example 95 except that Reference Example 98 was used in place of Reference Example 94.

### Reference Example 100

### tert-butyl (R)-3-(3-((tert-butoxycarbonyl)(methyl)amino)-4-(2-chloro-5-fluorobenzyl)-5-methylthiophe n-2-yl)-2-((diphenylmethylene)amino)propanoate

Reference Example 100 was synthesized in the same method as in Reference Example 96 except that Reference Example 99 was used in place of Reference Example 95.

### Reference Example 101

### (R)-6-amino-3-(2-chloro-5-fluorobenzyl)-2,4-dimethyl-6,7-dihydrothieno[3,2-b]pyridine-5(4 H)-one

Reference Example 101 was synthesized in the same method as in Reference Example 97 except that Reference Example 100 was used in place of Reference Example 96.

### Reference Example 102

### methyl 3-((tert-butoxycarbonyl)(methyl)amino)-4-(2-chloro-5-fluorobenzyl)-5-formylthiophene-2-ca rboxylate

Reference Example 102 was synthesized in the same method as in Reference Example 98 except that DMF was used in place of methyl iodide.

### Reference Example 103

### methyl 3-((tert-butoxycarbonyl)(methyl)amino)-5-(((tert-butyldimethylsilyl)oxy)methyl)-4-(2-chloro -5-fluorobenzyl)thiophene-2-carboxylate

Sodium borohydride (0.004 g) was added to a mixture of Reference Example 102 (0.100 g), THF (1.0 mL) and methanol (1.0 mL) under ice cooling, and the mixture was stirred under ice cooling for 30 minutes. A saturated ammonium chloride aqueous solution was added to the reaction mixture, and the mixture was extracted with DCM. The extract was concentrated under reduced pressure.

Imidazole (0.023 g) was added to a mixture of the residue, TBSCl (0.051 g) and DMF (1.0 mL), and the mixture was stirred at room temperature for 19 hours. TBSCl (0.017 g) was added to the reaction mixture, and the mixture was stirred at room temperature for 3 hours. Imidazole (0.023 g) was added to the reaction mixture, and the mixture was stirred at room temperature for 30 minutes. Water was added to the reaction mixture, and the mixture was extracted with n-hexane/ethyl acetate (1/1). The extract was washed with water and a saturated saline solution, then dried over anhydrous magnesium sulfate, and concentrated under reduced pressure. The residue was purified by silica gel column chromatography (eluent: n-hexane/ethyl acetate=100/0 to 85/15) to give the title compound (0.090 g).

### Reference Example 104

### tert-butyl (5-(((tert-butyldimethylsilyl)oxy)methyl)-4-(2-chloro-5-fluorobenzyl)-2-(hydroxymethyl)thio phen-3-yl)(methyl)carbamate

Reference Example 104 was synthesized in the same method as in Reference Example 95 except that Reference Example 103 was used in place of Reference Example 94.

### Reference Example 105

### tert-butyl (R)-3-(3-((tert-butoxycarbonyl)(methyl)amino)-5-(((tert-butyldimethylsilyl)oxy)methyl)-4-(2 -chloro-5-fluorobenzyl)thiophen-2-yl)-2-((diphenylmethylene)amino)propanoate

Reference Example 105 was synthesized in the same method as in Reference Example 96 except that Reference Example 104 was used in place of Reference Example 95.

### Reference Example 106

### (R)-6-amino-3-(2-chloro-5-fluorobenzyl)-2-(ethoxymethyl)-4-methyl-6,7-dihydrothieno[3,2-b]pyridine-5(4H)-one

Methanesulfonic acid (0.195 g) was added to a mixture of Reference Example 105 (0.082 g), ethanol (1.0 mL) and toluene (0.5 mL), and the mixture was stirred at 80°C for 3 hours. A 5 mol/L sodium hydroxide aqueous solution (0.406 mL) was added to the reaction mixture under ice cooling, and the mixture was extracted with DCM. The extract was concentrated under reduced pressure. The residue was purified by APS column chromatography (eluent: n-hexane/ethyl acetate=70/30 to 0/100) to give the title compound (0.025 g).

### Reference Example 107

### tert-butyl (4-formylthiophen-3-yl)carbamate

DIBAL-H (1.02 mol/L in n-hexane) (7.01 mL) was added to a mixture of methyl 4-((tert-butoxycarbonyl)amino)thiophene-3-carboxylate (0.800 g) and DCM (8.0 mL) at -78°C, and the mixture was stirred under ice cooling for 30 minutes. DIBAL-H (1.02 mol/L in n-hexane) (7.01 mL) was added to the reaction mixture at -78°C, and the mixture was stirred under ice cooling for 15 minutes. A 30% Rochelle salt aqueous solution was added to the reaction mixture, and the mixture was stirred at room temperature for 3 hours. The mixture was extracted with DCM. The extract was dried over anhydrous sodium sulfate and concentrated under reduced pressure.

DMP (2.15 g) was added to a mixture of the residue and DCM (10 mL) under ice cooling, and the mixture was stirred under ice cooling for 30 minutes. A 1 mol/L sodium thiosulfate aqueous solution was added to the reaction mixture under ice cooling, and the mixture was stirred under ice cooling for 30 minutes. The mixture was extracted with DCM. The extract was dried over anhydrous sodium sulfate and concentrated under reduced pressure. The residue was purified by silica gel column chromatography (eluent: n-hexane/ethyl acetate=95/5 to 80/20) to give the title compound (0.523 g).

### Reference Example 108

### tert-butyl (4-((2-chloro-5-fluorophenyl)(hydroxy)methyl)thiophen-3-yl)carbamate

Isopropyl magnesium chloride (2 mol/L in THF) (1.65 mL) was added to a mixture of 1-chloro-4-fluoro-2-iodobenzene (0.846 g) and THF (3.0 mL) at -78°C, and the mixture was stirred under ice cooling for 30 minutes. A mixture of Reference Example 107 (0.250 g) and THF (3.0 mL) was added to the reaction mixture at -78°C, and the mixture was stirred under ice cooling for 30 minutes. A saturated ammonium chloride aqueous solution and water were added to the reaction mixture, and the mixture was extracted with ethyl acetate. The extract was concentrated under reduced pressure. The residue was purified by silica gel column chromatography (eluent: n-hexane/ethyl acetate=95/5 to 80/20) to give the title compound (0.393 g).

### Reference Example 109

### tert-butyl (4-(2-chloro-5-fluorobenzoyl)thiophen-3-yl)carbamate

DMP (0.699 g) was added to a mixture of Reference Example 108 (0.393 g) and DCM (12 mL) under ice cooling, and the mixture was stirred under ice cooling for 4 hours. A 1 mol/L sodium thiosulfate aqueous solution was added to the reaction mixture, and the mixture was stirred under ice cooling for 30 minutes. The mixture was extracted with DCM, and the extract was concentrated under reduced pressure. The residue was purified by silica gel column chromatography (eluent: n-hexane/ethyl acetate=95/5 to 80/20) to give the title compound (0.355 g).

### Reference Example 110

### tert-butyl (4-(2-chloro-5-fluorobenzoyl)-2-formylthiophen-3-yl)carbamate

LDA (1.07 mol/L in THF/n-hexane) (0.742 mL) was added to a mixture of Reference Example 109 (0.157 g) and THF (5.0 mL) at -78°C, and the mixture was stirred at the same temperature for 10 minutes. DMF (0.097 g) was added to the reaction mixture, and the mixture was stirred at -78°C for 1.5 hours. A saturated ammonium chloride aqueous solution was added to the reaction mixture, and the mixture was extracted with ethyl acetate. The extract was concentrated under reduced pressure. The residue was purified by silica gel column chromatography (eluent: n-hexane/ethyl acetate=100/0 to 80/20) to give the title compound (0.065 g).

### Reference Example 111

### methyl (Z)-2-(((benzyloxy)carbonyl)amino)-3-(3-((tert-butoxycarbonyl)amino)-4-(2-chloro-5-fluoro benzoyl)thiophen-2-yl)acrylate

DBU (0.031 g) was added to a mixture of N-benzyloxycarbonyl-2-phosphonoglycine trimethyl (0.067 g) and DCM (1.0 mL) at room temperature, and the mixture was stirred at the same temperature for 15 minutes. A mixture of Reference Example 110 (0.065 g) and DCM (1.0 mL) was added to the reaction mixture, and the mixture was stirred at room temperature for 30 minutes. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate=95/5 to 50/50) to give the title compound (0.060 g).

### Reference Example 112

### methyl 2-(((benzyloxy)carbonyl)amino)-3-(3-((tert-butoxycarbonyl)amino)-4-(2-chloro-5-fluorobenz oyl)thiophen-2-yl)propanoate

(1,5-cyclooctadiene)(pyridine)(tricyclohexylphosphine)iridium(I) hexafluorophosphate (0.004 g) was added to a mixture of Reference Example 111 (0.030 g) and DCM (1.0 mL), and the mixture was stirred at a hydrogen pressure (0.4 MPa) and at room temperature for 9 hours. The reaction mixture was concentrated under reduced pressure. The residue was purified by silica gel column chromatography (eluent: n-hexane/ethyl acetate=95/5 to 50/50) to give the title compound (0.030 g).

### Reference Example 113

### methyl 3-(bromomethyl)isothiazole-4-carboxylate

A mixture of 3-methylisothiazole-4-carboxylic acid (1.00 g), hydrogen chloride (4 mol/L in 1,4-dioxane) (5.0 mL) and methanol (6.0 mL) was stirred at 80°C for 1 hour under microwave irradiation. The reaction mixture was concentrated under reduced pressure. A saturated sodium bicarbonate aqueous solution was added to the residue under ice cooling, and the mixture was extracted with DCM. The extract was concentrated under reduced pressure.

A mixture of the residue, NBS (0.905 g), benzoyl peroxide (0.410 g) and carbon tetrachloride (18 mL) was stirred at 70°C for 5 hours. A saturated sodium bicarbonate aqueous solution was added to the reaction mixture under ice cooling, and the mixture was extracted with DCM. The extract was concentrated under reduced pressure. The residue was purified by silica gel column chromatography (eluent: n-hexane/ethyl acetate=100/0 to 90/10) to give the title compound (0.864 g).

### Reference Example 114

### methyl 3-(2-chloro-5-fluorobenzyl)isothiazole-4-carboxylate

A mixture of Reference Example 113 (0.614 g), 2-chloro-5-fluorophenylboronic acid (0.382 g), bis(triphenylphosphine)palladium(II) chloride (0.059 g), sodium carbonate (0.268 g), water (2.5 mL), ethanol (5.0 mL) and toluene (5.0 mL) was stirred at 100°C for 1 hour under microwave irradiation. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate=100/0 to 90/10) to give the title compound (0.273 g).

### Reference Example 115

### tert-butyl (tert-butoxycarbonyl)(3-(2-chloro-5-fluorobenzyl)isothiazol-4-yl)carbamate

A mixture of Reference Example 114 (0.250 g), a 4 mol/L lithium hydroxide aqueous solution (0.875 mL), methanol (1.0 mL) and THF (1.0 mL) was stirred at 60°C for 30 minutes. After the reaction mixture was cooled to room temperature, 2 mol/L hydrochloric acid was added, and the mixture was extracted with DCM. The extract was concentrated under reduced pressure to give 3-(2-chloro-5-fluorobenzyl)isothiazole-4-carboxylic acid (0.240 g).

A mixture of the obtained Compound (0.125 g), DPPA (0.317 g), TEA (0.140 g), toluene (2.0 mL) and 1,4-dioxane (1.0 mL) was refluxed for 3 hours. The reaction mixture was cooled to room temperature, water was then added, and the mixture was extracted with ethyl acetate. The extract was dried over anhydrous sodium sulfate and concentrated under reduced pressure.

A mixture of the residue, Boc₂O (1.00 g), DMAP (0.056 g) and THF (2.0 mL) was stirred at room temperature for 3 hours. Water was added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate=98/2 to 80/20) to give the title compound (0.094 g).

### Reference Example 116

### tert-butyl (tert-butoxycarbonyl)(3-(1-(2-chloro-5-fluorophenyl)ethyl)-5-methylisothiazol-4-yl)carbamat e

LDA (1.01 mol/L in THF/n-hexane) (0.313 mL) was added to a mixture of Reference Example 115 (0.070 g) and THF (3.0 mL) at -78°C, and the mixture was stirred at the same temperature for 15 minutes. Methyl iodide (0.067 g) was added to the reaction mixture at -78°C, and the mixture was stirred at room temperature for 15 minutes. LDA (1.01 mol/L in THF/n-hexane) (0.156 mL) was added to the reaction mixture at -78°C, and the mixture was stirred at the same temperature for 10 minutes. A saturated ammonium chloride aqueous solution was added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate=98/2 to 80/20) to give the title compound (0.055 g).

### Reference Example 117

### tert-butyl (3-(1-(2-chloro-5-fluorophenyl)ethyl)-5-methylisothiazol-4-yl)carbamate

A mixture of Reference Example 116 (0.055 g), TFA (0.054 g) and DCM (2.0 mL) was stirred at room temperature for 5 hours. A saturated carbonic acid aqueous solution was added to the reaction mixture, and the mixture was extracted with DCM. The extract was concentrated under reduced pressure. The residue was purified by silica gel column chromatography (eluent: n-hexane/ethyl acetate=90/10 to 70/30) to give the title compound (0.030 g).

### Reference Example 118

### tert-butyl (3-(1-(2-chloro-5-fluorophenyl)ethyl)-5-methylisothiazol-4-yl)(methyl)carbamate

A mixture of Reference Example 117 (0.030 g), methyl iodide (0.115 g), potassium carbonate (0.034 g) and DMF (1.0 mL) was stirred at room temperature for 63 hours. Water was added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate=95/5 to 80/20) to give the title compound (0.028 g).

### Reference Example 119

### tert-butyl (5-(bromomethyl)-3-(1-(2-chloro-5-fluorophenyl)ethyl)isothiazol-4-yl)(methyl)carbamate

A mixture of Reference Example 118 (0.028 g), NBS (0.013 g), benzoyl peroxide (0.006 g) and carbon tetrachloride (1.0 mL) was stirred at 70°C for 30 minutes. Benzoyl peroxide (0.023 g) was added to the reaction mixture at room temperature, and the mixture was stirred at 70°C for 1 hour. A saturated sodium bicarbonate aqueous solution was added to the reaction mixture under ice cooling, and the mixture was extracted with DCM. The extract was dried over anhydrous sodium sulfate and concentrated under reduced pressure. The residue was purified by silica gel column chromatography (eluent: n-hexane/ethyl acetate=100/0 to 90/10) to give the title compound (0.015 g).

### Reference Example 120

### tert-butyl (2R)-3-(4-((tert-butoxycarbonyl)(methyl)amino)-3-(1-(2-chloro-5-fluorophenyl)ethyl)isothiaz ol-5-yl)-2-((diphenylmethylene)amino)propanoate

A mixture of potassium hydroxide (0.120 g) and water (0.5 mL) was added to a mixture of Reference Example 119 (0.015 g), tert-butyl 2-((diphenylmethylene)amino)acetate (0.014 g), (S)-4,4-dibutyl-2,6-bis(3,4,5-trifluorophenyl)-4,5-dihydro-3H-dinaphtho[2,1-c:1',2'-e]azepini um bromide (0.002 g) and toluene (1.0 mL) under ice cooling, and the mixture was stirred under ice cooling for 30 minutes and at room temperature for 1 hour. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate=98/2 to 80/20) to give the title compound (0.012 g).

Structural formulae of reference examples are shown in the following tables.

**[Table 1]**

| Ref. No. | Str. | Ref. No. | Str. |
|---|---|---|---|
| 1 | 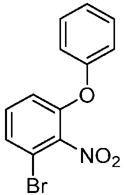 | 2 | 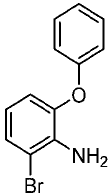 |
| 3 | 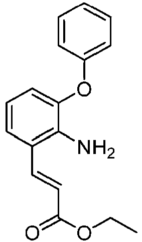 | 4 | 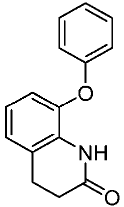 |
| 5 | 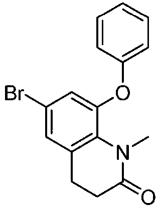 | 6 | 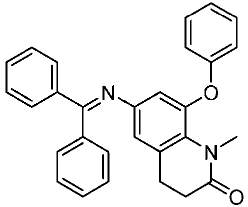 |
| 7 | 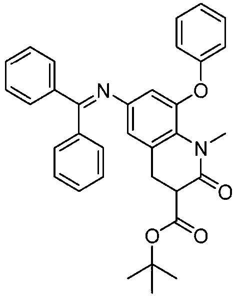 | 8 | 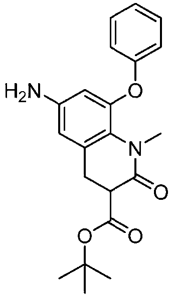 |
| 9 | 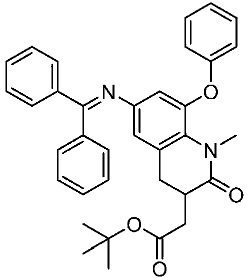 | 10 | 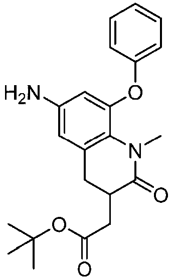 |

**[Table 2]**

| Ref. No. | Str. | Ref. No. | Str. |
|---|---|---|---|
| 11 | 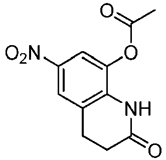 | 12 | 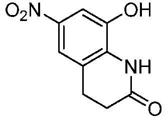 |
| 13 | 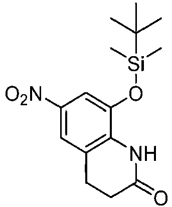 | 14 | 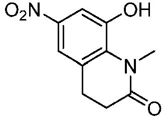 |
| 15 | 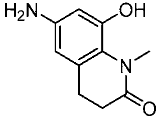 | 16 | 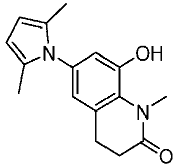 |
| 17 | 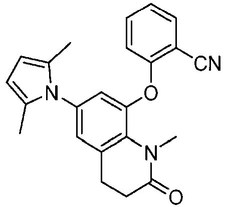 | 18 | 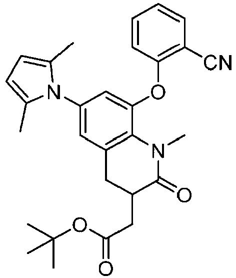 |
| 19 | 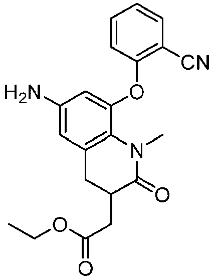 | 20 | 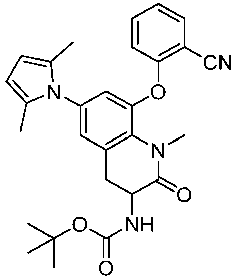 |

**[Table 3]**

| Ref. No. | Str. | Ref. No. | Str. |
|---|---|---|---|
| 21 | 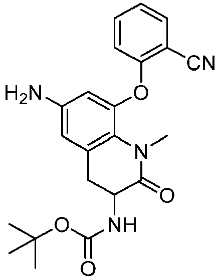 | 22 | 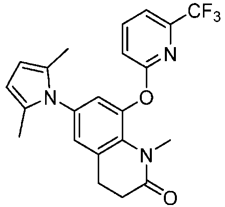 |
| 23 | 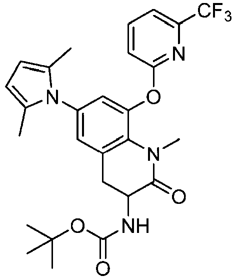 | 24 | 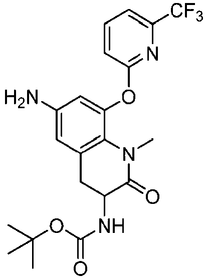 |
| 25 | 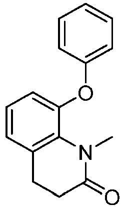 | 26 | 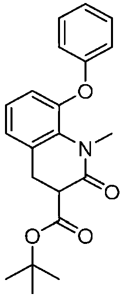 |
| 27 | 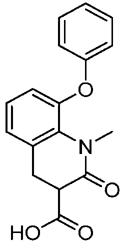 | 28 | 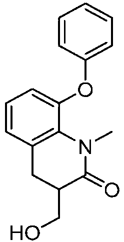 |

**[Table 4]**

| Ref. No. | Str. | Ref. No. | Str. |
|---|---|---|---|
| 29 | 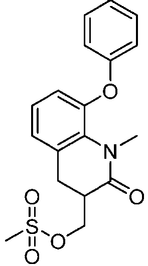 | 30 | 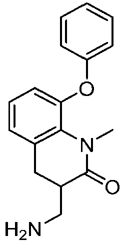 |
| 31 | 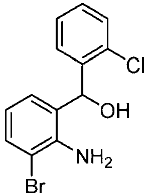 | 32 | 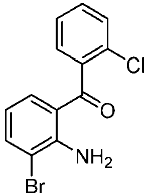 |
| 33 | 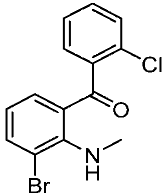 | 34 | 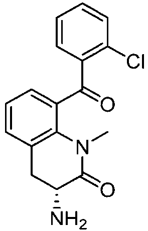 |
| 35 | 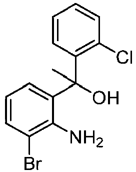 | 36 | 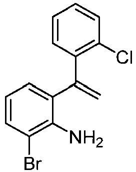 |
| 37 | 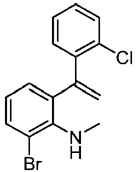 | 38 | 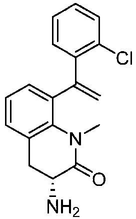 |

**[Table 5]**

| Ref. No. | Str. | Ref. No. | Str. |
|---|---|---|---|
| 39 | 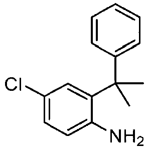 | 40 | 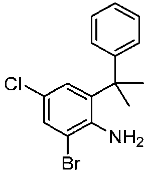 |
| 41 | 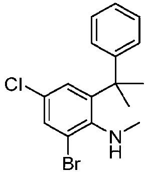 | 42 | 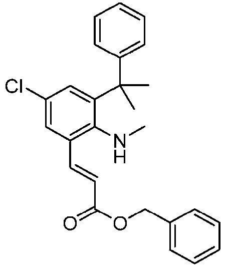 |
| 43 | 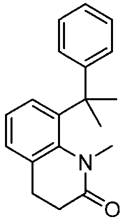 | 44 | 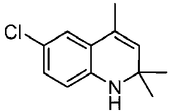 |
| 45 | 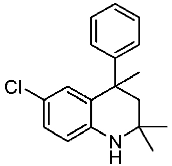 | 46 | 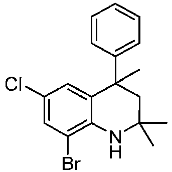 |
| 47 | 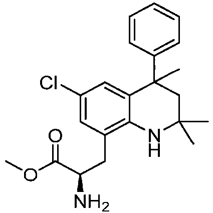 | 48 | 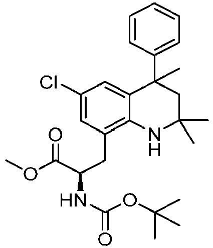 |

**[Table 6]**

| Ref. No. | Str. | Ref. No. | Str. |
|---|---|---|---|
| 49 | 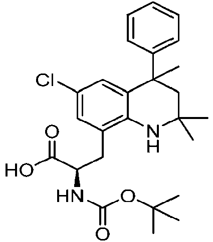 | 50 | 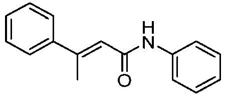 |
| 51 | 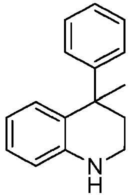 | 52 | 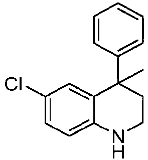 |
| 53 | 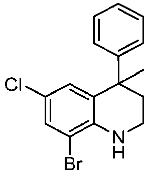 | 54 | 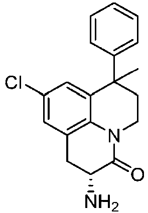 |
| 55 | 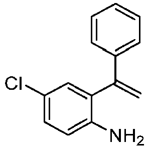 | 56 | 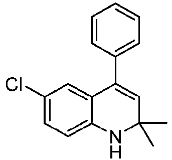 |
| 57 | 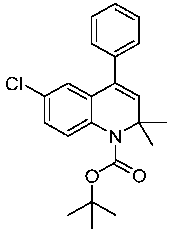 | 58 | 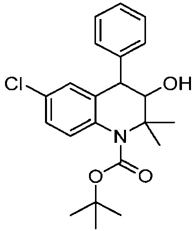 |

**[Table 7]**

| Ref. No. | Str. | Ref. No. | Str. |
|---|---|---|---|
| 59 | 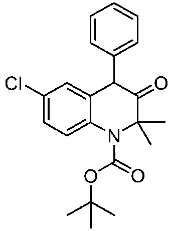 | 60 | 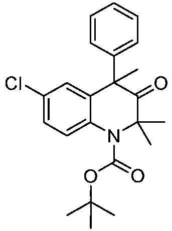 |
| 61 | 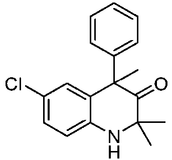 | 62 | 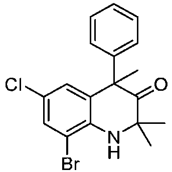 |
| 63 | 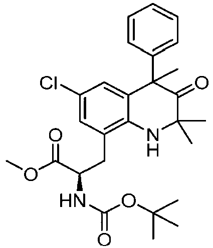 | 64 | 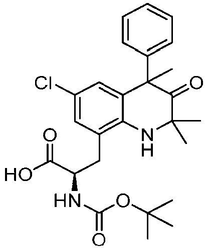 |
| 65 | 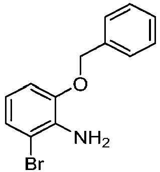 | 66 | 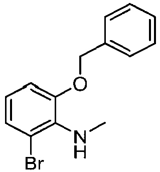 |
| 67 | 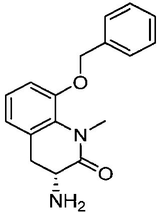 | 68 | 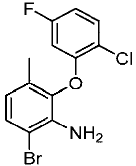 |

**[Table 8]**

| Ref. No. | Str. | Ref. No. | Str. |
|---|---|---|---|
| 69 | 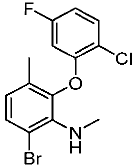 | 70 | 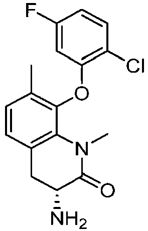 |
| 71 | 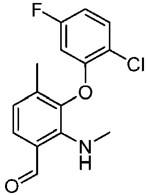 | 72 | 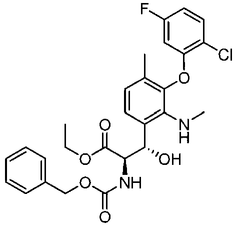 |
| 73 | 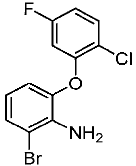 | 74 | 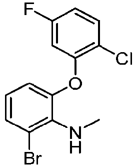 |
| 75 | 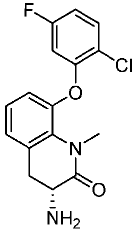 | 76 | 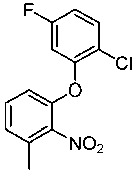 |
| 77 | 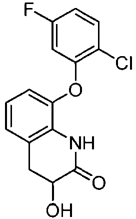 | 78 | 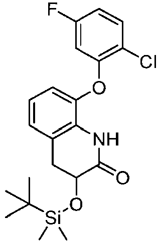 |

**[Table 9]**

| Ref. No. | Str. | Ref. No. | Str. |
|---|---|---|---|
| 79 | 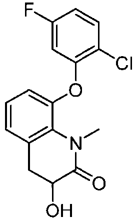 | 80 | 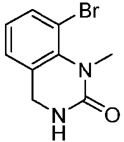 |
| 81 | 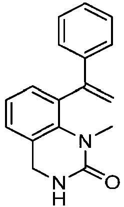 | 82 | 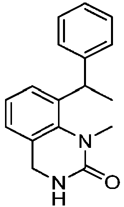 |
| 83 | 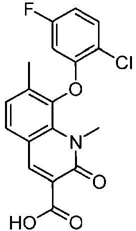 | | |

**[Table 10]**

| Ref. No. | Str. | Ref. No. | Str. |
|---|---|---|---|
| 84 | 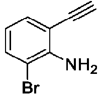 | 85 | 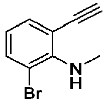 |
| 86 | 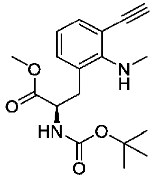 | 87 | 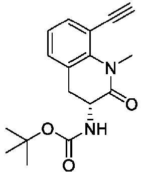 |

**[Table 11]**

| Ref. No. | Str. | Ref. No. | Str. |
|---|---|---|---|
| 88 | 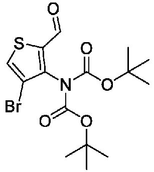 | 89 | 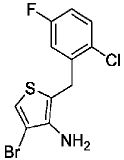 |
| 90 | 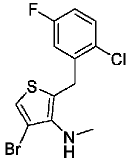 | 91 | 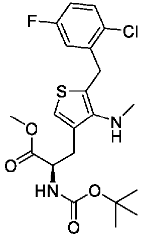 |
| 92 | 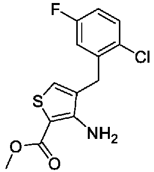 | 93 | 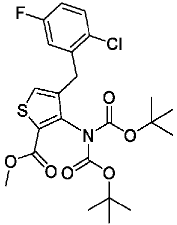 |
| 94 | 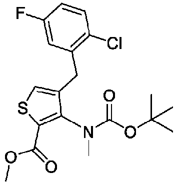 | 95 | 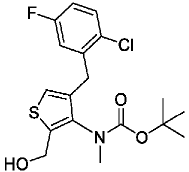 |
| 96 | 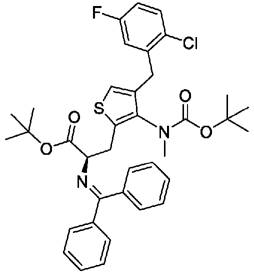 | 97 | 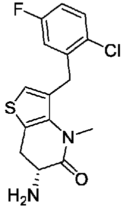 |

**[Table 12]**

| Ref. No. | Str. | Ref. No. | Str. |
|---|---|---|---|
| 98 | 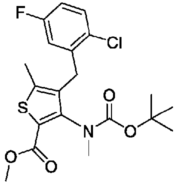 | 99 | 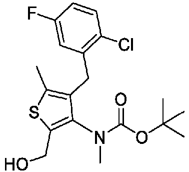 |
| 100 | 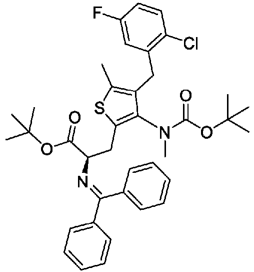 | 101 | 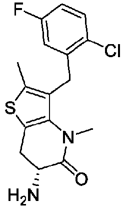 |
| 102 | 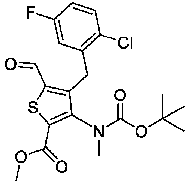 | 103 | 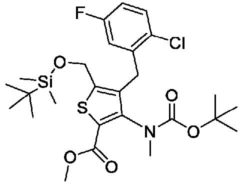 |
| 104 | 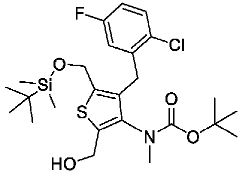 | 105 | 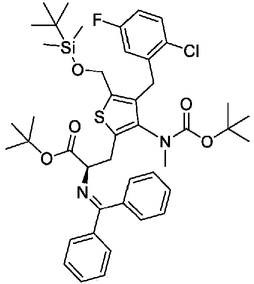 |
| 106 | 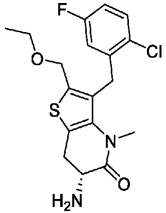 | 107 | 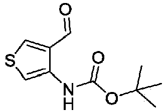 |

**[Table 13]**

| Ref. No. | Str. | Ref. No. | Str. |
|---|---|---|---|
| 108 | 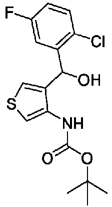 | 109 | 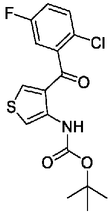 |
| 110 | 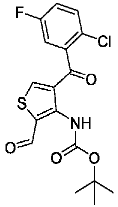 | 111 | 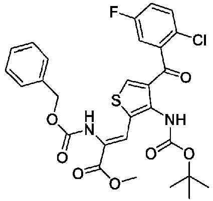 |
| 112 | 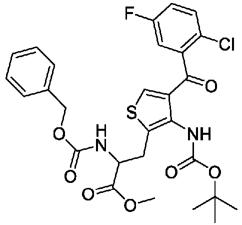 | 113 | 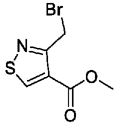 |
| 114 | 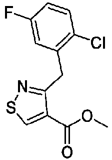 | 115 | 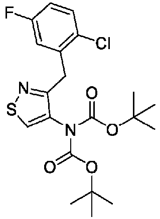 |
| 116 | 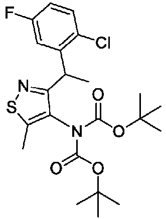 | 117 | 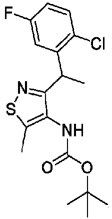 |

**[Table 14]**

| Ref. No. | Str. | Ref. No. | Str. |
|---|---|---|---|
| 118 | 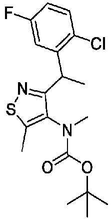 | 119 | 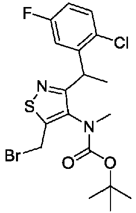 |
| 120 | 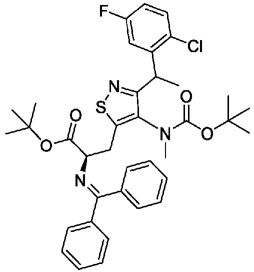 | | |

The notation of stereochemistry of Reference Example 72 in the table indicates a relative configuration.

### Example 1

### cyclopentyl (3-carbamoyl-1-methyl-2-oxo-8-phenoxy-1,2,3,4-tetrahydroquinoline-6-yl)carbamate

Triphosgene (0.014 g) was added to a mixture of cyclopentanol (0.013 g), TEA (0.015 g) and THF (1.0 mL) under ice cooling, and the mixture was stirred under ice cooling for 30 minutes. The reaction mixture was added to a mixture of Reference Example 8 (0.027 g), TEA (0.015 g) and THF (1.0 mL) under ice cooling, and the mixture was stirred at room temperature for 15 minutes. Water was added to the reaction mixture, and the mixture was extracted with DCM. The extract was concentrated under reduced pressure. The residue was purified by silica gel column chromatography (eluent: n-hexane/ethyl acetate=84/16 to 35/65) to give tert-butyl 6-(((cyclopentyloxy)carbonyl)amino)-1-methyl-2-oxo-8-phenoxy-1,2,3,4-tetrahydroquinoline -3-carboxylate (0.030 g).

A mixture of the obtained Compound (0.030 g) and hydrogen chloride (4 mol/L in 1,4-dioxane) (0.500 mL) was stirred at room temperature for 1 hour. The reaction mixture was concentrated under reduced pressure.

TEA (0.032 g), HATU (0.036 g) and ammonia (2 mol/L in methanol) (0.062 mL) were added to a mixture of the residue and THF (1.0 mL) at room temperature, and the mixture was stirred at the same temperature for 30 minutes. A saturated sodium bicarbonate aqueous solution was added to the reaction mixture, and the mixture was extracted with ethyl acetate. The extract was concentrated under reduced pressure. The residue was purified by silica gel column chromatography (eluent: n-hexane/ethyl acetate/methanol=40/60/0 to 0/100/0 to 0/53/47) to give the title compound (0.023 g).

### Example 2

### cyclopentyl (3-(2-amino-2-oxoethyl)-1-methyl-2-oxo-8-phenoxy-1,2,3,4-tetrahydroquinoline-6-yl)carbam ate

Example 2 was synthesized in the same method as in Example 1 except that Reference Example 10 was used in place of Reference Example 8.

### Example 3

### cyclopentyl (3-(2-amino-2-oxoethyl)-8-(2-cyanophenoxy)-1-methyl-2-oxo-1,2,3,4-tetrahydroquinoline-6-yl)carbamate

N-methylmorpholine (0.040 g) and cyclopentyl chloroformate (0.035 g) were added to a mixture of Reference Example 19 (0.060 g) and DCM (1.0 mL) under ice cooling, and the mixture was stirred at room temperature for 1 hour. Cyclopentyl chloroformate (0.035 g) was added to the reaction mixture at room temperature, and the mixture was stirred at the same temperature for 1 hour. N-methylmorpholine (0.064 g) and cyclopentyl chloroformate(0.070 g) were added to the reaction mixture under ice cooling, and the mixture was stirred at room temperature for 1 hour. A saturated ammonium chloride aqueous solution and water were added to the reaction mixture, and the mixture was extracted with DCM. The extract was concentrated under reduced pressure. The residue was purified by Method A (eluent: n-hexane/ethyl acetate=90/10 to 50/50) to give ethyl 2-(8-(2-cyanophenoxy)-6-(((cyclopentyloxy)carbonyl)amino)-1-methyl-2-oxo-1,2,3,4-tetrahy droquinoline-3-yl)acetate (0.062 g).

A 4 mol/L lithium hydroxide aqueous solution (0.095 mL) was added to a mixture of the obtained Compound (0.062 g), THF (0.5 mL) and methanol (0.5 mL) under ice cooling, and the mixture was stirred under ice cooling for 1 hour and at room temperature for 1 hour. Water and 1 mol/L hydrochloric acid (1.0 mL) were added to the reaction mixture under ice cooling, and the mixture was extracted with DCM. The extract was concentrated under reduced pressure.

DMT-MM (0.052 g) and 28% ammonia water (0.013 mL) were added to a mixture of the residue and MeCN (1.0 mL) at room temperature, and the mixture was stirred at the same temperature for 30 minutes. A saturated ammonium chloride aqueous solution was added to the reaction mixture, and the mixture was extracted with DCM. The extract was concentrated under reduced pressure. The residue was purified by Method A (eluent: n-hexane/ethyl acetate/methanol=49/49/2 to 0/98/2 to 0/86/14) to give the title compound (0.052 g).

### Example 4

### cyclopentyl (8-(2-cyanophenoxy)-1-methyl-2-oxo-3-ureido-1,2,3,4-tetrahydroquinoline-6-yl)carbamate

N-methylmorpholine (0.043 g) and cyclopentyl chloroformate (0.038 g) were added to a mixture of Reference Example 21 (0.070 g) and DCM (1.0 mL) under ice cooling, and the mixture was stirred at room temperature for 1 hour. Cyclopentyl chloroformate (0.038 g) was added to the reaction mixture at room temperature, and the mixture was stirred at the same temperature for 30 minutes. N-methylmorpholine (0.069 g) and cyclopentyl chloroformate (0.076 g) were added to the reaction mixture under ice cooling, and the mixture was stirred at room temperature for 30 minutes. A saturated sodium bicarbonate aqueous solution and water were added to the reaction mixture, and the mixture was extracted with DCM. The extract was concentrated under reduced pressure. The residue was purified by Method A (eluent: n-hexane/ethyl acetate=90/10 to 60/40) to give tert-butyl cyclopentyl (8-(2-cyanophenoxy)-1-methyl-2-oxo-1,2,3,4-tetrahydroquinoline-3,6-diyl)dicarbamate (0.080 g).

TFA (0.350 g) was added to a mixture of the obtained Compound (0.080 g) and DCM (1.0 mL) under ice cooling, and the mixture was stirred at room temperature for 1 hour. A 2 mol/L sodium hydroxide aqueous solution (2.0 mL) was added to the reaction mixture under ice cooling, and the mixture was extracted with DCM. The extract was concentrated under reduced pressure.

Potassium cyanate (0.016 g) and methanesulfonic acid (0.016 g) were added to a mixture of the residue, water (0.028 mL) and THF (1.0 mL) at room temperature, and the mixture was stirred at the same temperature for 5 hours. A saturated sodium bicarbonate aqueous solution and water were added to the reaction mixture, and the mixture was stirred at room temperature for 10 minutes. Insoluble substances were collected by filtration, and the obtained solid was washed with water and THF/water (1/2) and then dried under reduced pressure to give the title compound (0.048 g).

### Example 5

### cyclopentyl (1-methyl-2-oxo-8-((6-trifluoromethylpyridin-2-yl)oxy)-3-ureido-1,2,3,4-tetrahydroquinoline -6-yl)carbamate

Example 5 was synthesized in the same method as in Example 4 except that Reference Example 24 was used in place of Reference Example 21.

### Example 6

### (S)-1,1,1-trifluoropropan-2-yl (1-methyl-2-oxo-8-((6-trifluoromethylpyridin-2-yl)oxy)-3-ureido-1,2,3,4-tetrahydroquinoline -6-yl)carbamate

Pyridine (0.010 g) and 4-nitrophenyl chloroformate (0.027 g) were added to a mixture of Reference Example 24 (0.030 g) and DCM (1.0 mL) under ice cooling, and the mixture was stirred under ice cooling for 30 minutes. (S)-1,1,1-trifluoropropan-2-ol (0.023 g) and DMAP (0.024 g) were added to the reaction mixture under ice cooling, and the mixture was stirred at room temperature for 2 hours. A saturated sodium bicarbonate aqueous solution and water were added to the reaction mixture, and the mixture was extracted with DCM. The extract was concentrated under reduced pressure. The residue was purified by Method A (eluent: n-hexane/ethyl acetate=80/20 to 40/60) to give tert-Butyl ((S)-1,1,1-trifluoropropan-2-yl) (1-methyl-2-oxo-8-((6-trifluoromethylpyridin-2-yl)oxy)-1,2,3,4-tetrahydroquinoline-3,6-diyl) dicarbamate (0.034 g).

TFA (0.131 g) was added to a mixture of the obtained Compound (0.034 g) and DCM (1.0 mL) under ice cooling, and the mixture was stirred at room temperature for 2 hours. A 1 mol/L sodium hydroxide aqueous solution (2.0 mL) was added to the reaction mixture under ice cooling, and the mixture was extracted with DCM. The extract was concentrated under reduced pressure.

Potassium cyanate (0.006 g) and methanesulfonic acid (0.006 g) were added to a mixture of the residue, water (0.010 mL) and THF (0.5 mL) at room temperature, and the mixture was stirred at the same temperature for 1 hour. A saturated sodium bicarbonate aqueous solution and water were added to the reaction mixture, and the mixture was extracted with DCM. The extract was concentrated under reduced pressure. The residue was purified by Method A (eluent: ethyl acetate/methanol=98/2 to 88/12) to give the title compound (0.021 g).

### Example 7

### cyclopropylmethyl (1-methyl-2-oxo-8-((6-(trifluoromethyl)pyridin-2-yl)oxy)-3-ureido-1,2,3,4-tetrahydroquinoli ne-6-yl)carbamate

Example 7 was synthesized in the same method as in Example 6 except that cyclopropyl methanol was used in place of (S)-1,1,1-trifluoropropan-2-ol.

### Example 8

### 1-methylcyclopropyl (1-methyl-2-oxo-8-((6-(trifluoromethyl)pyridin-2-yl)oxy)-3-ureido-1,2,3,4-tetrahydroquinoli ne-6-yl)carbamate

Example 8 was synthesized in the same method as in Example 6 except that 1-methylcyclopropan-1-ol was used in place of (S)-1,1,1-trifluoropropan-2-ol.

### Example 9

### isopropyl (1-methyl-2-oxo-8-((6-trifluoromethylpyridin-2-yl)oxy)-3-ureido-1,2,3,4-tetrahydroquinoline -6-yl)carbamate

Example 9 was synthesized in the same method as in Example 6 except that propane-2-ol was used in place of (S)-1,1,1-trifluoropropan-2-ol.

### Example 10

### 1-((1-methyl-2-oxo-8-phenoxy-1,2,3,4-tetrahydroquinoline-3-yl)methyl)urea

A mixture of potassium cyanate (0.002 g) and water (0.100 mL), and methanesulfonic acid (0.002 g) were added to a mixture of Reference Example 30 (0.005 g) and THF (0.5 mL) at room temperature, and the mixture was stirred at the same temperature for 3 hours. A saturated sodium bicarbonate aqueous solution was added to the reaction mixture, and the mixture was extracted with ethyl acetate. The extract was concentrated under reduced pressure. APS was added to a mixture of the residue and methanol. The mixture was filtered, and the filtrate was concentrated under reduced pressure. The residue was purified by ODS column chromatography (eluent: water/MeCN=70/30 to 10/90) to give the title compound (0.002 g).

### Example 11

### (R)-1-(8-(2-chlorobenzoyl)-1-methyl-2-oxo-1,2,3,4-tetrahydroquinoline-3-yl)urea

Potassium cyanate (0.017 g) and methanesulfonic acid (0.016 g) were added to a mixture of Reference Example 34 (0.050 g), water (0.029 mL) and THF (1.0 mL) at room temperature, and the mixture was stirred at the same temperature for 10 hours. A saturated sodium bicarbonate aqueous solution and water were added to the reaction mixture, and the mixture was extracted with DCM. The extract was concentrated under reduced pressure. The residue was purified by APS column chromatography (eluent: ethyl acetate/methanol=100/0 to 90/10) to give the title compound (0.047 g).

### Example 12

### 1-((3R)-8-((2-chlorophenyl)(hydroxy)methyl)-1-methyl-2-oxo-1,2,3,4-tetrahydroquinoline-3-yl)urea

Sodium borohydride (0.006 g) was added to a mixture of Example 11 (0.030 g) and methanol (1.0 mL) under ice cooling, and the mixture was stirred at room temperature for 1 hour. A saturated ammonium chloride aqueous solution and water were added to the reaction mixture, and the mixture was extracted with DCM. The extract was concentrated under reduced pressure. The residue was purified by APS column chromatography (eluent: ethyl acetate/methanol=100/0 to 90/10) to give the title compound (0.025 g).

### Example 13

### (R)-1-(8-(1-(2-chlorophenyl)vinyl)-1-methyl-2-oxo-1,2,3,4-tetrahydroquinoline-3-yl)urea

Potassium cyanate (0.009 g), water (0.027 mL) and acetic acid (0.006 g) were added to a mixture of Reference Example 38 (0.024 g), methanol (0.2 mL) and THF (0.3 mL) at room temperature, and the mixture was stirred at the same temperature for 45 minutes. Water was added to the reaction mixture, and the mixture was extracted with DCM. The extract was concentrated under reduced pressure. The residue was purified by ODS column chromatography (eluent: water/MeCN=70/30 to 10/90) to give the title compound (0.015 g).

### Example 14

### 1-(1-methyl-2-oxo-8-(2-phenylpropan-2-yl)-1,2,3,4-tetrahydroquinoline-3-yl)urea

LDA (1.09 mol/L in THF/n-hexane) (0.550 mL) was added to a mixture of Reference Example 43 (0.110 g) and THF (2.0 mL) at -78°C, and the mixture was stirred at the same temperature for 30 minutes. DPPA (0.163 g) was added to the reaction mixture at -78°C, and the mixture was stirred at the same temperature for 1 hour. A mixture of Boc₂O (0.103 g) and THF (1.0 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After the reaction mixture was cooled to room temperature, a saturated ammonium chloride aqueous solution was added, and the mixture was extracted with DCM. The extract was concentrated under reduced pressure. The residue was purified by Method A (eluent: n-hexane/ethyl acetate=95/5 to 70/30) to give tert-butyl (1-methyl-2-oxo-8-(2-phenylpropan-2-yl)-1,2,3,4-tetrahydroquinoline-3-yl)carbamate (0.110 g).

TFA (0.636 g) was added to a mixture of the obtained Compound (0.110 g) and DCM (1.0 mL) under ice cooling, and the mixture was stirred at room temperature for 2 hours. The reaction mixture was concentrated under reduced pressure. The residue was purified by APS column chromatography (eluent: n-hexane/ethyl acetate/methanol=49/49/2 to 0/98/2 to 0/86/14) to give 3-amino-1-methyl-8-(2-phenylpropan-2-yl)-3,4-dihydroquinolin-2(1H)-one (0.079 g).

Potassium cyanate (0.029 g) and methanesulfonic acid (0.028 g) were added to a mixture of the obtained Compound (0.079 g), water (0.050 mL) and THF (1.0 mL) at room temperature, and the mixture was stirred at the same temperature for 1 hour. A saturated sodium bicarbonate aqueous solution and water were added to the reaction mixture, and the mixture was stirred at room temperature for 10 minutes. Insoluble substances were collected by filtration, and the obtained solid was washed with water and MTBE and then dried under reduced pressure to give the title compound (0.063 g).

### Example 15

### 1-((2R)-5,5,7-trimethyl-3-oxo-7-phenyl-2,3,6,7-tetrahydro-1H,5H-pyrido[3,2,1-ij]quinolin-2-yl)urea

DIPEA (0.024 g) was added to a mixture of Reference Example 49 (0.030 g) and MeCN (3.1 mL), and the mixture was stirred at room temperature for 5 minutes. T3P (1.7 mol/L in ethyl acetate) (0.073 mL) was added to the reaction mixture, and the mixture was stirred at room temperature for 10 minutes and at 60°C for 3.5 hours. DIPEA (0.024 g) and T3P (1.7 mol/L in ethyl acetate) (0.073 mL) were added to the reaction mixture at room temperature, and the mixture was stirred at the same temperature for 15 minutes and at 60°C for 1 hour. After the reaction mixture was cooled to room temperature, a saturated ammonium chloride aqueous solution and water were added, and the mixture was extracted with DCM. The extract was concentrated under reduced pressure. The residue was purified by silica gel column chromatography (eluent: n-hexane/ethyl acetate=100/0 to 70/30) to give tert-butyl ((2R)-9-chloro-5,5,7-trimethyl-3-oxo-7-phenyl-2,3,6,7-tetrahydro-1H,5H-pyrido[3,2,1-ij]quin olin-2-yl)carbamate (0.013 g).

TFA (0.060 g) was added to a mixture of the obtained Compound (0.012 g) and DCM (0.5 mL) under ice cooling, and the mixture was stirred at room temperature for 2.5 hours. A 1 mol/L sodium hydroxide aqueous solution (0.530 mL) was added to the reaction mixture under ice cooling, and the mixture was extracted with DCM. The extract was concentrated under reduced pressure.

Potassium cyanate (0.004 g) and acetic acid (0.003 g) were added to a mixture of the residue, water (0.010 mL), methanol (0.2 mL) and THF (0.3 mL) at room temperature, and the mixture was stirred at the same temperature for 2.5 hours. Water was added to the reaction mixture, and the mixture was extracted with DCM. The extract was concentrated under reduced pressure. The residue was purified by APS column chromatography (eluent: n-hexane/ethyl acetate/methanol=50/50/0 to 0/100/0 to 0/90/10) to give 1-((2R)-9-chloro-5,5,7-trimethyl-3-oxo-7-phenyl-2,3,6,7-tetrahydro-1H,5H-pyrido[3,2,1-ij]q uinoline-2-yl)urea (0.007 g).

A mixture of the obtained Compound (0.006 g), 10% Pd/C (0.003 g), THF (0.5 mL) and methanol (1.0 mL) was stirred under a hydrogen atmosphere at room temperature for 4.5 hours. The reaction mixture was filtered through Celite, and the filtrate was concentrated under reduced pressure. The residue was purified by APS column chromatography (eluent: ethyl acetate/methanol=100/0 to 80/20) to give the title compound (0.005 g).

### Example 16

### 1-((2R)-7-methyl-3-oxo-7-phenyl-2,3,6,7-tetrahydro-1H,5H-pyrido[3,2,1-ij]quinoline-2-yl)ur ea

Potassium cyanate (0.002 g) and acetic acid (0.002 g) were added to a mixture of Reference Example 54 (0.005 g), water (0.005 mL), methanol (0.2 mL) and THF (0.3 mL) at room temperature, and the mixture was stirred at the same temperature for 2 hours. Water was added to the reaction mixture, and the mixture was extracted with DCM. The extract was concentrated under reduced pressure. The residue was purified by APS column chromatography (eluent: n-hexane/ethyl acetate/methanol=50/50/0=0/100/0 to 0/85/15) to give 1-((2R)-9-chloro-7-methyl-3-oxo-7-phenyl-2,3,6,7-tetrahydro-1H,5H-pyrido[3,2,1-ij]quinolin e-2-yl)urea (0.003 g).

A mixture of the obtained Compound (0.003 g), 10% Pd/C (0.002 g) and methanol (1.0 mL) was stirred under a hydrogen atmosphere at room temperature for 3 hours. The reaction mixture was filtered through Celite, and the filtrate was concentrated under reduced pressure. The residue was purified by APS column chromatography (eluent: ethyl acetate/methanol=100/0 to 80/20) to give the title compound (0.003 g).

### Example 17

### 1-((2R)-5,5,7-trimethyl-3,6-dioxo-7-phenyl-2,3,6,7-tetrahydro-1H,5H-pyrido[3,2,1-ij]quinoli ne-2-yl)urea

Example 17 was synthesized in the same method as in Example 15 except that Reference Example 64 was used in place of Reference Example 49.

### Example 18

### (R)-1-(8-(benzyloxy)-1-methyl-2-oxo-1,2,3,4-tetrahydroquinoline-3-yl)urea

Potassium cyanate (0.037 g) and acetic acid (0.023 g) were added to a mixture of Reference Example 67 (0.100 g), water (0.128 mL), methanol (0.5 mL) and THF (1.0 mL) at room temperature, and the mixture was stirred at the same temperature for 4 hours. Water was added to the reaction mixture, and insoluble substances were collected by filtration.
The obtained solid was washed with water and MTBE and then dried under reduced pressure to give the title compound (0.098 g).

### Example 19

### (R)-N-(8-(2-chloro-5-fluorophenoxy)-1,7-dimethyl-2-oxo-1,2,3,4-tetrahydroquinoline-3-yl)m orpholine-4-carboxamide

Triphosgene (0.016 g) was added to a mixture of Reference Example 70 (0.036 g), DIPEA (0.069 g) and DCM (1.0 mL) at room temperature, and the mixture was stirred at the same temperature for 30 minutes. Morpholine (0.028 g) was added to the reaction mixture, and the mixture was stirred at room temperature for 15 minutes. Water was added to the reaction mixture, and the mixture was extracted with DCM. The extract was concentrated under reduced pressure. The residue was sequentially purified by silica gel column chromatography (eluent: n-hexane/ethyl acetate=40/60 to 0/100) and APS column chromatography (eluent: n-hexane/ethyl acetate=40/60 to 0/100). The obtained product was suspended in n-hexane/ethyl acetate (5/1), and insoluble substances were collected by filtration. The obtained solid was dried under reduced pressure to give the title compound (0.020 g).

### Example 20

### (R)-N-(8-(2-chloro-5-fluorophenoxy)-1,7-dimethyl-2-oxo-1,2,3,4-tetrahydroquinoline-3-yl)b enzamide

Benzoyl chloride (0.063 g) was added to a mixture of Reference Example 70 (0.115 g), DIPEA (0.089 g) and DCM (1.0 mL) under water cooling, and the mixture was stirred under water cooling for 30 minutes. Water was added to the reaction mixture, and the mixture was extracted with DCM. The extract was concentrated under reduced pressure. The residue was purified by APS column chromatography (eluent: n-hexane/ethyl acetate=95/5 to 50/50) to give the title compound (0.066 g).

### Example 21

### 1-((3R^{*},4S^{*})-8-(2-chloro-5-fluorophenoxy)-4-hydroxy-1,7-dimethyl-2-oxo-1,2,3,4-tetrahydro quinoline-3-yl)urea

Methanesulfonic acid (0.049 g) was added to a mixture of Reference Example 72 (0.090 g), ethanol (0.5 mL) and toluene (1.0 mL) at 70°C for 1.5 hours. After the reaction mixture was cooled to room temperature, a saturated sodium bicarbonate aqueous solution was added, and the mixture was extracted with DCM. The extract was concentrated under reduced pressure. The residue was purified by silica gel column chromatography (eluent: n-hexane/ethyl acetate=80/20 to 40/60) to give benzyl ((3R^{*},4S^{*})-8-(2-chloro-5-fluorophenoxy)-4-hydroxy-1,7-dimethyl-2-oxo-1,2,3,4-tetrahydroqu inoline-3-yl)carbamate (0.070 g).

Platinum(IV) oxide (0.010 g) was added to a mixture of the obtained Compound (0.070 g) and ethanol (2.0 mL), and the mixture was stirred under a hydrogen atmosphere at room temperature for 13 hours. The reaction mixture was filtered through Celite, and the filtrate was concentrated under reduced pressure.

Water (0.037 mL), potassium cyanate (0.022 g) and acetic acid (0.014 g) were added to a mixture of the residue and THF (0.5 mL), and the mixture was stirred at room temperature for 2 hours. Water was added to the reaction mixture, and the mixture was extracted with DCM. The extract was concentrated under reduced pressure. The residue was purified by APS column chromatography (eluent: n-hexane/ethyl acetate/methanol=49/49/2 to 0/98/2 to 0/86/14) to give the title compound (0.028 g).

### Example 22

### methyl (R)-(8-(2-chloro-5-fluorophenoxy)-1-methyl-2-oxo-1,2,3,4-tetrahydroquinoline-3-yl)carbama te

Methyl chloroformate (0.022 g) was added to a mixture of Reference Example 75 (0.050 g), DIPEA (0.040 g) and DCM (0.5 mL) at room temperature, and the mixture was stirred at the same temperature for 50 minutes. 1 mol/L hydrochloric acid was added to the reaction mixture, and the mixture was extracted with DCM. The extract was concentrated under reduced pressure. The residue was purified by silica gel column chromatography (eluent: n-hexane/ethyl acetate=50/50 to 0/100) to give the title compound (0.037 g).

### Example 23

### N-((3R)-8-(2-chloro-5-fluorophenoxy)-1-methyl-2-oxo-1,2,3,4-tetrahydroquinoline-3-yl)ami nosulfonamide

tert-Butanol (0.017 g) was added to a mixture of chlorosulfonyl isocyanate (0.033 g) and DCM (0.5 mL) at room temperature, and the mixture was stirred at the same temperature for 30 minutes. A mixture of Reference Example 75 (0.050 g) and DCM (0.5 mL), and subsequently, TEA (0.032 g) were added to the reaction mixture at room temperature, and the mixture was stirred at the same temperature for 1 hour. 1 mol/L hydrochloric acid was added to the reaction mixture, and the mixture was extracted with DCM. The extract was concentrated under reduced pressure.

TFA (0.178 g) was added to a mixture of the residue and DCM (0.5 mL) at room temperature, and the mixture was stirred at the same temperature for 1 hour. The reaction mixture was concentrated under reduced pressure. The residue was purified by silica gel column chromatography (eluent: n-hexane/ethyl acetate=50/50 to 0/100) to give the title compound (0.036 g).

### Example 24

### (R)-8-(2-chloro-5-fluorophenoxy)-1-methyl-3-(2-oxoimidazolidine-1-yl)-3,4-dihydroquinolin e-2(1H)-one

2-chloroethyl isocyanate (0.020 g) was added to a mixture of Reference Example 75 (0.050 g) and THF (1.0 mL) at room temperature, and the mixture was stirred at the same temperature for 1 hour. TEA (0.024 g) was added to the reaction mixture, and the mixture was stirred at room temperature for 1 hour. 2-Chloroethyl isocyanate (0.063 g) was added to the reaction mixture, and the mixture was stirred at room temperature for 1 hour. A saturated ammonium chloride aqueous solution was added to the reaction mixture, and the mixture was extracted with DCM. The extract was concentrated under reduced pressure.

Sodium hydride (about 60%) (0.012 g) was added to a mixture of the residue and THF (1.0 mL) under ice cooling, and the mixture was stirred under ice cooling for 30 minutes and at room temperature for 1 hour. A saturated ammonium chloride aqueous solution was added to the reaction mixture, and the mixture was extracted with DCM. The extract was concentrated under reduced pressure. DCM, and a small amount of methanol and MTBE were added to the residue, and insoluble substances were collected by filtration. The filtrate was concentrated under reduced pressure. The residue was purified by silica gel column chromatography (eluent: n-hexane/ethyl acetate/methanol=50/50/0 to 0/100/0 to 0/90/10) to give the title compound (0.025 g).

### Example 25

### (R)-3-(8-(2-chloro-5-fluorophenoxy)-1-methyl-2-oxo-1,2,3,4-tetrahydroquinoline-3-yl)oxazo lidine-2-one

2-Chloroethyl chloroformate (0.042 g) was added to a mixture of Reference Example 75 (0.063 g), TEA (0.040 g) and THF (0.63 mL) under ice cooling, and the mixture was stirred under ice cooling for 30 minutes. Insoluble substances were collected by filtration, and the mixture was washed with THF (0.63 mL). A 28% sodium methoxide methanol solution (0.075 mL) was added to the filtrate under ice cooling, and the mixture was stirred under ice cooling for 20 minutes. Water and a saturated ammonium chloride aqueous solution were added to the reaction mixture, and the mixture was extracted with DCM. The extract was concentrated under reduced pressure. The residue was purified by silica gel column chromatography (eluent: n-hexane/ethyl acetate=70/30 to 0/100) to give the title compound (0.056 g).

### Example 26

### 8-(2-chloro-5-fluorophenoxy)-1-methyl-2-oxo-1,2,3,4-tetrahydroquinoline-3-yl carbamate

4-Nitrophenyl chloroformate (0.018 g) was added to a mixture of Reference Example 79 (0.023 g), TEA (0.011 g), DMAP (0.002 g) and DCM (0.5 mL) at room temperature, and the mixture was stirred at the same temperature for 3 hours. 28% ammonia water (0.241 mL) was added to the reaction mixture, and the mixture was stirred at room temperature for 30 minutes. A saturated sodium bicarbonate aqueous solution was added to the reaction mixture, and the mixture was extracted with DCM. The extract was washed with a saturated sodium bicarbonate aqueous solution and then concentrated under reduced pressure. The residue was purified by Method A (eluent: n-hexane/ethyl acetate=70/30 to 0/100) to give the title compound (0.019 g).

### Example 27

### 1-(8-(2-chloro-5-fluorophenoxy)-1-methyl-2-thioxo-1,2,3,4-tetrahydroquinoline-3-yl)urea

A Lawesson's reagent (0.126 g) was added to a mixture of Reference Example 75 (0.020 g) and toluene (1.0 mL) at room temperature, and the mixture was stirred at 110°C for 14 hours. The reaction mixture was cooled to room temperature, water was then added, and the mixture was extracted with DCM. The extract was concentrated under reduced pressure. The residue was sequentially purified by silica gel column chromatography (eluent: ethyl acetate/methanol=100/0 to 50/50) and APS column chromatography (eluent: ethyl acetate/methanol=100/0 to 50/50) to give 3-amino-8-(2-chloro-5-fluorophenoxy)-1-methyl-3,4-dihydroquinoline-2(1H)-thione (0.022 g).

Potassium cyanate (0.007 g) and methanesulfonic acid (0.007 g) were added to a mixture of the obtained Compound (0.022 g), water (0.012 mL) and THF (1.0 mL) at room temperature, and the mixture was stirred at the same temperature for 14 hours. A saturated sodium bicarbonate aqueous solution and water were added to the reaction mixture, and the mixture was extracted with DCM. The extract was concentrated under reduced pressure. The residue was purified by silica gel column chromatography (eluent: ethyl acetate/methanol=100/0 to 90/10) to give the title compound (0.005 g).

### Example 28

### 1-(9-(2-chloro-5-fluorophenoxy)-4,5-dihydroimidazo[1,2-a]quinoline-4-yl)urea

Under an argon atmosphere, zinc powder (1.52 g) was added to a mixture of methyl (S)-2-((tert-butoxycarbonyl)amino)-3-iodopropanoate (5.21 g) and DMF (19 mL) under water cooling, and the mixture was stirred at room temperature for 45 minutes. A mixture of Reference Example 73 (3.34 g) and DMF (9.5 mL), and palladium(II) acetate (0.118 g) and Xphos (0.503 g) were added to the reaction mixture at room temperature, and the mixture was stirred at 40°C for 30 minutes and at room temperature for 15 hours. Acetic acid (1.90 g) was added to the reaction mixture, and the mixture was stirred at 70°C for 1.5 hours. After the reaction mixture was cooled to room temperature, water and n-hexane/ethyl acetate (1/1) were added, and the mixture was stirred at room temperature for 10 minutes. Insoluble substances were collected by filtration, and the organic layer of the filtrate was separated. The organic layer was washed with water and a saturated saline solution, then dried over anhydrous magnesium sulfate and concentrated under reduced pressure. The residue was purified by silica gel column chromatography (eluent: n-hexane/ethyl acetate=98/2 to 70/30) to give tert-butyl (R)-(8-(2-chloro-5-fluorophenoxy)-2-oxo-1,2,3,4-tetrahydroquinoline-3-yl)carbamate (4.03 g).

A Lawesson's reagent (4.20 g) was added to a mixture of the obtained Compound (4.03 g) and toluene (99 mL) at room temperature, and the mixture was stirred at 70°C for 30 minutes and at 90°C for 30 minutes. The reaction mixture was concentrated under reduced pressure. The residue was suspended in n-hexane/DCM (2/1), and insoluble substances were collected by filtration. The filtrate was concentrated under reduced pressure. The residue was purified by silica gel column chromatography (eluent: n-hexane/ethyl acetate=98/2 to 75/25) to give tert-butyl (8-(2-chloro-5-fluorophenoxy)-2-thioxo-1,2,3,4-tetrahydroquinoline-3-yl)carbamate (3.05 g).

Aminoacetaldehyde diethyl acetal (0.350 g) was added to a mixture of the obtained Compound (0.555 g), TEA (0.266 g) and ethanol (5.0 mL) at room temperature, and the mixture was stirred at 70°C for 2 hours. The reaction mixture was cooled to room temperature and then concentrated under reduced pressure.

A mixture of the residue and acetic acid (5.0 mL) was stirred at 100°C for 1.5 hours and at 120°C for 4 hours. A 5 mol/L sodium hydroxide aqueous solution (17.4 mL) was added to the reaction mixture under ice cooling, and the mixture was extracted with DCM. The extract was concentrated under reduced pressure. The residue was purified by silica gel column chromatography (eluent: n-hexane/ethyl acetate=90/10 to 50/50) to give tert-butyl (9-(2-chloro-5-fluorophenoxy)-4,5-dihydroimidazo[1,2-a]quinolin-4-yl)carbamate (0.078 g).

TFA (0.414 g) was added to a mixture of the obtained Compound (0.078 g) and DCM (0.78 mL) at room temperature, and the mixture was stirred at the same temperature for 45 minutes. A 5 mol/L sodium hydroxide aqueous solution (0.726 mL) was added to the reaction mixture under ice cooling, and the mixture was extracted with DCM. The extract was concentrated under reduced pressure.

Potassium cyanate (0.019 g) and acetic acid (0.011 g) were added to a mixture of the residue, water (0.078 mL) and THF (0.78 mL) under ice cooling, and the mixture was stirred under ice cooling for 2.5 hours. Water and MTBE were added to the reaction mixture, and insoluble substances were collected by filtration. The obtained solid was washed with water and MTBE and then dried under reduced pressure to give the title compound (0.042 g).

### Example 29

### 1-(9-(2-chloro-5-fluorophenoxy)-1-methyl-4,5-dihydroimidazo[1,2-a]quinolin-4-yl)urea

Under an argon atmosphere, zinc powder (1.52 g) was added to a mixture of methyl (S)-2-((tert-butoxycarbonyl)amino)-3-iodopropanoate (5.21 g) and DMF (19 mL) under water cooling, and the mixture was stirred at room temperature for 45 minutes. A mixture of Reference Example 73 (3.34 g) and DMF (9.5 mL), and palladium(II) acetate (0.118 g) and Xphos (0.503 g) were added to the reaction mixture at room temperature, and the mixture was stirred at 40°C for 30 minutes and at room temperature for 15 hours. Acetic acid (1.90 g) was added to the reaction mixture, and the mixture was stirred at 70°C for 1.5 hours. After the reaction mixture was cooled to room temperature, water and n-hexane/ethyl acetate (1/1) were added, and the mixture was stirred at room temperature for 10 minutes. Insoluble substances were collected by filtration, and the organic layer of the filtrate was separated. The organic layer was washed with water and a saturated saline solution and then concentrated under reduced pressure. The residue was purified by silica gel column chromatography (eluent: n-hexane/ethyl acetate=98/2 to 70/30) to give tert-butyl (R)-(8-(2-chloro-5-fluorophenoxy)-2-oxo-1,2,3,4-tetrahydroquinoline-3-yl)carbamate (4.03 g).

A Lawesson's reagent (4.20 g) was added to a mixture of the obtained Compound (4.03 g) and toluene (99 mL) at room temperature, and the mixture was stirred at 70°C for 30 minutes and at 90°C for 30 minutes. The reaction mixture was concentrated under reduced pressure. The residue was suspended in n-hexane/DCM (2/1), and insoluble substances were collected by filtration. The filtrate was concentrated under reduced pressure. The residue was purified by silica gel column chromatography (eluent: n-hexane/ethyl acetate=98/2 to 75/25) to give tert-butyl (8-(2-chloro-5-fluorophenoxy)-2-thioxo-1,2,3,4-tetrahydroquinoline-3-yl)carbamate (3.05 g).

Propargylamine (1.63 g) was added to a mixture of the obtained Compound (0.418 g) and 1-butanol (7.3 mL), and the mixture was stirred at 100°C for 15 minutes. After the reaction mixture was cooled to room temperature, a saturated ammonium chloride aqueous solution was added, and the mixture was extracted with DCM. The extract was concentrated under reduced pressure. The residue was purified by silica gel column chromatography (eluent: n-hexane/ethyl acetate=90/10 to 55/45) to give tert-butyl (9-(2-chloro-5-fluorophenoxy)-1-methyl-4,5-dihydroimidazo[1,2-a]quinolin-4-yl)carbamate (0.412 g).

TFA (0.514 g) was added to a mixture of the obtained Compound (0.100 g) and DCM (1.0 mL) at room temperature, and the mixture was stirred at the same temperature for 20 minutes. A 5 mol/L sodium hydroxide aqueous solution (0.902 mL) was added to the reaction mixture, and the mixture was extracted with DCM. The extract was concentrated under reduced pressure.

Potassium cyanate (0.024 g) and acetic acid (0.014 g) were added to a mixture of the residue, water (0.100 mL) and THF (1.0 mL) at room temperature, and the mixture was stirred at the same temperature for 2 hours. Water was added to the reaction mixture, and the mixture was extracted with DCM. The extract was concentrated under reduced pressure. The residue was purified by silica gel column chromatography (eluent: n-hexane/ethyl acetate=70/30 to 0/100) to give the title compound (0.049 g).

### Example 30

### 2-(1-methyl-2-oxo-8-(1-phenylethyl)-1,4-dihydroquinazoline-3(2H)-yl)acetamide

A mixture of Reference Example 82 (0.030 g) and DMF (0.5 mL) was added to a mixture of sodium hydride (about 60%) (0.005 g) and DMF (0.5 mL) under ice cooling, and the mixture was stirred under ice cooling for 30 minutes. 2-bromoacetamide (0.016 g) was added to the reaction mixture, and the mixture was stirred at room temperature for 3 hours. A saturated ammonium chloride aqueous solution and water were added to the reaction mixture, and the mixture was extracted with ethyl acetate. The extract was washed with water and a saturated saline solution, then dried over anhydrous magnesium sulfate, and concentrated under reduced pressure. The residue was purified by silica gel column chromatography (eluent: ethyl acetate/methanol=100/0 to 90/10) to give the title compound (0.010 g).

### Example 31

### 1-(8-(2-chloro-5-fluorophenoxy)-1,7-dimethyl-2-oxo-1,2-dihydroquinoline-3-yl)urea

TEA (0.056 g) and DPPA (0.114 g) were added to a mixture of Reference Example 83 (0.100 g), DMF (0.5 mL) and tert-butanol (1.0 mL), and the mixture was stirred at 60°C for 3 hours. After the reaction mixture was cooled to room temperature, a saturated sodium bicarbonate aqueous solution was added, and the mixture was extracted with ethyl acetate. The extract was washed with a saturated saline solution, then dried over anhydrous sodium sulfate, and concentrated under reduced pressure. The residue was purified by Method A (eluent: n-hexane/ethyl acetate=98/2 to 80/20) to give tert-butyl (8-(2-chloro-5-fluorophenoxy)-1,7-dimethyl-2-oxo-1,2-dihydroquinolin-3-yl)carbamate (0.040 g).

TFA (0.211 g) was added to a mixture of the obtained Compound (0.040 g) and DCM (1.0 mL), and the mixture was stirred at room temperature for 13 hours. A saturated sodium bicarbonate aqueous solution was added to the reaction mixture, and the mixture was extracted with DCM. The extract was concentrated under reduced pressure.

Triphosgene (0.027 g) and TEA (0.028 g) were added to a mixture of the residue and DCM (1.0 mL) under ice cooling, and the mixture was stirred under ice cooling for 30 minutes. Ammonium chloride (0.049 g) and DIEPA (0.119 g) were added to the reaction mixture, and the mixture was stirred at room temperature for 30 minutes. Water was added to the reaction mixture, and the mixture was stirred at room temperature for 10 minutes. Insoluble substances were collected by filtration, and the obtained solid was washed with water and MTBE and then dried under reduced pressure to give the title compound (0.014 g).

### Example 32

### 1-(4-(2-chloro-5-fluorophenoxy)-3,5-dimethyl-2-oxo-1,2,3,7b-tetrahydro-1aH-cyclopropa[c] quinoline-1a-yl)urea

Under an argon atmosphere, methyl iodide (0.084 g) was added to a mixture of diethylzinc (1 mol/L in n-hexane) (0.158 mL) and DCM (0.5 mL) under ice cooling, and the mixture was stirred under ice cooling for 10 minutes. A mixture of Example 31 (0.024 g) and DCM (0.5 mL) was added to the reaction mixture under ice cooling, and the mixture was stirred at room temperature for 6 hours. A saturated ammonium chloride aqueous solution was added to the reaction mixture under ice cooling, and the mixture was extracted with DCM. The extract was dried over anhydrous sodium sulfate and concentrated under reduced pressure. The residue was purified by silica gel column chromatography (eluent: n-hexane/ethyl acetate/methanol=30/70/0 to 0/100/0 to 0/95/5) to give the title compound (0.001 g).

### Example 33

### (R)-8-(2-chloro-5-fluorophenoxy)-1-methyl-3-((4-oxo-4,5-dihydro-1H-imidazole-2-yl)amino )-3,4-dihydroquinoline-2(1H)-one

DMAP (0.053 g) was added to a mixture of 2-thioxoimidazolidine-4-one (0.500 g), methyl iodide (2.44 g), DIPEA (1.11 g) and DCM (1.5 mL) at room temperature, and the mixture was stirred at the same temperature for 14 hours. The reaction mixture was poured into water, and the mixture was extracted with DCM. The extract was dried over anhydrous sodium sulfate and concentrated under reduced pressure. The residue was purified by silica gel column chromatography (eluent: n-hexane/ethyl acetate=100/0 to 0/100) to give 2-(methylthio)-1,5-dihydro-4H-imidazole-4-one (0.354 g).

A mixture of the obtained Compound (0.041 g), Reference Example 75 (0.100 g) and ethanol (3.0 mL) was stirred at 140°C for 1 hour under microwave irradiation. The reaction mixture was concentrated under reduced pressure. The residue was sequentially purified by silica gel column chromatography (eluent: n-hexane/ethyl acetate=100/0 to 65/35) and ODS column chromatography (eluent: water/MeCN=70/30 to 10/90) to give the title compound (0.025 g).

### Example 34

### (R)-3-((1H-imidazole-2-yl)amino)-8-(2-chloro-5-fluorophenoxy)-1-methyl-3,4-dihydroquinol ine-2(1H)-one

A mixture of Reference Example 75 (0.150 g), benzoyl isothiocyanate (0.114 g), TEA (0.095 g) and DCM (5.0 mL) was stirred at room temperature for 30 minutes. The reaction mixture was poured into water, and the mixture was extracted with DCM. The extract was dried over anhydrous sodium sulfate and concentrated under reduced pressure.

A mixture of the residue, a 2 mol/L sodium hydroxide aqueous solution (1.17 mL) and THF (5.0 mL) was stirred at room temperature for 3 hours. The reaction mixture was poured into a saturated ammonium chloride aqueous solution, and the mixture was extracted with DCM. The extract was dried over anhydrous sodium sulfate and concentrated under reduced pressure. The residue was purified by silica gel column chromatography (eluent: n-hexane/ethyl acetate=100/0 to 20/80) to give (R)-1-(8-(2-chloro-5-fluorophenoxy)-1-methyl-2-oxo-1,2,3,4-tetrahydroquinoline-3-yl)thiour ea (0.050 g).

A mixture of the obtained Compound (0.050 g), methyl iodide (0.021 g) and acetone (1.3 mL) was stirred at 55°C for 1 hour under microwave irradiation. The reaction mixture was filtered. Insoluble substances were washed with Et₂O and then dried under reduced pressure to give methyl (R)-(8-(2-chloro-5-fluorophenoxy)-1-methyl-2-oxo-1,2,3,4-tetrahydroquinoline-3-yl)carbami midothioate (0.043 g).

A mixture of the obtained Compound (0.021 g), 2,2-diethoxyethane-1-amine (0.007 g) and MeCN (0.5 mL) was stirred at 90°C for 10 hours under microwave irradiation. The reaction mixture was concentrated under reduced pressure.

A mixture of the residue, 6 mol/L hydrochloric acid (0.087 mL) and THF (0.5 mL) was stirred at room temperature for 30 minutes. Water and a saturated sodium bicarbonate aqueous solution were added to the reaction mixture under ice cooling, and the mixture was extracted with DCM. The extract was concentrated under reduced pressure. The residue was sequentially purified by silica gel column chromatography (eluent: n-hexane/ethyl acetate=100/0 to 0/100) and ODS column chromatography (eluent: water/MeCN=70/30 to 10/90) to give the title compound (0.006 g).

### Example 35

### (R)-1-(1-methyl-2-oxo-8-phenethyl-1,2,3,4-tetrahydroquinoline-3-yl)urea

Under an argon atmosphere, 10% Pd/C (0.016 g) was added to a mixture of Reference Example 87 (0.080 g), ethanol (1.5 mL) and THF (1.5 mL) at room temperature, and the mixture was stirred under a hydrogen atmosphere at the same temperature for 5 minutes. The reaction mixture was filtered through Celite, and the filtrate was concentrated under reduced pressure to give tert-butyl

### (R)-(1-methyl-2-oxo-8-vinyl-1,2,3,4-tetrahydroquinoline-3-yl)carbamate (0.081 g).

A mixture of the obtained Compound (0.041 g), bromobenzene (0.042 g), palladium(II) acetate (0.003 g), tris(2-methylphenyl)phosphine (0.008 g), TEA (0.054 g) and MeCN (1.0 mL) was stirred at 140°C for 10 minutes under microwave irradiation. A saturated ammonium chloride aqueous solution was added to the reaction mixture, and the mixture was extracted with ethyl acetate. The extract was dried over anhydrous sodium sulfate and concentrated under reduced pressure. The residue was sequentially purified by silica gel column chromatography (eluent: n-hexane/ethyl acetate=100/0 to 85/15) and ODS column chromatography (eluent: water/MeCN=70/30 to 10/90) to give tert-butyl (R,E)-(1-methyl-2-oxo-8-styryl-1,2,3,4-tetrahydroquinoline-3-yl)carbamate (0.007 g).

Under an argon atmosphere, 10% Pd/C (0.001 g) was added to a mixture of the obtained Compound (0.007 g), methanol (0.5 mL) and THF (0.5 mL) at room temperature, and the mixture was stirred under a hydrogen atmosphere at the same temperature for 2.5 hours. The reaction mixture was filtered through Celite, and the filtrate was concentrated under reduced pressure.

Under an argon atmosphere, TFA (0.042 g) was added to a mixture of the residue and DCM (0.5 mL) at room temperature, and the mixture was stirred at the same temperature for 1 hour. A 2 mol/L sodium hydroxide aqueous solution was added to the reaction mixture under ice cooling, and the mixture was extracted with DCM. The extract was concentrated under reduced pressure.

Under an argon atmosphere, potassium cyanate (0.002 g) and acetic acid (0.001 g) were added to a mixture of the residue, water (0.007 mL) and THF (0.5 mL) under ice cooling, and the mixture was stirred at room temperature for 22 hours. Potassium cyanate (0.002 g) and acetic acid (0.001 g) were added to the reaction mixture, and the mixture was stirred at room temperature for 18 hours. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate/methanol=30/70/0 to 0/100/0 to 0/95/5) to give the title compound (0.004 g).

### Example 36

### (R)-1-(7-(2-chloro-5-fluorobenzyl)-1-methyl-2-oxo-1,2,3,4-tetrahydrothieno[3,4-b]pyridine-3 -yl)urea

Under an argon atmosphere, methanesulfonic acid (0.021 g) was added to a mixture of Reference Example 91(0.034 g), ethanol (0.3 mL) and toluene (0.5 mL) at room temperature, and the mixture was stirred at 80°C for 5 hours and at room temperature for 15 hours. Methanesulfonic acid (0.036 g) was added to the reaction mixture at room temperature, and the mixture was stirred at 80°C for 3 hours. A saturated sodium bicarbonate aqueous solution was added to the reaction mixture under ice cooling, and the mixture was extracted with DCM. The extract was dried over anhydrous sodium sulfate and concentrated under reduced pressure. The residue was purified by APS column chromatography (eluent: n-hexane/ethyl acetate=50/50 to 20/80) to give (R)-3-amino-7-(2-chloro-5-fluorobenzyl)-1-methyl-3,4-dihydrothieno[3,4-b]pyridine-2(1H)-o ne (0.024 g).

Under an argon atmosphere, potassium cyanate (0.008 g) and acetic acid (0.005 g) were added to a mixture of the obtained Compound (0.024 g), water (0.027 mL) and THF (0.5 mL) at room temperature, and the mixture was stirred at the same temperature for 2 hours. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate/methanol=30/70/0 to 0/100/0 to 0/95/5) to give the title compound (0.010 g).

### Example 37

### (R)-1-(3-(2-chloro-5-fluorobenzyl)-4-methyl-5-oxo-4,5,6,7-tetrahydrothieno[3,2-b]pyridine-6 -yl)urea

Potassium cyanate (0.018 g) and acetic acid (0.011 g) were added to a mixture of Reference Example 97 (0.054 g), water (0.060 mL) and THF (0.54 mL) at room temperature, and the mixture was stirred at the same temperature for 16 hours. Water was added to the reaction mixture, and the mixture was extracted with DCM. The extract was concentrated under reduced pressure. The residue was purified by Method A (eluent: n-hexane/ethyl acetate/methanol=70/30/0 to 0/100/0 to 0/85/15). The obtained product was suspended in MTBE, and insoluble substances were collected by filtration. The obtained solid was washed with MTBE and then dried under reduced pressure to give the title compound (0.030 g).

### Example 38

### (R)-1-(3-(2-chloro-5-fluorobenzyl)-2,4-dimethyl-5-oxo-4,5,6,7-tetrahydrothieno[3,2-b]pyridi n-6-yl)urea

Example 38 was synthesized in the same method as in Example 37 except that Reference Example 101 was used in place of Reference Example 97.

### Example 39

### (R)-1-(2-bromo-3-(2-chloro-5-fluorobenzyl)-4-methyl-5-oxo-4,5,6,7-tetrahydrothieno[3,2-b] pyridin-6-yl)urea

NBS (0.006 g) was added to a mixture of Example 37 (0.010 g) and DCM (1.0 mL) under ice cooling, and the mixture was stirred under ice cooling for 45 minutes. A sodium thiosulfate aqueous solution was added to the reaction mixture, and the mixture was extracted with DCM. The extract was concentrated under reduced pressure. The residue was purified by silica gel column chromatography (eluent: n-hexane/ethyl acetate/methanol=70/30/0 to 0/100/0 to 0/90/10) to give the title compound (0.011 g).

### Example 40

### (R)-1-(3-(2-chloro-5-fluorobenzyl)-2-cyano-4-methyl-5-oxo-4,5,6,7-tetrahydrothieno[3,2-b]p yridin-6-yl)urea

Under an argon atmosphere, bis(tri-tert-butylphosphine)palladium(0) (0.001 g) was added to a mixture of Example 39 (0.009 g), zinc(II) cyanide (0.005 g), zinc powder (0.0004 g) and DMF (1.0 mL), and the mixture was stirred at 80°C for 2 hours. After the reaction mixture was cooled to room temperature, a saturated sodium bicarbonate aqueous solution and water were added, and the mixture was extracted twice with n-hexane/ethyl acetate (1/1) and twice with n-hexane/ethyl acetate (1/2). The combined extract was washed with water and a saturated saline solution, then dried over anhydrous magnesium sulfate, and concentrated under reduced pressure. The residue was purified by silica gel column chromatography (eluent: n-hexane/ethyl acetate/methanol=70/30/0 to 0/100/0 to 0/80/20), Method A (eluent: ethyl acetate/methanol=100/0 to 95/5), Method B (eluent: n-hexane/ethyl acetate=80/20 to 0/100), and PLC (developing solvent: ethyl acetate) to give the title compound (0.002 g).

### Example 41

### (R)-1-(3-(2-chloro-5-fluorobenzyl)-2-(hydroxymethyl)-4-methyl-5-oxo-4,5,6,7-tetrahydrothie no[3,2-b]pyridin-6-yl)urea

A mixture of Boc₂O (0.021 g) and DCM (0.5 mL) was added to a mixture of Reference Example 106 (0.024 g) and DCM (0.5 mL) at room temperature, and the mixture was stirred at 2 hours. A saturated ammonium chloride aqueous solution was added to the reaction mixture, and the mixture was extracted with DCM. The extract was concentrated under reduced pressure. The residue was purified by Method A (eluent: n-hexane/ethyl acetate=100/0 to 70/30) to give tert-butyl (R)-(3-(2-chloro-5-fluorobenzyl)-2-(ethoxymethyl)-4-methyl-5-oxo-4,5,6,7-tetrahydrothieno[ 3,2-b]pyridin-6-yl)carbamate (0.032 g).

TFA (0.109 g) was added to a mixture of the obtained Compound (0.023 g) and DCM (0.5 mL) at room temperature, and the mixture was stirred at the same temperature for 8 hours. A 5 mol/L sodium hydroxide aqueous solution (0.190 mL) was added to the reaction mixture under ice cooling, and the mixture was extracted with DCM. The extract was concentrated under reduced pressure.

Potassium cyanate (0.005 g) and acetic acid (0.003 g) were added to a mixture of the residue, water (0.050 mL) and THF (0.5 mL) under ice cooling, and the mixture was stirred at room temperature for 22 hours. Potassium cyanate (0.005 g) and acetic acid (0.003 g) were added to the reaction mixture at room temperature, and the mixture was stirred at the same temperature for 30 hours. Water was added to the reaction mixture, and the mixture was extracted with DCM. The extract was concentrated under reduced pressure. The residue was sequentially purified by silica gel column chromatography (eluent: n-hexane/ethyl acetate/methanol=50/50/0 to 0/100/0 to 0/80/20) and PLC (developing solvent: ethyl acetate/methanol=95/5) to give the title compound (0.002 g).

### Example 42

### 1-(3-(2-chloro-5-fluorobenzoyl)-4-methyl-5-oxo-4,5,6,7-tetrahydrothieno[3,2-b]pyridin-6-yl) urea

A mixture of Reference Example 112 (0.030 g) and hydrogen chloride (4 mol/L in 1,4-dioxane) (1.0 mL) was stirred at room temperature for 1 hour. The reaction mixture was concentrated under reduced pressure.

A mixture of the residue and DMF (1.0 mL) was stirred at 70°C for 1 hour. Potassium carbonate (0.011 g) and methyl iodide (0.014 g) were added to the reaction mixture at room temperature, and the mixture was stirred at the same temperature for 1 hour. Water was added to the reaction mixture, and the mixture was extracted with ethyl acetate. The extract was washed with water and a saturated saline solution, then dried over anhydrous sodium sulfate, and concentrated under reduced pressure. The residue was purified by silica gel column chromatography (eluent: n-hexane/ethyl acetate=98/2 to 50/50) to give benzyl (3-(2-chloro-5-fluorobenzoyl)-4-methyl-5-oxo-4,5,6,7-tetrahydrothieno[3,2-b]pyridin-6-yl)ca rbamate (0.016 g).

TMSCl (0.011 g) was added to a mixture of sodium iodide (0.016 g) and MeCN (0.5 mL) at room temperature, and the mixture was stirred at the same temperature for 15 minutes. The mixture of benzyl (3-(2-chloro-5-fluorobenzoyl)-4-methyl-5-oxo-4,5,6,7-tetrahydrothieno[3,2-b]pyridin-6-yl)ca rbamate (0.010 g) and MeCN (0.5 mL) was added to the reaction mixture at room temperature, and the mixture was stirred at 50°C for 2 hours. A 1 mol/L sodium thiosulfate aqueous solution was added to the reaction mixture under ice cooling, and the mixture was extracted with ethyl acetate. The extract was dried over anhydrous sodium sulfate and concentrated under reduced pressure.

A mixture of the residue, water (0.008 g), potassium cyanate (0.003 g), acetic acid (0.003 g) and THF (1.0 mL) was stirred at room temperature for 63 hours. Water was added to the reaction mixture, and the mixture was extracted with ethyl acetate. The extract was dried over anhydrous sodium sulfate and concentrated under reduced pressure. The residue was purified by ODS column chromatography (eluent: water/MeCN=70/30 to 10/90) to give the title compound (0.003 g).

### Example 43

### 1-((6R)-3-(1-(2-chloro-5-fluorophenyl)ethyl)-4-methyl-5-oxo-4,5,6,7-tetrahydroisothiazolo[4 ,5-b]pyridine-6-yl)urea

A mixture of Reference Example 120 (0.012 g), methanesulfonic acid (0.124 g), ethanol (0.4 mL) and toluene (0.2 mL) was stirred at room temperature for 30 minutes and at 70°C for 1 hour. After the reaction mixture was cooled to room temperature, water and ethyl acetate were added, and the aqueous layer was separated. A saturated sodium bicarbonate aqueous solution was added to the aqueous layer, and the mixture was extracted with DCM. The extract was concentrated under reduced pressure.

A mixture of the residue, water (0.005 g), potassium cyanate (0.002 g), acetic acid (0.002 g) and THF (1.0 mL) was stirred at room temperature for 3 hours. Water was added to the reaction mixture, and the mixture was extracted with DCM. The extract was concentrated under reduced pressure. The residue was purified by ODS column chromatography (eluent: 0.2% NH₃ in water/0.2% NH₃ in MeCN=68.3/31.7 to 5/95) to give the title compound (0.001 g).

The following tables show the structural formulae, physical property values, and TSHR antagonist activity (refer to Test Example 1) of examples.

**[Table 15]**

| Ex. No. | Str. | Phys. data | IC₅₀ |
|---|---|---|---|
| 1 | 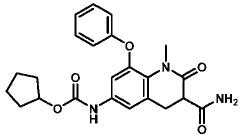 | MS m/z : 424 (M+H)⁺ | B |
| 2 | 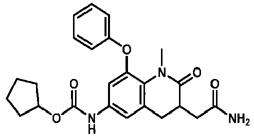 | ¹H-NMR (CDCl₃) δ ppm : 1.50-1.95 (9H, m), 2. 47 (1H, dd, J=4.9, 14.5 Hz), 2.72-3.01 (3H, m), 3.36 (3H, s), 5.09-5.21 (1H, m), 5.32-5.52 (1H, m), 6.25-6.42 (1H, m), 6.58-6.75 (1H, m), 6.86-7. 17 (5H, m), 7.28-7.38 (2H, m) | A |
| | | MS m/z : 438 (M+H)⁺ | |
| 3 | 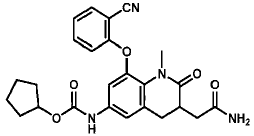 | ¹H-NMR (DMSO-d6) δ ppm : 1.47-1.93 (8H, m), 2.12 (1H, dd, J=8. 2, 15.5 Hz), 2.56-2.91 (4H, m), 3. 16 (3H, s), 5.00-5.09 (1H, m), 6.81-6.94 (2H, m), 7.13-7.45 (4H, m), 7.61-7.71 (1H, m), 7.88-7.96 (1H, m), 9.71 (1H, br) | A |
| | | MS m/z : 463 (M+H)⁺ | |
| 4 | 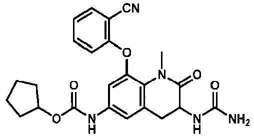 | MS m/z : 464 (M+H)⁺ | A |
| 5 | 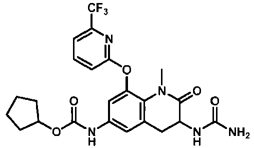 | MS m/z : 508 (M+H)⁺ | A |
| 6 | 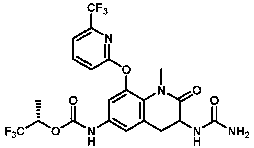 | MS m/z : 536 (M+H)⁺ | A |

**[Table 16]**

| Ex. No. | Str. | Phys. data | IC₅₀ |
|---|---|---|---|
| 7 | 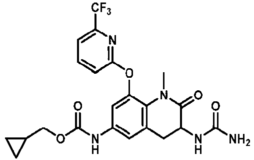 | MS m/z : 494 (M+H)⁺ | A |
| 8 | 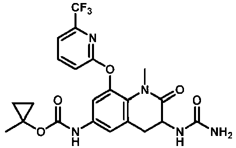 | MS m/z : 494 (M+H)⁺ | A |
| 9 | 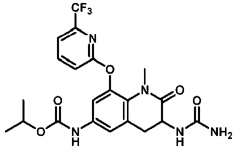 | MS m/z : 482 (M+H)⁺ | A |
| 10 | 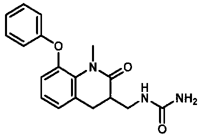 | MS m/z : 326 (M+H)⁺ | B |
| 11 | 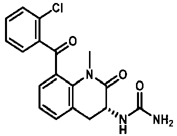 | MS m/z : 358 (M+H)⁺ | A |
| 12 | 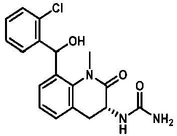 | MS m/z : 360 (M+H)⁺ | C |

**[Table 17]**

| Ex. No. | Str. | Phys. data | IC₅₀ |
|---|---|---|---|
| 13 | 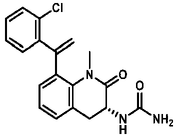 | ¹H-NMR (CDCl₃) δ ppm : 2.64-2.79 (1H, m), 3.28 (3H, s), 3.37 (1H, dd, J=5.3, 14.8 Hz), 4.21-4.32 (1H, m), 4.44 (2H, s), 5. 66-5. 90 (3H, m), 6.96-7.56 (7H, m) | A |
| | | MS m/z : 356 (M+H)⁺ | |
| 14 | 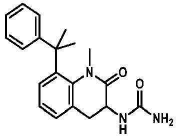 | MS m/z : 338 (M+H)⁺ | B |
| 15 | 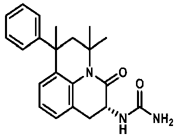 | ¹H-NMR (CDCl₃) δ ppm : 0.95 (3H, s), 1. 53 (3H, s), 1.73 (3H, s), 2.09 (1H, d, J=13.9 Hz), 2.38 (1H, d, J=13.9 Hz), 2.67-2.79 (1H, m), 3.38 (1H, dd, J=5.4, 14.4 Hz), 4.20-4.33 (1H, m), 4.47 (2H, s), 5.91 (1H, d, J=4.4 Hz), 6.86-6.99 (2H, m), 7.05-7.37 (6H, m) MS m/z : 364 (M+H)⁺ | A |
| 16 | 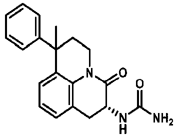 | MS m/z : 336 (M+H)⁺ | B |
| 17 | 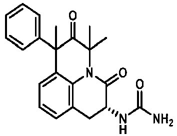 | ¹H-NMR (CDCl₃) δ ppm : 1.46 (3H, s), 1. 52 (3H, s), 1.82 (3H, s), 2.68-2.81 (1H, m), 3.47 (1H, dd, J=5.6, 15.1 Hz), 4.22-4.35 (1H, m), 4.41 (2H, s), 5.75-5.86 (1H, m), 6.75-6.86 (2H, m), 7.13-7.41 (6H, m) | A |
| | | MS m/z : 378 (M+H)⁺ | |
| 18 | 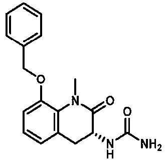 | MS m/z : 326 (M+H)⁺ | C |

**[Table 18]**

| Ex. No. | Str. | Phys. data | IC₅₀ |
|---|---|---|---|
| 19 | 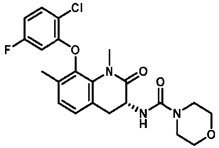 | MS m/z : 448 (M+H)⁺ | A |
| 20 | 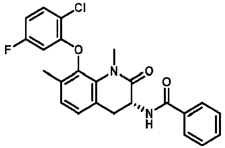 | MS m/z : 439 (M+H)⁺ | A |
| 21 | 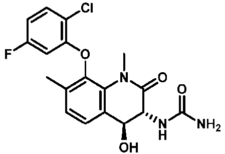 | ¹H-NMR (DMSO-d6) δ ppm : 2.06 (3H, s), 3. 17 (3H, s), 4. 38 (1H, dd, J=7.2, 10.8 Hz), 4.57 (1H, dd, J=5.0, 10.8 Hz), 5.94 (2H, br), 6. 09-6. 45 (3H, m), 6. 90-7.01 (1H, m), 7.23 (1H, d, J=7.9 Hz), 7.39 (1H, d, J=7.9 Hz), 7. 65 (1H, dd, J=5. 6, 8.8 Hz) | A |
| | | MS m/z : 394 (M+H)⁺ | |
| 22 | 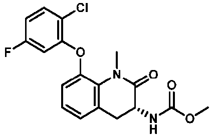 | MS m/z : 379 (M+H)⁺ | A |
| 23 | 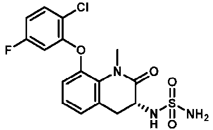 | MS m/z : 398 (M-H)⁻ | A |
| 24 | 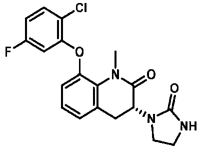 | MS m/z : 390 (M+H)⁺ | B |

**[Table 19]**

| Ex. No. | Str. | Phys. data | IC₅₀ |
|---|---|---|---|
| 25 | 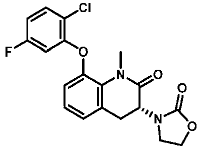 | MS m/z : 391 (M+H)⁺ | C |
| 26 | 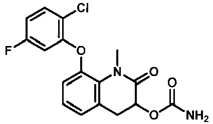 | ¹H-NMR (CDCl₃) δ ppm : 3.06-3.27 (2H, m), 3.43 (3H, s), 4.93 (2H, br), 5.25-5.35 (1H, m), 6.43-6.51 (1H, m), 6.75-6.91 (2H, m), 7.04-7.13 (2H, m), 7.42 (1H, dd, J=5.8, 8.9 Hz) | A |
| | | MS m/z : 365 (M+H)⁺ | |
| 27 | 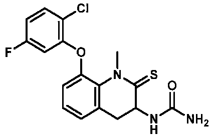 | ¹H-NMR (CDCl₉) δ ppm : 2.58-2.73 (1H, m), 3.37 (1H, dd, J=4.7, 14.9 Hz), 3.86-3.93 (3H, m), 4.27-4.41 (1H, m), 4.55 (2H, br), 6.12-6.34 (2H, m), 6.78-6.89 (2H, m), 7.06-7.21 (2H, m), 7.44 (1H, dd, J=5.7, 8.9 Hz) | A |
| | | MS m/z : 380 (M+H)⁺ | |
| 28 | 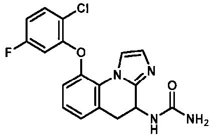 | MS m/z : 373 (M+H)⁺ | B |
| 29 | 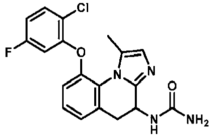 | MS m/z : 387 (M+H)⁺ | B |
| 30 | 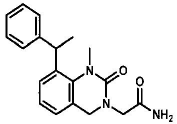 | MS m/z : 324 (M+H)⁺ | C |

**[Table 20]**

| Ex. No. | Str. | Phys. data | IC₅₀ |
|---|---|---|---|
| 31 | 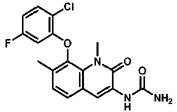 | MS m/z : 376 (M+H)⁺ | C |

**[Table 21]**

| Ex. No. | Str. | Phys. data | IC₅₀ |
|---|---|---|---|
| 32 | 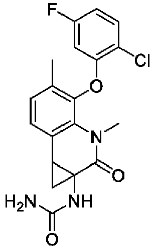 | MS m/z : 390 (M+H)⁺ | B |
| 33 | 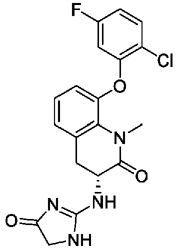 | MS m/z : 403 (M+H)⁺ | B |
| 34 | 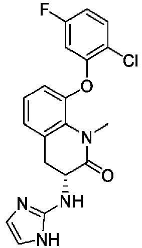 | MS m/z : 387 (M+H)⁺ | B |

**[Table 22]**

| Ex. No. | Str. | Phys. data | IC₅₀ |
|---|---|---|---|
| 35 | 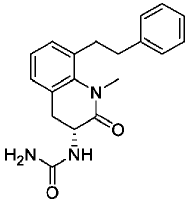 | MS m/z : 324 (M+H)⁺ | B |
| 36 | 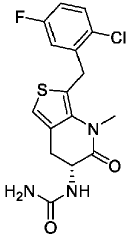 | MS m/z : 368 (M+H)⁺ | A |
| 37 | 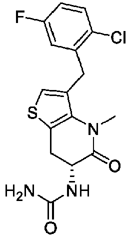 | MS m/z : 368 (M+H)⁺ | A |
| 38 | 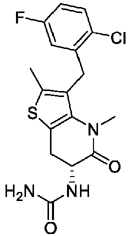 | MS m/z : 382 (M+H)⁺ | A |
| 39 | 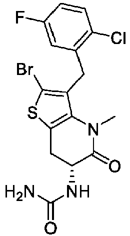 | MS m/z : 447 (M+H)⁺ | A |

**[Table 23]**

| Ex. No. | Str. | Phys. data | IC₅₀ |
|---|---|---|---|
| 40 | 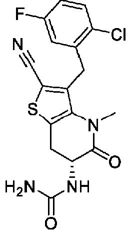 | MS m/z : 391 (M-H)⁻ | A |
| 41 | 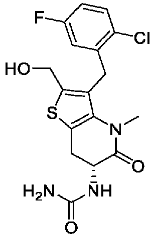 | MS m/z : 398 (M+H)⁺ | A |
| 42 | 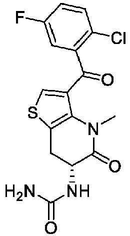 | MS m/z : 382 (M+H)⁺ | A |
| 43 | 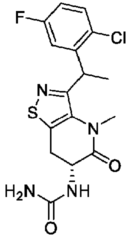 | ¹H-NMR (CDCl₃) δ ppm : 1.73 (3H, d, J=6.8 Hz), 2.70 (1H, dd, J=14.0, 15.6 Hz), 3.30-3.50 (3H, m), 3.77 (1H, dd, J=6.0, 15.6 Hz), 4.20-4.35 (1H, m), 4.48 (2H, br), 4.86 (1H, q, J=6.8 Hz), 5.78 (1H, d, J=4.0 Hz), 6.48 (1H, dd, J=2.8, 9.2 Hz), 6.80-6.95 (1H, m), 7.32 (1H, dd, J=5.2, 8.8 Hz) | A |
| | | MS m/z : 383 (M+H)⁺ | |

The notation of stereochemistry of Example 21 in the table indicates a relative configuration.

### Test Example 1

### Measurement of antagonist activity using TSH-induced cAMP production in human TSHR-stably expressing CHO cells as an indicator

The gene sequence (reference number NM_000369.2) of human TSHR was inserted into the multiple cloning site of pcDNA3.1(+). The prepared plasmid vector was introduced into CHO cells using a lipofection method to establish human TSHR-stably expressing CHO cells. The obtained cells were seeded at 5 x 10⁴ cells/well in a 96-well poly-D-lysine-coated plate, and cultured in an F12 culture medium containing 10% FBS, 400 µg/mL of G418, 50 U/mL of penicillin and 50 µg/mL of streptomycin under conditions of 37°C and 5% CO₂ for 1 day. The culture medium was removed, and the cells were then washed twice with 100 µL of an assay buffer (Hanks' Balanced Salt Solution containing 20 mM HEPES and 1 mM IBMX) per well. 30 µL of an assay buffer containing a test compound was added to the well, and incubation was performed at room temperature for 15 minutes. Then, 30 µL of an assay buffer containing human TSH (a final concentration of 50 ng/mL, R&D Systems, Inc.) was added, and incubation was performed at 37°C for 1 hour. The supernatant was removed, a Lysis and Detection Buffer 2 (Cisbio) was added, and incubation was performed at room temperature for 1 hour to prepare a cell lysate. According to the instruction manual of a cAMP Gs HiRange kit (Cisbio), the cell lysate, d2-labeled cAMP and anti-cAMP Europium Cryptate labeled antibodies (Cisbio) were reacted in a 384-well white microplate. Then, a fluorescence intensity ratio (measurement wavelength 665 nm/620 nm) was measured using a multi-plate reader (PHERAstar FSX, BMG LABTECH Japan). The fluorescence intensity ratio of each sample was converted to a cAMP content using a standard curve. The cAMP content was converted as a percentage of the control value to calculate a cAMP production rate. The cAMP production rate was plotted against the test compound concentration using Prism (Graph Pad Software Inc.) to calculate the IC₅₀ value. The IC₅₀ of each test compound is shown in the table. In the tables, IC₅₀<3.0 µM was indicated as A, and 3.0 µM≤IC₅₀<30 µM was indicated as B. If activity was observed but the IC₅₀ value could not be calculated, it was indicated as C.

As shown in the above tables, it was found that the compound of the present invention had human TSHR antagonist activity.

### [Industrial Applicability]

The compound of the present invention or a pharmacologically acceptable salt thereof is useful as a therapeutic agent for thyroid-related diseases because it has TSHR antagonist activity.

## Claims

1. A compound represented by Formula (A-I): 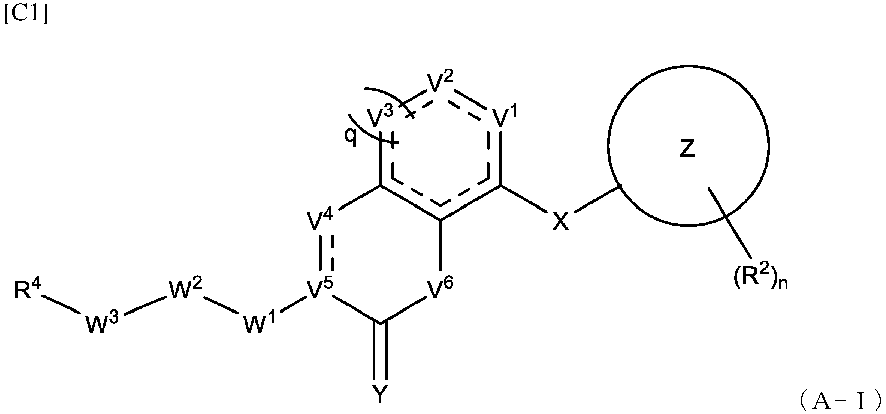 [wherein,
the ring Z is C₆₋₁₀ aryl, 5- or 6-membered heteroaryl, C₃₋₈ cycloalkyl, or 3- to 8-membered heterocycloalkyl;
V¹ is CR^{V1}R^{V1'}, CR^{V1}, NR^{V1}, N, O or S;
V² is CR^{V2}R^{V2'}, CR^{V2}, NR^{V2}, N, O or S;
V³ is CR^{V3}R^{V3'}, CR^{V3}, NR^{V3}, N, O, or S;
V⁴ is a single bond, CR^{T1}R^{T2}, CR^{T1}, O, NR^{T3}, or N;
V⁵ is CR³, C, or N;
V⁶ is NR¹;
X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0}, R^{X0'}, R^{X1}, and R^{X2} are each independently a hydrogen atom, a halogen atom, hydroxy, C₁₋₆ alkyl, or halo C₁₋₆ alkyl;
R^{X3} is a hydrogen atom, C₁₋₆ alkyl, or halo C₁₋₆ alkyl;
R^{X0} may, together with R^{X0'}, form a 3- to 8-membered saturated carbocyclic ring which is unsubstituted or substituted with 1 to 6 groups selected from Substituent Group B;
R^{X1} may, together with R^{X2}, form a 3- to 8-membered saturated carbocyclic ring which is unsubstituted or substituted with 1 to 6 groups selected from Substituent Group B;
R^{X1} may, together with R^{X3}, form a 3- to 8-membered saturated heterocyclic ring which is unsubstituted or substituted with 1 to 6 groups selected from Substituent Group B;
R¹ is a hydrogen atom, C₁₋₆ alkyl, or halo C₁₋₆ alkyl;
R¹ may, together with R^{X0}, R^{X1}, or R^{X3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
when there are two or more groups selected from Substituent Group B, these groups may be the same as or different from each other;
R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
R^{X0} may, together with R², form a 5- to 8-membered ring which is unsubstituted or substituted with 1 to 6 groups selected from Substituent Group B;
R^{X1} may, together with R², form a 5- to 8-membered ring which is unsubstituted or substituted with 1 to 6 groups selected from Substituent Group B;
R^{X3} may, together with R², form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
q is 0 or 1;
R^{V1}, R^{V1'}, R^{V2}, R^{V2'}, R^{V3}, and R^{V3'} are each independently a hydrogen atom, a halogen atom, hydroxy, cyano, C₁₋₆ alkyl, C₁₋₆ alkoxy, halo C₁₋₆ alkyl, hydroxy C₁₋₆ alkyl, C₁₋₆ alkoxy C₁₋₆ alkyl, C₆₋₁₀ aryl C₁₋₆ alkyl, halo C₁₋₆ alkoxy, C₆₋₁₀ aryl C₁₋₆ alkoxy, NR^{V4}COR^{V5}, NR^{V4}COOR^{V6}, or NR^{V4}CONR^{V7}R^{V8};
R^{V4} is a hydrogen atom or C₁₋₆ alkyl;
R^{V5}, R^{V6}, R^{V7}, and R^{V8} are each independently a hydrogen atom or a group selected from the group consisting of the following (i) to (ix):
(i) C₁₋₆ alkyl which is unsubstituted or substituted with 1 to 6 groups selected from Substituent Group A,
(ii) C₃₋₈ cycloalkyl which is unsubstituted or substituted with 1 to 6 groups selected from Substituent Group A,
(iii) C₃₋₈ cycloalkyl C₁₋₆ alkyl which is unsubstituted or substituted with 1 to 6 groups selected from Substituent Group A,
(iv) C₆₋₁₀ aryl which is unsubstituted or substituted with 1 to 6 groups selected from Substituent Group A,
(v) C₆₋₁₀ aryl C₁₋₆ alkyl which is unsubstituted or substituted with 1 to 6 groups selected from Substituent Group A,
(vi) 3- to 8-membered heterocycloalkyl which is unsubstituted or substituted with 1 to 6 groups selected from Substituent Group A,
(vii) 3- to 8-membered heterocycloalkyl C₁₋₆ alkyl which is unsubstituted or substituted with 1 to 6 groups selected from Substituent Group A,
(viii) 5- to 10-membered heteroaryl which is unsubstituted or substituted with 1 to 6 groups selected from Substituent Group A, and
(ix) 5- to 10-membered heteroaryl C₁₋₆ alkyl which is unsubstituted or substituted with 1 to 6 groups selected from Substituent Group A;
Substituent Group A is a group consisting of a halogen atom, cyano, hydroxy, N(C₁₋₆ alkyl)₂, C₁₋₆ alkyl, halo C₁₋₆ alkyl, C₁₋₆ alkoxy, halo C₁₋₆ alkoxy, C₆₋₁₀ aryl C₁₋₆ alkoxy, and C₃₋₈ cycloalkyl;
when there are two or more groups selected from Substituent Group A, these groups may be the same as or different from each other;
R^{T1} and R^{T2} are each independently a hydrogen atom, a halogen atom, hydroxy, C₁₋₆ alkyl, or halo C₁₋₆ alkyl;
R^{T3} is a hydrogen atom, C₁₋₆ alkyl, or halo C₁₋₆ alkyl;
R³ is a hydrogen atom, a halogen atom, or C₁₋₆ alkyl;
R⁴ is C₁₋₆ alkyl, halo C₁₋₆ alkyl, C₁₋₆ alkoxy C₁₋₆ alkyl, OR⁵, NR⁵R^{5'}, C₃₋₈ cycloalkyl, 3- to 8-membered heterocycloalkyl which is unsubstituted or substituted with 1 to 6 groups selected from Substituent Group C, C₆₋₁₀ aryl or 5- or 6-membered heteroaryl which is unsubstituted or substituted with 1 to 4 groups selected from Substituent Group C;
Substituent Group C is a group consisting of a halogen atom, cyano, hydroxy, C₁₋₆ alkyl, halo C₁₋₆ alkyl, hydroxy C₁₋₆ alkyl, C₁₋₆ alkoxy, halo C₁₋₆ alkoxy, oxo, CONH₂, CONH-(C₁₋₆ alkyl), and CONH-(C₃₋₈ cycloalkyl);
when there are two or more groups selected from Substituent Group C, these groups may be the same as or different from each other;
R⁵ and R^{5'} are each independently a hydrogen atom, C₁₋₆ alkyl, halo C₁₋₆ alkyl, or C₃₋₈ cycloalkyl;
R⁵ may, together with R^{5'}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
R³ may, together with R^{T1}, R^{T2}, or R^{T3}, form a 3- to 10-membered ring which is unsubstituted or substituted with 1 to 6 groups selected from Substituent Group B;
R³ may, together with R⁵, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
R⁴ may, together with R^{T1}, R^{T2}, or R^{T3}, form a 3- to 10-membered ring which is unsubstituted or substituted with 1 to 6 groups selected from Substituent Group B;
R⁵ may, together with R^{T1}, R^{T2}, or R^{T3}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
R^{V7} may, together with R^{V8}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
W¹ is CR^{W1}R^{W2}, O, NR^{W3}, or a single bond;
W² is CR^{W1}R^{W2}, O, NR^{W3}, or a single bond;
R^{W1} and R^{W2} are each independently a hydrogen atom, a halogen atom, C₁₋₆ alkyl, or halo C₁₋₆ alkyl;
R^{W3} is a hydrogen atom, C₁₋₆ alkyl, or halo C₁₋₆ alkyl;
W³ is CO, CNR^{W4}, SO, SO₂, or a single bond;
R^{W4} is a hydrogen atom, C₁₋₆ alkyl, cyano, hydroxy, C₁₋₆ alkoxy, CO-(C₁₋₆ alkyl), or SO₂-(C₁₋₆ alkyl);
n is an integer of 0 to 3;
when n is 2 or 3, these R²s may be the same as or different from each other;
R⁴ and R⁵ may, together with R^{W1} or R^{W3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
Y is O, S, or NR^{Y};
R^{Y} is a hydrogen atom or C₁₋₆ alkyl;
R^{Y} may, together with R¹, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
[C2] - - -
indicates a single bond or a double bond]
or a pharmacologically acceptable salt thereof;
(provided that, in Formula (A-I), when W¹ is a single bond; W² is CH₂ or NH; W³ is CO; and R⁴ is C₁₋₆ alkyl, halo C₁₋₆ alkyl, NR⁵R^{5'}, or C₃₋₈ cycloalkyl, any of the following (a) to (f) is satisfied;
(a) R^{T1} and R^{T2} are not both hydrogen atoms
(b) X is not O, and when X is CHR^{X0}, R^{X0} is neither a hydrogen atom nor C₁₋₆ alkyl
(c) R¹ is halo C₁₋₆ alkyl
(d) V² is CR^{V2}, and R^{V2} is halo C₁₋₆ alkoxy, NR^{V4}COR^{V5}, NR^{V4}COOR^{V6}, or NR^{V4}CONR^{V7}R^{V8}
(e) Y is not O
(f) q is 0).

2. The compound according to claim 1,
wherein the compound is represented by Formula (A-II): 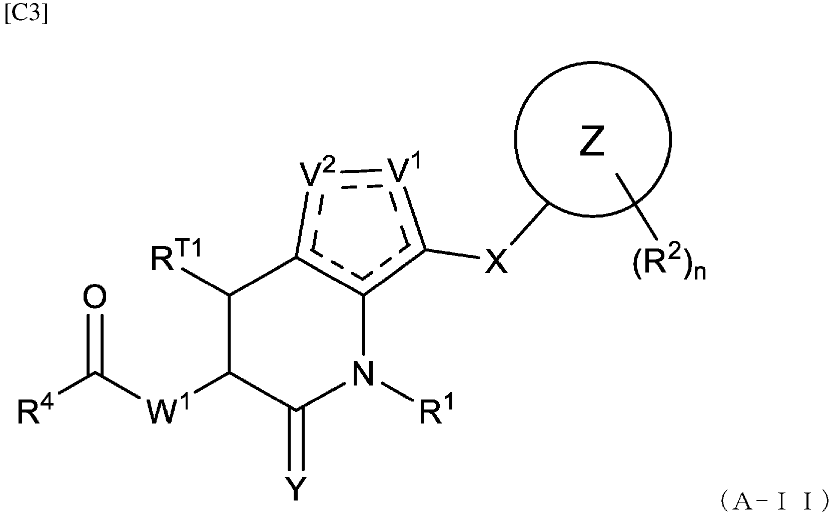 [wherein,
the ring Z is C₆₋₁₀ aryl, 5- or 6-membered heteroaryl, C₃₋₈ cycloalkyl, or 3- to 8-membered heterocycloalkyl;
V¹ is CR^{V1}R^{V1'}, CR^{V1}, NR^{V1}, N, O or S;
V² is CR^{V2}R^{V2'}, CR^{V2}, NR^{V2}, N, O or S;
R^{V1}, R^{V1'}, R^{V2}, and R^{V2'} are each independently a hydrogen atom, a halogen atom, hydroxy, cyano, C₁₋₆ alkyl, C₁₋₆ alkoxy, halo C₁₋₆ alkyl, hydroxy C₁₋₆ alkyl, C₁₋₆ alkoxy C₁₋₆ alkyl, halo C₁₋₆ alkoxy, NR^{V4}COR^{V5}, NR^{V4}COOR^{V6}, or NR^{V4}CONR^{V7}R^{V8};
R^{V4} is a hydrogen atom or C₁₋₆ alkyl;
R^{V5}, R^{V6}, R^{V7}, and R^{V8} are each independently a hydrogen atom or a group selected from the following groups (i) to (v):
(i) C₁₋₆ alkyl which is unsubstituted or substituted with 1 to 6 groups selected from Substituent Group A,
(ii) C₃₋₈ cycloalkyl which is unsubstituted or substituted with 1 to 6 groups selected from Substituent Group A,
(iii) C₃₋₈ cycloalkyl C₁₋₆ alkyl which is unsubstituted or substituted with 1 to 6 groups selected from Substituent Group A,
(iv) C₆₋₁₀ aryl which is unsubstituted or substituted with 1 to 6 groups selected from Substituent Group A, and
(v) C₆₋₁₀ aryl C₁₋₆ alkyl which is unsubstituted or substituted with 1 to 6 groups selected from Substituent Group A;
Substituent Group A is a group consisting of a halogen atom, C₁₋₆ alkyl, halo C₁₋₆ alkyl, C₁₋₆ alkoxy, halo C₁₋₆ alkoxy, and C₃₋₈ cycloalkyl;
when there are two or more groups selected from Substituent Group A, these groups may be the same as or different from each other;
X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0}, R^{X0'}, R^{X1}, and R^{X2} are each independently a hydrogen atom, a halogen atom, hydroxy, C₁₋₆ alkyl, or halo C₁₋₆ alkyl;
R^{X3} is a hydrogen atom, C₁₋₆ alkyl, or halo C₁₋₆ alkyl;
R^{X0} may, together with R^{X0'}, form a 3- to 8-membered saturated carbocyclic ring which is unsubstituted or substituted with 1 to 6 groups selected from Substituent Group B;
R^{X1} may, together with R^{X2}, form a 3- to 8-membered saturated carbocyclic ring which is unsubstituted or substituted with 1 to 6 groups selected from Substituent Group B;
R^{X1} may, together with R^{X3}, form a 3- to 8-membered saturated heterocyclic ring which is unsubstituted or substituted with 1 to 6 groups selected from Substituent Group B;
R¹ is a hydrogen atom, C₁₋₆ alkyl, or halo C₁₋₆ alkyl;
R¹ may, together with R^{X0}, R^{X1}, or R^{X3}, form a 5- to 8-membered saturated heterocyclic ring which is unsubstituted or substituted with 1 to 6 groups selected from Substituent Group B or a 5- to 8-membered unsaturated heterocyclic ring which is unsubstituted or substituted with 1 to 6 groups selected from Substituent Group B;
Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
when there are two or more groups selected from Substituent Group B, these groups may be the same as or different from each other;
R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
R^{T1} is a hydrogen atom, a halogen atom, hydroxy, C₁₋₆ alkyl, or halo C₁₋₆ alkyl;
R⁴ is C₁₋₆ alkyl, halo C₁₋₆ alkyl, C₁₋₆ alkoxy C₁₋₆ alkyl, OR⁵, NR⁵R^{5'}, C₃₋₈ cycloalkyl, or 3- to 8-membered heterocycloalkyl;
R⁵ and R^{5'} are each independently a hydrogen atom, C₁₋₆ alkyl, halo C₁₋₆ alkyl, or C₃₋₈ cycloalkyl;
R⁵ may, together with R^{5'}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
W¹ is CR^{W1}R^{W2}, O, NR^{W3}, or a single bond;
R^{W1} and R^{W2} are each independently a hydrogen atom, a halogen atom, C₁₋₆ alkyl, or halo C₁₋₆ alkyl;
R^{W3} is a hydrogen atom, C₁₋₆ alkyl, or halo C₁₋₆ alkyl;
n is an integer of 0 to 3;
when n is 2 or 3, these R²s may be the same as or different from each other; and
Y is O or S;
[C4] - - -
indicates a single bond or a double bond]
or a pharmacologically acceptable salt thereof.

3. The compound according to claim 2,
wherein R^{T1} is a hydrogen atom,
or a pharmacologically acceptable salt thereof.

4. The compound according to claim 3,
wherein R¹ is a hydrogen atom or C₁₋₆ alkyl,
or a pharmacologically acceptable salt thereof.

5. The compound according to claim 4,
wherein the ring Z is C₆₋₁₀ aryl or 5- or 6-membered heteroaryl,
or a pharmacologically acceptable salt thereof.

6. The compound according to claim 5,
wherein X is CR^{X0}R^{X0'}, O, CO or -(CR^{X1}R^{X2})₂-; and
R^{X0} and R^{X0'} are each independently a hydrogen atom, C₁₋₆ alkyl, or halo C₁₋₆ alkyl, or a pharmacologically acceptable salt thereof.

7. The compound according to claim 6,
wherein R^{V1}, R^{V1'}, R^{V2}, and R^{V2'} are each independently a hydrogen atom, a halogen atom, cyano, C₁₋₆ alkyl, C₁₋₆ alkoxy, halo C₁₋₆ alkyl, hydroxy C₁₋₆ alkyl, or C₁₋₆ alkoxy C₁₋₆ alkyl,
or a pharmacologically acceptable salt thereof.

8. The compound according to claim 7,
wherein Y is O,
or a pharmacologically acceptable salt thereof.

9. The compound according to claim 8,
wherein R⁴ is NR⁵R^{5'}; and
R⁵ and R^{5'} have the same meanings as in claim 1,
or a pharmacologically acceptable salt thereof.

10. The compound according to claim 9,
wherein W¹ is NR^{W3}; and
R^{W3} has the same meaning as in claim 1,
or a pharmacologically acceptable salt thereof.

11. The compound according to claim 10,
wherein R⁵ and R^{5'} are a hydrogen atom,
or a pharmacologically acceptable salt thereof.

12. The compound according to claim 1,
wherein the compound is represented by Formula (A-III): 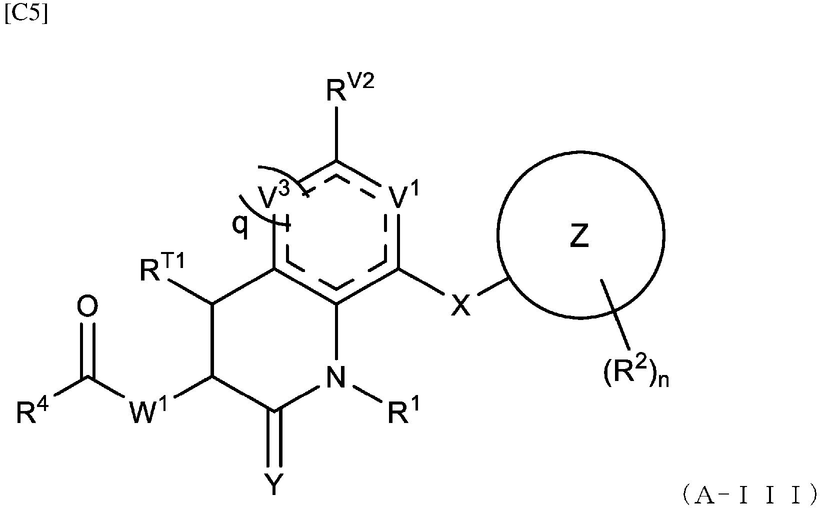 [wherein,
the ring Z is C₆₋₁₀ aryl, 5- or 6-membered heteroaryl, C₃₋₈ cycloalkyl, or 3- to 8-membered heterocycloalkyl;
V¹ is CR^{V1}R^{V1'}, CR^{V1}, NR^{V1}, N, O or S;
V³ is CR^{V3}R^{V3'}, CR^{V3}, NR^{V3}, N, O, or S;
R^{V1}, R^{V1'}, R^{V3}, and R^{V3'} are each independently a hydrogen atom, a halogen atom, hydroxy, cyano, C₁₋₆ alkyl, C₁₋₆ alkoxy, halo C₁₋₆ alkyl, hydroxy C₁₋₆ alkyl, C₁₋₆ alkoxy C₁₋₆ alkyl, halo C₁₋₆ alkoxy, NR^{V4}COR^{V5}, NR^{V4}COOR^{V6}, or NR^{V4}CONR^{V7}R^{V8};
R^{V2} is halo C₁₋₆ alkoxy, NR^{V4}COR^{V5}, NR^{V4}COOR^{V6}, or NR^{V4}CONR^{V7}R^{V8};
R^{V4} is a hydrogen atom or C₁₋₆ alkyl;
R^{V5}, R^{V6}, R^{V7}, and R^{V8} are each independently a hydrogen atom or a group selected from the group consisting of the following (i) to (ix):
(i) C₁₋₆ alkyl which is unsubstituted or substituted with 1 to 6 groups selected from Substituent Group A,
(ii) C₃₋₈ cycloalkyl which is unsubstituted or substituted with 1 to 6 groups selected from Substituent Group A,
(iii) C₃₋₈ cycloalkyl C₁₋₆ alkyl which is unsubstituted or substituted with 1 to 6 groups selected from Substituent Group A,
(iv) C₆₋₁₀ aryl which is unsubstituted or substituted with 1 to 6 groups selected from Substituent Group A,
(v) C₆₋₁₀ aryl C₁₋₆ alkyl which is unsubstituted or substituted with 1 to 6 groups selected from Substituent Group A,
(vi) 3- to 8-membered heterocycloalkyl which is unsubstituted or substituted with 1 to 6 groups selected from Substituent Group A,
(vii) 3- to 8-membered heterocycloalkyl C₁₋₆ alkyl which is unsubstituted or substituted with 1 to 6 groups selected from Substituent Group A,
(viii) 5- to 10-membered heteroaryl which is unsubstituted or substituted with 1 to 6 groups selected from Substituent Group A, and
(ix) 5- to 10-membered heteroaryl C₁₋₆ alkyl which is unsubstituted or substituted with 1 to 6 groups selected from Substituent Group A;
Substituent Group A is a group consisting of a halogen atom, cyano, hydroxy, N(C₁₋₆ alkyl)₂, C₁₋₆ alkyl, halo C₁₋₆ alkyl, C₁₋₆ alkoxy, halo C₁₋₆ alkoxy, C₆₋₁₀ aryl C₁₋₆ alkoxy, and C₃₋₈ cycloalkyl;
when there are two or more groups selected from Substituent Group A, these groups may be the same as or different from each other;
X is CR^{X0}R^{X0'}, O, NR^{X3}, CO, -C(=CR^{X1}R^{X2})-, -(CR^{X1}R^{X2})₂-, OCR^{X1}R^{X2}, CR^{X1}R^{X2}O, NR^{X3}CR^{X1}R^{X2}, CR^{X1}R^{X2}NR^{X3}, or -CR^{X1}=CR^{X2}-;
R^{X0}, R^{X0'}, R^{X1}, and R^{X2} are each independently a hydrogen atom, a halogen atom, hydroxy, C₁₋₆ alkyl, or halo C₁₋₆ alkyl;
R^{X3} is a hydrogen atom, C₁₋₆ alkyl, or halo C₁₋₆ alkyl;
R^{X0} may, together with R^{X0'}, form a 3- to 8-membered saturated carbocyclic ring which is unsubstituted or substituted with 1 to 6 groups selected from Substituent Group B;
R^{X1} may, together with R^{X2}, form a 3- to 8-membered saturated carbocyclic ring which is unsubstituted or substituted with 1 to 6 groups selected from Substituent Group B;
R^{X1} may, together with R^{X3}, form a 3- to 8-membered saturated heterocyclic ring which is unsubstituted or substituted with 1 to 6 groups selected from Substituent Group B;
R¹ is a hydrogen atom, C₁₋₆ alkyl, or halo C₁₋₆ alkyl;
R¹ may, together with R^{X0}, R^{X1}, or R^{X3}, form a 5- to 8-membered ring which is unsubstituted or substituted with 1 to 6 groups selected from Substituent Group B;
Substituent Group B is a group consisting of a halogen atom, hydroxy, C₁₋₆ alkyl, halo C₁₋₆ alkyl, C₁₋₆ alkoxy, halo C₁₋₆ alkoxy, and oxo;
when there are two or more groups selected from Substituent Group B, these groups may be the same as or different from each other;
R² is a halogen atom, cyano, hydroxy, amino, C₁₋₆ alkyl, halo C₁₋₆ alkyl, halohydroxy C₁₋₆ alkyl, SF₅, C₁₋₆ alkoxy, halo C₁₋₆ alkoxy, C₆₋₁₀ aryl C₁₋₆ alkyl, or C₆₋₁₀ aryl C₁₋₆ alkoxy;
R^{T1} is a hydrogen atom, a halogen atom, hydroxy, C₁₋₆ alkyl, or halo C₁₋₆ alkyl;
R⁴ is C₁₋₆ alkyl, halo C₁₋₆ alkyl, C₁₋₆ alkoxy C₁₋₆ alkyl, OR⁵, NR⁵R^{5'}, C₃₋₈ cycloalkyl, or 3- to 8-membered heterocycloalkyl;
R⁵ and R^{5'} are each independently a hydrogen atom, C₁₋₆ alkyl, halo C₁₋₆ alkyl, or C₃₋₈ cycloalkyl;
R⁵ may, together with R^{5'}, form a 3- to 10-membered saturated heterocyclic ring which is unsubstituted or substituted with 1 to 6 groups selected from Substituent Group B or a 3- to 10-membered unsaturated heterocyclic ring which is unsubstituted or substituted with 1 to 6 groups selected from Substituent Group B;
W¹ is CR^{W1}R^{W2}, O, NR^{W3}, or a single bond;
R^{W1} and R^{W2} are each independently a hydrogen atom, a halogen atom, C₁₋₆ alkyl, or halo C₁₋₆ alkyl;
R^{W3} is a hydrogen atom, C₁₋₆ alkyl, or halo C₁₋₆ alkyl;
n is an integer of 0 to 3;
when n is 2 or 3, these R²s may be the same as or different from each other;
q is 0 or 1; and
Y is O or S;
[C6] - - -
indicates a single bond or a double bond]
or a pharmacologically acceptable salt thereof.

13. A pharmaceutical composition comprising the compound or a pharmacologically acceptable salt thereof according to any one of claims 1 to 12 and a pharmaceutical additive.

14. The pharmaceutical composition according to claim 13, which is a pharmaceutical composition for treating thyroid-related diseases.

15. The pharmaceutical composition according to claim 14,
wherein the thyroid-related diseases are hyperthyroidism, Graves' disease or thyroid eye disease.
